(19) Europäisches Patentamt
European Patent Office
Office européen des brevets

(11) EP 1 419 173 B1

(12) **EUROPEAN PATENT SPECIFICATION**

(45) Date of publication and mention of the grant of the patent:
**26.11.2008 Bulletin 2008/48**

(21) Application number: **02772136.4**

(22) Date of filing: **09.08.2002**

(51) Int Cl.:
*C07K 4/00* (2006.01)

(86) International application number:
**PCT/EP2002/008942**

(87) International publication number:
**WO 2003/014145 (20.02.2003 Gazette 2003/08)**

# (54) PEPTIDES THAT BIND TO ATHEROSCLEROTIC LESIONS

PEPTIDE, DIE ATHEROSKLEROTISCHE SCHÄDIGUNGEN BINDEN

PEPTIDES SE LIANT AVEC LES LESIONS ATHEROSCLEREUSES

(84) Designated Contracting States:
**AT BE BG CH CY CZ DE DK EE ES FI FR GB GR IE IT LI LU MC NL PT SE SK TR**

(30) Priority: **10.08.2001 US 311507 P**

(43) Date of publication of application:
**19.05.2004 Bulletin 2004/21**

(73) Proprietors:
- **NOVARTIS AG**
  **4056 Basel (CH)**
  Designated Contracting States:
  **BE BG CH CY CZ DE DK EE ES FI FR GB GR IE IT LI LU MC NL PT SE SK TR**
- **Novartis Pharma GmbH**
  **1230 Wien (AT)**
  Designated Contracting States:
  **AT**
- **THE SCRIPPS RESEARCH INSTITUTE**
  **La Jolla, CA 92037 (US)**

(72) Inventors:
- **LIU, Cheng**
  **Carlsbad, CA 92009 (US)**
- **EDGINGTON, Thomas, S.**
  **La Jolla, CA 92037 (US)**
- **PRESCOTT, Margaret, Forney**
  **Millburn, NJ 07041 (US)**

(74) Representative: **de Weerd, Petrus G.W. et al**
**Novartis International AG**
**Corporate Intellectual Property**
**4002 Basel (CH)**

(56) References cited:

| | |
|---|---|
| **EP-A- 0 580 550** | **WO-A-00/51971** |
| **WO-A-01/32853** | **WO-A-94/13327** |
| **WO-A-94/29333** | **WO-A-02/094854** |
| **US-A- 5 827 516** | |

- **LUU T N ET AL: "Calcitonin gene-related peptide in healthy and atheromatous human epicardial coronary arteries: Function and receptor characterization." JOURNAL OF VASCULAR RESEARCH, vol. 32, no. 2, 1995, pages 93-99, XP009010613 ISSN: 1018-1172**
- **FRANK, RONALD: "Spot-synthesis: an easy technique for the positionally addressable, parallel chemical synthesis on a membrane support" TETRAHEDRON, vol. 48, no. 42, 16 October 1992 (1992-10-16), pages 9217-9232, XP000353580 Oxford, GB, ISSN: 0040-4020**
- **FAVRE ET AL.: "STRUCTURAL MIMICRY OF CANONICAL CONFORMATIONS IN ANTIBODY HYPERVARIABLE LOOPS USING CYCLIC PEPTIDES CONTAINING A HETEROCHIRAL DIPROLINE TEMPLATE" JOURNAL OF THE AMERICAN CHEMICAL SOCIETY, vol. 121, no. 12, 31 March 1999 (1999-03-31), pages 2679-2685, XP002137023 ISSN: 0002-7863**
- **BENGTSSON M. ET AL: 'Convergent synthesis of neoglycopeptides by coupling of 2-bromoethyl glycosides to cysteine and homocysteine residues in T cell stimulating peptides' GLYCOCONJUGATE JOURNAL vol. 15, no. 3, March 1998, UK, pages 223 - 231, XP009076741**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 419 173 B1

SEQUENCE LISTING

<110> Liu, C.
Edgington, T.S.
Prescott, M.
The Scripps Research Institute

<120> Peptides that Bind to Atherosclerotic Lesions

<130> 1361.010WO1

<150> US 60/311,507
<151> 2001-08-10

<160> 474

<170> FastSEQ for Windows Version 4.0

<210> 1
<211> 21
<212> DNA
<213> Artificial Sequence

<220>
<223> A sequence from a combinatorial phage display library.

<400> 1
gcgccgggtc cgtctaagag t 21

<210> 2
<211> 7
<212> PRT
<213> Artificial Sequence

<220>
<223> A sequence from a combinatorial phage display library.

<400> 2

Ala Pro Gly Pro Ser Lys Ser
1 5

<210> 3
<211> 27 <212> DNA
<213> Artificial Sequence

<220>
<223> A sequence from a combinatorial phage display library.

<400> 3
tgtgcgccgg gtccgtctaa gagttgc 27

<210> 4
<211> 9
<212> PRT
<213> Artificial Sequence

<220>
<223> A sequence from a combinatorial phage display library.

<400> 4

Cys Ala Pro Gly Pro Ser Lys Ser Cys
1 5

<210> 5
<211> 21
<212> DNA
<213> Artificial Sequence

<220>
<223> A sequence from a combinatorial phage display library.

<400> 5
caggagccga cgcggctgaa g 21

<210> 6
<211> 7
<212> PRT
<213> Artificial Sequence

<220>
<223> A sequence from a combinatorial phage display library.

<400> 6

Gln Glu Pro Thr Arg Leu Lys
1 5

<210> 7
<211> 27
<212> DNA
<213> Artificial Sequence

<220>
<223> A sequence from a combinatorial phage display library.

<400> 7
tgtcaggagc cgacgcggct gaagtgc 27

<210> 8
<211> 9
<212> PRT
<213> Artificial Sequence

<220>
<223> A sequence from a combinatorial phage display library.

<400> 8

Cys Gln Glu Pro Thr Arg Leu Lys Cys
1 5

<210> 9

<211> 21
<212> DNA
<213> Artificial Sequence

<220>
<223> A sequence from a combinatorial phage display library.

<400> 9
aaggagccta cgcgggcgca t 21

<210> 10
<211> 7
<212> PRT
<213> Artificial Sequence

<220>
<223> A sequence from a combinatorial phage display library.

<400> 10

Lys Glu Pro Thr Arg Ala His
1 5

<210> 11
<211> 27
<212> DNA
<213> Artificial Sequence

<220>
<223> A sequence from a combinatorial phage display library.

<400> 11
tgtaaggagc ctacgcgggc gcattgc 27

<210> 12
<211> 9
<212> PRT
<213> Artificial Sequence

<220>
<223> A sequence from a combinatorial phage display library.

<400> 12

Cys Lys Glu Pro Thr Arg Ala His Cys
1 5

<210> 13
<211> 21
<212> DNA
<213> Artificial Sequence

<220>
<223> A sequence from a combinatorial phage display library.

<400> 13
ttggcgatgc ttatggatac g 21

<210> 14
<211> 7
<212> PRT
<213> Artificial Sequence

<220>
<223> A sequence from a combinatorial phage display library.

<400> 14

Leu Ala Met Leu Met Asp Thr
1 5

<210> 15
<211> 27
<212> DNA
<213> Artificial Sequence

<220>
<223> A sequence from a combinatorial phage display library.

<400> 15
tgtttggcga tgcttatgga tacgtgc 27

<210> 16
<211> 9
<212> PRT
<213> Artificial Sequence

<220>
<223> A sequence from a combinatorial phage display library.

<400> 16

Cys Leu Ala Met Leu Met Asp Thr Cys
1 5

<210> 17
<211> 21
<212> DNA
<213> Artificial Sequence

<220>
<223> A sequence from a combinatorial phage display library.

<400> 17
aataagcata ctaggccgct t 21

<210> 18
<211> 7
<212> PRT

<213> Artificial Sequence

<220>
<223> A sequence from a combinatorial phage display library.

<400> 18

Asn Lys His Thr Arg Pro Leu
1 5

<210> 19
<211> 27
<212> DNA
<213> Artificial Sequence

<220>
<223> A sequence from a combinatorial phage display library.

<400> 19
tgtaataagc atactaggcc gctttgc 27

<210> 20
<211> 9
<212> PRT
<213> Artificial Sequence

<220>
<223> A sequence from a combinatorial phage display library.

<400> 20

Cys Asn Lys His Thr Arg Pro Leu Cys
1 5

<210> 21
<211> 21
<212> DNA
<213> Artificial Sequence

<220>
<223> A sequence from a combinatorial phage display library.

<400> 21
gtgcataagc tgcctgagtc t 21

<210> 22
<211> 7
<212> PRT
<213> Artificial Sequence

<220>
<223> A sequence from a combinatorial phage display library.

<400> 22

Val His Lys Leu Pro Glu Ser
1 5

<210> 23
<211> 27
<212> DNA
<213> Artificial Sequence

<220>
<223> A sequence from a combinatorial phage display library.

<400> 23
tgtgtgcata agctgcctga gtcttgc 27

<210> 24
<211> 9
<212> PRT
<213> Artificial Sequence

<220>
<223> A sequence from a combinatorial phage display library.

<400> 24

Cys Val His Lys Leu Pro Glu Ser Cys
1 5

<210> 25
<211> 21
<212> DNA
<213> Artificial Sequence

<220>
<223> A sequence from a combinatorial phage display library.

<400> 25
ccgactcagg cttctcttca t 21

<210> 26
<211> 7
<212> PRT
<213> Artificial Sequence

<220>
<223> A sequence from a combinatorial phage display library.

<400> 26

Pro Thr Gln Ala Ser Leu His
1 5

<210> 27

<211> 27
<212> DNA
<213> Artificial Sequence

<220>
<223> A sequence from a combinatorial phage display library.

<400> 27
tgtccgactc aggcttctct tcattgc 27

<210> 28
<211> 9
<212> PRT
<213> Artificial Sequence

<220>
<223> A sequence from a combinatorial phage display library.

<400> 28

Cys Pro Thr Gln Ala Ser Leu His Cys
1 5

<210> 29
<211> 21
<212> DNA
<213> Artificial Sequence

<220>
<223> A sequence from a combinatorial phage display library.

<400> 29
gatactgcgc ctccgtcgtc g 21

<210> 30
<211> 7
<212> PRT
<213> Artificial Sequence

<220>
<223> A sequence from a combinatorial phage display library.

<400> 30

Asp Thr Ala Pro Pro Ser Ser
1 5

<210> 31
<211> 27
<212> DNA
<213> Artificial Sequence

<220>
<223> A sequence from a combinatorial phage display library.

<400> 31
tgtgatactg cgcctccgtc gtcgtgc 27

<210> 32
<211> 9
<212> PRT
<213> Artificial Sequence

<220>
<223> A sequence from a combinatorial phage display library.

<400> 32

Cys Asp Thr Ala Pro Pro Ser Ser Cys
1 5

<210> 33
<211> 21
<212> DNA
<213> Artificial Sequence

<220>
<223> A sequence from a combinatorial phage display library.

<400> 33
ggggtgcaga ctctgcttgc t 21

<210> 34
<211> 7
<212> PRT
<213> Artificial Sequence

<220>
<223> A sequence from a combinatorial phage display library.

<400> 34

Gly Val Gln Thr Leu Leu Ala
1 5

<210> 35
<211> 27
<212> DNA
<213> Artificial Sequence

<220>
<223> A sequence from a combinatorial phage display library.

<400> 35
tgtggggtgc agactctgct tgcttgc 27

<210> 36
<211> 9
<212> PRT

<213> Artificial Sequence

<220>
<223> A sequence from a combinatorial phage display library.

<400> 36

Cys Gly Val Gln Thr Leu Leu Ala Cys
1 5

<210> 37
<211> 21
<212> DNA
<213> Artificial Sequence

<220>
<223> A sequence from a combinatorial phage display library.

<400> 37
gatcctgtga cgaagcatac t 21

<210> 38
<211> 7
<212> PRT
<213> Artificial Sequence

<220>
<223> A sequence from a combinatorial phage display library.

<400> 38

Asp Pro Val Thr Lys His Thr
1 5

<210> 39
<211> 27
<212> DNA
<213> Artificial Sequence

<220>
<223> A sequence from a combinatorial phage display library.

<400> 39
tgtgatcctg tgacgaagca tacttgc 27

<210> 40
<211> 9
<212> PRT
<213> Artificial Sequence

<220>
<223> A sequence from a combinatorial phage display library.

<400> 40

Cys Asp Pro Val Thr Lys His Thr Cys
1 5

<210> 41
<211> 21
<212> DNA
<213> Artificial Sequence

<220>
<223> A sequence from a combinatorial phage display library.

<400> 41
gatcagagta cgattcgggc g 21

<210> 42
<211> 7
<212> PRT
<213> Artificial Sequence

<220>
<223> A sequence from a combinatorial phage display library.

<400> 42

Asp Gln Ser Thr Ile Arg Ala
1 5

<210> 43
<211> 27
<212> DNA
<213> Artificial Sequence

<220>
<223> A sequence from a combinatorial phage display library.

<400> 43
tgtgatcaga gtacgattcg ggcgtgc 27

<210> 44
<211> 9
<212> PRT
<213> Artificial Sequence

<220>
<223> A sequence from a combinatorial phage display library.

<400> 44

Cys Asp Gln Ser Thr Ile Arg Ala Cys
1 5

<210> 45

<211> 21
<212> DNA
<213> Artificial Sequence

<220>
<223> A sequence from a combinatorial phage display library.

<400> 45
cgggctgcta ctccttcgat t 21

<210> 46
<211> 7
<212> PRT
<213> Artificial Sequence

<220>
<223> A sequence from a combinatorial phage display library.

<400> 46

Arg Ala Ala Thr Pro Ser Ile
1 5

<210> 47
<211> 27
<212> DNA
<213> Artificial Sequence

<220>
<223> A sequence from a combinatorial phage display library.

<400> 47
tgtcgggctg ctactccttc gatttgc 27

<210> 48
<211> 9
<212> PRT
<213> Artificial Sequence

<220>
<223> A sequence from a combinatorial phage display library.

<400> 48

Cys Arg Ala Ala Thr Pro Ser Ile Cys
1 5

<210> 49
<211> 21
<212> DNA
<213> Artificial Sequence

<220>

<223> A sequence from a combinatorial phage display library.

<400> 49
aagacgtcgc atgcgcagga g 21

<210> 50
<211> 7
<212> PRT
<213> Artificial Sequence

<220>
<223> A sequence from a combinatorial phage display library.

<400> 50

Lys Thr Ser His Ala Gln Glu
1 5

<210> 51
<211> 27
<212> DNA
<213> Artificial Sequence

<220>
<223> A sequence from a combinatorial phage display library.

<400> 51
tgtaagacgt cgcatgcgca ggagtgc 27

<210> 52
<211> 9
<212> PRT
<213> Artificial Sequence

<220>
<223> A sequence from a combinatorial phage display library.

<400> 52

Cys Lys Thr Ser His Ala Gln Glu Cys
1 5

<210> 53
<211> 21
<212> DNA
<213> Artificial Sequence

<220>
<223> A sequence from a combinatorial phage display library.

<221> misc_feature
<222> (1) ... (21)
<223> n = A,T,C or G

<400> 53
aagcatcctg ttggncgggt g 21

<210> 54
<211> 7
<212> PRT
<213> Artificial Sequence

<220>
<223> A sequence from a combinatorial phage display library.

<400> 54

Lys His Pro Val Gly Arg Val
1 5

<210> 55
<211> 27
<212> DNA
<213> Artificial Sequence

<220>
<223> A sequence from a combinatorial phage display library.

<221> misc_feature
<222> (1) ... (27)
<223> n = A,T,C or G

<400> 55
tgtaagcatc ctgttggncg ggtgtgc 27

<210> 56
<211> 9
<212> PRT
<213> Artificial Sequence

<220>
<223> A sequence from a combinatorial phage display library.

<400> 56

Cys Lys His Pro Val Gly Arg Val Cys
1 5

<210> 57
<211> 21
<212> DNA
<213> Artificial Sequence

<220>
<223> A sequence from a combinatorial phage display library.

<400> 57
acggatacta agaattcgca g 21

<210> 58
<211> 7
<212> PRT
<213> Artificial Sequence

<220>
<223> A sequence from a combinatorial phage display library.

<400> 58

Thr Asp Thr Lys Asn Ser Gln
1 5

<210> 59
<211> 27
<212> DNA
<213> Artificial Sequence

<220>
<223> A sequence from a combinatorial phage display library.

<400> 59
tgtacggata ctaagaattc gcagtgc 27

<210> 60
<211> 9
<212> PRT
<213> Artificial Sequence

<220>
<223> A sequence from a combinatorial phage display library.

<400> 60

Cys Thr Asp Thr Lys Asn Ser Gln Cys
1 5

<210> 61
<211> 21
<212> DNA
<213> Artificial Sequence

<220>
<223> A sequence from a combinatorial phage display library.

<400> 61
cagccgccga tggggcggta t 21

<210> 62
<211> 7
<212> PRT
<213> Artificial Sequence

<220>
<223> A sequence from a combinatorial phage display library.

<400> 62

Gln Pro Pro Met Gly Arg Tyr
1 5

<210> 63
<211> 27
<212> DNA
<213> Artificial Sequence

<220>
<223> A sequence from a combinatorial phage display library.

<400> 63
tgtcagccgc cgatggggcg gtattgc 27

<210> 64
<211> 9
<212> PRT
<213> Artificial Sequence

<220>
<223> A sequence from a combinatorial phage display library.

<400> 64

Cys Gln Pro Pro Met Gly Arg Tyr Cys
1 5

<210> 65
<211> 21
<212> DNA
<213> Artificial Sequence

<220>
<223> A sequence from a combinatorial phage display library.

<400> 65
aatgagaggc tgaataagga t 21

<210> 66
<211> 7
<212> PRT
<213> Artificial Sequence

<220>
<223> A sequence from a combinatorial phage display library.

<400> 66

Asn Glu Arg Leu Asn Lys Asp
1 5

<210> 67
<211> 27
<212> DNA
<213> Artificial Sequence

<220>
<223> A sequence from a combinatorial phage display library.

<400> 67
tgtaatgaga ggctgaataa ggattgc 27

<210> 68
<211> 9
<212> PRT
<213> Artificial Sequence

<220>
<223> A sequence from a combinatorial phage display library.

<400> 68

Cys Asn Glu Arg Leu Asn Lys Asp Cys
1 5

<210> 69
<211> 21
<212> DNA
<213> Artificial Sequence

<220>
<223> A sequence from a combinatorial phage display library.

<400> 69
ccgccgtcga ataagcagat g 21

<210> 70
<211> 7
<212> PRT
<213> Artificial Sequence

<220>
<223> A sequence from a combinatorial phage display library.

<400> 70

Pro Pro Ser Asn Lys Gln Met
1 5

<210> 71

<211> 27
<212> DNA
<213> Artificial Sequence

<220>
<223> A sequence from a combinatorial phage display library.

<400> 71
tgtccgccgt cgaataagca gatgtgc 27

<210> 72
<211> 9
<212> PRT
<213> Artificial Sequence

<220>
<223> A sequence from a combinatorial phage display library.

<400> 72

Cys Pro Pro Ser Asn Lys Gln Met Cys
1 5

<210> 73
<211> 21
<212> DNA
<213> Artificial Sequence

<220>
<223> A sequence from a combinatorial phage display library.

<400> 73
gattcttcgt cgcctgctcg g 21

<210> 74
<211> 7
<212> PRT
<213> Artificial Sequence

<220>
<223> A sequence from a combinatorial phage display library.

<400> 74

Asp Ser Ser Ser Pro Ala Arg
1 5

<210> 75
<211> 27
<212> DNA
<213> Artificial Sequence

<220>
<223> A sequence from a combinatorial phage display library.

<400> 75
tgtgattctt cgtcgcctgc tcggtgc 27

<210> 76
<211> 9
<212> PRT
<213> Artificial Sequence

<220>
<223> A sequence from a combinatorial phage display library.

<400> 76

Cys Asp Ser Ser Ser Pro Ala Arg Cys
1 5

<210> 77
<211> 21
<212> DNA
<213> Artificial Sequence

<220>
<223> A sequence from a combinatorial phage display library.

<400> 77
acgcagtctg ataataggcg t 21

<210> 78
<211> 7
<212> PRT
<213> Artificial Sequence

<220>
<223> A sequence from a combinatorial phage display library.

<400> 78

Thr Gln Ser Asp Asn Arg Arg
1 5

<210> 79
<211> 27
<212> DNA
<213> Artificial Sequence

<220>
<223> A sequence from a combinatorial phage display library.

<400> 79
tgtacgcagt ctgataatag gcgttgc 27

<210> 80
<211> 9
<212> PRT

<213> Artificial Sequence

<220>
<223> A sequence from a combinatorial phage display library.

<400> 80

Cys Thr Gln Ser Asp Asn Arg Arg Cys
1 5

<210> 81
<211> 21
<212> DNA
<213> Artificial Sequence

<220>
<223> A sequence from a combinatorial phage display library.

<400> 81
aagggtctgc cggctaagac t 21

<210> 82
<211> 7
<212> PRT
<213> Artificial Sequence

<220>
<223> A sequence from a combinatorial phage display library.

<400> 82

Lys Gly Leu Pro Ala Lys Thr
1 5

<210> 83
<211> 27
<212> DNA
<213> Artificial Sequence

<220>
<223> A sequence from a combinatorial phage display library.

<400> 83
tgtaagggtc tgccggctaa gacttgc 27

<210> 84
<211> 9
<212> PRT
<213> Artificial Sequence

<220>
<223> A sequence from a combinatorial phage display library.

<400> 84

Cys Lys Gly Leu Pro Ala Lys Thr Cys
1 5

<210> 85
<211> 21
<212> DNA
<213> Artificial Sequence

<220>
<223> A sequence from a combinatorial phage display library.

<400> 85
ttgcagccgc atctgagtct t 21

<210> 86
<211> 7
<212> PRT
<213> Artificial Sequence

<220>
<223> A sequence from a combinatorial phage display library.

<400> 86

Leu Gln Pro His Leu Ser Leu
1 5

<210> 87
<211> 27
<212> DNA
<213> Artificial Sequence

<220>
<223> A sequence from a combinatorial phage display library.

<400> 87
tgtttgcagc cgcatctgag tctttgc 27

<210> 88
<211> 9
<212> PRT
<213> Artificial Sequence

<220>
<223> A sequence from a combinatorial phage display library.

<400> 88

Cys Leu Gln Pro His Leu Ser Leu Cys
1 5

<210> 89
<211> 21
<212> DNA
<213> Artificial Sequence

<220>
<223> A sequence from a combinatorial phage display library.

<400> 89
gcggttccgt agaatcgttc t 21

<210> 90
<211> 7
<212> PRT
<213> Artificial Sequence

<220>
<223> A sequence from a combinatorial phage display library.

<400> 90

Ala Val Pro Gln Asn Arg Ser
1 5

<210> 91
<211> 27
<212> DNA
<213> Artificial Sequence

<220>
<223> A sequence from a combinatorial phage display library.

<400> 91
tgtgcggttc cgtagaatcg ttcttgc 27

<210> 92
<211> 9
<212> PRT
<213> Artificial Sequence

<220>
<223> A sequence from a combinatorial phage display library.

<400> 92

Cys Ala Val Pro Gln Asn Arg Ser Cys
1 5

<210> 93
<211> 21
<212> DNA
<213> Artificial Sequence

<220>
<223> A sequence from a combinatorial phage display library.

<400> 93
atgaatcaga ctcctgattt g 21

<210> 94
<211> 7
<212> PRT
<213> Artificial Sequence

<220>
<223> A sequence from a combinatorial phage display library.

<400> 94

Met Asn Gln Thr Pro Asp Leu
1 5

<210> 95
<211> 27
<212> DNA
<213> Artificial Sequence

<220>
<223> A sequence from a combinatorial phage display library.

<400> 95
tgtatgaatc agactcctga tttgtgc 27

<210> 96
<211> 9
<212> PRT
<213> Artificial Sequence

<220>
<223> A sequence from a combinatorial phage display library.

<400> 96

Cys Met Asn Gln Thr Pro Asp Leu Cys
1 5

<210> 97
<211> 21
<212> DNA
<213> Artificial Sequence

<220>
<223> A sequence from a combinatorial phage display library.

<400> 97
tttcagatgc agcctactct t 21

<210> 98
<211> 7
<212> PRT
<213> Artificial Sequence

<220>
<223> A sequence from a combinatorial phage display library.

<400> 98

Phe Gln Met Gln Pro Thr Leu
1 5

<210> 99
<211> 27
<212> DNA
<213> Artificial Sequence

<220>
<223> A sequence from a combinatorial phage display library.

<400> 99
tgttttcaga tgcagcctac tctttgc 27

<210> 100
<211> 9
<212> PRT
<213> Artificial Sequence

<220>
<223> A sequence from a combinatorial phage display library.

<400> 100

Cys Phe Gln Met Gln Pro Thr Leu Cys
1 5

<210> 101
<211> 21
<212> DNA
<213> Artificial Sequence

<220>
<223> A sequence from a combinatorial phage display library.

<400> 101
agtggggctt ctaataagac g 21

<210> 102
<211> 7
<212> PRT
<213> Artificial Sequence

<220>

<223> A sequence from a combinatorial phage display library.

<400> 102

Ser Gly Ala Ser Asn Lys Thr
1 5

<210> 103
<211> 27
<212> DNA
<213> Artificial Sequence

<220>
<223> A sequence from a combinatorial phage display library.

<400> 103
tgtagtgggg cttctaataa gacgtgc 27

<210> 104
<211> 9
<212> PRT
<213> Artificial Sequence

<220>
<223> A sequence from a combinatorial phage display library.

<400> 104

Cys Ser Gly Ala Ser Asn Lys Thr Cys
1 5

<210> 105
<211> 21
<212> DNA
<213> Artificial Sequence

<220>
<223> A sequence from a combinatorial phage display library.

<400> 105
actaagatgc ggttggagca g 21

<210> 106
<211> 7
<212> PRT
<213> Artificial Sequence

<220>
<223> A sequence from a combinatorial phage display library.

<400> 106

Thr Lys Met Arg Leu Glu Gln
1 5

<210> 107
<211> 27
<212> DNA '
<213> Artificial Sequence

<220>
<223> A sequence from a combinatorial phage display library.

<400> 107
tgtactaaga tgcggttgga gcagtgc 27

<210> 108
<211> 9
<212> PRT
<213> Artificial Sequence

<220>
<223> A sequence from a combinatorial phage display library.

<400> 108

Cys Thr Lys Met Arg Leu Glu Gln Cys
1 5

<210> 109
<211> 21
<212> DNA
<213> Artificial Sequence

<220>
<223> A sequence from a combinatorial phage display library.

<400> 109
acgtctccta tttatccggg t 21

<210> 110
<211> 7
<212> PRT
<213> Artificial Sequence

<220>
<223> A sequence from a combinatorial phage display library.

<400> 110

Thr Ser Pro Ile Tyr Pro Gly
1 5

<210> 111

<211> 27
<212> DNA
<213> Artificial Sequence

<220>
<223> A sequence from a combinatorial phage display library.

<400> 111
tgtacgtctc ctatttatcc gggttgc 27

<210> 112
<211> 9
<212> PRT
<213> Artificial Sequence

<220>
<223> A sequence from a combinatorial phage display library.

<400> 112

Cys Thr Ser Pro Ile Tyr Pro Gly Cys
1 5

<210> 113
<211> 21
<212> DNA
<213> Artificial Sequence

<220>
<223> A sequence from a combinatorial phage display library.

<400> 113
aagactccgt cgcagagtca g 21

<210> 114
<211> 7
<212> PRT
<213> Artificial Sequence

<220>
<223> A sequence from a combinatorial phage display library.

<400> 114

Lys Thr Pro Ser Gln Ser Gln
1 5

<210> 115
<211> 27
<212> DNA
<213> Artificial Sequence

<220>
<223> A sequence from a combinatorial phage display library.

<400> 115
tgtaagactc cgtcgcagag tcagtgc 27

<210> 116
<211> 9
<212> PRT
<213> Artificial Sequence

<220>
<223> A sequence from a combinatorial phage display library.

<400> 116

**Cys Lys Thr Pro Ser Gln Ser Gln Cys**
**1 5**

<210> 117
<211> 21
<212> DNA
<213> Artificial Sequence

<220>
c223> A sequence from a combinatorial phage display library.

<400> 117
cttcaggctt ttaaggcgac t 21

<210> 118
<211> 7
<212> PRT
<213> Artificial Sequence

<220>
<223> A sequence from a combinatorial phage display library.

<400> 118

**Leu Gln Ala Phe Lys Ala Thr**
**1 5**

<210> 119
<211> 27
<212> DNA
<213> Artificial Sequence

<220>
<223> A sequence from a combinatorial phage display library.

<400> 119
tgtcttcagg cttttaaggc gacttgc 27

<210> 120
<211> 9

<212> PRT
<213> Artificial Sequence

<220>
<223> A sequence from a combinatorial phage display library.

<400> 120

Cys Leu Gln Ala Phe Lys Ala Thr Cys
1 5

<210> 121
<211> 21
<212> DNA
<213> Artificial Sequence

<220>
<223> A sequence from a combinatorial phage display library.

<400> 121
tctactactg agcttaataa g 21

<210> 122
<211> 7
<212> PRT
<213> Artificial Sequence

<220>
<223> A sequence from a combinatorial phage display library.

<400> 122

Ser Thr Thr Glu Leu Asn Lys
1 5

<210> 123
<211> 27
<212> DNA
<213> Artificial Sequence

<220>
<223> A sequence from a combinatorial phage display library.

<400> 123
tgttctacta ctgagcttaa taagtgc 27

<210> 124
<211> 9 <212> PRT
<213> Artificial Sequence

<220>
<223> A sequence from a combinatorial phage display library.

<400> 124

Cys Ser Thr Thr Glu Leu Asn Lys Cys
1 5

<210> 125
<211> 21
<212> DNA
<213> Artificial Sequence

<220>
<223> A sequence from a combinatorial phage display library.

<400> 125
aggactcata gttctccgac t 21

<210> 126
<211> 7
<212> PRT
<213> Artificial Sequence

<220>
<223> A sequence from a combinatorial phage display library.

<400> 126

Arg Thr His Ser Ser Pro Thr
1 5

<210> 127
<211> 27
<212> DNA
<213> Artificial Sequence

<220>
<223> A sequence from a combinatorial phage display library.

<400> 127
tgtaggactc atagttctcc gacttgc 27

<210> 128
<211> 9
<212> PRT
<213> Artificial Sequence

<220>
<223> A sequence from a combinatorial phage display library.

<400> 128

Cys Arg Thr His Ser Ser Pro Thr Cys
1 5

<210> 129
<211> 21
<212> DNA
<213> Artificial Sequence

<220>
<223> A sequence from a combinatorial phage display library.

<400> 129
aatgagaatt ttaaggggct g 21

<210> 130
<211> 7
<212> PRT
<213> Artificial Sequence

<220>
<223> A sequence from a combinatorial phage display library.

<400> 130

Asn Glu Asn Phe Lys Gly Leu
1 5

<210> 131
<211> 27
<212> DNA
<213> Artificial Sequence

<220>
<223> A sequence from a combinatorial phage display library.

<400> 131
tgtaatgaga attttaaggg gctgtgc 27

<210> 132
<211> 9
<212> PRT
<213> Artificial Sequence

<220>
<223> A sequence from a combinatorial phage display library.

<400> 132

Cys Asn Glu Asn Phe Lys Gly Leu Cys
1 5

<210> 133
<211> 21
<212> DNA
<213> Artificial Sequence

<220>

<223> A sequence from a combinatorial phage display library.

<400> 133
agtaagacta atcatgcttc t 21

<210> 134
<211> 7
<212> PRT
<213> Artificial Sequence

<220>
<223> A sequence from a combinatorial phage display library.

<400> 134

Ser Lys Thr Asn His Ala Ser
1 5

<210> 135
<211> 27
<212> DNA
<213> Artificial Sequence

<220>
<223> A sequence from a combinatorial phage display library.

<400> 135
tgtagtaaga ctaatcatgc ttcttgc 27

<210> 136
<211> 9
<212> PRT
<213> Artificial Sequence

<220>
<223> A sequence from a combinatorial phage display library.

<400> 136

Cys Ser Lys Thr Asn His Ala Ser Cys
1 5

<210> 137
<211> 21
<212> DNA
<213> Artificial Sequence

<220>
<223> A sequence from a combinatorial phage display library.

<400> 137
acgccgtatc cttctaattc g 21

<210> 138

<211> 7
<212> PRT
<213> Artificial Sequence

<220>
<223> A sequence from a combinatorial phage display library.

<400> 138

Thr Pro Tyr Pro Ser Asn Ser
1 5

<210> 139
<211> 27
<212> DNA
<213> Artificial Sequence

<220>
<223> A sequence from a combinatorial phage display library.

<400> 139
tgtacgccgt atccttctaa ttcgtgc 27

<210> 140
<211> 9
<212> PRT
<213> Artificial Sequence

<220>
<223> A sequence from a combinatorial phage display library.

<400> 140

Cys Thr Pro Tyr Pro Ser Asn Ser Cys
1 5

<210> 141
<211> 21
<212> DNA
<213> Artificial Sequence

<220>
<223> A sequence from a combinatorial phage display library.

<400> 141
acgctgtctg ctgctccgca t 21

<210> 142
<211> 7
<212 > PRT
<213> Artificial Sequence

<220>
<223> A sequence from a combinatorial phage display library.

<400> 142

Thr Leu Ser Ala Ala Pro His
1 5

<210> 143
<211> 27
<212> DNA
<213> Artificial Sequence

<220>
<223> A sequence from a combinatorial phage display library.

<400> 143
tgtacgctgt ctgctgctcc gcattgc 27

<210> 144
<211> 9
<212> PRT
<213> Artificial Sequence

<220>
<223> A sequence from a combinatorial phage display library.

<400> 144

Cys Thr Leu Ser Ala Ala Pro His Cys
1 5

<210> 145
<211> 21
<212> DNA
<213> Artificial Sequence

<220>
<223> A sequence from a combinatorial phage display library.

<400> 145
cttaataatt ctcaggctca t 21

<210> 146
<211> 7
<212> PRT
<213> Artificial Sequence

<220>
<223> A sequence from a combinatorial phage display library.

<400> 146

Leu Asn Asn Ser Gln Ala His
1 5

<210> 147
<211> 27
<212> DNA
<213> Artificial Sequence

<220>
<223> A sequence from a combinatorial phage display library.

<400> 147
tgtcttaata attctcaggc tcattgc 27

<210> 148
<211> 9
<212> PRT
<213> Artificial Sequence

<220>
<223> A sequence from a combinatorial phage display library.

<400> 148

Cys Leu Asn Asn Ser Gln Ala His Cys
1 5

<210> 149
<211> 21
<212> DNA
<213> Artificial Sequence

<220>
<223> A sequence from a combinatorial phage display library.

<400> 149
attgagcatt cggcgcagca g 21

<210> 150
<211> 7
<212> PRT
<213> Artificial Sequence

<220>
<223> A sequence from a combinatorial phage display library.

<400> 150

Ile Glu His Ser Ala Gln Gln
1 5

<210> 151
<211> 27
<212> DNA
<213> Artificial Sequence

<220>
<223> A sequence from a combinatorial phage display library.

<400> 151
tgtattgagc attcggcgca gcagtgc 27

<210> 152
<211> 9
<212> PRT
<213> Artificial Sequence

<220>
<223> A sequence from a combinatorial phage display library.

<400> 152

Cys Ile Glu His Ser Ala Gln Gln Cys
1 5

<210> 153
<211> 21
<212> DNA
<213> Artificial Sequence

<220>
<223> A sequence from a combinatorial phage display library.

<400> 153
tcggctgcgg ggcatcatac t 21

<210> 154
<211> 7
<212> PRT
<213> Artificial Sequence

<220>
<223> A sequence from a combinatorial phage display library.

<400> 154

Ser Ala Ala Gly His His Thr
1 5

<210> 155
<211> 27
<212> DNA
<213> Artificial Sequence

<220>
<223> A sequence from a combinatorial phage display library.

<400> 155
tgttcggctg cggggcatca tacttgc 27

<210> 156

<211> 9
<212> PRT
<213> Artificial Sequence

<220>
<223> A sequence from a combinatorial phage display library.

<400> 156

Cys Ser Ala Ala Gly His His Thr Cys
1 5

<210> 157
<211> 21
<212> DNA
<213> Artificial Sequence

<220>
<223> A sequence from a combinatorial phage display library.

<400> 157
cataatcaga agttgaatcg t 21

<210> 158
<211> 7
<212> PRT
<213> Artificial Sequence

<220>
<223> A sequence from a combinatorial phage display library.

<400> 158

His Asn Gln Lys Leu Asn Arg
1 5

<210> 159
<211> 27
<212> DNA
<213> Artificial Sequence

<220>
<223> A sequence from a combinatorial phage display library.

<400> 159
tgtcataatc agaagttgaa tcgttgc 27

<210> 160
<211> 9
<212> PRT
<213> Artificial Sequence

<220>
<223> A sequence from a combinatorial phage display library.

<400> 160

Cys His Asn Gln Lys Leu Asn Arg Cys
1 5

<210> 161
<211> 21
<212> DNA
<213> Artificial Sequence

<220>
<223> A sequence from a combinatorial phage display library.

<400> 161
aagtcgactt ctcatagtat g 21

<210> 162
<211> 7
<212> PRT
<213> Artificial Sequence

<220>
<223> A sequence from a combinatorial phage display library.

<400> 162

Lys Ser Thr Ser His Ser Met
1 5

<210> 163
<211> 27
<212> DNA
<213> Artificial Sequence

<220>
<223> A sequence from a combinatorial phage display library.

<400> 163
tgtaagtcga cttctcatag tatgtgc 27

<210> 164
<211> 9
<212> PRT
<213> Artificial Sequence

<220>
<223> A sequence from a combinatorial phage display library.

<400> 164

Cys Lys Ser Thr Ser His Ser Met Cys
1 5

<210> 165
<211> 21
<212> DNA
<213> Artificial Sequence

<220>
<223> A sequence from a combinatorial phage display library.

<400> 165
cctaataata agtcggcttc g 21

<210> 166
<211> 7
<212> PRT
<213> Artificial Sequence

<220>
<223> A sequence from a combinatorial phage display library.

<400> 166

Pro Asn Asn Lys Ser Ala Ser
1 5

<210> 167
<211> 27
<212> DNA
<213> Artificial Sequence

<220>
<223> A sequence from a combinatorial phage display library.

<400> 167
tgtcctaata ataagtcggc ttcgtgc 27

<210> 168
<211> 9
<212> PRT
<213> Artificial Sequence

<220>
<223> A sequence from a combinatorial phage display library.

<400> 168

Cys Pro Asn Asn Lys Ser Ala Ser Cys
1 5

<210> 169
<211> 21
<212> DNA
<213> Artificial Sequence

<220>

<223> A sequence from a combinatorial phage display library.

<400> 169
gaggatccta cgttgaaggt g 21

<210> 170
<211> 7
<212> PRT
<213> Artificial Sequence

<220>
<223> A sequence from a combinatorial phage display library.

<400> 170

Glu Asp Pro Thr Leu Lys Val
1 5

<210> 171
<211> 27
<212> DNA
<213> Artificial Sequence

<220>
<223> A sequence from a combinatorial phage display library.

<400> 171
tgtgaggatc ctacgttgaa ggtgtgc 27

<210> 172
<211> 9
<212> PRT
<213> Artificial Sequence

<220>
<223> A sequence from a combinatorial phage display library.

<400> 172

Cys Glu Asp Pro Thr Leu Lys Val Cys
1 5

<210> 173
<211> 21
<212> DNA
<213> Artificial Sequence

<220>
<223> A sequence from a combinatorial phage display library.

<400> 173
atgtctgcga tgtctcgtca g 21

<210> 174
<211> 7
<212> PRT
<213> Artificial Sequence

<220>
<223> A sequence from a combinatorial phage display library.

<400> 174

Met Ser Ala Met Ser Arg Gln
1 5

<210> 175
<211> 27
<212> DNA
<213> Artificial Sequence

<220>
<223> A sequence from a combinatorial phage display library.

<400> 175
tgtatgtctg cgatgtctcg tcagtgc 27

<210> 176
<211> 9
<212> PRT
<213> Artificial Sequence

<220>
<223> A sequence from a combinatorial phage display library.

<400> 176

Cys Met Ser Ala Met Ser Arg Gln Cys
1 5

<210> 177
<211> 21
<212> DNA
<213> Artificial Sequence

<220>
<223> A sequence from a combinatorial phage display library.

<400> 177
cctgggaaga tttcgcgtag t 21

<210> 178
<211> 7
<212> PRT
<213> Artificial Sequence

<220>

<223> A sequence from a combinatorial phage display library.

<400> 178

Pro Gly Lys Ile Ser Arg Ser
1 5

<210> 179
<211> 27
<212> DNA
<213> Artificial Sequence

<220>
<223> A sequence from a combinatorial phage display library.

<400> 179
tgtcctggga agatttcgcg tagttgc 27

<210> 180
<211> 9
<212> PRT
<213> Artificial Sequence

<220>
<223> A sequence from a combinatorial phage display library.

<400> 180

Cys Pro Gly Lys Ile Ser Arg Ser Cys
1 5

<210> 181
<211> 21
<212> DNA
<213> Artificial Sequence

<220>
<223> A sequence from a combinatorial phage display library.

<400> 181
ctgaagctgg ggtcgaagca g 21

<210> 182
<211> 7
<212> PRT
<213> Artificial Sequence

<220>
<223> A sequence from a combinatorial phage display library.

<400> 182

Leu Lys Leu Gly Ser Lys Gln
1 5

<210> 183
<211> 27
<212> DNA
<213> Artificial Sequence

<220>
<223> A sequence from a combinatorial phage display library.

<400> 183
tgtctgaagc tggggtcgaa gcagtgc 27

<210> 184
<211> 9
<212> PRT
<213> Artificial Sequence

<220>
<223> A sequence from a combinatorial phage display library.

<400> 184

Cys Leu Lys Leu Gly Ser Lys Gln Cys
1 5

<210> 185
<211> 21
<212> DNA
<213> Artificial Sequence

<220>
<223> A sequence from a combinatorial phage display library.

<400> 185
aagactagtc cggagtctac t 21

<210> 186
<211> 7
<212> PRT
<213> Artificial Sequence

<220>
<223> A sequence from a combinatorial phage display library.

<400> 186

Lys Thr Ser Pro Glu Ser Thr
1 5

<210> 187

<211> 27
<212> DNA
<213> Artificial Sequence

<220>
<223> A sequence from a combinatorial phage display library.

<400> 187
tgtaagacta gtccggagtc tacttgc 27

<210> 188
<211> 9
<212> PRT
<213> Artificial Sequence

<220>
<223> A sequence from a combinatorial phage display library.

<400> 188

Cys Lys Thr Ser Pro Glu Ser Thr Cys
1 5

<210> 189
<211> 21
<212> DNA
<213> Artificial Sequence

<220>
<223> A sequence from a combinatorial phage display library.

<400> 189
acgctttttc cggggaattc t 21

<210> 190
<211> 7
<212> PRT
<213> Artificial Sequence

<220>
<223> A sequence from a combinatorial phage display library.

<400> 190

Thr Leu Phe Pro Gly Asn Ser
1 5

<210> 191
<211> 27
<212> DNA
<213> Artificial Sequence

<220>
<223> A sequence from a combinatorial phage display library.

<400> 191
tgtacgcttt ttccggggaa ttcttgc 27

<210> 192
<211> 9
<212> PRT
<213> Artificial Sequence

<220>
<223> A sequence from a combinatorial phage display library.

<400> 192

Cys Thr Leu Phe Pro Gly Asn Ser Cys
1 5

<210> 193
<211> 21
<212> DNA
<213> Artificial Sequence

<220>
<223> A sequence from a combinatorial phage display library.

<400> 193
ttgccgtcgt ctacgaggct g 21

<210> 194
<211> 7
<212> PRT
<213> Artificial Sequence

<220>
<223> A sequence from a combinatorial phage display library.

<400> 194

Leu Pro Ser Ser Thr Arg Leu
1 5

<210> 195
<211> 27
<212> DNA
<213> Artificial Sequence

<220>
<223> A sequence from a combinatorial phage display library.

<400> 195
tgtttgccgt cgtctacgag gctgtgc 27

<210> 196
<211> 9

<212> PRT
<213> Artificial Sequence

<220>
<223> A sequence from a combinatorial phage display library.

<400> 196

Cys Leu Pro Ser Ser Thr Arg Leu Cys
1 5

<210> 197
<211> 21
<212> DNA
<213> Artificial Sequence

<220>
<223> A sequence from a combinatorial phage display library.

<400> 197
tcgagtcaga ggactcctcc g 21

<210> 198
<211> 7
<212> PRT

<220>
<213> Artificial Sequence

<223> A sequence from a combinatorial phage display library.

<400> 198

Ser Ser Gln Arg Thr Pro Pro
1 5

<210> 199
<211> 27
<212> DNA
<213> Artificial Sequence

<220>
<223> A sequence from a combinatorial phage display library.

<400> 199
tgttcgagtc agaggactcc tccgtgc 27

<210> 200
<211> 9
<212> PRT
<213> Artificial Sequence

<220>
<223> A sequence from a combinatorial phage display library.

<400> 200

Cys Ser Ser Gln Arg Thr Pro Pro Cys
1 5

<210> 201
<211> 21
<212> DNA
<213> Artificial Sequence

<220>
<223> A sequence from a combinatorial phage display library.

<400> 201
ctgcctacga tgacgccgac g 21

<210> 202
<211> 7
<212> PRT
<213> Artificial Sequence

<220>
<223> A sequence from a combinatorial phage display library.

<400> 202

Leu Pro Thr Met Thr Pro Thr
1 5

<210> 203
<211> 27
<212> DNA
<213> Artificial Sequence

<220>
<223> A sequence from a combinatorial phage display library.

<400> 203
tgtctgccta cgatgacgcc gacgtgc 27

<210> 204
<211> 9
<212> PRT
<213> Artificial Sequence

<220>
<223> A sequence from a combinatorial phage display library.

<400> 204

Cys Leu Pro Thr Met Thr Pro Thr Cys
1 5

<210> 205
<211> 21
<212> DNA
<213> Artificial Sequence

<220>
<223> A sequence from a combinatorial phage display library.

<400> 205
cttatgacgc cgtcgaagag g 21

<210> 206
<211> 7
<212> PRT
<213> Artificial Sequence

<220>
<223> A sequence from a combinatorial phage display library.

<400> 206

Leu Met Thr Pro Ser Lys Arg
1 5

<210> 207
<211> 27
<212> DNA
<213> Artificial Sequence

<220>
<223> A sequence from a combinatorial phage display library.

<400> 207
tgtcttatga cgccgtcgaa gaggtgc 27

<210> 208
<211> 9
<212> PRT
<213> Artificial Sequence

<220>
<223> A sequence from a combinatorial phage display library.

<400> 208

Cys Leu Met Thr Pro Ser Lys Arg Cys
1 5

<210> 209
<211> 21
<212> DNA
<213> Artificial Sequence

<220>
<223> A sequence from a combinatorial phage display library.

<400> 209
gagcattttt ttagtcggtc t 21

<210> 210
<211> 7
<212> PRT
<213> Artificial Sequence

<220>
<223> A sequence from a combinatorial phage display library.

<400> 210

Glu His Phe Phe Ser Arg Ser
1 5

<210> 211
<211> 27
<212> DNA
<213> Artificial Sequence

<220>
<223> A sequence from a combinatorial phage display library.

<400> 211
tgtgagcatt tttttagtcg gtcttgc 27

<210> 212
<211> 9
<212> PRT
<213> Artificial Sequence

<220>
<223> A sequence from a combinatorial phage display library.

<400> 212

Cys Glu His Phe Phe Ser Arg Ser Cys
1 5

<210> 213
<211> 21
<212> DNA
<213> Artificial Sequence

<220>
<223> A sequence from a combinatorial phage display library.

<400> 213
acgaatcagt ttttgcagca g 21

<210> 214
<211> 7
<212> PRT

<213> Artificial Sequence

<220>
<223> A sequence from a combinatorial phage display library.

<400> 214

Thr Asn Gln Phe Leu Gln Gln
1 5

<210> 215
<211> 27
<212> DNA
<213> Artificial Sequence

<220>
<223> A sequence from a combinatorial phage display library.

<400> 215
cgtacgaatc agtttttgca gcagtgc 27

<210> 216
<211> 9
<212> PRT
<213> Artificial Sequence

<220>
<223> A sequence from a combinatorial phage display library.

<400> 216

Cys Thr Asn Gln Phe Leu Gln Gln Cys
1 5

<210> 217
<211> 21
<212> DNA
<213> Artificial Sequence

<220>
<223> A sequence from a combinatorial phage display library.

<400> 217
cctgcgaata agtcttcgtt t 21

<210> 218
<211> 7
<212> PRT
<213> Artificial Sequence

<220>
<223> A sequence from a combinatorial phage display library.

<400> 218

Pro Ala Asn Lys Ser Ser Phe
1 5

<210> 219
<211> 27
<212> DNA
<213> Artificial Sequence

<220>
<223> A sequence from a combinatorial phage display library.

<400> 219
tgtcctgcga ataagtcttc gttttgc 27

<210> 220
<211> 9
<212> PRT
<213> Artificial Sequence

<220>
<223> A sequence from a combinatorial phage display library.

<400> 220

Cys Pro Ala Asn Lys Ser Ser Phe Cys
1 5

<210> 221
<211> 21
<212> DNA
<213> Artificial Sequence

<220>
<223> A sequence from a combinatorial phage display library.

<400> 221
agtacgacgc agtctagttg g 21

<210> 222
<211> 7
<212> PRT
<213> Artificial Sequence

<220>
<223> A sequence from a combinatorial phage display library.

<400> 222

Ser Thr Thr Gln Ser Ser Trp
1 5

<210> 223

<211> 27
<212> DNA
<213> Artificial Sequence

<220>
<223> A sequence from a combinatorial phage display library.

<400> 223
tgtagtacga cgcagtctag ttggtgc 27

<210> 224
<211> 9 <212> PRT
<213> Artificial Sequence

<220>
<223> A sequence from a combinatorial phage display library.

<400> 224

Cys Ser Thr Thr Gln Ser Ser Trp Cys
1 5

<210> 225
<211> 21
<212> DNA
<213> Artificial Sequence

<220>
<223> A sequence from a combinatorial phage display library.

<400> 225
gtgactccgg atcggctgac g 21

<210> 226
<211> 7
<212> PRT
<213> Artificial Sequence

<220>
<223> A sequence from a combinatorial phage display library.

<400> 226

Val Thr Pro Asp Arg Leu Thr
1 5

<210> 227
<211> 27
<212> DNA
<213> Artificial Sequence

<220>
<223> A sequence from a combinatorial phage display library.

<400> 227
tgtttgactc cggatcggct gacgtgc 27

<210> 228
<211> 9
<212> PRT
<213> Artificial Sequence

<220>
<223> A sequence from a combinatorial phage display library.

<400> 228

Cys Val Thr Pro Asp Arg Leu Thr Cys
1 5

<210> 229
<211> 21
<212> DNA
<213> Artificial Sequence

<220>
<223> A sequence from a combinatorial phage display library.

<400> 229
acgtggcaga cttagaggtc g 21

<210> 230
<211> 7
<212> PRT
<213> Artificial Sequence

<220>
<223> A sequence from a combinatorial phage display library.

<400> 230

Thr Trp Gln Thr Gln Arg Ser
1 5

<210> 231
<211> 27
<212> DNA
<213> Artificial Sequence

<220>
<223> A sequence from a combinatorial phage display library.

<400> 231
tgtacgtggc agacttagag gtcgtgc 27

<210> 232
<211> 9
<212> PRT

<213> Artificial Sequence

<220>
<223> A sequence from a combinatorial phage display library.

<400> 232

Cys Thr Trp Gln Thr Gln Arg Ser Cys
1 5

<210> 233
<211> 21
<212> DNA
<213> Artificial Sequence

<220>
<223> A sequence from a combinatorial phage display library.

<400> 233
ccgcatcctg ggactcgtca t 21

<210> 234
<211> 7
<212> PRT
<213> Artificial Sequence

<220>
<223> A sequence from a combinatorial phage display library.

<400> 234

Pro His Pro Gly Thr Arg His
1 5

<210> 235
<211> 27
<212> DNA
<213> Artificial Sequence

<220>
<223> A sequence from a combinatorial phage display library.

<400> 235
tgtccgcatc ctgggactcg tcattgc 27

<210> 236
<211> 9
<212> PRT
<213> Artificial Sequence

<220>
<223> A sequence from a combinatorial phage display library.

<400> 236

Cys Pro His Pro Gly Thr Arg His Cys
1 5

<210> 237
<211> 21
<212> DNA
<213> Artificial Sequence

<220>
<223> A sequence from a combinatorial phage display library.

<400> 237
gcgccgaagc cgcagtctca g 21

<210> 238
<211> 7
<212> PRT
<213> Artificial Sequence

<220>
<223> A sequence from a combinatorial phage display library.

<400> 238

Ala Pro Lys Pro Gln Ser Gln
1 5

<210> 239
<211> 27
<212> DNA
<213> Artificial Sequence

<220>
<223> A sequence from a combinatorial phage display library.

<400> 239
tgtgcgccga agccgcagtc tcagtgc 27

<210> 240
<211> 9
<212> PRT
<213> Artificial Sequence

<220>
<223> A sequence from a combinatorial phage display library.

<400> 240

Cys Ala Pro Lys Pro Gln Ser Gln Cys
1 5

<210> 241
<211> 21
<212> DNA
<213> Artificial Sequence

<220>
<223> A sequence from a combinatorial phage display library.

<400> 241
agtcaggcgt agattcctgc g 21

<210> 242
<211> 7
<212> PRT
<213> Artificial Sequence

<220>
<223> A sequence from a combinatorial phage display library.

<400> 242

Ser Gln Ala Gln Ile Pro Ala
1 5

<210> 243
<211> 27
<212> DNA
<213> Artificial Sequence

<220>
<223> A sequence from a combinatorial phage display library.

<400> 243
tgtagtcagg cgtagattcc tgcgtgc 27

<210> 244
<211> 9
<212> PRT
<213> Artificial Sequence

<220>
<223> A sequence from a combinatorial phage display library.

<400> 244

Cys Ser Gln Ala Gln Ile Pro Ala Cys
1 5

<210> 245
<211> 21
<212> DNA
<213> Artificial Sequence

<220>

<223> A sequence from a combinatorial phage display library.

<400> 245
ccgcagaata aggggaaggc t 21

<210> 246
<211> 7
<212> PRT
<213> Artificial Sequence

<220>
<223> A sequence from a combinatorial phage display library.

<400> 246

Pro Gln Asn Lys Gly Lys Ala
1 5

<210> 247
<211> 27
<212> DNA
<213> Artificial Sequence

<220>
<223> A sequence from a combinatorial phage display library.

<400> 247
tgtccgcaga ataaggggaa ggcttgc 27

<210> 248
<211> 9
<212> PRT
<213> Artificial Sequence

<220>
<223> A sequence from a combinatorial phage display library.

<400> 248

Cys Pro Gln Asn Lys Gly Lys Ala Cys
1 5

<210> 249
<211> 21
<212> DNA
<213> Artificial Sequence

<220>
<223> A sequence from a combinatorial phage display library.

<400> 249
catactgcgc atccgcggtc t 21

<210> 250
<211> 7
<212> PRT
<213> Artificial Sequence

<220>
<223> A sequence from a combinatorial phage display library.

<400> 250

His Thr Ala His Pro Arg Ser
1 5

<210> 251
<211> 27
<212> DNA
<213> Artificial Sequence

<220>
<223> A sequence from a combinatorial phage display library.

<400> 251
tgtcatactg cgcatccgcg gtcttgc 27

<210> 252
<211> 9
<212> PRT
<213> Artificial Sequence

<220>
<223> A sequence from a combinatorial phage display library.

<400> 252

Cys His Thr Ala His Pro Arg Ser Cys
1 5

<210> 253
<211> 21
<212> DNA
<213> Artificial Sequence

<220>
<223> A sequence from a combinatorial phage display library.

<400> 253
aagcagtctg gtcctgtttc t 21

<210> 254
<211> 7
<212> PRT
<213> Artificial Sequence

<220>

<223> A sequence from a combinatorial phage display library.

<400> 254

Lys Gln Ser Gly Pro Val Ser
1 5

<210> 255
<211> 27
<212> DNA
<213> Artificial Sequence

<220>
<223> A sequence from a combinatorial phage display library.

<400> 255
tgtaagcagt ctggtcctgt ttcttgc 27

<210> 256
<211> 9
<212> PRT
<213> Artificial Sequence

<220>
<223> A sequence from a combinatorial phage display library.

<400> 256

Cys Lys Gln Ser Gly Pro Val Ser Cys
1 5

<210> 257
<211> 21
<212> DNA
<213> Artificial Sequence

<220>
<223> A sequence from a combinatorial phage display library.

<400> 257
agtcagtatc cgtcgcgttc t 21

<210> 258
<211> 7
<212> PRT
<213> Artificial Sequence

<220>
<223> A sequence from a combinatorial phage display library.

<400> 258

Ser Gln Tyr Pro Ser Arg Ser
1 5

<210> 259
<211> 27
<212> DNA
<213> Artificial Sequence

<220>
<223> A sequence from a combinatorial phage display library.

<400> 259
tgtagtcagt atccgtcgcg ttcttgc 27

<210> 260
<211> 9
<212> PRT
<213> Artificial Sequence

<220>
<223> A sequence from a combinatorial phage display library.

<400> 260

Cys Ser Gln Tyr Pro Ser Arg Ser Cys
1 5

<210> 261
<211> 21
<212> DNA
<213> Artificial Sequence

<220>
<223> A sequence from a combinatorial phage display library.

<400> 261
tcgagggatg ggaagactac g 21

<210> 262
<211> 7
<212> PRT
<213> Artificial Sequence

<220>
<223> A sequence from a combinatorial phage display library.

<400> 262

Ser Arg Asp Gly Lys Thr Thr
1 5

<210> 263

<211> 27
<212> DNA
<213> Artificial Sequence

<220>
<223> A sequence from a combinatorial phage display library.

<400> 263
tgttcgaggg atgggaagac tacgtgc 27

<210> 264
<211> 9
<212> PRT
<213> Artificial Sequence

<220>
<223> A sequence from a combinatorial phage display library.

<400> 264

Cys Ser Arg Asp Gly Lys Thr Thr Cys
1 5

<210> 265
<211> 21
<212> DNA
<213> Artificial Sequence

<220>
<223> A sequence from a combinatorial phage display library.

<400> 265
acgacgctga tgcctaatat t 21

<210> 266
<211> 7
<212> PRT
<213> Artificial Sequence

<220>
<223> A sequence from a combinatorial phage display library.

<400> 266

Thr Thr Leu Met Pro Asn Ile
1 5

<210> 267
<211> 27
<212> DNA
<213> Artificial Sequence

<220>
<223> A sequence from a combinatorial phage display library.

<400> 267
tgtacgacgc tgatgcctaa tatttgc 27

<210> 268
<211> 9
<212> PRT
<213> Artificial Sequence

<220>
<223> A sequence from a combinatorial phage display library.

<400> 268

Cys Thr Thr Leu Met Pro Asn Ile Cys
1 5

<210> 269
<211> 21
<212> DNA
<213> Artificial Sequence

<220>
<223> A sequence from a combinatorial phage display library.

<400> 269
acgaataagc ttgataatac t 21

<210> 270
<211> 7
<212> PRT
<213> Artificial Sequence

<220>
<223> A sequence from a combinatorial phage display library.

<400> 270

Thr Asn Lys Leu Asp Asn Thr
1 5

<210> 271
<211> 27
<212> DNA
<213> Artificial Sequence

<220>
<223> A sequence from a combinatorial phage display library.

<400> 271
tgtacgaata agcttgataa tacttgc 27

<210> 272
<211> 9
<212> PRT

<213> Artificial Sequence

<220>
<223> A sequence from a combinatorial phage display library.

<400> 272

Cys Thr Asn Lys Leu Asp Asn Thr Cys
1 5

<210> 273
<211> 21
<212> DNA
<213> Artificial Sequence

<220>
<223> A sequence from a combinatorial phage display library.

<400> 273
actaagatgc ggttggagca g 21

<210> 274
<211> 7
<212> PRT
<213> Artificial Sequence

<220>
<223> A sequence from a combinatorial phage display library.

<400> 274

Thr Lys Met Arg Leu Glu Gln
1 5

<210> 275
<211> 27
<212> DNA
<213> Artificial Sequence

<220>
<223> A sequence from a combinatorial phage display library.

<400> 275
tgtactaaga tgcggttgga gcagtgc 27

<210> 276
<211> 9
<212> PRT
<213> Artificial Sequence

<220>
<223> A sequence from a combinatorial phage display library.

<400> 276

Cys Thr Lys Met Arg Leu Glu Gln Cys
1 5

<210> 277
<211> 21
<212> DNA
<213> Artificial Sequence

<220>
<223> A sequence from a combinatorial phage display library.

<400> 277
tcgccggatc cgggtagtaa g 21

<210> 278
<211> 7
<212> PRT
<213> Artificial Sequence

<220>
<223> A sequence from a combinatorial phage display library.

<400> 278

Ser Pro Asp Pro Gly Ser Lys
1 5

<210> 279
<211> 27
<212> DNA
<213> Artificial Sequence

<220>
<223> A sequence from a combinatorial phage display library.

<400> 279
tgttcgccgg atccgggtag taagtgc 27

<210> 280
<211> 9
<212> PRT
<213> Artificial Sequence

<220>
<223> A sequence from a combinatorial phage display library.

<400> 280

Cys Ser Pro Asp Pro Gly Ser Lys Cys
1 5

<210> 281
<211> 21

<212> DNA
<213> Artificial Sequence

<220>
<223> A sequence from a combinatorial phage display library.

<400> 281
gagcattttt ttagtcggtc t 21

<210> 282
<211> 7
<212> PRT
<213> Artificial Sequence

<220>
<223> A sequence from a combinatorial phage display library.

<400> 282

Glu His Phe Phe Ser Arg Ser
1 5

<210> 283
<211> 27
<212> DNA
<213> Artificial Sequence

<220>
<223> A sequence from a combinatorial phage display library.

c400> 283
tgtgagcatt tttttagtcg gtcttgc 27

<210> 284
<211> 9
<212> PRT
<213> Artificial Sequence

<220>
<223> A sequence from a combinatorial phage display library.

<400> 284

Cys Glu His Phe Phe Ser Arg Ser Cys
1 5

<210> 285
<211> 21
<212> DNA
<213> Artificial Sequence

<220>
<223> A sequence from a combinatorial phage display library.

<400> 285
ggggcgccgt ctgatcatgt g 21

<210> 286
<211> 7
<212> PRT
<213> Artificial Sequence

<220>
<223> A sequence from a combinatorial phage display library.

<400> 286

Gly Ala Pro Ser Asp His Val
1 5

<210> 287
<211> 27
<212> DNA
<213> Artificial Sequence

<220>
<223> A sequence from a combinatorial phage display library.

<400> 287
tgtggggcgc cgtctgatca tgtgtgc 27

<210> 288
<211> 9
<212> PRT
<213> Artificial Sequence

<220>
<223> A sequence from a combinatorial phage display library.

<400> 288

Cys Gly Ala Pro Ser Asp His Val Cys
1 5

<210> 289
<211> 21
<212> DNA
<213> Artificial Sequence

<220>
<223> A sequence from a combinatorial phage display library.

<400> 289
ccgcatcctg ggactcgtca t 21

<210> 290
<211> 7
<212> PRT

<213> Artificial Sequence

<220>
<223> A sequence from a combinatorial phage display library.

<400> 290

Pro His Pro Gly Thr Arg His
1 5

<210> 291
<211> 27
<212> DNA
<213> Artificial Sequence

<220>
<223> A sequence from a combinatorial phage display library.

<400> 291
tgtccgcatc ctgggactcg tcattgc 27

<210> 292
<211> 9
<212> PRT
<213> Artificial Sequence

<220>
<223> A sequence from a combinatorial phage display library.

<400> 292

Cys Pro His Pro Gly Thr Arg His Cys
1 5

<210> 293
<211> 21
<212> DNA
<213> Artificial Sequence

<220>
<223> A sequence from a combinatorial phage display library.

<400> 293
attaagcagt cgctgtctcg t 21

<210> 294
<211> 7
<212> PRT
<213> Artificial Sequence

<220>
<223> A sequence from a combinatorial phage display library.

<400> 294

Ile Lys Gln Ser Leu Ser Arg
1 5

<210> 295
<211> 27
<212> DNA
<213> Artificial Sequence

<220>
<223> A sequence from a combinatorial phage display library.

<400> 295
tgtattaagc agtcgctgtc tcgttgc 27

<210> 296
<211> 9
<212> PRT
<213> Artificial Sequence

<220>
<223> A sequence from a combinatorial phage display library.

<400> 296

Cys Ile Lys Gln Ser Leu Ser Arg Cys
1 5

<210> 297
<211> 21
<212> DNA
<213> Artificial Sequence

<220>
<223> A sequence from a combinatorial phage display library.

<400> 297
acgacgcata atgcgaagtg g 21

<210> 298
<211> 7
<212> PRT
<213> Artificial Sequence

<220>
<223> A sequence from a combinatorial phage display library.

<400> 298

Thr Thr His Asn Ala Lys Trp
1 5

<210> 299

<211> 27
<212> DNA
<213> Artificial Sequence

<220>
<223> A sequence from a combinatorial phage display library.

<400> 299
tgtacgacgc ataatgcgaa gtggtgc 27

<210> 300
<211> 9
<212> PRT
<213> Artificial Sequence

<220>
<223> A sequence from a combinatorial phage display library.

<400> 300

Cys Thr Thr His Asn Ala Lys Trp Cys
1 5

<210> 301
<211> 21
<212> DNA
<213> Artificial Sequence

<220>
<223> A sequence from a combinatorial phage display library.

<400> 301
ctgactacga agcctaggat g 21

<210> 302
<211> 7
<212> PRT
<213> Artificial Sequence

<220>
<223> A sequence from a combinatorial phage display library.

<400> 302

Leu Thr Thr Lys Pro Arg Met
1 5

<210> 303
<211> 27
<212> DNA
c213> Artificial Sequence

<220>
<223> A sequence from a combinatorial phage display library.

<400> 303
tgtctgacta cgaagcctag gatgtgc 27

<210> 304
<211> 9
<212> PRT
<213> Artificial Sequence

<220>
<223> A sequence from a combinatorial phage display library.

<400> 304

Cys Leu Thr Thr Lys Pro Arg Met Cys
1 5

<210> 305
<211> 21
<212> DNA
<213> Artificial Sequence

<220>
<223> A sequence from a combinatorial phage display library.

<400> 305
aagctgaagt cggggtcgct g 21

<210> 306
<211> 7
<212> PRT
<213> Artificial Sequence

<220>
<223> A sequence from a combinatorial phage display library.

<400> 306

Lys Leu Lys Ser Gly Ser Leu
1 5

<210> 307
<211> 27
<212> DNA
<213> Artificial Sequence

<220>
<223> A sequence from a combinatorial phage display library.

<400> 307
tgtaagctga agtcggggtc gctgtgc 27

<210> 308
<211> 9
<212> PRT

<213> Artificial Sequence

<220>
<223> A sequence from a combinatorial phage display library.

<400> 308

Cys Lys Leu Lys Ser Gly Ser Leu Cys
1 5

<210> 309
<211> 21
<212> DNA
<213> Artificial Sequence

<220>
<223> A sequence from a combinatorial phage display library.

<400> 309
cttccgtcga aggtgtctcg g 21

<210> 310
<211> 7
<212> PRT
<213> Artificial Sequence

<220>
<223> A sequence from a combinatorial phage display library.

<400> 310

Leu Pro Ser Lys Val Ser Arg
1 5

<210> 311
<211> 27
<212> DNA
<213> Artificial Sequence

<220>
<223> A sequence from a combinatorial phage display library.

c400> 311
tgtcttccgt cgaaggtgtc tcggtgc 27

<210> 312
<211> 9
<212> PRT
<213> Artificial Sequence

<220>
<223> A sequence from a combinatorial phage display library.

<400> 312

Cys Leu Pro Ser Lys Val Ser Arg Cys
1 5

<210> 313
<211> 21
<212> DNA
<213> Artificial Sequence

<220>
<223> A sequence from a combinatorial phage display library.

<400> 313
gcgccgggtc cgtctaagag t 21

<210> 314
<211> 7
<212> PRT
<213> Artificial Sequence

<220>
<223> A sequence from a combinatorial phage display library.

<400> 314

Ala Pro Gly Pro Ser Lys Ser
1 5

<210> 315
<211> 27
<212> DNA
<213> Artificial Sequence

<220>
<223> A sequence from a combinatorial phage display library.

<400> 315
tgtgcgccgg gtccgtctaa gagttgc 27

<210> 316
<211> 9
<212> PRT
<213> Artificial Sequence

<220>
<223> A sequence from a combinatorial phage display library.

<400> 316

Cys Ala Pro Gly Pro Ser Lys Ser Cys
1 5

<210> 317

<211> 21
<212> DNA
<213> Artificial Sequence

<220>
<223> A sequence from a combinatorial phage display library.

<400> 317
tctccgctta agtctctttc g 21

<210> 318
<211> 7
<212> PRT
<213> Artificial Sequence

<220>
<223> A sequence from a combinatorial phage display library.

<400> 318

Ser Pro Leu Lys Ser Leu Ser
1 5

<210> 319
<211> 27
<212> DNA
<213> Artificial Sequence

<220>
<223> A sequence from a combinatorial phage display library.

<400> 319
tgttctccgc ttaagtctct ttcgtgc 27

<210> 320
<211> 9
<212> PRT
<213> Artificial Sequence

<220>
<223> A sequence from a combinatorial phage display library.

<400> 320

Cys Ser Pro Leu Lys Ser Leu Ser Cys
1 5

<210> 321
<211> 21
<212> DNA
<213> Artificial Sequence

<220>
<223> A sequence from a combinatorial phage display library.

<400> 321
gcgccgggtc cgtctaagag t 21

<210> 322
<211> 7
<212> PRT
<213> Artificial Sequence

<220>
<223> A sequence from a combinatorial phage display library.

<400> 322

Ala Pro Gly Pro Ser Lys Ser
1 5

<210> 323
<211> 27
<212> DNA
<213> Artificial Sequence

<220>
<223> A sequence from a combinatorial phage display library.

<400> 323
tgtgcgccgg gtccgtctaa gagttgc 27

<210> 324
<211> 9
<212> PRT
<213> Artificial Sequence

<220>
<223> A sequence from a combinatorial phage display library.

<400> 324

Cys Ala Pro Gly Pro Ser Lys Ser Cys
1 5

<210> 325
<211> 21
<212> DNA
<213> Artificial Sequence

<220>
<223> A sequence from a combinatorial phage display library.

<400> 325
ccgtcgggtc ttactaagca g 21

<210> 326
<211> 7

<212> PRT
<213> Artificial Sequence

<220>
<223> A sequence from a combinatorial phage display library.

<400> 326

Pro Ser Gly Leu Thr Lys Gln
1 5

<210> 327
<211> 27
<212> DNA
<213> Artificial Sequence

<220>
<223> A sequence from a combinatorial phage display library.

<400> 327
tgtccgtcgg gtcttactaa gcagtgc 27

<210> 328
<211> 9

<212> PRT
<213> Artificial Sequence

<220>
<223> A sequence from a combinatorial phage display library.

<400> 328

Cys Pro Ser Gly Leu Thr Lys Gln Cys
1 5

<210> 329
<211> 21
<212> DNA
<213> Artificial Sequence

c220>
<223> A sequence from a combinatorial phage display library.

<400> 329
aagtcgaata tgcctctgag t 21

<210> 330
<211> 7
<212> PRT
<213> Artificial Sequence

<220>
<223> A sequence from a combinatorial phage display library.

<400> 330

Lys Ser Asn Met Pro Leu Thr
1 5

<210> 331
<211> 27
<212> DNA
<213> Artificial Sequence

<220>
<223> A sequence from a combinatorial phage display library.

<400> 331
tgtaagtcga atatgcctct gagttgc 27

<210> 332
<211> 9
<212> PRT
<213> Artificial Sequence

<220>
<223> A sequence from a combinatorial phage display library.

<400> 332

Cys Lys Ser Asn Met Pro Leu Thr Cys
1 5

<210> 333
<211> 21
<212> DNA
<213> Artificial Sequence

<220>
<223> A sequence from a combinatorial phage display library.

<400> 333
aatcagcggc attagatgtc t 21

<210> 334
<211> 7
<212> PRT
<213> Artificial Sequence

<220>
<223> A sequence from a combinatorial phage display library.

<400> 334

Asn Gln Arg His Gln Met Ser
1 5

<210> 335
<211> 27
<212> DNA
<213> Artificial Sequence

<220>
<223> A sequence from a combinatorial phage display library.

<400> 335
tgtaatcagc ggcattagat gtcttgc 27

<210> 336
<211> 9
<212> PRT
<213> Artificial Sequence

<220>
<223> A sequence from a combinatorial phage display library.

<400> 336

Cys Asn Gln Arg His Gln Met Ser Cys
1 5

<210> 337
<211> 21
<212> DNA
<213> Artificial Sequence

<220>
<223> A sequence from a combinatorial phage display library.

<400> 337
cagcgggcgg atcagaagca g 21

<210> 338
<211> 7
<212> PRT
<213> Artificial Sequence

<220>
<223> A sequence from a combinatorial phage display library.

<400> 338

Gln Arg Ala Asp Gln Lys Gln
1 5

<210> 339
<211> 27
<212> DNA
<213> Artificial Sequence

<220>

<223> A sequence from a combinatorial phage display library.

<400> 339
tgtcagcggg cggatcagaa gcagtgc 27

<210> 340
<211> 9
<212> PRT
<213> Artificial Sequence .

<220>
<223> A sequence from a combinatorial phage display library.

<400> 340

Cys Gln Arg Ala Asp Gln Lys Gln Cys
1 5

<210> 341
<211> 21
<212> DNA
<213> Artificial Sequence

<220>
<223> A sequence from a combinatorial phage display library.

<400> 341
aatcatcggt atatgcagat g 21

<210> 342
<211> 7
<212> PRT
<213> Artificial Sequence

<220>
<223> A sequence from a combinatorial phage display library.

<400> 342

Asn His Arg Tyr Met Gln Met
1 5

<210> 343
<211> 27
<212> DNA
<213> Artificial Sequence

<220>
<223> A sequence from a combinatorial phage display library.

<400> 343
tgtaatcatc ggtatatgca gatgtgc 27

<210> 344

<211> 9
<212> PRT
<213> Artificial Sequence

<220>
<223> A sequence from a combinatorial phage display library.

<400> 344

Cys Asn His Arg Tyr Met Gln Met Cys
1 5

<210> 345
<211> 21
<212> DNA
<213> Artificial Sequence

<220>
<223> A sequence from a combinatorial phage display library.

<400> 345
attactccta tgtctcgtac t 21

<210> 346
<211> 7
<212> PRT
<213> Artificial Sequence

<220>
<223> A sequence from a combinatorial phage display library.

<400> 346

Ile Thr Pro Met Ser Arg Thr
1 5

<210> 347
<211> 27
<212> DNA
<213> Artificial Sequence

<220>
<223> A sequence from a combinatorial phage display library.

<400> 347
tgtattactc ctatgtctcg tacttgc 27

<210> 348
<211> 9
<212> PRT
<213> Artificial Sequence

<220>

<223> A sequence from a combinatorial phage display library.

<400> 348

Cys Ile Thr Pro Met Ser Arg Thr Cys
1 5

<210> 349
<211> 21
<212> DNA
<213> Artificial Sequence

<220>
<223> A sequence from a combinatorial phage display library.

<400> 349
agtcctacga ttgggcagaa g 21

<210> 350
<211> 7
<212> PRT
<213> Artificial Sequence

<220>
<223> A sequence from a combinatorial phage display library.

<400> 350

Ser Pro Thr Ile Gly Gln Lys
1 5

<210> 351
<211> 27
<212> DNA
<213> Artificial Sequence

<220>
<223> A sequence from a combinatorial phage display library.

<400> 351
tgtagtccta cgattgggca gaagtgc 27

<210> 352
<211> 9
<212> PRT
<213> Artificial Sequence

<220>
<223> A sequence from a combinatorial phage display library.

<400> 352

Cys Ser Pro Thr Ile Gly Gln Lys Cys
1 5

<210> 353
<211> 21
<212> DNA
<213> Artificial Sequence

<220>
<223> A sequence from a combinatorial phage display library.

<400> 353
tctaattatt cgctgggtat g 21

<210> 354
<211> 7
<212> PRT
<213> Artificial Sequence

<220>
<223> A sequence from a combinatorial phage display library.

<400> 354

Ser Asn Tyr Ser Leu Gly Met
1 5

<210> 355
<211> 27
<212> DNA
<213> Artificial Sequence

<220>
<223> A sequence from a combinatorial phage display library.

<400> 355
tgttctaatt attcgctggg tatgtgc 27

<210> 356
<211> 9
<212> PRT
<213> Artificial Sequence

<220>
<223> A sequence from a combinatorial phage display library.

<400> 356

Cys Ser Asn Tyr Ser Leu Gly Met Cys
1 5

<210> 357

<211> 21
<212> DNA
<213> Artificial Sequence

<220>
<223> A sequence from a combinatorial phage display library.

<400> 357
acgaatacgg ggcataggca t 21

<210> 358
<211> 7
<212> PRT
<213> Artificial Sequence

<220>
<223> A sequence from a combinatorial phage display library.

<400> 358

Thr Asn Thr Gly His Arg His
1 5

<210> 359
<211> 27
<212> DNA
<213> Artificial Sequence

<220>
c223> A sequence from a combinatorial phage display library.

c400> 359
tgtacgaata cggggcatag gcattgc 27

<210> 360
<211> 9
<212> PRT
<213> Artificial Sequence

<220>
<223> A sequence from a combinatorial phage display library.

<400> 360

Cys Thr Asn Thr Gly His Arg His Cys
1 5

<210> 361
<211> 21
<212> DNA
<213> Artificial Sequence

<220>
<223> A sequence from a combinatorial phage display library.

<400> 361
actatgcgta ctaattctag t 21

<210> 362
<211> 7
<212> PRT
<213> Artificial Sequence

<220>
<223> A sequence from a combinatorial phage display library.

<400> 362

Thr Met Arg Thr Asn Ser Ser
1 5

<210> 363
<211> 27
<212> DNA
<213> Artificial Sequence

<220>
<223> A sequence from a combinatorial phage display library.

<400> 363
tgtactatgc gtactaattc tagttgc 27

<210> 364
<211> 9
<212> PRT
<213> Artificial Sequence

<220>
<223> A sequence from a combinatorial phage display library.

<400> 364

Cys Thr Met Arg Thr Asn Ser Ser Cys
1 5

<210> 365
<211> 21
<212> DNA
<213> Artificial Sequence

<220>
<223> A sequence from a combinatorial phage display library.

<400> 365
acggcgccgt tggagcggag g 21

<210> 366
<211> 7
<212> PRT

<213> Artificial Sequence

<220>
<223> A sequence from a combinatorial phage display library.

<400> 366

Thr Ala Pro Leu Glu Arg Arg
1 5

<210> 367
<211> 27
<212> DNA
<213> Artificial Sequence

<220>
<223> A sequence from a combinatorial phage display library.

<400> 367
tgtacggcgc cgttggagcg gaggtgc 27

<210> 368
<211> 9
<212> PRT
<213> Artificial Sequence

<220>
<223> A sequence from a combinatorial phage display library.

<400> 368

Cys Thr Ala Pro Leu Glu Arg Arg Cys
1 5

<210> 369
<211> 21
<212> DNA
<213> Artificial Sequence

<220>
<223> A sequence from a combinatorial phage display library.

<400> 369
ctgcttgggg agcctcggac t 21

<210> 370
<211> 7
<212> PRT
<213> Artificial Sequence

<220>
<223> A sequence from a combinatorial phage display library.

<400> 370

Leu Leu Gly Glu Pro Arg Thr
1 5

<210> 371
<211> 27
<212> DNA
<213> Artificial Sequence

<220>
<223> A sequence from a combinatorial phage display library.

<400> 371
tgtctgcttg gggagcctcg gacttgc 27

<210> 372
<211> 9
<212> PRT
<213> Artificial Sequence

<220>
<223> A sequence from a combinatorial phage display library.

<400> 372

Cys Leu Leu Gly Glu Pro Arg Thr Cys
1 5

<210> 373
<211> 21
<212> DNA
<213> Artificial Sequence

<220>
<223> A sequence from a combinatorial phage display library.

<400> 373
tctcgtgcta gtactaatga t 21

<210> 374
<211> 7
<212> PRT
<213> Artificial Sequence

<220>
<223> A sequence from a combinatorial phage display library.

<400> 374

Ser Arg Ala Ser Thr Asn Asp
1 5

<210> 375
<211> 27
<212> DNA
<213> Artificial Sequence

<220>
<223> A sequence from a combinatorial phage display library.

<400> 375
tgttctcgtg ctagtactaa tgattgc 27

<210> 376
<211> 9
<212> PRT
<213> Artificial Sequence

<220>
<223> A sequence from a combinatorial phage display library.

<400> 376

Cys Ser Arg Ala Ser Thr Asn Asp Cys
1 5

<210> 377

<211> 21
<212> DNA
<213> Artificial Sequence

<220>
<223> A sequence from a combinatorial phage display library.

<400> 377
aataagtcga ataaggagtt t 21

<210> 378
<211> 7
<212> PRT
<213> Artificial Sequence

<220>
<223> A sequence from a combinatorial phage display library.

<400> 378

Asn Lys Ser Asn Lys Glu Phe
1 5

<210> 379
<211> 27
<212> DNA
<213> Artificial Sequence

<220>
<223> A sequence from a combinatorial phage display library.

<400> 379
tgtaataagt cgaataagga gttttgc 27

<210> 380
<211> 9
<212> PRT
<213> Artificial Sequence

<220>
<223> A sequence from a combinatorial phage display library.

<400> 380

Cys Asn Lys Ser Asn Lys Glu Phe Cys
1 5

<210> 381 <211> 21
<212> DNA
<213> Artificial Sequence

<220>
<223> A sequence from a combinatorial phage display library.

<400> 381
catgcgcggg tgccgctggt t 21

<210> 382
<211> 7
<212> PRT
<213> Artificial Sequence

<220>
<223> A sequence from a combinatorial phage display library.

<400> 382

His Ala Arg Val Pro Leu Val
1 5

<210> 383
<211> 27
<212> DNA
<213> Artificial Sequence

<220>
<223> A sequence from a combinatorial phage display library.

<400> 383
tgtcatgcgc gggtgccgct ggtttgc 27

<210> 384

<211> 9
<212> PRT
<213> Artificial-Sequence

<220>
<223> A sequence from a combinatorial phage display library.

<400> 384

Cys His Ala Arg Val Pro Leu Val Cys
1 5

<210> 385
<211> 21
<212> DNA
<213> Artificial Sequence

<220>
<223> A sequence from a combinatorial phage display library.

<400> 385
cttaataatt ctcaggctca t 21

<210> 386
<211> 7
<212> PRT
<213> Artificial Sequence

<220>
<223> A sequence from a combinatorial phage display library.

<400> 386

Leu Asn Asn Ser Gln Ala His
1 5

<210> 387
<211> 27
<212> DNA
<213> Artificial Sequence

<220>
<223> A sequence from a combinatorial phage display library.

<400> 387
tgtcttaata attctcaggc tcattgc 27

<210> 388
<211> 9
<212> PRT
<213> Artificial Sequence

<220>
<223> A sequence from a combinatorial phage display library.

<400> 388

Cys Leu Asn Asn Ser Gln Ala His Cys
1 5

<210> 389
<211> 21
<212> DNA
<213> Artificial Sequence

<220>
<223> A sequence from a combinatorial phage display library.

<400> 389
aatccgtcgc gttctacgtc g 21

<210> 390
<211> 7
<212> PRT
<213> Artificial Sequence

<220>
<223> A sequence from a combinatorial phage display library.

<400> 390

Asn Pro Ser Arg Ser Thr Ser
1 5

<210> 391
<211> 27
<212> DNA
<213> Artificial Sequence

<220>
<223> A sequence from a combinatorial phage display library.

<400> 391
tgtaatccgt cgcgttctac gtcgtgc 27

<210> 392
<211> 9
<212> PRT
<213> Artificial Sequence

<220>
<223> A sequence from a combinatorial phage display library.

<400> 392

Cys Asn Pro Ser Arg Ser Thr Ser Cys
1 5

<210> 393
<211> 21
<212> DNA
<213> Artificial Sequence

<220>
<223> A sequence from a combinatorial phage display library.

<400> 393
acgccgactt agaagagttt g 21

<210> 394
<211> 7
<212> PRT
<213> Artificial Sequence

<220>
<223> A sequence from a combinatorial phage display library.

<400> 394

Thr Pro Thr Gln Lys Ser Leu
1 5

<210> 395
<211> 27
<212> DNA
<213> Artificial Sequence

<220>
<223> A sequence from a combinatorial phage display library.

<400> 395
tgtacgccga cttagaagag tttgtgc 27

<210> 396
<211> 9
<212> PRT
<213> Artificial Sequence

<220>
<223> A sequence from a combinatorial phage display library.

<400> 396

Cys Thr Pro Thr Gln Lys Ser Leu Cys
1 5

<210> 397
<211> 21
<212> DNA
<213> Artificial Sequence

<220>

<223> A sequence from a combinatorial phage display library.

<400> 397
tcgcagcggc ctgttcagat g 21

<210> 398
<211> 7
<212> PRT
<213> Artificial Sequence

<220>
<223> A sequence from a combinatorial phage display library.

<400> 398

Ser Gln Arg Pro Val Gln Met
1 5

<210> 399
<211> 27
<212> DNA
<213> Artificial Sequence

<220>
<223> A sequence from a combinatorial phage display library.

<400> 399
tgttcgcagc ggcctgttca gatgtgc 27

<210> 400
<211> 9
<212> PRT
<213> Artificial Sequence

<220>
<223> A sequence from a combinatorial phage display library.

<400> 400

Cys Ser Gln Arg Pro Val Gln Met Cys
1 5

<210> 401
<211> 21
<212> DNA
<213> Artificial Sequence

<220>
<223> A sequence from a combinatorial phage display library.

<400> 401
gcgccgggtc cgtctaagag t 21

<210> 402
<211> 7
<212> PRT
<213> Artificial Sequence

<220>
<223> A sequence from a combinatorial phage display library.

<400> 402

Ala Pro Gly Pro Ser Lys Ser
1 5

<210> 403
<211> 27
<212> DNA
<213> Artificial Sequence

<220>
<223> A sequence from a combinatorial phage display library.

<400> 403
tgtgcgccgg gtccgtctaa gagttgc 27

<210> 404
<211> 9
<212> PRT
<213> Artificial Sequence

<220>
<223> A sequence from a combinatorial phage display library.

<400> 404

Cys Ala Pro Gly Pro Ser Lys Ser Cys
1 5

<210> 405
<211> 21
<212> DNA
<213> Artificial Sequence

<220>
<223> A sequence from a combinatorial phage display library.

<400> 405
aaggggtcgt ctattcttaa t 21

<210> 406
<211> 7
<212> PRT
<213> Artificial Sequence

<220>

<223> A sequence from a combinatorial phage display library.

<400> 406

Lys Gly Ser Ser Ile Leu Asn
1               5

<210> 407
<211> 27
<212> DNA
<213> Artificial Sequence

<220>
<223> A sequence from a combinatorial phage display library.

<400> 407
tgtaaggggt cgtctattct taattgc 27

<210> 408
<211> 9
<212> PRT
<213> Artificial Sequence

<220>
<223> A sequence from a combinatorial phage display library.

<400> 408

Cys Lys Gly Ser Ser Ile Leu Asn Cys
1               5

<210> 409
<211> 21
<212> DNA
<213> Artificial Sequence

<220>
<223> A sequence from a combinatorial phage display library.

<400> 409
gttaatcgga gtgatgggat g 21

<210> 410
<211> 7
<212> PRT
<213> Artificial Sequence

<220>
<223> A sequence from a combinatorial phage display library.

<400> 410

Val Asn Arg Ser Asp Gly Met
1 5

<210> 411
<211> 27
<212> DNA
<213> Artificial Sequence

<220>
<223> A sequence from a combinatorial phage display library.

<400> 411
tgtgttaatc ggagtgatgg gatgtgc 27

<210> 412
<211> 9
<212> PRT
<213> Artificial Sequence

<220>
<223> A sequence from a combinatorial phage display library.

<400> 412

Cys Val Asn Arg Ser Asp Gly Met Cys
1 5

<210> 413
<211> 21
<212> DNA
<213> Artificial Sequence

<220>
<223> A sequence from a combinatorial phage display library.

<400> 413
ccgcatcctg ggactcgtca t 21

<210> 414
<211> 7
<212> PRT
<213> Artificial Sequence

<220>
<223> A sequence from a combinatorial phage display library.

<400> 414

Pro His Pro Gly Thr Arg His
1 5

<210> 415
<211> 27
<212> DNA
<213> Artificial Sequence

<220>
<223> A sequence from a combinatorial phage display library.

<400> 415
tgtccgcatc ctgggactcg tcattgc 27

<210> 416
<211> 9
<212> PRT
<213> Artificial Sequence

<220>
<223> A sequence from a combinatorial phage display library.

<400> 416

Cys Pro His Pro Gly Thr Arg His Cys
1 5

<210> 417
<211> 21
<212> DNA
<213> Artificial Sequence

<220>
<223> A sequence from a combinatorial phage display library.

<400> 417
atgaatcagc gggttcagaa t 21

<210> 418
<211> 7
<212> PRT
<213> Artificial Sequence

<220>
<223> A sequence from a combinatorial phage display library.

<400> 418

Met Asn Gln Arg Val Gln Asn
1 5

<210> 419
<211> 27
<212> DNA
<213> Artificial Sequence

<220>

<223> A sequence from a combinatorial phage display library.

<400> 419
tgtatgaatc agcgggttca gaattgc 27

<210> 420
<211> 9
<212> PRT
<213> Artificial Sequence

<220>
<223> A sequence from a combinatorial phage display library.

<400> 420

Cys Met Asn Gln Arg Val Gln Asn Cys
1 5

<210> 421
<211> 21
<212> DNA
<213> Artificial Sequence

<220>
<223> A sequence from a combinatorial phage display library.

<400> 421
aatcagtgga agtcggtgtc t 21

<210> 422
<211> 7
<212> PRT
<213> Artificial Sequence

<220>
<223> A sequence from a combinatorial phage display library.

<400> 422

Asn Gln Trp Lys Ser Val Ser
1 5

<210> 423
<211> 27
<212> DNA
<213> Artificial Sequence

<220>
<223> A sequence from a combinatorial phage display library.

<400> 423
tgtaatcagt ggaagtcggt gtcttgc 27

<210> 424
<211> 9

<212> PRT
<213> Artificial Sequence

<220>
<223> A sequence from a combinatorial phage display library.

<400> 424

Cys Asn Gln Trp Lys Ser Val Ser Cys
1 5

<210> 425
<211> 21
<212> DNA
<213> Artificial Sequence

<220>
<223> A sequence from a combinatorial phage display library.

<400> 425
cagacgcatg ctcggcatgt t 21

<210> 426
<211> 7
<212> PRT
<213> Artificial Sequence

<220>
<223> A sequence from a combinatorial phage display library.

<400> 426

Gln Thr His Ala Arg His Val
1 5

<210> 427
<211> 27
<212> DNA
<213> Artificial Sequence

<220>
<223> A sequence from a combinatorial phage display library.

<400> 427
tgtcagacgc atgctcggca tgtttgc 27

<210> 428
<211> 9
<212> PRT
<213> Artificial Sequence

<220>
<223> A sequence from a combinatorial phage display library.

<400> 428

Cys Gln Thr His Ala Arg His Val Cys
1 5

<210> 429
<211> 21
<212> DNA
<213> Artificial Sequence

<220>
<223> A sequence from a combinatorial phage display library.

<400> 429
tttcagaatc gtcagccgat g 21

<210> 430
<211> 7
<212> PRT
<213> Artificial Sequence

<220>
<223> A sequence from a combinatorial phage display library.

<400> 430

Phe Gln Asn Arg Gln Pro Met
1 5

<210> 431
<211> 27
<212> DNA
<213> Artificial Sequence

<220>
<223> A sequence from a combinatorial phage display library.

<400> 431
tgttttcaga atcgtcagcc gatgtgc 27

<210> 432
<211> 9
<212> PRT
<213> Artificial Sequence

<220>
<223> A sequence from a combinatorial phage display library.

<400> 432

Cys Phe Gln Asn Arg Gln Pro Met Cys
1 5

<210> 433
<211> 21
<212> DNA
<213> Artificial Sequence

<220>
<223> A sequence from a combinatorial phage display library.

<400> 433
agggctctgg atacggcgaa t 21

<210> 434
<211> 7
<212> PRT
<213> Artificial Sequence

<220>
<223> A sequence from a combinatorial phage display library.

<400> 434

Arg Ala Leu Asp Thr Ala Asn
1 5

<210> 435
<211> 27
<212> DNA
<213> Artificial Sequence

<220>
<223> A sequence from a combinatorial phage display library.

<400> 435
tgtagggctc tggatacggc gaattgc 27

<210> 436
<211> 9
<212> PRT
<213> Artificial Sequence

<220>
<223> A sequence from a combinatorial phage display library.

<400> 436

Cys Arg Ala Leu Asp Thr Ala Asn Cys
1 5

<210> 437

<211> 21
<212> DNA
<213> Artificial Sequence

<220>
<223> A sequence from a combinatorial phage display library.

<400> 437
caggagccga cgcggctgaa g 21

<210> 438
<211> 7
<212> PRT
<213> Artificial Sequence

<220>
<223> A sequence from a combinatorial phage display library.

<400> 438

Gln Glu Pro Thr Arg Leu Lys
1 5

<210> 439
<211> 27
<212> DNA
<213> Artificial Sequence

<220>
<223> A sequence from a combinatorial phage display library.

<400> 439
tgtcaggagc cgacgcggct gaagtgc 27

<210> 440
<211> 9
<212> PRT
<213> Artificial Sequence

<220>
<223> A sequence from a combinatorial phage display library.

<400> 440

Cys Gln Glu Pro Thr Arg Leu Lys Cys
1 5

<210> 441
<211> 21
<212> DNA .
<213> Artificial Sequence

<220>
<223> A sequence from a combinatorial phage display library.

<400> 441
aaggagccta cgaaggcgca t 21

<210> 442
<211> 7
<212> PRT
<213> Artificial Sequence

<220>
<223> A sequence from a combinatorial phage display library.

<400> 442

Lys Glu Pro Thr Lys Ala His
1 5

<210> 443
<211> 27
<212> DNA
<213> Artificial Sequence

<220>
<223> A sequence from a combinatorial phage display library.

<400> 443
tgtaaggagc ctacgaaggc gcattgc 27

<210> 444
<211> 9
<212> PRT
<213> Artificial Sequence

<220>
<223> A sequence from a combinatorial phage display library.

<400> 444

Cys Lys Glu Pro Thr Lys Ala His Cys
1 5

<210> 445
<211> 21
<212> DNA
<213> Artificial Sequence

<220>
<223> A sequence from a combinatorial phage display library.

<400> 445
aatggtaagg ctaattggaa g 21

<210> 446
<211> 7
<212> PRT

<213> Artificial Sequence

<220>
<223> A sequence from a combinatorial phage display library.

<400> 446

Asn Gly Lys Ala Asn Trp Lys
1 5

<210> 447
<211> 27
<212> DNA
<213> Artificial Sequence

<220>
<223> A sequence from a combinatorial phage display library.

<400> 447
tgtaatggta aggctaattg gaagtgc 27

<210> 448
<211> 9
<212> PRT
<213> Artificial Sequence

<220>
<223> A sequence from a combinatorial phage display library.

<400> 448

Cys Asn Gly Lys Ala Asn Trp Lys Cys
1 5

<210> 449

<400> 449
000

<210> 450
<211> 4
<212> PRT
<213> Artificial Sequence

<220>
<223> A region of similarity between SEQ ID NO:8 and SEQ ID NO:12.

<400> 450

Glu Pro Thr Arg
1

<210> 451

<400> 451
000

<210> 452
<211> 9
<212> PRT
<213> Artificial Sequence

<220>
<223> A sequence from a combinatorial phage display library.

<400>.452

Cys Ile Lys Arg Ser Asp Gly Ser Cys
1 5

<210> 453

<400> 453
000

<210> 454
<211> 8
<212> PRT
<213> Artificial Sequence

<220>
<223> A sequence from a combinatorial phage display library.

<400> 454

Asn Gln Arg Tyr Gln Met Gly Cys
1 5

<210> 455

<400> 455
000

<210> 456
<211> 8
<212> PRT
<213> Artificial Sequence

<220>
<223> A sequence from a combinatorial phage display library.

<400> 456

Asn His Arg Tyr Gln Met Gly Cys
1 5

<210> 457

<400> 457
000

<210> 458
<211> 6
<212> PRT
<213> Artificial Sequence

<220>
<223> A sequence from a combinatorial phage display library.

<400> 458

Cys Ala Pro Gly Pro Ser
1 5

<210> 459

<400> 459
000

<210> 460
<211> 6
<212> PRT
<213> Artificial Sequence

<220>
<223> A sequence from a combinatorial phage display library.

<400> 460

Gly Pro Ser Lys Ser Cys
1 5

<210> 461

<400> 461
000

<210> 462
<211> 6
<212> PRT
<213> Artificial Sequence

<220>
<223> A sequence from a combinatorial phage display library.

<400> 462

Ala Pro Gly Pro Ser Lys
1 5

<210> 463

<400> 463
000

<210> 464
<211> 9
<212> PRT
<213> Artificial Sequence

<220>
<223> A sequence from a combinatorial phage display library.

<400> 464

Cys Tyr Asn Arg Ser Asp Gly Met Cys
1 5

<210> 465

<400> 465
000

<210> 466
<211> 10
<212> PRT
<213> Artificial Sequence

<220>
<223> A control peptide.

<400> 466

Ala Cys Val Asn Arg Ser Asp Gly Met Cys
1 5 10

<210> 467

<400> 467
000

<210> 468
<211> 15
<212> PRT
<213> Artificial Sequence

<220>

<223> A sequence from a combinatorial phage display library.

<400> 468

Ala Cys Ala Pro Gly Pro Ser Lys Ser Cys Gly Gly Ser Tyr Lys
1               5                   10                  15

<210> 469

<400> 469
000

<210> 470
<211> 15
<212> PRT
<213> Artificial Sequence

<220>
<223> A sequence from a combinatorial phage display library.

<400> 470

Ala Cys Asn His Arg Tyr Met Gln Met Cys Gly Gly Ser Tyr Lys
1               5                   10                  15

<210> 471

<400> 471
000

<210> 472
<211> 15
<212> PRT
<213> Artificial Sequence

<220>
<223> A sequence from a combinatorial phage display library.

<400> 472 '

Ala Cys Asn Gln Arg His Gln Met Ser Cys Gly Gly Ser Tyr Lys
1               5                   10                  15

<210> 473

<400> 473
000

<210> 474
<211> 15
<212> PRT

<213> Artificial Sequence

<220>
<223> A sequence from a combinatorial phage display library.

<400> 474

```
Ala Cys Val Asn Arg Ser Asp Gly Met Cys Gly Gly Ser Tyr Lys
 1               5                  10                  15
```

## Description

### FIELD OF THE INVENTION

The present invention relates to detecting and treating vascular problems. In one embodiment, the invention provides peptides that selectively bind and home to atherosclerotic lesions *in vivo*. Methods to select such peptides by *in vivo* assays using phage display libraries are also provided, as well as methods to identify the target biomolecule bound by those peptides. The invention also provides methods to diagnose or treat pathologic conditions of endothelial tissues, for example, atherosclerotic lesions, by administering a peptide conjugated to a reporter molecule or to a therapeutic agent.

### BACKGROUND TO THE INVENTION

Atherosclerosis is the pathologic process that principally contributes to the pathogenesis of myocardial and cerebral infarction. A whole range of pathological changes occurs during progression of atherosclerotic disease.
While some of these changes can be described, the underlying molecular mechanisms and the exact sequence of events are not sufficiently well defined to accurately predict when a patient may suffer myocardial or cerebral infarction.
One of the characteristic early changes in the intima of a developing atherosclerotic plaque is the accumulation of lipid-laden foam cells, derived from blood-derived monocytes. An injury that causes endothelium dysfunction and localized inflammatory responses likely contributes to atherosclerotic lesion formation. The endothelial dysfunction is manifested, most notably at branch points in the arterial tree, by accumulation of lipoprotein and derived lipids in arterial wall and the appearance of specific glycoproteins (such as E-selectin) on the surface of the endothelial cells. These surface changes increase the attachment and migration of monocytes and T lymphocytes, probably through the influence of growth regulatory molecules released by the altered endothelium, the adherent leukocytes, and the underlying smooth muscle cells. As the process continues, the monocytes differentiate to become macrophages, which accumulate lipid and become foam cells. Together with the accompanying lymphocytes, they are believed to be the basis of fatty streak formation.
Further development of atherosclerotic lesions involves the proliferation of smooth muscle cells and the formation of connective tissue by smooth muscle cells. A matrix of connective tissue can form that comprises elastic fibre proteins, collagen and proteoglycans, cell death (apoptosis) and the accumulation of lipid in the surrounding matrix. The atherosclerotic process is a recurring one. Cell influx and proliferation leads to increases in size of advanced lesions. Progression of atherosclerosis is marked by the accumulation of smooth muscle cells and lipid-laden macrophages with an overlying fibrous cap, the disruptions or dissolution of which is proposed to be a frequent cause of the acute thrombosis that occurs in the vulnerable plaque and extends into the lumen of the blood vessel leading to occlusion and infarction of the local tissue (see, e.g., Libby, P. (2000). Changing concepts of atherogenesis. J. Internal Medicine 247: 349-58).
Central to the initiation and progression of atherosclerotic lesions are the associated dysfunctional changes in the endothelium. Early lesions develop at sites of morphologically intact endothelium. Several classes of adhesive cell surface glycoproteins, such as VCAM-1, are implicated in atherogenesis, but many changes in gene expression within endothelial cells during atherogenesis are not well understood.
Some of the cellular events that occur during the progression of atherosclerosis in humans are similar to those observed in hypercholesterolemia animal models. Apo E is a cholesterol-rich plasma lipoprotein that is found in humans and hypercholesterolemic mice. Apo E appears to participate in the binding ofVLDL and chylomicrons lipoproteins to hepatic LDL receptors and to the LDL R-related proteins. When mice with an inactivated Apo E gene ("knockout" mice) are fed a high fat diet, they display severe hypercholesterolemia with elevated cholesterol levels, including elevated VLDL, IDL, and to a lesser degree, LDL levels. The presence of high levels of Apo E is accompanied by low levels of HDL cholesterol. Much of the Apo E expressed in mice is truncated and lacks the LDL-R binding domain. Apo E knockout mice develop early fatty streak lesions within a few months of birth that progress into moderate atherosclerotic lesions with time.
While the general course of atherosclerosis is understood, the location and progression of potentially problematic atherosclerotic lesions are difficult to identify *in vivo.* Moreover, most therapeutic agents currently administered to a patient are not targeted to a particular site, resulting in systemic delivery of the agent to cells and tissues of the body where it is unnecessary, and often undesirable. This may result in adverse drug side effects, and often limits the dose of a drug (e.g., cytotoxic agents and other anti-cancer or anti-viral drugs) that can be administered. Although oral administration of drugs is generally recognized as a convenient and economical method of administration, oral administration can result in either (a) uptake of the drug through the epithelial barrier, resulting in undesirable systemic distribution, or (b) temporary residence of the drug within the gastrointestinal tract. Accordingly, new methods and targeting agents are needed for specifically delivering reporter molecules and therapeutic agents to cells and tissues that may benefit from detection and treatment of disease conditions or injuries. Such methods and targeting agents can avoid

the general physiological effects of inappropriate delivery of such agents to other cells and tissues.

One commonly used method for identifying new molecules involves screening collections of natural materials, such as fermentation broths of plant extracts, or libraries of synthesized molecules. Assays that range in complexity from simple binding assays to elaborate physiological tests are utilized. Such screening methods can provide leads on possible active molecules, but such molecules often require extensive testing or design modifications before a truly useful molecule is identified. Moreover, such testing and design modifications are time-consuming and costly.

Libraries of peptides or polynucleotides have been utilized to identify molecules useful for a variety of purposes. The methods were originally developed to speed up the determination of epitopes recognized by monoclonal antibodies. For example, one standard method involves in parallel synthesis of large arrays of peptides and progressive serial screening with acceptor molecules labeled with fluorescent or other reporter groups. The sequence of an effective peptide can be decoded from its address in the array. *See,* for example, Geysen et al., Proc. Natl. Acad. Sci. USA, 81: 3998-4002 (1984); Maeji et al., J. Immunol. Methods, 146: 83-90 (1992); and Fodor et al., Science, 251: 767-775 (1991).

In another approach, Lam et al., Nature, 354: 82-84 (1991) describe combinatorial libraries of peptides that are synthesized on resin beads such that each resin bead contains about 20 pmoles of the same peptide. The beads are screened with labeled acceptor molecules and those with bound acceptor are searched for by visual inspection, physically removed, and the peptide identified by direct sequence analysis. This method requires, however, sensitive methods for sequence determination.

A different approach for identification in a combinatorial peptide library is used by Houghten et al., Nature, 354: 84-86 (1991). For hexapeptides of the twenty natural amino acids, four hundred separate libraries are synthesized, each with the first two amino acids fixed and the remaining four positions occupied by all possible combinations. An assay, based on competition for binding or other activity, is then used to find the library with an active peptide. Then twenty new libraries are synthesized and assayed to determine the effective amino acid in the third position, and the process is repeated until all six positions in the peptide were identified.

More recently, Houghten (Abstract, European Peptide Society 1992 symposium, Interlaken, Switzerland) suggested a different approach. Starting with twenty amino acids, a total of 20 x 6=120 peptide mixtures are synthesized. In twenty mixtures, position 6 contains a unique amino acid, and positions 1-5 contain a mixture of all natural amino acids. In another twenty mixtures, position 5 contains a unique amino acid and all other positions contain a mixture of all twenty amino acids, etc. Once synthesized, all of the 120 peptide mixtures are tested simultaneously and the most active of each of the twenty mixtures representing each position is identified.

Another approach involves presentation of peptides on the surface of a bacteriophage. A library of peptides can be displayed, where each phage contains a DNA sequence that codes for an individual peptide. The library is made by synthesizing a large number of random oligonucleotides to generate all combinations of peptide sequences. Useful peptides can be selected by finding those that bind to the particular target. This method is known as biopanning. Phage recovered by such binding assays can be amplified and selection for binding can be repeated to eliminate phage that bind non-specifically. The sequences of peptides that bind specifically to a target are identified by DNA sequencing. See, for example, Cwirla et al., Proc. Natl. Acad. Sci. USA, 87: 6378-6382 (1990); Scott et al., Science, 249: 386-390 (1990); and Devlin et al., Science, 249: 404-406 (1990); Felici et al., Gene, 128: 21-27 (1993).

One such peptide library was made by O'Neil et al. (Proteins: Structure, Function and Genetics, 14: 509-515 (1992). These authors have constructed a random circular hexapeptide sequence inserted in the pIII phage protein. The library was used to select ligands to the receptor glycoprotein IIb/IIIa, a member of the integrin family of cell adhesion molecules that mediate platelet aggregation through the binding of fibrinogen and von Willebrand factor. However, all of this work was done *in vitro.*

If methods could be found to directly search for peptides that bind biomolecules and tissues *in vivo,* one of skill in the art could move more quickly toward identification of peptides useful for in vivo imaging and *in vivo* therapeutic purposes.

In US 5,827,516, naturally processed peptides that bind to certain human MHC II DR allotypes (HLA-DR1 to 4, and HLA DR7 and DR8) are disclosed.

Frank. R (1992) Tetrahedron, VoL. 48, No. 42, pp 9217-9232 discloses a technique for the preparation of a series of pre-defined peptides.

In WO 94/29333 an immunoreagent comprising the active site of a monoamine oxidase (MAOI), a linking group and an immunoreactive material is disclosed.

In WO 01/32853 a B cell clonal toxin is disclosed comprising an antigen which is internalised by a B cell and biologically acceptable toxin.

Favre M. et al (1999) J. Am. Chem. Soc. 121,2679-2685 analysed the structural mimicry of canonical conformations of the hypervariable loop of antibodies using various cyclic peptides.

In WO 94/13327 describes an immunoreagent comprising the active site of DHFR enzyme, a linking group and an immunoreactive material.

Bengtsson M et al (1998) Glycoconjugate Journal, 15, 223-231 describes a method of synthesing glycopeptides

by coupling 2-bromoethyl glycosides to cysteine and homocysteine.

## SUMMARY OF THE INVENTION

The invention provides isolated peptides comprising SEQ. I.D. No 4, capable of binding to an atherosclerotic lesion in a mammal The disclosure makes available isolated peptide variants that include peptides with sequences identical at four of the amino acid positions and that can bind to an atherosclerotic lesion.

In a preferred embodiment, the disclosure makes available isolated peptide variants that include peptides with sequences identical at five, more preferably six, of the amino acid positions and that can bind to an atherosclerotic lesion.

The invention is further directed to an isolated nucleic acid encoding a peptide comprising SEQ. I.D No 4.

In another embodiment, the invention discloses an isolated nucleic acid capable of hybridizing under stringent conditions to an isolated nucleic acid encoding a peptide comprising SEQ ID NO:4. wherein the stringent hybridization conditions comprise 6xSSC and at 55˚C

The disclosure still further makes available a method of identifying a peptide capable of binding to mammalian vascular tissues that includes, circulating a phage display library through the vascular tissues of a mammal; isolating a phage that selectively adheres to the vascular tissues of the mammal; and identifying a peptide displayed on the phage, wherein the peptide is capable of binding to the vascular tissues of the mammal. Such a method can also include minimally amplifying the isolated phage isolated to provide a population of phage that selectively adhere to the vascular tissues of the mammal; circulating the population of phage through the vascular tissues of a mammal; isolating a second selected phage that selectively adheres to the vascular tissues of the mammal; and identifying a peptide displayed on the second selected phage, wherein the peptide is capable of binding to the vascular tissues of the mammal.

The disclosure also makes available a method of identifying a protein bound by a peptide that is capable of binding to the vascular tissues of a mammal that includes, separating a mixture of proteins prepared from the vascular tissues of a mammal; contacting the mixture of proteins with a peptide that is capable of binding to the vascular tissues of the mammal; and identifying a protein that binds the peptide.

The disclosure further makes available a method of identifying a peptide capable of binding to an atherosclerotic lesion in a mammal that includes, circulating a phage display library through the vascular tissues of a mammal; isolating a phage that selectively adheres to an atherosclerotic lesion in the mammal; and identifying a peptide displayed on the phage, wherein the peptide is capable of binding to an atherosclerotic lesion of the mammal. The isolated phage can also be minimally amplified to provide a population of phage that selectively adhere to an atherosclerotic lesion in the mammal. That population of phage can be circulated through the vascular tissues of a mammal and a second selected phage that selectively adheres to an atherosclerotic lesion in the mammal can be isolated. A peptide displayed on the second selected phage can be isolated that is capable of binding to an atherosclerotic lesion in the mammal.

The disclosure still further provides a method of identifying a protein bound by the peptide of the invention that is capable of binding to an atherosclerotic lesion of a mammal that includes separating a mixture of proteins prepared from atherosclerotic lesions from a mammal, contacting the separated mixture of proteins with a peptide that is capable of binding to an atherosclerotic lesion of the mammal, and identifying a protein that binds the peptide.

The disclosure also provides a method of identifying the location of atherosclerotic lesions in a mammal that includes, administering a peptide of the invention conjugated to a reporter molecule to the vascular system of a mammal and observing the location of the reporter molecule. The peptide used is conjugated to the reporter molecule can bind to an atherosclerotic lesion in a mammal.

The disclosure further provides a method of identifying the severity of an atherosclerotic lesion in a mammal that includes administering a peptide of the invention conjugated to a reporter molecule to the vascular system of a mammal and observing the amount, localization, shape, density, or relative distribution of reporter molecules on an atherosclerotic lesion in the mammal. The peptide conjugated to the reporter molecule can bind to an atherosclerotic lesion in a mammal through a specific target biomolecule. Such binding permits visualization not only of the atherosclerotic lesion, but also of the amount, localization, shape, density, or relative distribution of target biomolecules on the atherosclerotic lesion.

The disclosure is also directed to methods of treating diseases such as stroke, atherosclerosis, acute coronary syndromes including unstable angina, thrombosis and myocardial infarction, plaque rupture, both primary and secondary (in-stent) restenosis in coronary or peripheral arteries, transplantation-induced sclerosis, peripheral limb disease, intermittent claudication and diabetic complications (including ischemic heart disease, peripheral artery disease, congestive heart failure, retinopathy, neuropathy and nephropathy), or thrombosis. These methods involve administering a therapeutically effective amount of a peptide of the invention conjugated to a therapeutic agent, wherein the peptide can bind to an atherosclerotic lesion in the mammal and the therapeutic agent can beneficially treat any of these diseases. For example, the therapeutic agent may be able to reduce or control the size of an atherosclerotic lesion.

In one embodiment, the disclosure also provides a method of treating atherosclerosis in a mammal that includes administering a therapeutically effective amount of a peptide of the invention conjugated to a therapeutic agent, wherein

the peptide of the invention can bind to an atherosclerotic lesion in the mammal and the therapeutic agent can beneficially treat atherosclerosis. Such therapeutic agents include, for example, thrombolytic agents such as streptokinase, tissue plasminogen activator, plasmin and urokinase, anti-thrombotic agents such as tissue factor protease inhibitors (TFPI), nematode-extraded anticoagulant proteins (NAPs) and the like, metalloproteinase inhibitors, anti-inflammatory agents or liposomes that contain thrombolytic agents such as streptokinase, tissue plasminogen activator, plasmin and urokinase, anti-thrombotic agents such as tissue factor protease inhibitors (TFPI), nematode-extracted anticoagulant proteins (NAPs) and the like, metalloproteinase inhibitors, or anti-inflammatory agents.

The disclosure further makes available a method of preventing heart attack in a mammal that includes administering a therapeutically effective amount of a peptide of the invention conjugated to a therapeutic agent, wherein the peptide can bind to an atherosclerotic lesion in the mammal and the therapeutic agent can help prevent heart attack. Such therapeutic agents include, for example, thrombolytic agents such as streptokinase, tissue plasminogen activator, plasmin and urokinase, anti-thrombotic agents such as tissue factor protease inhibitors (TFPI), nematode-extracted anticoagulant proteins (NAPs) and the like, metalloproteinase inhibitors, anti inflammatory agents or liposomes that contain thrombolytic agents as streptokinase, tissue plasminogen activator, plasmin and urokinase, anti-thrombotic agents such as tissue factor protease inhibitors (TFPI), nematode-extracted anticoagulant proteins (NAPs) and the like, metalloproteinase inhibitors, or anti inflammatory agents. The therapeutic agent can also be an enzyme capable of converting a prodrug into an active drug. Hence the peptides of the invention can deliver catalysts to atherosclerotic lesions where the catalyst can promote formation of an agent useful for treating or preventing atherosclerosis, vascular diseases and/or heart disease.

## DESCRIPTION OF THE FIGURES

Figure 1 provides images of aorta from mice injected with $10^{11}$ phage bearing CAPGPSKSC (SEQ ID NO:4) or control phage without this peptide sequence. Binding of phage to aorta was visualized with biotinylated anti-phage antibody and streptavidin-linked enzyme activation of DAB.

Figure 1A shows a young non-atherosclerotic Apo E knockout mouse injected with CAPGPSKSC (SEQ ID NO:4) phage. In the aorta of these mice, atherosclerotic lesions have not yet developed, and no phage binding to the aorta is detectable. These results suggest that phage bearing the CAPGPSKSC (SEQ ID NO:4) peptide bind target molecules that are associated with atherosclerotic lesions and whose expression is not up- regulated by the deficiency of ApoE.

Figure 1B shows a normal aorta from a Balb/C mouse. No association of phage with the aortic surface is observed suggesting the biomolecule that binds CAPGPSKSC (SEQ ID NO:4) phage is not present in detectable quantities on the surface of normal aorta endothelium.

Figure 1C shows an aorta from an Apo B knockout mouse fed a high fat diet. Atherosclerotic lesions (white) are clearly visible. This mouse was injected with $10^{11}$ control phage. No detectable association of control phage with the lesions is observed.

Figure ID shows an aorta of an atherosclerotic Apo E knockout mouse fed a high fat diet. This mouse was injected with $10^{11}$ CAPGPSKSC (SEQ ID NO:4) phage. Phage binding to the lesions is specifically visualized as red-brown staining of the white atherosclerotic lesions.

Figure 2A depicts an aorta from an atherosclerotic ApoE knockout mouse fed high fat diet and infused with biotinylated CAPGPSKSC (SEQ ID NO:4) peptide (visualized as red-brown staining). The staining pattern of the peptide is like that of phage displaying the peptide.

Figure 2B shows a histologic section of a typical atherosclerotic lesion from the aorta of an atherosclerotic ApoE knockout mouse. The binding of this peptide is most intense in the endothelium overlying the atherosclerotic lesion (black arrows), and peptide positivity is diminished towards the adjacent apparently uninvolved endothelium (grey arrows). Binding of peptide is not observed in the center of the lesion where the endothelium is not present (white arrows), consistent with endothelial cell localization of the target molecule.

Figure 3 provides two images of an atherosclerotic lesion in a human arterial specimen. Figure 3A is an image of the specimen before application of a biotinylated CAPGPSKSC (SEQ ID NO:4) peptide of the invention. Following application of the biotinylated CAPGPSKSC (SEQ ID NO:4) peptide to the luminal surface of the specimen, the specimen was washed and any peptide bound and was visualised by enzyme-linked avidin conversion of DAB substrate. Bound peptide is apparent in Figure 3B against the white tissue as darker, brownish red staining. The association of CAPGPSKSC (SEQ ID NO:4) phage with the lesion, but not with non-atherosclerotic surfaces of arteries and veins, indicated that humans express the target biomolecule of this peptide in atherosclerotic lesions, and suggests that the peptide may be used for diagnostic and therapeutic purposes in humans.

Figure 4 is a photograph of a Western blot identifying the target biomolecule of the CAPGPSKSC (SEQ ID NO:4) peptide in mouse endothelial cells (bEND.3). The total protein lysate (lane 1) and membrane preparations were

separated on a SDS polyacrylamide gel and then transferred to a nitrocellulose membrane. The membrane was probed with biotinylated peptide CAPGPSKSC (SEQ ID NO:4). Two sharp bands were observed on a Western blot of a whole cell lysate of mouse endothelial cell line (bEND.3), using the biotinylated CAPGPSKSC (SEQ ID NO:4) peptide to detect target proteins. The sizes of the proteins bound by CAPGPSKSC (SEQ ID NO:4) peptide were about 82 kilodaltons (P82) and about 120 kilodaltons (P120). The sharpness of the detected bands suggests that these target proteins are not glycoproteins. The P82 protein was also detected in membrane fractions partially purified by Triton X 114 extraction. These data suggest that the P82 protein is a membrane protein.

## DETAILED DESCRIPTION OF THE INVENTION

The invention provides peptides that bind to selected biomolecules and tissues, for example, those biomolecules and tissues within discrete regions of the mammalian vascular system. In one embodiment the peptides bind to atherosclerotic lesions. Such peptides can selectively interact *in vivo* with atherosclerotic lesions and can be used as probes to characterize the changing expression profile of surface molecules in the lesion endothelium. These peptides can also be used for non-invasive imaging of atherosclerotic lesions and for therapy to control or diminish the growth and development of the lesions.

The disclosure also makes available methods for isolating peptides that bind to any biomolecules or tissues of interest. Peptides are isolated by *in vivo* screening procedures. Naked cyclized peptides or phage display libraries can be injected or otherwise introduced into a mammal. Peptides or phage displaying peptides that bind to specific biomolecules or tissues of interest are identified and/or isolated.

Selected animal models that express biomolecules of interest or that develop conditions mimicking problematic human conditions can be used as the mammals for testing peptide and phage binding. For example, Apo E knockout mice on high fat atherogenic diets develop atherosclerotic lesions. Such mice can be used to isolate peptides that bind to atherosclerotic lesions. Animals bearing tumors can be used for identifying peptides that bind to the vascular endothelium and pseudoendothelium of those tumors, and to the vessels associated with the tumors. Through repeated exposure, collection and isolation of peptide bearing phage by the "panning" methods provided herein it is possible to obtain peptides that are capable of recognizing biochemical entities on the surface of a variety of cell and tissue types.

For example, after four rounds of *in vivo* panning, four peptide sequences were repeatedly identified as showing preferential binding to the atherosclerotic lesions in atherosclerotic Apo E knockout mice, but not in non-atherosclerotic Apo E knockout mice. These peptides were found to bind to atherosclerotic lesions in atherosclerotic Apo E knockout mice as well as to atherosclerotic lesions in human vascular tissue specimens.

### Definitions

The term "control the size of an atherosclerotic lesion" refers to the ability of a therapeutic agent to prevent further enlargement of an atherosclerotic lesion or to prevent occlusion of a blood vessel by an atherosclerotic lesion. In many instances, the therapeutic agents linked to peptides of the invention can reduce the size of an atherosclerotic lesion.

A "deletion" is defined as a change in either nucleotide or amino acid sequence in which one or more nucleotides or amino acid residues, respectively, are absent.

"Hybridization" as used herein means "any process by which a strand of nucleic acid joins with a complementary strand through base pairing" (Coombs J (1994) Dictionary of Biotechnology, Stockton Press, New York N.Y.).

An "insertion" or "addition" is that change in a nucleotide or amino acid sequence that has resulted in the addition of one or more nucleotides or amino acid residues, respectively.

A "phage-display library" is a protein expression library that expresses a collection of peptide sequences as fusion proteins joined with a phage coat protein. Thus, in the context of the invention, a combinatorial library of peptide sequences is expressed on the exterior of the phage particle. Those of skill in the art will recognize that phage clones that express peptides specific for atherosclerotic lesions can be substantially purified by serial rounds of phage binding to an atherosclerotic lesion.

"Polynucleotide", "nucleotide" and "nucleic acid", used interchangeably herein, is defined as a polymeric form of nucleotides of any length, either ribonucleotides or deoxyribonucleotides. These terms include a single-, double-or triple-stranded DNA, genomic DNA, cDNA, RNA, DNA -RNA hybrids, polymers comprising purine and pyrimidine bases, or other natural, chemically, biochemically modified, non-natural or derivatized nucleotide bases. The backbone of the polynucleotide can comprise sugars and phosphate groups (as may typically be found in RNA or DNA), or modified or substituted sugar or phosphate groups. Polynucleotides or nucleic acids of the invention may be in the form of RNA or in the form of DNA, which DNA includes cDNA, genomic DNA or synthetic DNA. As used herein, "DNA" includes not only bases A, T, C, and G, but also includes any of their analogs or modified forms of these bases, such as methylated nucleotides, internucleotide modifications such as uncharged linkages and thioates, use of sugar analogs, and modified

and/or alternative backbone structures, such as polyamides.

A "reporter molecule" is any labeling or signaling moiety known to one of skill in the art including chemicals, proteins, peptides, biotin, radionuclides, enzymes, fluorescent, chemiluminescent, contrast agents, liposomes, MRI, NMR, and ESR signaling agents, and chromogenic agents as well as substrates, cofactors, inhibitors, magnetic particles and the like. Patents teaching the use of such labels include U.S. Pat. Nos. 3,817,837; 3,850,752; 3,939,350; 3,996,345; 4,277,437; 4,275,149 and 4,366,241.

The peptides of the present invention selectively bind to target molecules *in vivo.* A peptide "selectively binds" a target molecule when it interacts with a binding domain of the target molecule with a greater affinity, or is more specific for that binding domain as compared with other binding domains of other physiological molecules. The phrase "is specific for" refers to the degree of selectivity shown by a peptide with respect to the number and types of interacting molecules with which the peptide interacts and the rates and extent of these reactions, e.g. the degree of selectivity shown by an antibody with respect to the number and types of antigens with which the antibody combines and the rates and the extent of these reactions. The phrase "selectively binds" in the present context also means binding sufficient to be useful in the method of the invention. As is known in the art, useful selective binding, for instance, to a receptor, depends on both the binding affinity and the concentration of ligand achievable in the vicinity of the receptor. Thus, binding affinities lower than that found for any naturally occurring competing ligands may be useful, as long as the cell or tissue to be treated can tolerate concentrations of added ligand sufficient to compete, for example, for binding to a target biomolecule.

"Stringency" typically occurs in a range from about Tm -5 ˚C (5 ˚C below the Tm of the probe) to about 20˚C to 25˚C below Tm. As will be understood by those of skill in the art, a stringent hybridization can be used to identify or detect identical polynucleotide sequences or to identify or detect similar or related polynucleotide sequences.

A "substitution" results from the replacement of one or more nucleotides or amino acids by different nucleotides or amino acids, respectively.

As used herein, a "target" is a biomolecule or tissue to which a peptide identified according to the invention can bind.

A "therapeutic agent" is any drug, enzyme, protein, viral particle, toxin or other agent that one of skill in the art can use to beneficially treat a mammal having a target bound by a peptide of the invention. Such therapeutic agents include, for example, thrombolytic agents such as streptokinase, tissue plasminogen activator, plasmin and urokinase, anti-thrombotic agents such as tissue factor protease inhibitors (TFPI), nematode-extracted anticoagulant proteins (NAPs) and the like, metalloproteinase inhibitors, anti-inflammatory agents or liposomes that contain thrombolytic agents such as streptokinase, tissue plasminogen activator, plasmin and urokinase, anti-thrombotic agents such as tissue factor protease inhibitors (TFPI), nematode-extracted anticoagulant proteins (NAPs) and the like, metalloproteinase inhibitors, or anti-inflammatory agents. In another embodiment, the therapeutic agent is a nucleic acid useful for gene therapy. Such a nucleic acid can be directly attached to a peptide of the invention, or it can be present in a phage particle, liposome or other vector available to one of skill in the art.

A "variant" peptide is defined as a peptide with an amino acid sequence that differs by one or more amino acids from a reference peptide or amino acid sequence. Variant peptides will have substantially the same physical, chemical, and/or functional properties as the reference peptide. In general, a variant may have "conservative" changes, wherein a substituted amino acid has similar structural or chemical properties, for example, replacement of leucine with isoleucine. Similar minor variations may also include amino acid deletions or insertions, or both. In contrast to a variant peptide, a derivative peptide may have somewhat different physical, chemical and/or functional properties compared to the reference peptide. For example, a derivative peptide can have enhanced binding properties relative to the reference peptide. A derivative may therefore have "nonconservative" changes, e.g., replacement of a glycine with a tryptophan. Guidance in determining which and how many amino acid residues may be substituted, inserted or deleted to retain or enhance the physical, chemical and/or functional properties (e.g. binding properties) of a peptide is provided herein and is available in the art, for example, in certain computer programs such as DNAStar.

**Peptides**

Peptides isolated by the *in vivo* methods made available by the invention bind to biomolecules and tissues of interest. In many instances, such peptides selectively bind to such biomolecules and tissues. In some embodiments, a peptide that selectively binds to a biomolecule or tissue of interest, binds with sufficient selectivity to permit the peptide to become localized *in vivo* at the site of the biomolecule or tissue. Peptides that selectively bind to biomolecules and tissues can therefore be detected at the site of such biomolecules and tissues. Desirable peptides that selectively bind to biomolecules and tissues permit reliable detection of those biomolecules and tissues *in vivo.* Desirable peptides that selectively bind to biomolecules and tissues may also permit reliable delivery of a therapeutic agent to the site of the biomolecule or tissue. However, because various types of reporter molecules and therapeutic agents may alter the physical and chemical properties of the peptide or sterically hinder binding, a peptide conjugated to such a reporter molecule or therapeutic agent may still "selectively bind" even though some modification of the peptide conjugated,

reporter molecule or therapeutic agent is needed to optimize binding.

Additional amino acids can be added or inserted into these peptides, for example, to enhance binding activity, to permit cyclization or to link a reporter molecule or a therapeutic agent to the peptide.

**Peptide Variants and Derivatives**

The disclosure makes available variants and derivatives of the isolated peptides that can bind to the biomolecule or tissue to which the isolated peptide bound. Such variants and derivatives have identify with at least about four of the amino acid positions and are capable of binding to an atherosclerotic lesion. In a preferred embodiment, the variants and derivatives have identify with at least about five of the amino acid positions and can bind to an atherosclerotic lesion. More preferably, peptide variants and derivatives have identity with at least about six of the amino acid positions and can bind to an atherosclerotic lesion.

Amino acid residues of the isolated peptides and peptide variants can be genetically encoded L-amino acids, naturally occurring non-genetically encoded L-amino acids, synthetic L-amino acids or D-enantiomers of any of the above. The amino acid notations used herein for the twenty genetically encoded L-amino acids and common non-encoded amino acids are conventional and are as shown in Table 2.

**Table 2**

| Amino Acid | One-letter Symbol | Abbreviation |
|---|---|---|
| Alanine | A | Ala |
| Arginine | R | Arg |
| Asparagine | N | Asn |
| Aspartic Acid | D | Asp |
| Cysteine | C | Cys |
| Glutamine | Q | Gln |
| Glutamaic Acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |
| β-Alanine | | bAla |
| 2,3-Diaminopropionic acid | | Dpr |
| α-Aminoisobutyric acid | | Aib |
| N-Methylglycine (sarcosine) | | MeGly |
| Ornithine | | Orn |
| Citrulline | | Cit |

(continued)

| Amino Acid | One-letter Symbol | Abbreviation |
|---|---|---|
| t-Butylalanine | | t-BuA |
| t-Butylglycine | | t-BuG |
| N-methylisoleucine | | MeIle |
| Phenylglycine | | Phg |
| Cyclohexylalanine | | Cha |
| Norleucine | | Nle |
| Naphthylalanine | | Nal |
| Pyridylalanine | | |
| 3-Benzothienyl alanine | | |
| 4-Chlorophenylalanine | | Phe(4-Cl) |
| 2-Fluorophenylalanine | | Phe(2-F) |
| 3-Fluorophenylalanine | | Phe(3-F) |
| 4-Fluorophenylalanine | | Phe(4-F) |
| Penicillamine | | Pen |
| 1,2,3,4-Tetrahydro-isoquinoline-3-carboxylic acid | | Tic |
| β-2-thienylalanine | | Thi |
| Methionine sulfoxide | | MSO |
| Homoarginine | | hArg |
| N-acetyl lysine | | AcLys |
| 2,4-Diamino butyric acid | | Dbu |
| P-Aminophenylalanine | | Phe($pNH_2$) |
| N-methylvaline | | MeVal |
| Homocysteine | | hCys |
| Homoserine | | hSer |
| E-Amino hexanoic acid | | Aha |
| δ-Amino valeric acid | | Ava |
| 2,3-Diaminobutyric acid | | Dab |

Peptides that are encompassed within the scope of the invention can have one or more amino acids substituted with an amino acid of similar or different chemical and/or physical properties, so long as these variant and derivative peptides retain the ability to bind to the biomolecule or tissue.

When generating a variant or derivative peptide, amino acids that reside within similar classes or subclasses can be substituted for amino acids in a reference peptide or amino acid sequence. As known to one of skill in the art, amino acids can be placed into three main classes: hydrophilic amino acids, hydrophobic amino acids and cysteine-like amino acids, depending primarily on the characteristics of the amino acid side chain. These main classes may be further divided into subclasses.

Hydrophilic amino acids include amino acids having acidic, basic or polar side chains and hydrophobic amino acids include amino acids having aromatic or apolar side chains. Apolar amino acids may be further subdivided to include, among others, aliphatic amino acids. The definitions of the classes of amino acids as used herein are as follows:

"Hydrophobic Amino Acid" refers to an amino acid having a side chain that is uncharged at physiological pH and that is repelled by aqueous solution. Examples of genetically encoded hydrophobic amino acids include Ile, Leu and Val. Examples of non-genetically encoded hydrophobic amino acids include t-BuA.

"Aromatic Amino Acid" refers to a hydrophobic amino acid having a side chain containing at least one ring

having a conjugated π-electron system (aromatic group). The aromatic group may be further substituted with substituent groups such as alkyl, alkenyl, alkynyl, hydroxyl, sulfonyl, nitro and amino groups, as well as others. Examples of genetically encoded aromatic amino acids include phenylalanine, tyrosine and tryptophan. Commonly encountered non-genetically encoded aromatic amino acids include phenylglycine, 2-naphthylalanine, β-2-thienylalanine, 1,2,3,4-tetrahydroisoquinoline-3-carboxylic acid, 4-chlorophenylalanine, 2-fluorophenylalanine, 3-fluorophenylalanine and 4-fluorophenylalanine.

"Apolar Amino Acid" refers to a hydrophobic amino acid having a side chain that is generally uncharged at physiological pH and that is not polar. Examples of genetically encoded apolar amino acids include glycine, proline and methionine. Examples of non-encoded apolar amino acids include Cha.

"Aliphatic Amino Acid" refers to an apolar amino acid having a saturated or unsaturated straight chain, branched or cyclic hydrocarbon side chain. Examples of genetically encoded aliphatic amino acids include Ala, Leu, Val and Ile. Examples of non-encoded aliphatic amino acids include Nle.

"Hydrophilic Amino Acid" refers to an amino acid having a side chain that is attracted by aqueous solution. Examples of genetically encoded hydrophilic amino acids include Ser and Lys. Examples of non-encoded hydrophilic amino acids include Cit and hCys.

"Acidic Amino Acid" refers to a hydrophilic amino acid having a side chain pK value of less than 7. Acidic amino acids typically have negatively charged side chains at physiological pH due to loss of a hydrogen ion. Examples of genetically encoded acidic amino acids include aspartic acid (aspartate) and glutamic acid (glutamate).

"Basic Amino Acid" refers to a hydrophilic amino acid having a side chain pK value of greater than 7. Basic amino acids typically have positively charged side chains at physiological pH due to association with hydronium ion. Examples of genetically encoded basic amino acids include arginine, lysine and histidine. Examples of non-genetically encoded basic amino acids include the non-cyclic amino acids ornithine, 2,3-diaminopropionic acid, 2,4-diaminobutyric acid and homoarginine.

"Polar Amino Acid" refers to a hydrophilic amino acid having a side chain that is uncharged at physiological pH, but where a bond in the side chain has a pair of electrons that are held more closely by one of the atoms involved in the bond. Examples of genetically encoded polar amino acids include asparagine and glutamine. Examples of non-genetically encoded polar amino acids include citrulline, N-acetyl lysine and methionine sulfoxide.

"Cysteine-Like Amino Acid" refers to an amino acid having a side chain capable of forming a covalent linkage with a side chain of another amino acid residue, such as a disulfide linkage. Typically, cysteine-like amino acids generally have a side chain containing at least one thiol (SH) group. An example of a genetically encoded cysteine-like amino acid is cysteine. Examples of non-genetically encoded cysteine-like amino acids include homocysteine and penicillamine.

As will be appreciated by those having skill in the art, the above classifications are not absolute. Several amino acids exhibit more than one characteristic property, and can therefore be included in more than one category. For example, tyrosine has both an aromatic ring and a polar hydroxyl group. Thus, tyrosine has dual properties and can be included in both the aromatic and polar categories. Similarly, in addition to being able to form disulfide linkages, cysteine also has an apolar character. Thus, while not strictly classified as a hydrophobic or an apolar amino acid, in many instances cysteine can be used to confer hydrophobicity to a peptide.

Certain commonly encountered amino acids that are not genetically encoded and that can be present, or substituted for an amino acid, in the peptides, peptide variants and peptide derivatives of the invention include, but are not limited to, β-alanine (b-Ala) and other omega-amino acids such as 3-aminopropionic acid (Dap), 2,3-diaminopropionic acid (Dpr), 4-aminobutyric acid and so forth; α-aminoisobutyric acid (Aib); ε-aminohexanoic acid (Aha); δ-aminovaleric acid (Ava); N-methylglycine (MeGly); ornithine (Orn); citrulline (Cit); t-butylalanine (t-BuA); t-butylglycine (t-BuG); N-methylisoleucine (MeIle); phenylglycine (Phg); cyclohexylalanine (Cha); norleucine (Nle); 2-naphthylalanine (2-Nal); 4-chlorophenylalanine (Phe(4-Cl)); 2-fluorophenylalanine (Phe(2-F)); 3-fluorophenylalanine (Phe(3-F)); 4-fluorophenylalanine (Phe(4-F)); penicillamine (Pen); 1,2,3,4-tetrahydroisoquinoline-3-carboxylic acid (Tic); β-2-thienylalanine (Thi); methionine sulfoxide (MSO); homoarginine (hArg); N-acetyl lysine (AcLys); 2,3-diaminobutyric acid (Dab); 2,3-diaminobutyric acid (Dbu); p-aminophenylalanine (Phe($pNH_2$)); N-methyl valine (MeVal); homocysteine (hCys) and homoserine (hSer). These amino acids also fall into the categories defined above.

The classifications of the above-described genetically encoded and non-encoded amino acids are summarized in Table 3, below. It is to be understood that Table 3 is for illustrative purposes only and does not purport to be an exhaustive list of amino acid residues that may comprise the peptides, variants and derivatives described herein. Other amino acid residues that are useful for making the peptides, peptide variants and peptide derivatives described herein can be found, e.g., in Fasman, 1989, CRC Practical Handbook of Biochemistry and Molecular Biology, CRC Press, Inc. , and the references cited therein. Amino acids not specifically mentioned herein can be conveniently classified into the above-described categories on the basis of known behaviour and/or their characteristic chemical and/or physical properties as compared with amino acids specifically identified.

**TABLE 3**

| Classification | Genetically Encoded | Genetically Non-Encoded |
|---|---|---|
| Hydrophobic | | |
| Aromatic | F, Y, W | Phg, Nal, Thi, Tic, Phe(4-Cl), Phe(2-F), Phe(3-F), Phe(4-F), Pyridyl Ala, Benzothienyl Ala |
| Apolar | M, G, P | Cha |
| Aliphatic | A, V, L, I | t-BuA, t-BuG, MeIle, Nle, MeVal, Cha, bAla, MeGly, Aib |
| Hydrophilic | | |
| Acidic | D, E, | |
| Basic | H, K, R | Dpr, Orn, hArg, Phe(p-$NH^2$), DBU, $A^2$ BU |
| Polar | Q, N, S, T, Y | Cit, AcLys, MSO, hSer |
| Cysteine-Like | C | Pen, hCys, β-methyl-Cys |

Peptides of the invention can have any amino acid substituted by any similarly classified amino acid to create a variant or derivative peptide, so long as the peptide variant or derivative retains an ability to bind to the biomolecule or tissue to which the unaltered or reference peptide bound.

**Peptides Conjugated to Reporter Molecules**

According to the invention, peptides identified as described herein can be attached or conjugated to any known reporter molecule or other label or signaling agent. While the peptides of the invention have utility for identifying the location of, and for imaging, atherosclerotic lesions, the invention is not limited to imaging just atherosclerotic lesions. The peptides of the invention can be used to detect, identify, locate and/or image any target molecule to which a peptide of the invention can bind, either *in vitro* and *in vivo.*

In one embodiment, the peptides and methods provided herein can be used to diagnose the location, extent, and pathologic composition of atherosclerotic lesions anywhere within the body of a mammal. For example, detection of a peptide-conjugate capable of binding to atherosclerotic lesion can provide information regarding the location, shape, extent and pattern of expression of a target biomolecule in relation to the lesion. Peptides isolated as being able to bind to atherosclerotic lesions of different stages can be used to diagnose the staging or severity of the lesions and potential risk of thrombosis. Any reporter molecule, label or signaling agent known to one of skill in the art can be attached to the peptides of the invention as well as any and all agents used as diagnostic tools or to enhance diagnostic tools. Such peptide-conjugates can then be used *in vivo* or *in vitro* to image, locate or otherwise detect the biomolecule or tissue to which the peptide binds.

The peptide-conjugates of the invention can serve as a signal enhancing agent for medical diagnostic imaging, for example, for MRI, ultrasound, infrared and other imaging procedures. Peptide conjugates used for MRI, and radiodiagnostic imaging can, for example, have one or more amino acid side chains or linkers that are attached to chelating moieties, contrast agents or liposomes, such as unilamellar gadolinium-liposomes, manganese-liposomes, and iron-DTPA-stearate-liposomes.

One of skill in the art can conjugate such reporter molecules, labels and signaling agents to the present peptides using known techniques. For example, the followings references provide guidance on conjugation and use of such reporter molecules, labels and signaling agents in various diagnostic imaging procedures.

Bacic, G., M. R. Niesman, et al. (1990). "NMR and ESR study of liposome delivery of Mn2+ to murine liver." Magn Reson Med 13(1): 44-61.

Bartolozzi, C., F. Donati, et al. (2000). "MnDPDP-enhanced MRI vs dual-phase spiral CT in the detection of hepatocellular carcinoma in cirrhosis." Eur Radiol 10(11): 1697-702.

Bockhorst, K., M. Hoehn-Berlage, et al. (1993). "NMR-contrast enhancement of experimental brain tumors with MnTPPS: qualitative evaluation by in vivo relaxometry." Magn Reson Imaging 11(5): 655-63.

Colet, J. M., L. Vander Elst, et al. (1998). "Dynamic evaluation of the hepatic uptake and clearance of manganese-based MRI contrast agents: a 31P NMR study on the isolated and perfused rat liver." J Magn Reson Imaging 8 (3): 663-9.

Diehl, S. J., K. J. Lehmann, et al. (1999). "MR imaging of pancreatic lesions. Comparison of manganese-DPDP and gadolinium chelate." Invest Radiol 34(9): 589-95.

Fiel, R., E. Mark, et al. (1993). "Tumor-selective contrast enhancing agent, Mn(III)meso- [tri(4-sulfonatophenyl)phenyl]porphine (MnTPPS3)." Magn Reson Imaging 11(7): 1079-81.

Kim, S. W. and T. Kozuka (1990). "[Mn-TPPS4; a potential MRI contrast agent for localizing the normal aortic wall in rabbits]." Nippon Igaku Hoshasen Gakkai Zasshi 50(2): 192-94.

Laniado, M. and A. F. Kopp (1997). "[Current status of the clinical development of MR contrast media]." Rofo Fortschr Geb Rontgenstr Neuen Bildgeb Verfahr 167(6): 541-50.

Marchal, G., X. Zhang, et al. (1993). "Comparison between Gd-DTPA, Gd-EOB-DTPA, and Mn-DPDP in induced HCC in rats: a correlation study of MR imaging, microangiography, and histology." Magn Reson Imagine 11(5): 665-74.

Maurer, J., A. Strauss, et al. (2000). "Contrast-enhanced high resolution magnetic resonance imaging of pigmented malignant melanoma using Mn-TPPS4 and Gd-DTPA: experimental results." Melanoma Res 10(1): 40-6.

Maurer, J., A. Strauss, et al. (1999). "[Mn-TPPS4 in the diagnosis of malignant skin tumors. In vivo studies with high resolution magnetic resonance tomography in melanotic melanoma]." Radiologe 39(5): 422-7.

Navon, G., R. Panigel, et al. (1986). "Liposomes containing paramagnetic macromolecules as MRI contrast agents." Magn Reson Med 3(6): 876-80.

Ni, Y., G. Marchal, et al. (1994). "Prolonged positive contrast enhancement with Gd-EOB-DTPA in experimental liver tumors: potential value in tissue characterization." J Magn Reson Imaging 4(3): 355-63.

Plowchalk, D. R., J. P. Jordan, et al. (1987). "Effects of manganese (Mn++) and iron (Fe+++) on magnetic resonance imaging (MRI) characteristics of human placenta and amniotic fluid." Physiol Chem Phys Med NMR 19(1): 35-41.

Rofsky, N. M. and J. C. Weinreb (1992). "Manganese (II) N,N'-dipyridoxylethylene-diamine-N,N'-diacetate 5,5'-bis(phosphate): clinical experience with a new contrast agent." Magn Reson Q 8(3): 156-68.

Runge, V. M. (2000). "Safety of approved MR contrast media for intravenous injection." J Magn Reson Imaging 12(2): 205-13.

Saeed, M., S. Wagner, et al. (1989). "Occlusive and reperfused myocardial infarcts: differentiation with Mn-DPDP--enhanced MR imaging." Radiology 172(1): 59-64.

Schmiedl, U. P., J. A. Nelson, et al. (1992). "Hepatic contrast-enhancing properties of manganese-mesoporphyrin and manganese-TPPS4. A comparative magnetic resonance imaging study in rats." Invest Radiol 27(7): 536-42.

Schwendener, R. A., R. Wuthrich, et al. (1990). "A pharmacokinetic and MRI study of unilamellar gadolinium-, manganese-, and iron-DTPA-stearate liposomes as organ-specific contrast agents." Invest Radiol 25(8): 922-32.

Wang, C. (1998). "Mangafodipir trisodium (MnDPDP)-enhanced magnetic resonance imaging of the liver and pancreas." Acta Radiol Suppl 415: 1-31.

Wilmes, L. J., M. Hoehn-Berlage, et al. (1993). "In vivo relaxometry of three brain tumors in the rat: effect of Mn-TPPS, a tumor-selective contrast agent." J Magn Reson Imaging 3(1): 5-12.

Wolf, G. L., K. R. Burnett, et al. (1985). "Contrast agents for magnetic resonance imaging." Magn Reson Annu: 231-66.

Wyttenbach, R., M. Saeed, et al. (1999). "Detection of acute myocardial ischemia using first-pass dynamics of MnDPDP on inversion recovery echoplanar imaging." J Magn Reson Imaging 9(2): 209-14.

Yamamoto, T., A. Matsumura, et al. (1998). "Manganese-metalloporphyrin (ATN-10) as a tumor-localizing agent: magnetic resonance imaging and inductively coupled plasma atomic emission spectroscopy study with experimental brain tumors." Neurosurgery 42(6): 1332-7; discussion 1337-8.

The present peptide-conjugates can also be used for ultrasound imaging. For example, the peptide can be grafted to the surface of a liposome that contains gas through conjugation of the peptide to a PEGylated lipid. The microbubbles so formed serve as signal enhancing agents for the ultrasound detection and imaging procedure. Such liposomes are described in U.S. Patent 6,139,819 to Unger et al.

Useful chelating moieties tightly bind metal ions such as technetium-99m and indium-111. One of skill in the art can employ known procedures to make such technetium-99m and indium-111 labeled peptides for diagnostic imaging.

See, for example, U.S. Patent 6,107,459 to Dean. Peptide conjugates with radionuclides are also useful for therapy, including radiotherapy.

The peptides of the invention can also be conjugated with any available dye or fluorescent moiety or intermediate such as biotin. Such peptide-dye conjugates can, for example, be used with infrared spectroscopy to detect and locate the biomolecules or tissues to which the peptide can bind.

In one embodiment, the peptide-conjugate is targeted to the luminal surface of atherosclerotic lesions. When coupled with a reporter molecule, the peptide-conjugate renders the surface of the atherosclerotic lesions visible using different appropriate detection methods. Lipid laden plaques that are soft and not fibrous tend to have subtle changes in the lesion surface contour under flow conditions, which are indicative of the characteristics of the plaque. These type of plaques are prone to rupture and initiation of a series of events leading the thrombotic occlusion of the affected artery. Such plaques are associated with unstable angina and sudden death; they are likely to rupture and to produce thrombosis and/or unstable angina and thereby myocardial infarction or subinfarctive myocardial injury. Currently, no markers are known that can uniquely identify these types of susceptible plaques. Zena and Michael A. Wiener, Clinical imaging of

the high-risk or vulnerable atherosclerotic plaque. (Aug. 17, 2001) CIRCULATION RESEARCH 89(4):305-16. However, these medical conditions can be diagnosed and treated by visualizing the surface of the atherosclerotic lesion using the present peptide-conjugates and then by targeting those lesions with a therapeutic agent conjugated to a peptide of the invention that uniquely associates or binds to those susceptible lesions. Aleternatively, conventional treatments maybe employed (e.g. stent insertion, angioplasty, etc.) after the susceptible plaques are identified.

**Therapeutic Agents**

The invention also contemplates conjugating peptides to any therapeutic agent available to one of skill in the art. In the present context "a therapeutic agent" is also intended to comprise active metabolites and prodrugs thereof. An active "metabolite" is an active derivative of a therapeutic agent produced when the therapeutic agent is metabolised. A "prodrug" is a compound that is either metabolised to a therapeutic agent or is metabolised to an active metabolite(s) of a therapeutic agent. This invention can be used to administer therapeutic agents such as small molecular weight compounds, radionuclides, drugs, enzymes, peptides and/or proteins with biological activity, nucleic acids or genes that encode therapeutic polypeptides, expression vectors or other nucleic acid constructs, for example, naked plasmid DNAs, any vector carrying one or more genes, any sense or antisense RNA, any ribozyme, or any antibody.

For example, the peptides of the invention can be used to deliver fusion proteins or fibrinolytic agents. Such therapeutic agents include, for example, thrombolytic agents such as streptokinase, tissue plasminogen activator, plasmin and urokinase, anti-thrombotic agents such as tissue factor protease inhibitors (TFPI), anti-inflammatory agents, metalloproteinase inhibitors, nematode-extracted anticoagulant proteins (NAPs) and the like. Liposomes can be used to facilitate delivery of such agents, for example, thrombolytic agents such as streptokinase, tissue plasminogen activator, plasmin and urokinase, anti-thrombotic agents such as tissue factor protease inhibitors (TFPI), anti-inflammatory agents, metalloproteinase inhibitors, nematode-extracted anticoagulant proteins (NAPs) and the like.

The peptides of the invention can be linked to such proteins or polypeptides. Upon administration, these therapeutic agents will become localized at the site of atherosclerotic lesions and will help control, diminish or otherwise facilitate improved arterial blood flow in the region of the atherosclerotic lesion. The peptides of the invention can also be used to deliver nanoparticles, such as vectors for gene therapies (in a manner similar to the phage particles used for isolation of the peptides), as well as liposomes containing therapeutic agents like those listed herein.

Examples of therapeutic agents that can be linked to the peptides of the invention include the following:

1) Agents that and modulate lipid levels (for example, HMG-CoA reductase inhibitors, thyromimetics, fibrates, agonists of peroxisome proliferator-activated receptors (PPAR) (including PPAR-alpha, PPAR-gamma and/or PPAR-delta).
2) Agents that control and modulate oxidative processes such as modifiers of reactive oxygen species or treatments that modify the production and/or activity of modified lipoproteins;
3) Agents that control and modulate insulin resistance and/or activity or glucose metabolism or activity including, but not limited to, agonists of PPAR-alpha, PPAR- gamma and/or PPAR-delta, modifiers of DPP-IV, and modifiers of glucocorticoid receptors;
4) Agents that control and modulate expression of receptors or adhesion molecules or integrins on endothelial cells or smooth muscle cells in any vascular location
5) Agents that control and modulate the activity of endothelial cells or smooth muscle cells in any vascular location;
6) Agents that control and modulate inflammation associated receptors including, but not limited to chemokine receptors, RAGE, toll-like receptors, angiotensin receptors, TGF receptors, interleukin receptors, TNF receptors, C-reactive protein receptors, and other receptors involved in inflammatory signaling pathways including the activation of NF-kb;.
7) Agents that control and modulate proliferation, apoptosis or necrosis of endothelial cells , vascular smooth muscle or lymphocytes, monocytes, and neutrophils adhering to or within the vessel;
8) Agents that control and modulate production, degradation, or cross-linking of any extracellular matrix proteins including, but not limited to, collagen, elastin, and proteoglycans;
9) Agents that control and modulate activation, secretion or lipid loading of any cell type within mammalian vessels;
10) Agents that control and modulate the activation, proliferation or any other modification of dendritic cells within mammalian vessels; and
11) Agents that control and modulate the activation, adhesion, or other processes that modify platelet events at the level of the vessel wall.

Examples of these types of agents and procedures for using these physiological agents are described in more detail below.

In one embodiment, the therapeutic agent is a pre-selected nucleic acid that encodes a protein whose activity

can benefit a mammal suffering from any atherosclerotic lesion. The gene therapy agent can, for example, reduce the size of a lesion, prevent platelet interaction with the lesion, reduce or prevent the growth of smooth muscle cells or otherwise stabilize or beneficially interact with the atherosclerotic lesion. In another embodiment, the therapeutic agent is a pre-selected nucleic acid that can generate an antisense RNA useful for reducing the expression of a deleterious protein at the site of the atherosclerotic lesion.

Such a therapeutic nucleic acids can be directly attached to a peptide of the invention, or it can be present in a phage particle, liposome or other transformation vector available to one of skill in the art.

For example, in one embodiment a peptide of the invention (e.g. CAPGPSKSC, SEQ ID NO:4) is attached to a pre-selected therapeutic nucleic acid, phage, liposome or other molecule to form a peptide-therapeutic agent. In one embodiment, a nucleic acid encoding a polypeptide therapeutic agent is directly administered *in vivo,* where it is targeted to the site of atherosclerotic lesions via linkage to a peptide of the invention (e.g. CAPGPSKSC, SEQ ID NO:4). A cell specific peptide of the invention can also be used to deliver "naked" DNA, for example, by use of controlled pressure-mediated delivery of the naked DNA using methods available in the art. See, e.g., von der Leyen, Braun-Dullaeus, et al, A pressure-mediated nonviral method for efficient arterial gene and oligonucleotide transfer, Hum. Gene Ther. 1999. 10:2355-64. Such methods provide safe and efficient arterial transfer to cells at the site of atherosclerotic lesions for nucleic acids, genes and oligonucleotides.

In another embodiment, a nucleic acid encoding a peptide of the invention is joined with another nucleic acid that encodes a biologically active therapeutic agent to form a hybrid or recombinant nucleic acid. The hybrid or recombinant nucleic acid is placed within an appropriate vector to generate a gene therapy construct that may be expressed in a cell type of interest. The peptide-therapeutic agent and/or the gene therapy construct can be delivered to the tissue of interest by attachment to a peptide of the invention followed by *in vivo* administration to a selected mammal. The peptide of the invention guides the agent or construct to the intended biomolecule or tissue. For example, if the CAPGPSKSC (SEQ ID NO:4) peptide is attached to such agents and therapeutic constructs, the CAPGPSKSC (SEQ ID NO: 4) peptide will home to and bind an 82 kilodalton and/or a 120 kilodalton target protein that is present in atherosclerotic lesions of a mammal. The 82 kilodalton target protein may be a membrane protein. Hence, the CAPGPSKSC (SEQ ID NO:4) peptide can deliver the therapeutic nucleic acid, phage, liposome or other vector or therapeutic agent to membranes of cells within the atherosclerotic lesion.

Nucleic acids that may be used with the peptides of the invention include the following.

1. Genes or nucleic acids that encode proteins or antisense RNAs that inhibit inflammatory events at the sites of atherosclerosis lesion progression or at sites of vulnerable atherosclerotric lesions. Such genes or nucleic acids include the dominant-negative form or mutants or decoys of various chemokine genes (e.g. any of the CCR, CXCR, or CX3CR chemokines including but not limited to RANTES, CCR1,2,3,4,5,6,7,8,9,10; CXCR2, CXCR5, CX3CR1) or soluble forms of the receptors for such chemokines. Likewise, dominant-negative forms or mutant genes or decoys for soluble forms of encoding toll-like receptors (e.g. TLR-1, TLR-2, TLR-3, TLR-4 or TLR-5), angiotensin I or II receptors, interleukin receptors, integrins such a I-CAM, V-CAM, E-selectin, P-selectin, LFA1, alpha(v)beta(3) or any other receptor or molecule that stimulates a signalling cascade involved in activation of NFkappaB, inflammation or proliferation, such as, but not limited to CD40, CD40L, GRO-alpha, Rho-kinase, MCP-1, 11-6, 11-8, leukptriene B4, or leukotactin-1.
2. Genes or nucleic acids that encode proteins or antisense RNAs that inhibit foam cell formation and thus retard progression and/or stimulate regression of atherosclerotic lesions. Such genes or nucleic acids can, for example, encode secreted "decoys" or mutants of macrophage scavenger receptors MSR (sMSR) (in particular, one containing an extracellular portion of the human MSR type AI), apoE, or mutants or decoys that would block activation of receptors including but not limited to platelet factor 4, Lox-1, Lox-2, Lox-3, leukotriene B4, lipoxygenases such as, but not limited to LO-12 or LO-15. In addition, other antisense nucleic acids that could be targeted to the site of atherosclerotic lesions via incorporation of a peptide of the invention include E2F decoy, NF-kappa B decoy, and other decoys.
3. Genes or nucleic acids that encode proteins or antisense RNAs that enhance vasodilation and/or stablize arterial vessel and/or prevent vascular spasm to prevent myocardial infartcion or stroke. Such genes or nucleic acids include: heme oxygenase-1 (HO-1), ecNOS, iNOS, superoxide dismutases, estrogen receptors or soluble or mutated forms of estrogen receptors.
4. Genes or nucleic acids that encode proteins or antisense RNAs that inhibit local thrombosis, for example, a tissue factor pathway inhibitor or tPA.
5. Genes or nucleic acids that encode proteins or antisense RNAs that stablize a plaque by promoting fibrous cap thickening and/or by healing the endothelial lining on top of such lesions. Examples include FGF, PDGF, TGFbeta, EGF, or HGF. Some genes or nucleic acids that would inhibit apoptosis in the fibrous cap include bcl2, crmA.
6. Genes or nucleic acids that encode proteins or antisense RNAs that inhibit inflammation such as NFkB decoys, dominant-negative Rho-kinase, p57Kip2, IL-18 binding protein, Il-10, mutants of MCP-1

7. Genes or nucleic acids that encode proteins or antisense RNAs that inhibit cell proliferation such as p57Kip2, cyclin dependant kinase inhibitors, kallikrein, p53 and the like.

8. Genes or nucleic acids that encode proteins or antisense RNAs that improve vascular dilation such as ecNOS, iNOS, superoxide dismutases, estrogen receptors and the like.

9. Genes or nucleic acids that encode proteins or antisense RNAs that decrease matrix degradation such as tissue inhibitors of matrix metalloproteinases (TIMPs) incuding but not limited to TIMP1,2,3,4,5,6,7

10. Genes or nucleic acids that encode proteins or antisense RNAs that have anti-angiogenesis and/or growth factor/ cytokine inhibitory activities. Examples include VEGF/VEGFR antagonists (sFlt-1, sFlk, sNRP1), Angiopoietin/Tie antagonists (sTie-2), anti-chemokines (IP-10,PF-4, Gro-beta, IFN-gamma (Mig)), FGF/FGFR antagonists (sFGFR), inhibitors of PDGF, TGFbeta, IGF-1,various fragments of extracellular matrix proteins (such as Angiostatin, Endostatin, Kininostatin, Fibrinogen-E fragment, Thrombospondin, Tumstatin, Canstatin, Restin) Ephrin/Eph antagonists (sEphB4, sephrinB2), vasostatin, PEDF, Prolactin fragment, proliferin-related protein, TrpRS fragments, METH-1 and METH-2.

The peptides of the invention can also be linked or incorporated into antisense nucleic acids or oligonucletodes that can inhibit or stimulate synthesis of target genes and nucleic acids, for example, those listed herein.

A preselected nucleic acid can encode an antisense RNA as the therapeutic agent. Such an antisense RNA is typically a "sense" DNA sequence cloned into an expression cassette in the opposite orientation relative to its normal orientation (i.e., 3' to 5' rather than 5' to 3'). When operably linked to a promoter in the expression cassette in such an opposite orientation, an RNA that is complementary to the natural mRNA encoded by the nucleic acid is synthesized.

Double-stranded RNA (dsRNA) can trigger silencing of homologous gene expression by a mechanism termed RNAi (for RNA-mediated interference) (Fire, A., Xu, S., Montgomery, M. K., Kostas, S. A., Driver, S. E. & Mello, C. C. (1998) Nature (London) 391, 806-811). RNAi is an evolutionarily conserved phenomenon and a multistep process that involves generation of active small interfering RNA (siRNA) *in vivo* through the action of an RNase III endonuclease, Dicer. The resulting 21- to 23-nt siRNA mediates degradation of the complementary homologous RNA (Bernstein, E. , Denli, A. M. & Hannon, G. J. (2001) RNA 7, 1509-1521; Sharp, P. A. (2001) Genes Dev. 15,485-490). Such RNAi technology could be employed with the current invention by linking or otherwise incorporating a peptide of the invention with the siRNAs. Hence, the peptides of the invention can be used to block expression of a specific gene by employing the RNAi. RNAs of interest whose expression can be blocked include those that encode the same target proteins, genes, RNA and DNA described herein.

Targeted delivery of a peptide of the invention that results in delivery of a polynucleotide or gene or ribozyme or growth factor to atherosclerotic plaque(s) can enhance endothelial coverage and healing at the site of atherosclerosis, especially in plaques that are vulnerable to rupturing and to producing thrombosis, unstable angina, myocardial infarction or subinfarctive myocardial injury. Other areas that could benefit from such targeted delivery to heal endothelium include the sites of vascular interventions such as angioplasty or stenting. Polynucleotides, DNA, peptides, growth factors (or agents that stimulate secretion of growth factors) that may be delivered to such sites would include, but not be limited to: VEGF (e.g. all forms of vascular endothelial growth factor including but not limited to VEGF121 and VEGF165), Fibroblast Growth Factors (e.g. FGF-1, 2), hepatocyte growth factor, placental growth factors (PIGFs), platelet derived endothelial cell growth factors, and TGF-beta.

Preselected nucleic acids useful for therapy can be placed in expression cassettes and/or expression vectors. To prepare expression cassettes for transformation, the nucleic acid encoding a therapeutic agent and/or peptide of the invention may be circular or linear, double-stranded or single-stranded.

Generally, a vector is used to facilitate delivery and expression of an expression cassette or nucleic acid.

A peptide of the invention can be incorporated into any vector available to one of skill in the art, along with a nucleic acid that encodes a useful therapeutic agent. Such vectors include viral (adenovirus, retrovirus, lentivirus or other viruses) vectors or synthetic vectors (such as but not limited to liposomes, microparticles or nanoparticles) to allow improved delivery of genes, ribozymes, antisense oligonucleotides, or DNA to atherosclerotic lesions as well as to allow improved endothelial cell transduction with these genes, ribozymes, antisense or naked DNA. In addition, non-vector mediated targeted delivery of oligonucleotides could be achieved by linking a peptide of the invention onto or within an oligonucleotide or other nucleic acid.

A number of vector systems are known for the introduction of foreign or native genes into mammalian cells. These include SV40 virus (Okayama et al., 1985); bovine papilloma virus (DiMaio et al., 1982); adenovirus (Morin et al., 1987; Dai et al., 1995; Yang et al., 1996; Tripathy et al., 1996; Quantin et al., 1992; Rosenfeld et al., 1991; Wagner, 1992; Curiel et al., 1992; Curiel, 1991; LeGal LaSalle et al., 1993; Kass-Eisler et al., 1993); adeno-associated virus (Muzyczka, 1994; Xiao et al., 1996); herpes simplex virus (Geller et al., 1988; Huard et al., 1995; U.S. Pat. No. 5,501,979); lentivirus (Douglas, et al., Hum Gene Ther. 12(4):401-413 (2001), Miyoshi, et al., Virol. 72:8150-8157 (1999), Garvey et al. Virology, 175:391-409, 1990, Berkowitz et al. J. Virol. 7(7):3371-3382 (2001), WO 01/44458, US patent application 09/734,836, US Patent No. 6,277,633 and 5,380,830. The targeting peptides can be used in any mammalian expression

vector to target the expression system to the appropriate target endothelial cells. See, for example, Wu et al. (1991); Wu and Wu (1988); Wu et al. (1989); Zenke et al. (1990); and Wagner et al. (1990). Grifman et al. (2001) describes the incorporation of tumor-targeting peptides into recombinant AAV capsids. For descriptive purposes only, this embodiment will be described with reference to an adenoviral vector, a preferred aspect of this embodiment. However, it will be understood that the embodiment is applicable to any of the previously mentioned vector systems and others known in the art.

Any such vector to which a peptide of the invention has been linked or within which such a peptide is encoded, can also contain an inducible promoter operably linked to a coding region for the peptide or a polypeptide therapeutic agent. Such a promoter permits controllable expression of the nucleic acid through an appropriate inducer of transcription.

A vector can be in the form of chimeric DNA that contains the coding region of the selected nucleic acid flanked by control sequences that promote the expression of the nucleic acid within target cells. As used herein, "chimeric" means that a vector comprises DNA from at least two different species, or comprises DNA from the same species, which is linked or associated in a manner that does not occur in the "native" or wild type of the species.

Aside from preselected nucleic acid that encodes a beneficial protein, RNA or antisense RNA, a portion of the preselected nucleic acid may be serve a regulatory or a structural function. For example, the preselected nucleic acid may itself comprise a promoter that is active in the mammal, particularly in the cells present in the atherosclerotic lesions of the mammal. Alternatively, a promoter that is present within the vector or the genome of the mammal can be used. Many promoter elements well known to the art may be employed in the practice of the invention.

The term "control sequences" is defined to mean DNA sequences necessary for the expression of an operably linked coding region in a particular host organism. Control sequences that are suitable for eukaryotic cells include promoters, polyadenylation signals, and enhancers. Prokaryotic cells that are useful for designing, amplifying and maintaining nucleic acids for eukaryotic gene therapy, utilize control sequences such as promoters, and, optionally operator sequences and ribosome binding sites.

Other elements functional in eukaryotic host cells, such as introns, enhancers, polyadenylation sequences and the like, may also be a part of the preselected nucleic acid. Such elements may or may not be necessary for the function of the nucleic acid, but may provide improved expression of RNA from the nucleic acid by affecting transcription, stability of the mRNA, or the like. Such elements may be included in the nucleic acid or vector as desired by one of skill in the art to obtain the optimal performance of the transforming nucleic acid or vector in the cell.

"Operably linked" is defined to mean that the nucleic acids are placed in a functional relationship with another nucleic acid sequence. For example, control sequences are operably linked to a nucleic acid encoding a beneficial protein; a promoter or enhancer is operably linked to a coding region if it affects the transcription of the coding region; or a ribosome binding site is operably linked to a coding region if it is positioned so as to facilitate translation. Generally, "operably linked" means that the nucleic acids being linked are contiguous and, in the case of a secretory leader, contiguous and in reading phase. However, enhancers do not have to be contiguous. Linking is generally accomplished by ligation at convenient restriction sites. If such sites do not exist, synthetic oligonucleotide adaptors or linkers can be used in accord with conventional practice.

The preselected nucleic acid to be introduced into target cells will generally also contain either a selectable marker gene or a reporter gene (or both) to facilitate identification and selection of transformed cells from the wider population of cells. Alternatively, the selectable marker may be carried on a separate nucleic acid and used in a co-transformation procedure. Both selectable markers and reporter genes may be flanked with appropriate regulatory sequences to enable expression in the host cells. Useful selectable markers are well known in the art and include, for example, antibiotic-resistance genes, such as *neo*, *hpt*, *dhfr*, and the like.

Reporter genes are used for identifying target cells and cells transformed by the vectors or therapeutic constructs of the invention. Reporter genes that encode for easily assayable proteins are well known in the art. In general, a reporter gene is a gene that is not present in or expressed by the recipient organism or tissue and that encodes a protein whose expression is manifested by some easily detectable property, e.g., enzymatic activity or fluorescence. Preferred genes include the chloramphenicol acetyl transferase gene (cat) from Tn9 of *E. coli*, the beta-glucuronidase gene (gus) of the *uidA* locus of *E. coli*, the green fluorescence protein and the luciferase gene from firefly *Photinus pyralis.* Expression of the reporter gene is assayed at a suitable time after the vector or recombinant nucleic acid has been introduced into the mammal or recipient cells.

The general methods for constructing recombinant nucleic acids that can transform target cells are well known to those skilled in the art, and the same compositions and methods of construction may be utilized to produce nucleic acids and vectors useful herein. For example, J. Sambrook et al., Molecular Cloning: A Laboratory Manual, Cold Spring Harbor Laboratory Press (2d ed., 1989), provides suitable methods of construction.

The peptides of the invention can also be used to deliver pharmaceutical compounds (drugs) to sites of atherosclerotic lesions or to sites of revascularization procedures including stenting or access ports for dialysis. Such pharmaceutical compounds can be directly linked to the peptide. Alternatively, the peptide can be incorporated into a chemical composition containing the therapeutic agent or compound. In another embodiment, the peptide can be incorporated

into an artificial carrier (e.g. a liposome or other microparticle) that contains the therapeutic agent or compound. Such drug therapies would include compounds regulate HMG-CoA reductase (e.g. statins), fibrates and other compounds effecting PPARs (e.g. PPAR alpha, gamma, and/or delta agonists), or thyromimetics.

HMG-CoA reductase inhibitors are also called β-hydroxy-β-methylglutaryl-coenzyme-A reductase inhibitors. These inhibitors are understood to be those active agents that may be used to lower the lipid levels including cholesterol, especially LDL-cholesterol, in blood. The class of HMG-Co-A reductase inhibitors comprises compounds having differing structural features. HMG-CoA reductase inhibitors suitable for use herein include, but are not limited to, pitavastatin, simvastatin, pravastatin, rivastatin, mevastatin, fluindostatin, cerivastatin, velostatin, fluvastatin, dalvastatin, dihydrocompactin, compactin, rosuvastatin, or lovastatin; or a pharmaceutically acceptable salt of pitavastatin, simvastatin, pravastatin, rivastatin, cerivastatin, mevastatin, fluindostatin, velostatin, fluvastatin, dalvastatin, dihydrocompactin, compactin, rosuvastatin and lovastatin or, in each case, a pharmaceutically acceptable salt thereof. Information on such compounds is available to one of skill in the art, see for example, the information on atorvastatin (EP 680320), cerivastatin (EP 491226), fluvastatin (US 5354772), pitavastatin (EP 304063), lovastatin (EP 22478), pravastatin (UK 2077264), rosuvastatin (ZD 4522or S 4522) and simvastatin (EP 33538). Desirable HMG-Co-A reductase inhibitors are those agents that have been marketed, for example, fluvastatin, atorvastatin, pitavastatin or simvastatin or, in each case, a pharmaceutically acceptable salt thereof.

Fibrates are known to lower the levels of triglyceride-rich lipoproteins, such as VLDL, to raise HDL levels, and to have variable effects on LDL levels. The effects on VLDL levels may result primarily from an increase in lipoprotein lipase activity, especially in muscle. This leads to enhanced hydrolysis of VLDL triglyceride content and enhanced VLDL catabolism. These compounds are also reported to decrease hepatic VLDL triglyceride synthesis, possibly by inhibiting fatty acid synthesis and by promoting fatty acid oxidation as a result of peroxisomal proliferation.

Fibrates include fibric acid derivatives such as, for example, clofibrate, gernfibrozil, fenofibrate, ciprofibrate, and bezafibrate. Fenofibrate is commercially available as Tricor™. Fenofibric acid, the active metabolite of fenofibrate, lowers plasma triglycerides apparently by inhibiting triglyceride synthesis, resulting in a reduction of VLDL released into the circulation, and also by stimulating the catabolism of triglycerides rich lipoprotein (i.e. VLDL). The recommended daily dose of fenofibrate is 67 mg, but this dosage may vary depending on the route of administration. For example, when linked to a peptide of the invention lower dosages of fenofibrate may be utilized, for example, because the peptide targets this therapeutic agent to the appropriate site.

Clofibrate is commercially available as Atromid-S™ capsules. Each capsule contains 500 mg of clofibrate. Clofibrate lowers elevated serum lipids by reducing the very low-density lipoprotein fraction that is rich in triglycerides and thereby reduces serum cholesterol. Clofibrate may also inhibit the hepatic release of lipoproteins (particularly VLDL) and potentiate the action of lipoprotein lipase. The recommended daily dose of clofibrate is 2 grams, administered in divided doses. However, this dosage may be modified as when clofibrate is linked to a peptide of the invention. In general, lower dosages of clofibrate may then be utilized.

Gemfibrozil is commercially available as Lopid™ tablets. Each tablet contains 600 mg of gernfibrozil. Gernfibrozil is a lipid regulating agent that decreases serum triglycerides and very low density lipoprotein cholesterol, and increases high density lipoprotein cholesterol. The recommended daily dose of gernfibrozil is 1200 mg, administered in two divided doses. However, this dosage may be modified as when gernfibrozil is linked to a peptide of the invention. In general, lower dosages of gernfibrozil may then be utilized.

Fibrate hypolipidemic agents can also be used as synthetic PPAR (peroxisome proliferator activated receptor) agonists. Fibrates include PPAR-alpha agonists that may also act as agonists for PPAR-gamma and/or PPAR-delta subtypes. Fibrates such as bezafibrate and fenofibrate exert hypolipidemic effects as PPARα agonists by decreasing apolipoprotein C-III production (enhancing lipoprotein lipase activity) and increasing lipoprotein lipase (LPL) production in the liver (Lefebvre et al., Arterioscler. Thromb. Vasc. Biol. 1997; 17:1756). Moreover, in man but not in rodents, PPAR agonists increase levels of high-density lipoprotein cholesterol (HDL-C) by inducing apoA-I gene expression in the liver (Staels and Auwerx, Atherosclerosis. 1998; 137 Suppl: S 19). PPARα may also have a role in obesity. For example, PPARα ligands lower body weight in rodents without any change in food intake (Guerre-Millo et al., J. Biol. Chem. 2000; 275:16638).

PPARs are ligand-activated transcription factors that belong to the nuclear hormone receptor superfamily (Djouadi et al., J. Clin. Invest. 1998; 102:1083, Kersten et al., Nature 2000; 405:421). These receptors function as heterodimeric complexes with the receptor for 9-cis retinoic acid (RXR). Activation of PPAR/RXR heterodimers occurs upon binding of the ligands, driving activation of specific PPAR-sensitive elements in the promoter region of target genes and leading to activation of gene expression (Kersten et al., Nature 2000; 405:421). The natural endogenous ligands shared by PPARs appear to be polyunsaturated and oxidized fatty acids. There are three subtypes of the PPARs: PPARα, PPARδ and PPARγ.

PPARα plays a key role in the control of lipid metabolism and is expressed primarily in tissues participating in fatty acid oxidation (e.g., liver, kidney, heart, and skeletal muscle). The natural ligands for this PPAR subtype include fatty acids and their acyl CoA derivatives (Issemann, J. Mol. Endocrinol. 1993; 11:37, Murakami et al., Biochem. Biophy.

Res. Comm. 1999; 260:609). Activation of PPARα results in the expression of genes encoding enzymes involved in the beta-oxidation of lipids. PPARα activation also up-regulates enzymes involved in fatty acid uptake, mitochondrial beta-oxidation and ketone body synthesis as well as the induction of fatty acid transport proteins and acyl-CoA synthetase, responsible for cellular fatty acid esterification and entrapment. PPARα up-regulates expression of liver and muscle carnitine palmitoyl transferase-1 (CPT-1) genes.

The importance of this nuclear hormone receptor in lipid homeostasis is supported by the observations that PPARα knockout mice display hepatic and cardiac lipid accumulation (Djouadi et al., J. Clin. Invest. 1998; 102:1083) and late onset obesity (Costet et al., J. Biol. Chem. 1998; 273:29577). Furthermore, PPARα may modulate inflammation, and has been implicated as a modulator of atherosclerosis and restenosis. PPARα also may play a role in the evolution of oxidative stress observed in aging (Devchand et al., Nature 1996; 384:39, Poynter and Daynes, J. Biol. Chem. 1998; 273:32833). PPARα modulates cardiac energy metabolic shifts and has been implicated in the processes accompanying cardiac hypertrophy, heart failure and myocardial infarction (Barger et al., Trends Cardiovasc. Med. 2001; 10:238). In addition, PPARα agonists are direct modulators of the vessel wall and thus might play a role in vascular diseases (Buchan et al., Med. Res. Rev. 2000; 20:350, Glass, J. Endocrinol. 2001; 169:461). PPARα may also function to regulate fat homeostasis in the islet beta cell (Zhou et al., Proc. Natl. Acad. Sci. U.S.A. 1998; 95:8898.

While PPARα promotes lipid oxidation, PPARy promotes lipid storage and consequently is expressed predominantly in adipocytes. PPARγ activation drives adipogenesis in part through the formation of new adipocytes, which serve as lipid reservoirs (Tontonoz et al., Cell 1994; 79:1147, Lehmann et al., J. Biol. Chem. 1995; 270:12953). PPARγ agonists induce expression of genes such as aP2, phosphoenol pyruvate carboxykinase (PEPCK), acyl-CoA synthetase (ACS), fatty acid binding protein (FABP) and lipoprotein lipase (LPL) (MacDougald and Lane, Annu. Rev. Biochem. 1995; 64: 345-73:345, Robinson et al., Biochem. Biophys. Res. Commun. 1998; 244:671, Clarke, Br. J. Nutr. 2000; 83 Suppl 1: S59). The induction of LPL promotes fatty acid delivery to the adipocytes while FABP and ACS induction enhance fatty acid uptake and storage by the small new adipocytes. While PPARγ is not expressed in the muscle, its activation increases skeletal muscle insulin sensitivity by a poorly characterized mechanism. Elevated muscle and β-cell lipid stores are correlated with muscle insulin resistance and β-cell secretory dysfunction (Zhou et al., Metabolism 1996; 45: 981).

PPARγ agonists may exert insulin-sensitizing effects by shifting lipid storage away from muscle, liver and β-cells, toward adipose tissue (Shimabukuro et al., Proc. Natl. Acad. Sci. U.S.A. 1998; 95:2498). PPARγ agonists also inhibit muscle PDK4 leading to increased PDH activity and increased glucose utilization. In the liver, treatment with PPARγ agonists inhibits PEPCK and G-6-Pase activities thereby decreasing gluconeogenesis, e.g., thiazolidinediones (TZDs), including troglitazone, rosiglitazone, and pioglitazone are synthetic PPARγ ligands that exert antidiabetic effects by alleviating insulin resistance at the level of the muscle and liver. PPARγ ligands also may play a role in vascular endothelial function by reducing the expression of endothelin-1 and thereby have a hypotensive effect (Satoh et al., Biochem. Biophys. Res. Commun. 1999; 254:757). It has been shown that two dominant negative mutations in PPARγ were associated with severe hypertension in humans (Barroso et al., Nature 1999; 402:880). The anti-hypertensive effects of PPARγ agonists have also been seen in animal models not associated with insulin resistance, suggesting the anti-hypertensive effects of PPARγ agonists may be independent of their insulin sensitizing actions (Willson et al., Annu. Rev. Biochem. 2001; 70:341). Like the other subtypes, PPARδ receptor is also activated by fatty acids. PPARδ subtype is widely expressed and plays a role in lipid metabolism (Leibowitz et al., FEBS Letters 2000; 473:333). In non-diabetic insulin resistant monkeys a specific PPARδ ligand, GW501516, increased HDL-C (80%) and apoA-I (43%) and lowered triglyceride, LDL-C and insulin levels. GW501516 also promotes reverse cholesterol transport via up-regulation of macrophage ABCA1 (Oliver et al., Proc. Nat. Acad. Sci. 2001; 98:5306).

PPARα and PPARγ ligands may also have an anti-atherosclerotic benefit through stimulation of macrophage ABCA1 expression and thereby promoting apoA1-mediated cholesterol efflux (Chinetti et al., Nature Medicine 2001; 7: 53). Hence, the peptides of the invention may be linked to PPAR, PPAR agonists and/or to PPAR ligands and used to treat conditions such as dyslipidemia, hyperlipidemia, hypercholesteremia, atherosclerosis, hypertriglyceridemia, heart failure, myocardial infarction, vascular diseases, cardiovascular diseases, and hypertension.

Compounds that are PPAR agonists include compounds such as those described in United States Patent 6,008,239, WO 9727847, WO 9727857, WO 9728115, WO 9728137, WO 9728149, Hulin et al., Current Pharm. Design (1996) 2, pp. 85-102, and Willson et al. J. Med. Chem. 1996 vol. 39 pp. 665-669. Non-glitazone type PPARγ agonists include N-(2-benzoylphenyl)-L-tyrosine analogues, e.g. GI-262570, and JTT501. Preferred dual PPARγ/PPARα agonists include ω-[(oxoquinazolinylalkoxy) phenyl]alkanoates and analogs thereof, very especially the compound DRF-554158, described in WO 99/08501 and the compound NC-2100 described by Fukui in Diabetes 2000, 49(5), 759-767. Pharmaceutically acceptable salts and esters of PPAR-agonists are likewise included within the scope of this invention.

PPAR-alpha, PPAR-gamma, and PPAR-delta agonists may be identified according to an assay described in U.S. Patent 6,008,239. PPAR agonists are also identified by the following assays.

Human PPAR-gamma 2, human PPAR-delta and human PPAR-alpha can be expressed as gst-fusion proteins in E. coli. Bacterial cells containing expression vectors encoding these fusion proteins can be propagated, expression

of the proteins can be induced, and bacterial cells can be harvested by centrifugation. The pellet can be resuspended, cells disrupted in a French press and debris removed by centrifugation at 12, 000 X g. Recombinant human PPAR receptors can be further purified by affinity chromatography on glutathione sepharose. After application to the column, washing to remove non-specifically bound material, receptors can be eluted with glutathione. Glycerol (10%) can be added to stabilize the receptors and aliquots of the receptors can be stored at - 80°C.

For binding to PPAR-gamma, an aliquot of receptor can be incubated in TEGM (10 mM Tris, pH 7.2, 1 mM EDTA, 10% glycerol, 7 μl/100 mL β-mercaptoethanol, 10 mM Na molybdate, 1 mM dithiothreitol, 5 μg/mL aprotinin, 2 μg/mL leupeptin, 2 μg/mL benzamidine and 0.5 mM PMSF) containing 0.1 % non-fat dry milk and 10 nM [$^3H_2$] AD5075 (21 Ci/mmole), ± test compound as described in Berger et al. (Novel peroxisome proliferator-activated receptor (PPAR-gamma) and PPAR-delta ligands produce distinct biological effects. J. Biol. Chem. (1999), 274: 6718-6725. Assays can be incubated for about 16 hr at 4°C, in a final volume of 150 μL. Unbound ligand can be removed by incubation with 100 μL dextran/gelatin-coated charcoal, on ice, for about 10 min. After centrifugation at 3000 rpm for 10 min at 4°C, 50 μL of the supernatant fraction can be counted in a Topcount.

For binding to PPAR-delta, an aliquot of receptor can be incubated in TEGM (10 mM Tris, pH 7.2, 1 mM EDTA, 10% glycerol, 7 μl/100 mL β-mercaptoethanol, 10 mM Na molybdate, 1 mM dithiothreitol, 5 μg/mL aprotinin, 2 μg/mL leupeptin, 2 μg/mL benzamidine and 0.5 mM PMSF) containing 0.1 % non-fat dry milk and 2.5 nM [$^3H_2$]L-783483, (17 Ci/mmole), ± test compound as described in Berger et al (Novel peroxisome proliferator-activated receptor-γ (PPAR-gamma) and PPAR-δ (PPAR-delta) ligands produce distinct biological effects. 1999 J Biol Chem 274: 6718 6725). (L-783483 is 3-chloro-4-(3-(7-propyl-3-trifluoromethyl-6-benz-[4,5]- isoxazoloxy)propylthio)-phenylacetic acid, Ex. 20 in WO 97128137). Assays can be incubated for about 16 hr at 4°C, in a final volume of 150 μL. Unbound ligand can be removed by incubation with 100 μL dextran/gelatin-coated charcoal, on ice, for about 10 min. After centrifugation at 3000 rpm for 10 min at 4°C, 50 μL of the supernatant fraction can be counted in a Topcount.

For binding to PPAR-α, an aliquot of receptor can be incubated in TEGM (10 mM Tris, pH 7.2, 1 mM EDTA, 10% glycerol, 7 μl/100 mL β-mercaptoethanol, 10 mM Na molybdate, 1 mM dithiothreitol, 5 μg/mL aprotinin, 2 μg/mL leupeptin, 2 μg/mL benzamidine and 0.5 mM PMSF) containing 0.1% non-fat dry milk and and 5.0 nM [$^3H_2$]L-797773, (34 Ci/mmole), ±test compound. (L-797733 is (3-(4-(3-phenyl-7 propyl-6-benz-[4,51-isoxazoloxy)butyloxy))phenylacetic acid, Ex.62 in WO 97/28137). Assays can be incubated for about 16 hr at 4° C., in a final volume of 150 μL. Unbound ligand can be removed by incubation with 100 μL dextran/gelatin-coated charcoal, on ice, for about 10 min. After centrifugation at 3000 rpm for 10 min at 4° C., 50 μL of the supernatant fraction can be counted in a Topcount.

The invention also includes a method for reducing cholesterol synthesis comprising administering a peptide of the invention linked to a thyroid hormone receptor beta agonist, e.g., selected from CGS23425 and CGS26214, and/or a fibrate, e.g., selected from clofribrate, gernfibrozil, fenofibrate, ciprofibrate and benzafibrate, in therapeutically effective amounts to a patient in need of such treatment. Fibrates therefore can be used in combination with thyroid hormone receptor beta agonist to practice the instant invention.

The term "DPP-IV inhibitor" is intended to indicate a molecule that exhibits inhibition of the-IV and functionally related enzymes, such as from 1-100% inhibition, and specially preserves the action of substrate molecules, including but not limited to glucagon-like peptide-1, gastric inhibitory polypeptide, peptide histidine methionine, substance P, neuropeptide Y, and other molecules typically containing alanine or proline residues in the second aminoterminal position. Treatment with DPP-IV inhibitors prolongs the duration of action of peptide substrates and increases levels of their intact, undegraded forms leading to a spectrum of biological activities relevant to the disclosed invention.

DPP-IV inhibitors that can be utilized are available to one of skill in the art. For example, DPP-IV inhibitors are generically and specifically disclosed, for example, in WO 98/19998, DE19616 486 A1, WO 00/34241, WO 95/15309, WO 01/72290, WO01/52825, WO 9310127, WO 9925719, WO 9938501, WO 9946272, WO 9967278 and WO 9967279.

Published patent application WO 98/19998 discloses N-(N'-substituted glycyl)-2-cyano pyrrolidines, in particular 1-[2-[5-Cyanopyridin-2-yl]amino]-ethylamino]acetyl-2-cyano-(S)-pyrrolidine (NVP-DPP728). DE19616 486 A1 discloses val-pyr, val-thiazolidide, isoleucyl-thiazolidide, isoleucyl-pyrrolidide, and fumar salts of isoleucyl-thiazolidide and isoleucyl-pyrrolidide. Published patent application WO 0034241 and published patent US 6110949 disclose N-substituted adamantyl-amino-acetyl-2-cyano pyrrolidines and W (substituted glycyl)-4-cyano pyrrolidines respectively. DPP-IV inhibitors of interest are specially those cited in claims 1 to 4. Published patent application WO 95/15309 discloses amino acid 2-cyanopyrrolidine amides as inhibitors of DPP-IV Published patent application WO 9529691 discloses peptidyl derivates of diesters of alpha-aminoalkylphosphonic acids, particularly those with proline or related structures. DPP-IV inhibitors of interest are specially those cited in Table 1 to 8. In WO 01/72290 DPP-IV inhibitors of interest are specially those cited in example 1 and claims 1, 4, and 6. WO 01/52825 specially discloses (S)-1-{2-[5-cyanopyridin-2yl)amino] ethyl-aminoacetyl)-2-cyano-pyrrolidine or (S)-1-[(3-hydroxy-1-adamantyl)amino]acetyl-2-cyano-pyrrolidine. Published patent application WO 9310127 discloses proline boronic esters useful as DPP-IV inhibitors. DPP-IV inhibitors of interest are specially those cited in examples 1 to 19. Published patent application WO 9925719 discloses sulphostin, a DPP-IV inhibitor prepared by culturing a Streptomyces microorganism. Published patent application WO 9938501 discloses N-substituted 4-8 membered heterocyclic rings. DPP-IV inhibitors of interest are specially those cited in claims 15 to

20. Published patent application WO 9946272 discloses phosphoric compounds as inhibitors of DPP-IV. DPP-IV inhibitors of interest are specially those cited in claims 1 to 23. Published patent applications WO 9967278 and WO 9967279 disclose DPP-IV prodrugs and inhibitors of the form A-B-C where C is either a stable or unstable inhibitor of DPP-IV. Any of the substances disclosed in the above mentioned patent documents are considered potentially useful as DPP-IV inhibitors to be used in carrying out the present invention.

Preferred DPP-IV inhibitors are N-substituted adamantyl-amino-acetyl-2-cyano pyrrolidines, N (substituted glycyl)-4-cyano pyrrolidines, N-(N'-substituted glycyl)-2-cyanopyrrolidines, N-aminoacyl thiazolidines, N-aminoacyl pyrrolidines, L-allo-isoleucyl thiazolidine, L-threo-isoleucyl pyrrolidine, and L-allo-isoleucyl pyrrolidine, 1-[2-[(5-cyanopyridin-2-yl)amino] ethylamino]acetyl-2-cyano-(S)-pyrrolidine and pharmaceutical salts thereof. Especially preferred are 1-{2-[(5-cyanopyridin-2-yl)amino]ethylamino}acetyl-2 (S)-cyano-pyrrolidine dihydrochloride, of the following formula:

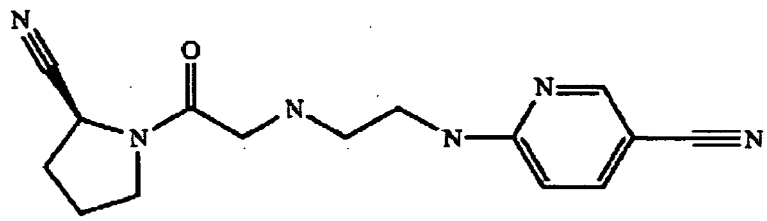

especially the dihydrochloride thereof Another preferred DPP-IV inhibitor is pyrrolidine, 1-[(3-hydroxy-1-adamantyl)amino]acetyl-2-cyano-, (S) of the following formula:

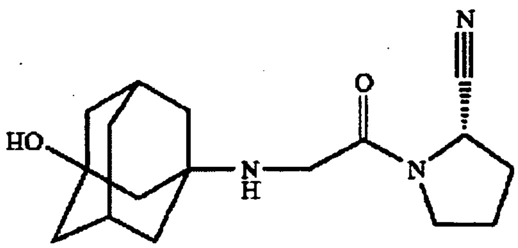

Another preferred DPP-IV inhibitor is L-threo-isoleucyl thiazolidine, and pharmaceutical salts thereof. Especially preferred are orally active DPP-IV inhibitors.

In another embodiment, the therapeutic agent is a thyromimetic compound. Examples of thyromimetic compounds to be employed in the present invention include those disclosed in EP 580 550, U.S. Patent Nos. 5654468, 5569674 and 5401772.

Thyromimetic compounds of the present invention also include those compounds disclosed in U.S. Patent Nos. 4069343; 4554290; 4766121; 4826876; 4910305; 5061798; 5232947; 5284971; 5401772; WO 00/58279, and those disclosed in Yokoyama N. et al., Journal of Medicinal Chemistry, 38(4):695-707 (1995) and Stephan Z. F. et al., Atherosclerosis, 126:53-63 (1996) especially the corresponding subject matter of the claims and the working examples directed to thyromimetic compounds.

Particularly preferred thyromimetic compounds also include compounds of formula I:

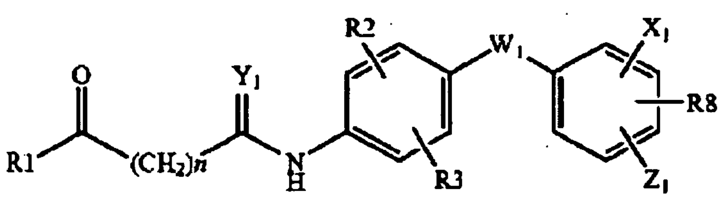

wherein:

$W_1$ is O, S, S(O) or $S(O)_2$;
$X_1$ is--$SR_4$,--$S(O)R_4$,--$S(O)_2R_4$, or--$S(O)_2$NR5R6; or $X_1$ is--C(O)NR5R6 provided that--C(O)NR5R6 is located at the 3'-, 4'-or 5'-position;
$Y_1$ is O or $H_2$;
$Z_1$ is hydrogen, halogen, hydroxy, optionally substituted alkoxy, aralkoxy, acyloxy or alkoxycarbonyloxy;
R1 is hydroxy, optionally substituted alkoxy, aryloxy, heteroaryloxy, aralkoxy, cycloalkoxy, heteroaralkoxy or--NR5R6;
R2 is hydrogen, halogen or alkyl;
R3 is halogen or alkyl;
R4 is optionally substituted alkyl, aryl, aralkyl, heteroaralkyl or heteroaryl;
R5, R6 and R7 are independently hydrogen, optionally substituted alkyl, cycloalkyl, aryl, aralkyl, heteroaryl, or heteroaralkyl; or R5 and R6 combined are alkylene optionally interrupted by O, S, S(O), $S(O)_2$ or NR7 which together with the nitrogen atom to which they are attached form a 5-to 7-membered ring;
R8 is hydrogen, halogen, trifluoromethyl, lower alkyl or cycloalkyl;
n represents zero or an integer from 1 to 4; and pharmaceutically acceptable salts thereof.

Preferred are the compounds of formula I as defined above with the proviso that when $X_1$ is -C(O)NR5R6, $Z_1$ is different from hydrogen (preferably hydroxy).

Preferred compounds of formula I include those compounds wherein:

R1 is hydroxy, lower alkoxy or NR5R6; R5 being hydrogen or lower alkyl and R6 being hydrogen, lower alkyl, lower alkoxy or R5 and R6 combined being alkylene or alkylene interrupted by O which together with the nitrogen atom to which they are attached form a 5- to 7- membered ring;
R1 is more preferably hydroxy, lower alkoxy or aryloxy.
R2 is hydrogen, halogen or lower alkyl.
R3 is halogen or lower alkyl.
R4 is phenyl or phenyl substituted by one or more substituents selected from the group consisting of lower alkyl, lower alkoxy, halogen and trifluoromethyl.
R5 is hydrogen.
R6 is phenyl or phenyl substituted by one or more substituents selected from the group consisting of lower alkyl, lower alkoxy, halogen and trifluoromethyl.
R8 is hydrogen or lower alkyl, more preferably hydrogen.
$W_1$ is O or S, more preferably O.
$X_1$ is -$S(O)_2$R4; R4 being lower alkyl, phenyl or phenyl substituted by one or more substituents selected from the group consisting of lower alkyl, lower alkoxy, halogen and trifluoromethyl; or is -$S(O)_2$NR5R6 or is -C(O)NR5R6; R5, in each case, being hydrogen or lower alkyl and R6, in each case, being hydrogen, lower alkyl, lower alkyl substituted by NR5R6, 3- to 7-membered cycloalkyl, phenyl, phenyl substituted by one or more substituents selected from the group consisting of lower alkyl, lower alkoxy, halogen and trifluoromethyl; pyridyl or N-lower alkyl-2-pyridone; or R5 and R6 combined, in each case, being alkylene or alkylene interrupted by O or $S(O)_2$ which together with the nitrogen atom to which they are attached form a 5- to 7- membered ring.
$X_1$ is preferably -$S(O)_2$R4 or -$S(O)_2$NR5R6.
$Y_1$ is O or $H_2$, more preferably O.
$Z_1$ is hydrogen or hydroxy.

The integer "n" preferably is zero, 1 or 2.

Preferred are the compounds of formula IA

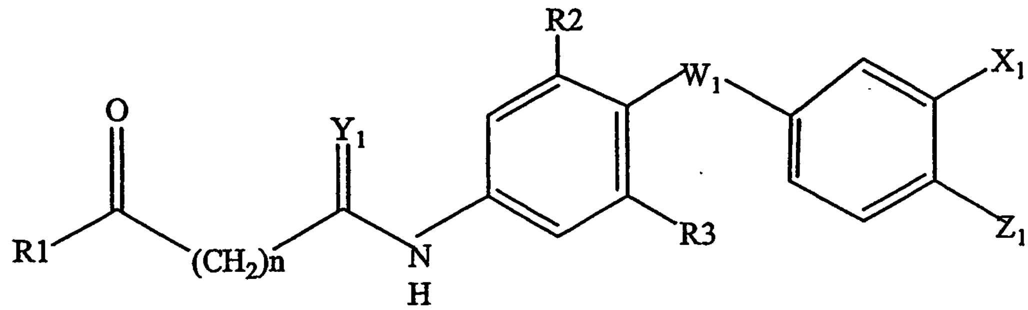

wherein

W$_1$ is O or S;

X$_1$ is -SR4, -S(O)R4, -S(O)$_2$R4, -S(O)$_2$NR5R6 or -C(O)NR5R6;

Y$_1$ is O or H$_2$;

Z$_1$ is hydrogen, halogen, hydroxy, alkoxy, aralkoxy, acyloxy or alkoxycarbonyloxy;

R1 is hydroxy, lower alkoxy or aryloxy;

R2 is hydrogen, halogen or lower alkyl;

R3 is halogen or lower alkyl;

R4 is optionally substituted alkyl, aryl, aralkyl, heteroaryl or heteroaralkyl;

R5, R6 and R7 are independently hydrogen, optionally substituted alkyl, cycloalkyl, aryl, aralkyl, heteroaryl, or heteroaralkyl; or R5 and R6 combined are alkylene optionally interrupted by O, S, S(O), S(O)$_2$ or NR7 which together with the nitrogen atom to which they are attached form a 5- to 7- membered ring;

n represents zero, 1 or 2;

and pharmaceutically acceptable salts thereof.

Further preferred are the compounds of formula IB

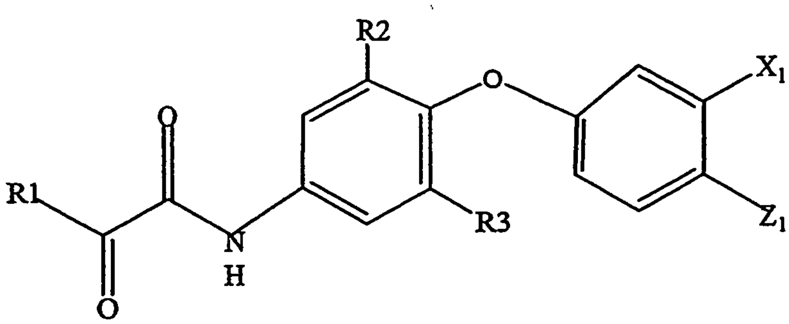

in which

X$_1$ is -S(O)$_2$R4, -S(O)$_2$NR5R6 or -C(O)NR5R6;

Z$_1$ is hydroxy, lower alkanoyloxy or lower alkoxy;

R1 is hydroxy or lower alkoxy;

R2 and R3 are lower alkyl;

R4 is aryl;

R5, R6 and R7 are independently hydrogen, optionally substituted alkyl, cycloalkyl, aryl, aralkyl, heteroaryl, or heteroaralkyl; or R5 and R6 combined are alkylene optionally interrupted by O, S, S(O), $S(O)_2$ or NR7 which together with the nitrogen atom to which they are attached form a 5- to 7- membered ring; and pharmaceutically acceptable salts thereof.

Preferred are compounds of formula I, IA and IB, and pharmaceutically acceptable salts thereof, wherein X is $-S(O)_2R4$ or $-S(O)_2$ NR5R6.

Also preferred are the compounds of formula IC

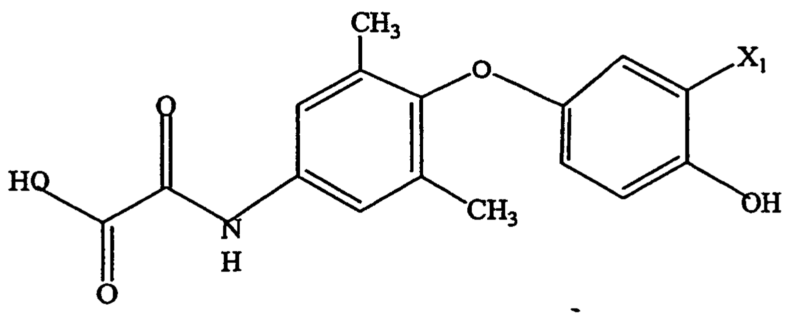

in which

$X_1$ is $-S(O)_2R4$ or $-S(O)_2NR5R6$;

R4 is monocyclic aryl;

R5, R6 and R7 are independently hydrogen, optionally substituted alkyl or aryl; or R5 and R6 combined are $CH_2CH_2$-Q-$CH_2CH_2$ wherein Q is $CH_2$, O, NR7, S, S(O) or $S(O)_2$ which together with the nitrogen atom to which they are attached from a 6-membered ring; pharmaceutically acceptable prodrug esters thereof; and pharmaceutically acceptable salts thereof.

Particularly preferred are the compounds of formula IC wherein $X_1$ is $S(O)_2R4$ and R4 is phenyl optionally substituted by lower alkyl, halo, lower alkoxy or trifluoromethyl; pharmaceutically acceptable salts thereof; and prodrug derivatives thereof. Most preferred is the compound N-(4-[3-(4-fluoro-benzenesulfonyl)-4-hydroxy-phenoxy]-3,5-dimethyl-phenyl)-oxamic acid.

Preferred thyromimetic compounds also include those disclosed in WO 00/51971, provided as formula II herein:

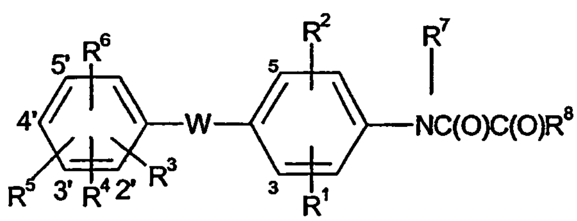

II

prodrugs thereof, geometric and optical isomers thereof, and pharmaceutically acceptable salts of said compounds, said prodrugs, and said isomers, wherein:

$R^1$, $R^2$ and $R^3$ are each independently hydrogen, halogen, $C_{1-6}$ alkyl, trifluoromethyl, -CN, -$OCF_3$ or -$OC_{1-6}$ alkyl;

$R^4$ is hydrogen, $C_{1-12}$ alkyl optionally substituted with one to three substitutents independently selected from Group Z, $C_{2-12}$ alkenyl, halogen, -CN, aryl, heteroaryl, $C_{3-10}$ cycloalkyl, heterocycloalkyl, -$S(O)_2NR^9R^{10}$, -$C(O)NR^9R^{10}$, -($C_{1-6}$ alkyl)-$NR^9R^{10}$, -$NR^9C(O)R^{10}$, -$NR^9C(O)NR^9R^{10}$, -$NR^9S(O)_2R^{10}$, -($C_{1-6}$ alkyl)-$OR^{11}$, -$OR^{11}$ or -$S(O)_aR^{12}$, provided that, where $R^5$ is not fluoro, $R^4$ is -$S(O)_2NR^9R^{10}$, -$C(O)NR^9R^{10}$, -($C_{1-6}$ alkyl)-$NR^9R^{10}$, -$NR^9C(O)R^{10}$, -$NR^9C(O)NR^9R^{10}$, -$NR^9S(O)_2R^{10}$, -($C_{1-6}$ alkyl)-$OR^{11}$, -$OR^{11}$or-$S(O)_aR^{12}$ ;

or $R^3$ and $R^4$ may be taken together to form a carbocyclic ring A of the formula -$(CH_2)_b$- or a heterocyclic ring A selected from the group consisting of- Q-$(CH_2)_c$- and -$(CH_2)_j$-Q-$(CH_2)_k$- wherein Q is O, S or $NR^{17}$, wherein said carbocyclic ring A and said heterocyclic ring A are each independently optionally substituted with one or more substituents independently selected from $C_{1-4}$ alkyl, halide or oxo;

$R^5$ is fluoro, hydroxy, $C_{1-4}$ alkoxy or $OC(O)R^9$;

or $R^4$ and $R^5$ may be taken together to form a heterocyclic ring B selected from the group consisting of -$CR^9$=$CR^{10}$ NH-, -N=$CR^9$-NH-, -$CR^9$=CH-O- and -$CR^9$=CH-S-;

$R^6$ is hydrogen, halogen, $C_{1-4}$ alkyl or trifluoromethyl;

$R^7$ is hydrogen or $C_{1-6}$ alkyl;

$R^8$ is -$OR^9$ or -$NR^{19}R^{20}$;

$R^9$ and $R^{10}$ for each occurrence are independently (A) hydrogen, (B) $C_{1-12}$ alkyl optionally substituted with one or more substituents independently selected from Group V, (C) $C_{2-12}$ alkenyl, (D) $C_{3-10}$ cycloalkyl optionally substituted with one or more substituents independently selected from $C_{1-6}$ alkyl, $C_{2-5}$ alkynyl, $C_{3-10}$ cycloalkyl, -CN, -$NR^{13}R^{14}$, oxo, -$OR^{18}$, -$COOR^{18}$ or aryl optionally substituted with X and Y, (E) aryl optionally substituted with X and Y, or (F) het optionally substituted with X and Y;

or $R^9$ and $R^{10}$ for any occurrence may be taken together to form a heterocyclic ring C optionally further containing a second heterogroup selected from the group consisting of -O-, -$NR^{13}$- and -S-, and optionally further substituted with one or more substituents independently selected from $C_{1-5}$ alkyl, oxo, -$NR^{13}R^{14}$, -$OR^{18}$, -$C(O)_2R^{18}$, -CN, -$C(O)R^9$, aryl optionally substituted with X and Y, het optionally substituted with X and Y, $C_{5-6}$ spirocycloalkyl, and a carbocyclic ring B selected from the group consisting of 5-, 6-, 7- and 8-membered partially and fully saturated, and unsaturated carbocyclic rings, and including any bicyclic group in which said carbocyclic ring B is fused to a carbocyclic ring C selected from the group consisting of 5-, 6-, 7- and 8-membered partially and fully saturated, and unsaturated carbocyclic rings;

$R^{11}$ is $C_{1-12}$ alkyl optionally substituted with one or more substituents independently selected from Group V, $C_{2-12}$ alkenyl, $C_{3-10}$ cycloalkyl, trifluoromethyl, difluoromethyl, monofluoromethyl, aryl optionally substituted with X and Y, het optionally substituted with X and Y, -$C(O)NR^9R^{10}$ or -$C(O)R^9$;

$R^{12}$ is $C_{1-12}$ alkyl optionally substituted with one or more substituents independently selected from Group V, $C_{2-12}$ alkenyl, $C_{3-10}$ cycloalkyl, aryl optionally substituted with X and Y, or het optionally substituted with X and Y;

$R^{13}$ and $R^{14}$ for each occurrence are independently hydrogen, $C_{1-6}$ alkyl, $C_{2-6}$ alkenyl, -($C_{1-6}$ alkyl)-$C_{1-6}$ alkoxy, aryl optionally substituted with X and Y, het optionally substituted with X and Y, -($C_{1-4}$ alkyl)-aryl optionally substituted with X and Y, -($C_{1-4}$ alkyl)-heterocycle optionally substituted with X and Y, -($C_{1-4}$ alkyl)-hydroxy, -($C_{1-4}$ alkyl)-halo, -($C_{1-4}$ alkyl)-poly-halo, -($C_{1-4}$ alkyl)-$CONR^{15}R^{16}$ or $C_{3-10}$ cycloalkyl;

$R^{15}$ and $R^{16}$ for each occurrence are independently hydrogen, $C_{1-6}$ alkyl, $C_{3-10}$ cycloalkyl or aryl optionally substituted with X and Y;

$R^{17}$ is hydrogen, alkyl, $C_{1-6}$ alkyl, -$COR^9$ or -$SO_2R^9$ ;

$R^{18}$ is hydrogen, $C_{1-6}$ alkyl, $C_{2-6}$ alkenyl, -($C_{1-6}$ alkyl)-$C_{1-6}$ alkoxy, aryl optionally substituted with X and Y, het optionally substituted with X and Y, -($C_{1-4}$ alkyl)-aryl optionally substituted with X and Y, -($C_{1-4}$ alkyl)-heterocycle

optionally substituted with X and Y, -($C_{1-4}$ alkyl)-hydroxy, -($C_{1-4}$ alkyl)-halo, -($C_{1-4}$ alkyl)-poly-halo, -($C_{1-4}$-alkyl)-$CONR^{15}R^{16}$, -($C_{1-4}$ alkyl)-($C_{1-4}$ alkoxy) or $C_{3-10}$ cycloalkyl;

$R^{19}$ is hydrogen or $C_{1-6}$ alkyl;

$R^{20}$ is hydrogen or $C_{1-6}$ alkyl;

W is 0, $S(O)_d$, $CH_2$ or $NR^9$;

Group Z is $C_{2-6}$ alkenyl, $C_{2-6}$ alkynyl, halogen, $-CF_3$, $-OCF_3$, hydroxy, oxo, -CN, aryl, heteroaryl, $C_{3-10}$ cycloalkyl, heterocycloalkyl, $-S(O)_aR^{12}$, $-S(O)_2NR^9R^{10}$, $-C(O)R^9R^{10}$, and $-NR^9R^{10}$;

Group V is halogen, $-NR^{13}R^{14}$, $-OCF_3$, $-OR^9$, oxo, trifluoromethyl, -CN, $C_{3-10}$ cycloalkyl, aryl optionally substituted with X and Y, and het optionally substituted with X and Y;

het for each occurrence is a heterocyclic ring D selected from the group consisting of 4-, 5-, 6-, 7-and 8-membered partially and fully saturated, and unsaturated, heterocyclic rings containing from one to four heteroatoms independently selected from the group consisting ofN, O and S, and including any bicyclic group in which said heterocyclic ring D is fused to a benzene ring or a heterocyclic ring E selected from the group consisting of 4-, 5-, 6-, 7- and 8-membered partially and fully saturated, and unsaturated, heterocyclic rings containing from one to four heteroatoms independently selected from the group consisting ofN, O and S;

X and Y for each occurrence are independently (A) hydrogen, (B) halogen, (C) trifluoromethyl, (D) $-OCF_3$, (E) -CN, (F) $C_{1-6}$ alkyl optionally substituted with one or more substituents independently selected from the group consisting of halogen, $-OCF_3$, $-CF_3$ and phenyl, (G) $C_{1-6}$ alkoxy, (H) aryl optionally substituted with one or more substituents independently selected from the group consisting of halogen, $-OCF_3$, $-CF_3$, $C_{1-4}$ alkyl and $C_{1-4}$ alkoxy, (I) $-C(O)_2R^{13}$, (J) $-C(O)NR^{13}R^{14}$, (K) $-C(O)R^{13}$, (L) $-NR^{13}C(O)NR^{13}R^{14}$ and (M) $-NR^{13}C(O)R^{14}$; or X and Y for any occurrence in the same variable may be taken together to form (a) a carbocyclic ring D of the formula $-(CH_2)_e-$ or (b) a heterocyclic ring F selected from the group consisting of $-O(CH_2)_fO-$, $(CH_2)_gNH$-and -CH=CHNH- ;

a and d are each independently 0, 1 or 2;

b is 3, 4, 5, 6 or 7;

c, f, g, j and k are each independently 2, 3, 4, 5 or 6; and

e is 3, 4, 5, 6 or 7.

A preferred group of compounds and pharmaceutically acceptable salts of such compounds, designated the A Group, contains those compounds of Formula II and pharmaceutically acceptable salts of such compounds, as shown above, wherein W is O.

A preferred group of compounds and pharmaceutically acceptable salts of such compounds, of the A Group, designated the B Group, contains those compounds of Formula II and pharmaceutically acceptable salts of such compounds, as shown above, wherein $R^1$ is located at the 3 position, $R^2$ is located at the 5 position, $R^3$ is located at the 2' position, $R^4$ is located at the 3' position, $R^5$ is located at the 4' position, and $R^6$ is located at the 5' position.

A preferred group of compounds and pharmaceutically acceptable salts of such compounds, of the B Group, designated the C Group, contains those compounds of Formula II and pharmaceutically acceptable salts of such compounds, as shown above, wherein $R^3$ is hydrogen, or $R^3$ and $R^4$ are taken together to form a carbocyclic ring A of the formula $-(CH_2)_b-$ or a heterocyclic ring A selected from the group consisting of $-Q-(CH_2)_c$ and $-(CH_2)_j-Q-(CH_2)_k$-wherein Q is O, S or $NR^{17}$ wherein said carbocyclic ring A and said heterocyclic ring A are each independently optionally substituted with one or more substituents independently selected from $C_{1-4}$ alkyl, halide or oxo, $R^5$ is hydroxy, $R^6$ is hydrogen and $R^7$ is hydrogen.

A preferred group of compounds pharmaceutically acceptable salts of such compounds, of the C Group, designated the D Group, contains those compounds of Formula II and pharmaceutically acceptable salts of such compounds, as shown above, wherein $R^1$ and $R^2$ are each independently methyl, bromo or chloro, and $R^8$ is hydroxy, methoxy, ethoxy, isopropoxy, $NH_2$ or $NH(CH_3)$.

A preferred group of compounds and pharmaceutically acceptable salts of such compounds, of the D Group, designated the E Group, contains those compounds of Formula II and pharmaceutically acceptable salts of such compounds, as shown above, wherein $R^4$ is $S(O)_2NR^9R^{10}$, and $R^{10}$ is hydrogen or methyl.

Particularly preferred compounds of the E Group are compounds wherein (a) $R^1$ is chloro, $R^2$ is methyl, $R^8$ is ethoxy or hydroxy, $R^9$ is ethyl and $R^{10}$ is hydrogen, (b) $R^1$ is chloro, $R^2$ is methyl, $R^3$ is ethoxy or hydroxy, $R^9$ is *n*-butyl and $R^{10}$ is hydrogen, (c) $R^1$ is chloro, $R^2$ is methyl, $R^8$ is ethoxy or hydroxy, $R^9$ is $-CH_2$-cyclopropyl and $R^{10}$ is hydrogen and (d) $R^1$ is chloro, $R^2$ is methyl, $R^3$ is isopropoxy or hydroxy, $R^9$ is cyclopropyl and $R^{10}$ is hydrogen; and pharmaceutically acceptable salts of said compounds.

Another preferred group of compounds and pharmaceutically acceptable salts of such compounds, of the D Group, designated the F Group, contains those compounds of Formula II and pharmaceutically acceptable salts of such compounds, as shown above, wherein $R^4$ is $S(O)_2NR^9R^{10}$, and $R^9$ and $R^{10}$ are taken together with the nitrogen atom to which they are attached to form $N(CH_2)_4$, $N(CH_2)_5$, morpholine or

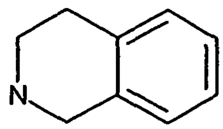

Particularly preferred compounds of the F Group are those wherein $R^9$ and $R^{10}$ are taken together with the nitrogen atom to which they are attached to form $N(CH_2)_4$.

A preferred group of compounds and pharmaceutically acceptable salts of such compounds, of the E Group, designated the G Group, contains those compounds of Formula II and pharmaceutically acceptable salts of such compounds, as shown above, wherein $R^9$ is hydrogen, isopropyl, $-CH_2$-2-thienyl, $-CH_2$-cyclopropyl, cyclopropyl, $-(CH_2)_2OH$, exo-2-norbornyl, methyl, ethyl, 4-fluorophenyl, cyclobutyl, cyclopentyl, cyclohexyl, *n*-propyl, *n*-butyl, *n*-pentyl, *n*-hexyl, *n*-octyl or *n*-decyl.

Particularly preferred compounds of the G Group are compounds wherein (a) $R^1$ is chloro, $R^2$ is methyl, $R^8$ is hydroxy or ethoxy, $R^9$ is cyclopropyl and $R^{10}$ is hydrogen, (b) $R^1$ is methyl, $R^2$ is methyl, $R^8$ is hydroxy or ethoxy, $R^9$ is cyclopropyl and $R^{10}$ is methyl, (c) $R^1$ is methyl, $R^2$ is methyl, $R^8$ is hydroxy or ethoxy, $R^9$ is cyclobutyl and $R^{10}$ is methyl, (d) $R^1$ is methyl, $R^2$ is methyl, $R^8$ is hydroxy or ethoxy, $R^9$ is cyclopropyl and $R^{10}$ is hydrogen and (e) $R^1$ is methyl, $R^2$ is methyl, $R^8$ is hydroxy or ethoxy, $R^9$ is cyclobutyl and $R^{10}$ is hydrogen; and pharmaceutically acceptable salts of said compounds.

A preferred group of compounds and pharmaceutically acceptable salts of such compounds, of the D Group, designated the J Group, contains those compounds of Formula II and pharmaceutically acceptable salts of such compounds, as shown above, wherein $R^4$ is $-C(O)NR^9R^{10}$, and $R^{10}$ is hydrogen, methyl or ethyl.

A preferred group of compounds and pharmaceutically acceptable salts of such compounds, of the J Group, designated the K group, contains those compounds of Formula II and pharmaceutically acceptable salts of such compounds, as shown above, wherein $R^9$ is methyl, ethyl, isopropyl, *n*-propyl, isobutyl, *n*-butyl, *n*-pentyl, *n*-hexyl, 4-fluorophenyl, $-CH_2$-2-thienyl, cyclopropyl, $-CH_2$-cyclopropyl, cyclobutyl, cyclopentyl, cyclohexyl, $-CH_2$-cyclohexyl, endo-2-norbornyl, exo-2-norbornyl, (S)-1-phenylethyl, (R)-1-phenylethyl, $-CH_2$-2-chlorophenyl, $-CH_2$-4-chlorophenyl, $-CH_2$-4-fluorophenyl, $-CH_2$-3-chloro-4-fluorophenyl, $-CH_2$-2-chloro-4-fluorophenyl, $-CH_2$-2-fluoro-4-chlorophenyl, $-CH_2$-3,4-difluorophenyl, $-CH_2$-4-isopropylphenyl, $-CH_2$-2,3-dichlorophenyl, $-CH_2$-2,4-dichlorophenyl, $-CH_2$-3,4-dichlorophenyl, $-CH_2$-3-trifluoromethyl-4-chlorophenyl, 4-phenylphenyl, 3-(2,4-dimethyl)pentyl, (R)-1-(1-naphthyl)ethyl, 1,1-dimethylpropyl, 1,2-dimethylpropyl, 2,2-dimethylpropyl, (R)-1-(2-naphthyl)ethyl, (R)-2-(1-naphthyl)ethyl, $-CH_2$-(1-naphthyl), (R)-1-cyclohexylethyl, (S)-1-cyclohexylethyl, $-CH_2$-3,4-methylenedioxyphenyl, $-CH_2$-4-*t*-butylphenyl, $-CH_2$-2,3-dichlorophenyl, 1-indanyl, (R)-1-indanyl, (S)-1-indanyl, 5-indanyl, 1-(1,2,3,4-tetrahydronaphthyl) or (R)-1-cyclohexylethyl.

Particularly preferred compounds of the K Group are compounds wherein (a) $R^1$ is chloro, $R^2$ is chloro, $R^8$ is hydroxy or ethoxy, $R^9$ is 3-(2,4-dimethyl)pentyl and $R^{10}$ is hydrogen, (b) $R^1$ is methyl, $R^2$ is methyl, $R^8$ is hydroxy or ethoxy, $R^9$ is cyclopropyl and $R^{10}$ is methyl, (c) $R^1$ is methyl, $R^2$ is methyl, $R^8$ is hydroxy or ethoxy, $R^9$ is cyclobutyl and $R^{10}$ is methyl, (d) $R^1$ is methyl, $R^2$ is methyl, $R^8$ is hydroxy or ethoxy, $R^9$ is 3-(2,4-dimethyl)pentyl and $R^{10}$ is hydrogen, (e) $R^1$ is methyl, $R^2$ is methyl, $R^8$ is hydroxy or ethoxy, $R^9$ is *n*-pentyl and $R^{10}$ is methyl, (g) $R^1$ is methyl, $R^2$ is methyl, $R^8$ is hydroxy or ethoxy, $R^9$ is isopropyl and $R^{10}$ is methyl, (h) $R^1$ is methyl, $R^2$ is methyl, $R^8$ is hydroxy, ethoxy or $NH_2$, $R^9$ is cyclobutyl and $R^{10}$ is methyl and (i) $R^1$ is chloro, $R^2$ is chloro, $R^8$ is hydroxy or ethoxy, $R^9$ is cyclobutyl and $R^{10}$ is methyl; and pharmaceutically acceptable salts of said compounds.

Another preferred group of compounds and pharmaceutically acceptable salts of such compounds, designated the L Group, contains those compounds of Formula II and pharmaceutically acceptable salts of such compounds, as shown above, wherein $R^4$ is $-C(O)NR^9R^{10}$, and $R^9$ and $R^{10}$ are taken together with the nitrogen atom to which they are attached to form $N(CH_2)_7$, $N(CH_2)_6$, $N(CH_2)_5$, $N(CH_2)_4$, morpholine,

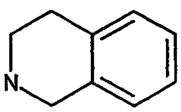, 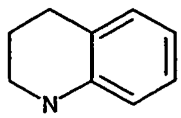, 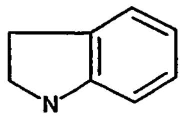, 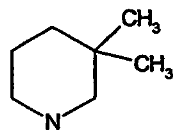, 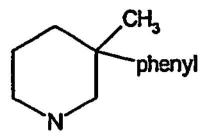,

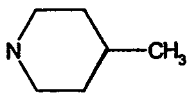, 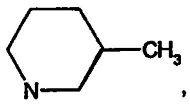, 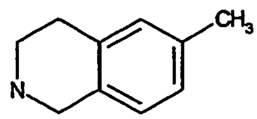, 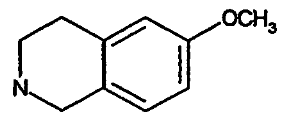

or

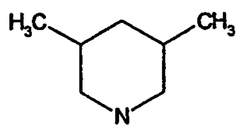

A preferred group of compounds and pharmaceutically acceptable salts of such compounds, of the D Group, designated the M Group, contains those compounds of Formula II and pharmaceutically acceptable salts of such compounds, as shown above, wherein $R^4$ is $-CH_2NR^9R^{10}$, and $R^{10}$ is hydrogen, methyl or $-COCH_3$.

A preferred group of compounds and pharmaceutically acceptable salts of such compounds, of the M Group, designated the N group, contains those compounds of Formula II and pharmaceutically acceptable salts of such compounds, as shown above, wherein $R^9$ is methyl, *n*-propyl, isopropyl, cyclopropyl, cyclobutyl, cyclopentyl, cyclohexyl, exo-2-norbornyl, $-CH_2$-4-fluorophenyl, $-CH_2$-4-chlorophenyl, $-CH_2$-4-isopropylphenyl, $-CH_2$-3,4-methylenedioxyphenyl, (R)-1-(1-naphthyl)ethyl, (R)-1-phenylethyl, (S)-1-phenylethyl, (R)-1-cyclohexylethyl, 1-(1,2,3,4-tetrahydronaphthyl), 1-indanyl or $-CH_2$-(1-naphthyl).

A preferred group of compounds and pharmaceutically acceptable salts of such compounds, of the M Group, designated the O group, contains those compounds of Formula II and pharmaceutically acceptable salts of such compounds, as shown above, wherein $R^9$ and $R^{10}$ are taken together with the nitrogen atom to which they are attached to form $N(CH_2)_6$, morpholine,

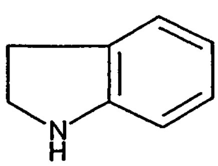, 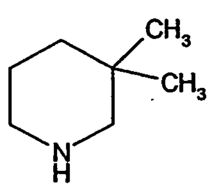, 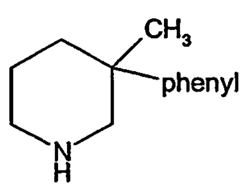, or 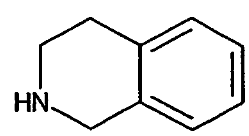

A preferred group of compounds and pharmaceutically acceptable salts of such compounds, of the D Group, designated the P Group, contains those compounds of Formula II and pharmaceutically acceptable salts of such compounds, as shown above, wherein $R^4$ is $-NHCOR^9$.

A preferred group of compounds and pharmaceutically acceptable salts of such compounds, of the P Group, designated the Q Group, contains those compounds of Formula II and pharmaceutically acceptable salts of such compounds, as shown above, wherein $R^9$ is cyclopropyl or cyclobutyl.

A preferred group of compounds and pharmaceutically acceptable salts of such compounds, of the D Group, designated the R Group, contains those compounds of Formula II and pharmaceutically acceptable salts of such compounds, as shown above, wherein $R^4$ is $-S(O)_2R^{12}$, and $R^{12}$ is 4-chlorophenyl, phenyl, 1-naphthyl, 2-naphthyl, $CH_2$-cyclopropyl, isopropyl, $CH_2$-cyclobutyl, $CH_2$-cyclohexyl, cyclopentyl, $CH_2$-4-fluorophenyl, 4-tolyl, methyl, ethyl, *n*-butyl, $CH_2$-phenyl or *n*-propyl.

Particularly preferred compounds of the R Group are compounds wherein (a) $R^1$ is chloro, $R^2$ is chloro, $R^8$ is

hydroxy or ethoxy, and $R^{12}$ is ethyl, (b) $R^1$ is chloro, $R^2$ is chloro, $R^8$ is hydroxy or ethoxy and $R^{12}$ is $-CH_2$-cyclobutyl, (c) $R^1$ is chloro, $R^2$ is chloro, $R^8$ is hydroxy or ethoxy and $R^{12}$ is -CH2-cyclohexyl, (d) $R^1$ is chloro, $R^2$ is chloro, $R^8$ is hydroxy or ethoxy and $R^{12}$ is cyclopentyl, (e) $R^1$ is chloro, $R^2$ is chloro, $R^8$ is hydroxy or ethoxy, and $R^{12}$ is $-CH_2$-cyclopropyl, (f) $R^1$ is chloro, $R^2$ is chloro, $R^8$ is hydroxy or ethoxy, and $R^{12}$ is $-CH_2$-cyclobutyl, and (g) $R^1$ is methyl, $R^2$ is methyl, $R^8$ is hydroxy or ethoxy, and $R^{12}$ is $-CH_2$-cyclopropyl; and pharmaceutically acceptable salts of said compounds.

A preferred group of compounds and pharmaceutically acceptable salts of such compounds, of the B Group, designated the S Group, contains those compounds of Formula II and pharmaceutically acceptable salts of such compounds, as shown above, wherein $R^1$ and $R^2$ are each independently methyl, bromo or chloro, $R^3$ is hydrogen, $R^4$ and $R^5$ are taken together to form

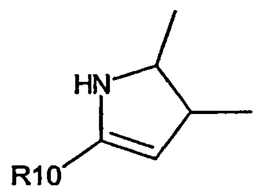

$R^6$ is hydrogen, $R^7$ is hydrogen, $R^8$ is ethoxy, hydroxy or $NH_2$, and $R^{10}$ is hydrogen or methyl.

A preferred group of compounds and pharmaceutically acceptable salts of such compounds, of the D Group, designated the T Group, contains those compounds of Formula II and pharmaceutically acceptable salts of such compounds, as shown above, wherein $R^3$ is hydrogen, and $R^4$ is $-OR^{11}$.

A preferred group of compounds and pharmaceutically acceptable salts of such compounds, of the T Group, designated the U Group, contains those compounds of Formula II and pharmaceutically acceptable salts of such compounds, as shown above, wherein $R^{11}$ is phenyl, 4-chlorophenyl or 4-fluorophenyl.

A preferred group of compounds and pharmaceutically acceptable salts of such compounds, of the D Group, designated the V Group, contains those compounds of Formula II and pharmaceutically acceptable salts of such compounds, as shown above, wherein $R^3$ is hydrogen, and $R^4$ is $-(C_{1-6}$ alkyl)$-OR^{11}$. Particularly preferred compounds of the V Group are compounds wherein $R^4$ is $-CH_2-OR^{11}$.

A preferred group of compounds and pharmaceutically acceptable salts of such compounds, of the V Group, designated the W Group, contains those compounds of Formula II and pharmaceutically acceptable salts of such compounds, as shown above, wherein $R^{11}$ is phenyl or 4-fluorophenyl.

A preferred group of compounds and pharmaceutically acceptable salts of such compounds, of the D Group, designated the X Group, contains those compounds of Formula II and pharmaceutically acceptable salts of such compounds, as shown above, wherein $R^3$ and $R^4$ are taken together to form a carbocyclic ring A of the formula $-(CH_2)_b-$ or a heterocyclic ring A selected from the group consisting of $-Q-(CH_2)_c$ and $-(CH_2)_j-Q-(CH_2)_k-$ wherein Q is O, S or $NR^{17}$, wherein said carbocyclic ring A and said heterocyclic ring A are each independently optionally substituted with one or more substituents independently selected from $C_{1-4}$ alkyl, halide or oxo.

A preferred group of compounds and pharmaceutically acceptable salts of such compounds, of the X Group, designated the Y Group, contains those compounds of Formula II and pharmaceutically acceptable salts of such compounds, as shown above, wherein $R^3$ and $R^4$ are taken together to form said carbocyclic ring A.

A preferred group of compounds and pharmaceutically acceptable salts of such compounds, of the Y Group, designated the Z Group, contains those compounds of Formula II and pharmaceutically acceptable salts of such compounds, as shown above, wherein $R^3$ and $R^4$ are taken together to form $-(CH_2)_3-$, $-CH_2-C(CH_3)_2-CH_2-$ or $-(CH_2)_4-$.

Particularly preferred compounds of the Z Group are compounds wherein (a) $R^1$ is methyl, $R^2$ is methyl, $R^8$ is hydroxy or ethoxy, and $R^3$ and $R^4$ are taken together to form $-(CH_2)_3-$, (b) $R^1$ is chloro, $R^2$ is methyl, $R^8$ is hydroxy or ethoxy, and $R^3$ and $R^4$ are taken together to form $-(CH_2)_3-$ and (c) $R^1$ is methyl, $R^2$ is methyl, $R^8$ is hydroxy or ethoxy, and $R^3$ and $R^4$ are taken together to form $-(CH_2)_4-$; and pharmaceutically acceptable salts of said compounds.

A preferred group of compounds and pharmaceutically acceptable salts of such compounds, designated the AA Group, contains those compounds of Formula II and pharmaceutically acceptable salts of such compounds, as shown above, wherein $R^8$ is $-OR^9$.

A preferred group of compounds and pharmaceutically acceptable salts of such compounds, of the AA Group, designated the AB Group, contains those compounds of Formula II and pharmaceutically acceptable salts of such compounds, as shown above, wherein $R^9$ is $C_{1-12}$ alkyl.

A preferred group of compounds and pharmaceutically acceptable salts of such compounds, of the AB Group, designated the AC Group, contains those compounds of Formula II and pharmaceutically acceptable salts of such

compounds, as shown above, wherein $R^9$ is methyl, isopropyl or ethyl.

A preferred group of compounds and pharmaceutically acceptable salts of such compounds, of the AC Group, designated the AD Group, contains those compounds of Formula II and pharmaceutically acceptable salts of such compounds, as shown above, wherein $R^9$ is ethyl.

A preferred group of the pharmaceutically acceptable salts of the compounds of Formula II, and the prodrugs, geometric and optical isomers thereof, contains those pharmaceutically acceptable salts of the compounds, prodrugs, and geometric and optical isomers wherein the salt is a potassium or a sodium salt.

A preferred group of compounds of Formula n, designated the AE Group, includes the specific compounds:

N-[3-chloro-4-(3-cylopropylsulfamoyl-4-hydroxy-phenoxy)-5-methyl-p henyl]- oxamic acid,

N-[4-(3-cyclopropylsulfamoyl-4-hydroxy-phenoxy)-3,5-dimethyl-phenyl ]-oxamic acid,

N-{4-[3-(cyclobutyl-methyl-carbamoyl)-4-hydroxy-phenoxy]-3,5-dimeth yl- phenyl}-oxamic acid,

N-{3-chloro-4-[3-(cyclobutyl-methyl-carbamoyl)-4-hydroxy-phenoxy]-5 - methyl-phenyl}-oxamic acid,

N-[4-(7-hydroxy-indan-4-yloxy)-3,5-dimethyl-phenyl]-oxamic acid,

N-(3,5-dichloro-4-[3-(cyclobutyl-methyl-carbamoyl)-4-hydroxy-phenoxy]-phenyl}-oxamic acid,

N-[3,5-dichloro-4-(3-cyclopentanesulfonyl-4-hydroxy-phenoxy)-phenyl]-oxamic acid,

N-[3,5-dichloro-4-(3-cyclopropylmethanesulfonyl-4-hydroxy-phenoxy)-phenyl]-oxamic acid,

N-[3,5-dichloro-4-(3-cyclobutylmethanesulfonyl-4-hydroxy-phenoxy)-phenyl]-oxamic acid,

N-[4-(3-cyclopropylmethanesulfonyl-4-hydroxy-phenoxy)-3,5-dimethylphenyl]-oxamic acid,

N-[3-chloro-4-(3-cyclobutylmethanesulfonyl-4-hydroxy-phenoxy)-5-methyl-phenyl]-oxamic acid,

N-[4-(3-cyclobutylmethanesulfonyl-4-hydroxy-phenoxy)-3,5-dimethylphenyl]-oxamic acid,

N-[4-(3-cyclopentylmethanesulfonyl-4-hydroxy-phenoxy)-3,5-dimethylphenyl]-oxamic acid,

N-[3-chloro-4-(3-cyclopentylmethanesulfonyl-4-hydroxy-phenoxy)-5-methyl-phenyl]-oxamic acid,

N-[3,5-dichloro-4-(3-cyclopentylmethanesulfonyl-4-hydroxy-phenoxy)-phenyl]-oxamic acid,

N-[4-(3-cyclohexylmethanesulfonyl-4-hydroxy-phenoxy)-3,5-dimethylphenyl]-oxamic acid,

N-[3-chloro-4-(3-cyclohexylmethanesulfonyl-4-hydroxy-phenoxy)-5-methyl-phenyl]-oxamic acid,

N-[3,5-dichloro-4-(3-cyclohexylmethanesulfonyl-4-hydroxy-phenoxy)-phenyl]-oxamic acid,

N-[3,5-dichloro-4-[3-(4-fluoro-benzenesulfonyl)-4-hydroxy-phenoxy)-phenyl]-oxamic acid,

N- {4-[3-(4-fluoro-benzenesulfonyl)-4-hydroxy-phenoxy]-3,5-dimethylphenyl}-oxamic acid,

N-{3-chloro-4-[3-(4-fluoro-benzenesulfonyl)-4-hydroxy-phenoxy]-5-methyl-phenyl}-oxamic acid, and the prodrugs and geometric and optical isomers thereof, and the pharmaceutically acceptable salts of the compounds, prodrugs and isomers.

A preferred group of the pharmaceutically acceptable salts of the compounds, prodrugs, and geometric and optical isomers of the AE Group, designated the AF Group, contains those pharmaceutically acceptable salts of the compounds, prodrugs, and geometric and optical isomers wherein the salt is a potassium or a sodium salt.

A preferred group of the compounds, and geometric and optical isomers thereof, of the compounds of the AE group, designated the AG Group, contains the ethyl esters of those compounds.

A preferred group of compounds and pharmaceutically acceptable salts of such compounds, of the B Group, designated the AH Group, contains those compounds of Formula II and pharmaceutically acceptable salts of such compounds, as shown above, wherein $R^5$ is fluoro.

A preferred group of compounds and pharmaceutically acceptable salts of such compounds, of the AH Group, designated the AI Group, contains those compounds of Formula II and pharmaceutically acceptable salts of such compounds, as shown above, wherein $R^4$ is hydrogen, fluoro, chloro, methyl or cyclobutyl-methyl-carbamoyl.

A preferred group of compounds and pharmaceutically acceptable salts of such compounds, of the AI Group, designated the AJ Group, contains those compounds of Formula II and pharmaceutically acceptable salts of such compounds, as shown above, wherein $R^1$ and $R^2$ are each independently methyl or chloro.

A preferred group of compounds and pharmaceutically acceptable salts of such compounds, of the AJ Group, designated the AK Group, contains those compounds of Formula II and pharmaceutically acceptable salts of such compounds, as shown above, wherein $R^1$ and $R^2$ are each methyl.

A preferred group of compounds and pharmaceutically acceptable salts of such compounds, of the AJ Group, designated the AL Group, contains those compounds of Formula II and pharmaceutically acceptable salts of such compounds, as shown above, wherein $R^1$ and $R^2$ are each chloro.

A preferred group of compounds and pharmaceutically acceptable salts of such compounds, of the AJ Group, designated the AM Group, contains those compounds of Formula II and pharmaceutically acceptable salts of such compounds, as shown above, wherein $R^7$ is hydrogen, and $R^8$ is hydrogen or $-OR^9$.

A preferred group of compounds and pharmaceutical acceptable salts of such compounds, of the AM Group, designated the AN Group, contains those compounds of Formula II and pharmaceutically acceptable salts of such compounds, as shown above, wherein $R^9$ is methyl or ethyl.

A preferred group of compounds of Formula II, designated the AO Group, includes the specific compounds:

N-[4-(4-Fluoro-phenoxy)-3,5-dimethyl-phenyl]-oxamic acid,

N-[3,5-Dichloro-4-(4-fluoro-phenoxy)-phenyl]-oxamic acid,

N-[3,5-Dichloro-4-(3,4-difluoro-phenoxy)-phenyl]-oxamic acid,

N-[4-(3-Methyl-4-Fluoro-phenoxy)-3,5-dichloro-phenyl]-oxamic acid,

N-[3,5-Dichloro-4-(3-chloro-4-fluoro-phenoxy)-phenyl]-oxamic acid,

N-[4-(3,4-Difluoro-phenoxy)-3,5-dimethyl-phenyl]-oxamic acid,

N-[4-(3-Chloro-4-fluoro-phenoxy)-3,5-dimethyl-phenyl]-oxamic acid,

N-[4-(3-Methyl-4-fluoro-phenoxy)-3,5-dimethyl-phenyl]-oxamic acid,

N-[3,5-Dichloro-4-(4-fluoro-phenoxy)-phenyl]-oxamic acid,

N-[3,5-Dichloro-4-(3,4-difluoro-phenoxy)-phenyl]-oxamic acid,

N-{4-[3-(Cyclobutyl-methyl-carbamoyl)-4-fluoro-phenoxy)-3,5-dimethyl - phenyl]-oxamic acid,

N-[4-(4-Fluoro-phenoxy)-3,5-dimethyl-phenyl]-oxamic acid, and the prodrugs and geometric and optical isomers thereof, and the pharmaceutically acceptable salts of the compounds, prodrugs and isomers.

A preferred group of the pharmaceutically acceptable salts of the compounds, prodrugs, and geometric and optical isomers of the AO Group, designated the AP Group, contains those pharmaceutically acceptable salts of the compounds, prodrugs, and geometric and optical isomers wherein the salt is a potassium or a sodium salt.

A preferred group of the compounds, and geometric and optical isomers thereof, of the compounds of the AO group, designated the AQ Group, contains the ethyl esters of those compounds.

Also preferred are those compounds disclosed in EP 580 550 and U. S. Patent No. 5,569,674 and 5,654,468 of formula III:

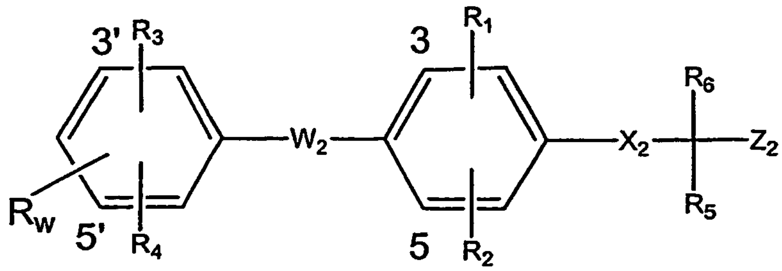

wherein $R_w$ is hydroxy, esterified hydroxy or etherified hydroxy;
$R_1$ is halogen, trifluoromethyl or lower alkyl;
$R_2$ is halogen, trifluoromethyl or lower alkyl;
$R_3$ is halogen, trifluoromethyl, lower alkyl, aryl, aryl-lower alkyl, cycloalkyl or cycloalkyl-lower alkyl; or
$R_3$ is the radical

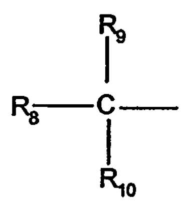

wherein $R^8$ is hydrogen, lower alkyl, aryl, cycloalkyl, aryl-lower alkyl or cycloalkyl-lower alkyl; $R_9$ is hydroxy or acyloxy; $R_{10}$ represents hydrogen or lower alkyl; or $R_9$ and $R_{10}$ together represent oxo;
$R_4$ is hydrogen, halogen, trifluoromethyl or lower alkyl;
$X_2$ is $-NR_7$;
$W_2$ is O or S;
$R_5$ and $R_6$ together represent oxo;
$R_7$ represents hydrogen or lower alkyl;
$Z_2$ represents carboxyl, carboxyl derivatized as a pharmaceutically acceptable ester or as a pharmaceutically acceptable amide; or a pharmaceutically acceptable salt thereof.

Listed below are definitions of various terms used to describe the thyromimetic compounds of the present invention. These definitions apply to the terms as they are used throughout the specification (unless they are otherwise limited in specific instances either individually or as part of a larger group).

The term "optionally substituted alkyl" refers to unsubstituted or substituted straight or branched chain hydrocarbon groups having 1 to 20 carbon atoms, preferably 1 to 7 carbon atoms. Exemplary unsubstituted alkyl groups include methyl, ethyl, propyl, isopropyl, n-butyl, *t*-butyl, isobutyl, pentyl, hexyl, isohexyl, heptyl, 4,4-dimethylpenthyl, octyl and the like. Substituted alkyl groups include, but are not limited to, alkyl groups substituted by one or more (e.g. two or three) of the following groups: halo, lower alkenyl, hydroxy, cycloalkyl, alkanoyl, alkoxy, alkyloxyalkoxy, alkanoyloxy, amino, alkylamino, dialkylamino, dialkylaminocarbonyl, alkanoylamino, thiol, alkylthio, alkylthiono, alkylsulfonyl, arylsulfonyl, heteroarylsulfonyl, sulfonamido, nitro, cyano, carboxy, alkoxycarbonyl, aryl, arakyl, aralkoxy, guanidino, heterocyclyl including indolyl, imidazolyl, furyl, thienyl, thiazolyl, pyrrolidyl, pyridyl, pyrimidyl, piperidyl, morpholinyl and the like. Preferred substituents of substituted alkyl, especially of substituted alkyl of variable R1 being substituted alkoxy, are lower alkyl, cycloalkyl, lower alkenyl, benzyl, mono or disubstituted lower alkyl, e.g. ω-(amino, mono- or di-lower alkylamino, carboxy, lower alkoxycarbonyl)-lower alkyl, □-(lower alkanoyloxy, lower alkoxycarbonyl or di-lower alkylaminocarbonyl)-lower alkyl, such as pivaloyloxy-methyl.

The term "lower alkyl" refers to those alkyl groups as described above having 1 to 7, preferably 1 to 4 carbon atoms.

The term "halogen" or "halo" refers to fluorine, chlorine, bromine and iodine.

The term "alkenyl" refers to any of the above alkyl groups having at least 2 carbon atoms and further containing at least one carbon to carbon double bond. Groups having two to four carbon atoms are preferred.

The term "alkylene" refers to a straight chain bridge of 1 to 6 carbon atoms connected by single bonds (e.g., $-(CH_2)_X-$ wherein x is 1 to 6), which may be substituted with 1 to 3 lower alkyl groups.
The term "cycloalkyl" refers to cyclic hydrocarbon groups of 3 to 8 carbon atoms.
The term "alkoxy" refers to alkyl-O-.
The term "acyl" refers to alkanoyl, aroyl, heteroaroyl, arylalkanoyl or heteroarylalkanoyl.
The term "alkanoyl" refers to alkyl-C(O)-.
The term "alkanoyloxy" refers to alkyl-C(O)-O-.
The terms "alkylamino" and "dialkylamino" refer to (alkyl)NH- and $(alkyl)_2N-$, respectively.
The term "alkanoylamino" refers to alkyl-C(O)-NH-.
The term "alkylthio" refers to alkyl-S-.
The term "alkylthiono" refers to alkyl-S(O)-.
The term "alkylsulfonyl" refers to alkyl-$S(O)_2$-.
The term "alkoxycarbonyl" refers to alkyl-O-C(O)-.
The term "alkoxycarbonyloxy" refers to alkyl-O-C(O)O-.
The term "alkyl" as referred to in the above definitions relates to optionally substituted alkyl as defined above.
The term "aryl" refers to monocyclic or bicyclic aromatic hydrocarbon groups having 6 to 12 carbon atoms in the ring portion, such as phenyl, naphthyl, tetrahydronaphthyl, and biphenyl groups, each of which may optionally be substituted by one to four substituents such as alkyl, halo, hydroxy, alkoxy, alkanoyl, alkanoyloxy, amino, alkylamino, dialkylamino, alkanoyl-amino, thiol, alkylthio, nitro, cyano, carboxy, carboxyalkyl, alkoxycarbonyl, alkyl-thiono, alkylsulfonyl, sulfonamido, heterocyclyl and the like.
The term "monocyclic aryl" refers to optionally substituted phenyl as described under aryl.
The term "aralkyl" refers to an aryl group bonded directly through an alkyl group, such as benzyl.
The term "aralkoxy" refers to an aryl group bonded through an alkoxy group.
The term "arylsulfonyl" refers to aryl-$S(O)_2$-.
The term "aroyl" refers to aryl-C(O)-.
The term "heterocyclyl" refers to an optionally substituted, fully saturated or unsaturated, aromatic or nonaromatic cyclic group, for example, which is a 4 to 7 membered monocyclic, 7 to 11 membered bicyclic, or 10 to 15 membered tricyclic ring system, which has at least one heteroatom in at least one carbon atom-containing ring. Each ring of the heterocyclic group containing a heteroatom may have 1, 2 or 3 heteroatoms selected from nitrogen atoms, oxygen atoms and sulfur atoms, where the nitrogen and sulfur heteroatoms may also optionally be oxidized. The heterocyclic group may be attached at a heteroatom or carbon atom.
Exemplary monocyclic heterocyclic groups include pyrrolidinyl, pyrrolyl, pyrazolyl, oxetanyl, pyrazolinyl, imidazolyl, imidazolinyl, imidazolidinyl, oxazolyl, oxazolidinyl, isoxazolinyl, isoxazolyl, thiazolyl, thiadiazolyl, thiazolidinyl, isothiazolyl, isothiazolidinyl, furyl, tetrahydrofuryl, thienyl, oxadiazolyl, piperidinyl, piperazinyl, 2-oxopiperazinyl, 2-oxopiperidinyl, 2-oxopyrrolodinyl, 2-oxoazepinyl, azepinyl, 4-piperidonyl, pyridyl, 2-pyridone, N-lower alkyl-pyridone, e.g. N-lower alkyl-2-pyridone, pyrazinyl, pyrimidinyl, pyridazinyl, tetrahydropyranyl, morpholinyl, thiamorpholinyl, S-oxo-thiamorpholinyl S,S-dioxo-thiamorpholinyl, 1,3-dioxolane and tetrahydro-1,1-dioxothienyl, and the like.
Exemplary bicyclic heterocyclic groups include indolyl, benzothiazolyl, benzoxazolyl, benzothienyl, quinuclidinyl, quinolinyl, tetrahydroisoquinolinyl, isoquinolinyl, benzimidazolyl, benzopyranyl, indolizinyl, benzofuryl, chromonyl, coumarinyl, benzopyranyl, cinnolinyl, quinoxalinyl, indazolyl, pyrrolopyridyl, furopyridinyl (such as furo[2,3-c]pyridinyl, furo[3,2-b]-pyridinyl] or furo[2,3-b]pyridinyl), dihydroisoindolyl, dihydroquinazolinyl (such as 3,4-dihydro-4-oxo-quinazolinyl) and the like.
Exemplary tricyclic heterocyclic groups include carbazolyl, benzindolyl, phenanthrolinyl, acridinyl, phenanthridinyl, xanthenyl and the like.
The term "heterocyclyl" includes substituted heterocyclic groups. Substituted heterocyclic groups refer to heterocyclic groups substituted with 1, 2 or 3 of the following:

(a) alkyl;
(b) hydroxy (or protected hydroxy);
(c) halo;
(d) oxo (i.e. = O);
(e) amino, alkylamino or dialkylamino;
(f) alkoxy;
(g) cycloalkyl;
(h) carboxy;
(i) heterocyclooxy;
(j) alkoxycarbonyl, such as unsubstituted lower alkoxycarbonyl;
(k) mercapto;

(l) nitro;
(m) cyano;
(n) sulfonamido, sulfonamidoalkyl or sulfonamidodialkyl;
(o) aryl;
(p) alkylcarbonyloxy;
(q) arylcarbonyloxy;
(r) arylthio;
(s) aryloxy;
(t) alkylthio;
(u) formyl;
(v) aralkyl; or
(w) aryl substituted with alkyl, cycloalkyl, alkoxy, hydroxy, amino, alkylamino, dialkylamino or halo.

The term "heterocyclooxy" denotes a heterocyclic group bonded through an oxygen bride.

The term "heteroaryl" refers to an aromatic heterocycle, for example monocyclic or bicyclic aryl, such as pyrrolyl, pyrazolyl, imidazolyl, oxazolyl, isoxazolyl, thiazolyl, isothiazolyl, furyl, thienyl, pyridyl, pyrazinyl, pyrimidinyl, pyridazinyl, indolyl, benzothiazolyl, benzoxazolyl, benzothienyl, quinolinyl, isoquinolinyl, benzimidazolyl, benzofuryl, and the like, optionally substituted by one or more substituents as described in connection with substituted aryl, e.g. by lower alkyl, lower alkoxy or halo.

The term "heteraryloxy" refers to heteroaryl-O-.

The term "heteroarylsulfonyl" refers to heteroaryl-$S(O)_2$-.

The term "heteroaroyl" refers to heteroaryl-C(O)-.

The term "heteroarallcyl" refer to a heteroaryl group bonded through an alkyl group.

Encompassed by the invention are prodrug derivatives, e.g., any pharmaceutically acceptable prodrug ester derivatives of the carboxylic acids of the invention ($COR_x$ being carboxy) which are convertible by solvolysis or under physiological conditions to the free carboxylic acids. Examples of such carboxylic acid esters include esters defined by $COR_x$, and are preferably lower alkyl esters, cycloalkyl esters, lower alkenyl esters, benzyl esters, mono or disubstituted lower alkyl esters, e.g. the ω-(amino, mono- or di-lower alkylamino, carboxy, lower alkoxycarbonyl)-lower alkyl esters, the α-, β-, γ-, or δ-(lower alkanoyloxy, lower alkoxycarbonyl or di-lower alkylaminocarbonyl)-lower alkyl esters, such as the pivaloyloxy-methyl ester, and the like conventionally used in the art.

The thyromimetic compounds of the invention depending on the nature of the substituents, may possess one or more asymmetric centers. The resulting diastereoisomers, enantiomers and geometric isomers are encompassed by the instant invention.

Pharmaceutically acceptable salts of any acidic compounds of the invention are salts formed with bases, namely cationic salts such as alkali and alkaline earth metal salts, such as sodium, lithium, potassium, calcium, magnesium, as well as ammonium salts, such as ammonium, trimethylammonium, diethylammonium, and tris-(hydroxymethyl)-methylammonium salts.

The compounds described above may be prepared and administered in accordance with the methods set forth in WO 00/58279. Similarly acid addition salts, such as of mineral acids, organic carboxylic, and organic sulfonic acids e.g. hydrochloric acid, methanesulfonic acid, maleic acid, are possible provided a basic group, such as pyridyl, constitutes part of the structure.

**Conjugation, Cyclization and Other Modifications of the Peptides**

The invention contemplates conjugation of the peptides to other entities and cyclization of peptides using amino acids or other moieties that are capable of forming covalent linkages without interruption of peptide linkages.

Amino acids can be conjugated to other entities and/or peptides cyclized by any method available to one of skill in the art. For example, functional groups present on the side chains of amino acids in the peptides can be combined with functional groups in the entity to which the peptide is to conjugated. Functional groups that can form covalent bonds include --COOH and --OH; -COOH and -$NH_2$; and --COOH and --SH. Pairs of amino acids that can be used to conjugate proteins to the present peptides or to cyclize a peptide include, Asp and Lys; Glu and Lys; Asp and Arg; Glu and Arg; Asp and Ser; Glu and Ser; Asp and Thr; Glu and Thr; Asp and Cys; and Glu and Cys. Other examples of amino acid residues that are capable of forming covalent linkages with one another include cysteine-like amino acids such Cys, hCys, β-methyl-Cys and Pen, which can form disulfide bridges with one another. Preferred cysteine-like amino acid residues include Cys and Pen. Other pairs of amino acids that can be used for conjugation and cyclization of the peptide will be apparent to those skilled in the art.

The groups used to conjugate or cyclize a peptide need not be amino acids. Examples of functional groups capable of forming a covalent linkage with the amino terminus of a peptide include carboxylic acids and esters. Examples

of functional groups capable of forming a covalent linkage with the carboxyl terminus of a peptide include --OH, --SH, $--NH_2$ and --NHR where R is ($C_1$ - $C_6$) alkyl, ($C_1$ - $C_6$) alkenyl and ($C_1$ - $C_6$) alkynyl.

The variety of reactions between two side chains with functional groups suitable for forming such interlinkages, as well as reaction conditions suitable for forming such interlinkages, will be apparent to those of skill in the art. Preferably, the reaction conditions used to conjugate or cyclize the peptides are sufficiently mild so as not to degrade or otherwise damage the peptide. Suitable groups for protecting the various functionalities as necessary are well known in the art (see, e.g., Greene & Wuts, 1991, 2nd ed., John Wiley & Sons, NY), as are various reaction schemes for preparing such protected molecules.

Peptide conjugates and cyclic peptides as well as methods for preparing such peptides are well-known in the art (see, e.g., Spatola, 1983, Vega Data 1(3) for a general review); Spatola, 1983, "Peptide Backbone Modifications" In: Chemistry and Biochemistry of Amino Acids Peptides and Proteins (Weinstein, ed.), Marcel Dekker, New York, p. 267 (general review); Morley, 1980, Trends Pharm. Sci. 1:463-468; Hudson et al., 1979, Int. J. Prot. Res. 14:177-185 ($--CH_2$ NH--, $--CH_2$ $CH_2$ --); Spatola et al., 1986, Life Sci. 38:1243-1249 ($--CH_2$ --S); Hann, 1982, J. Chem. Soc. Perkin Trans. I. 1:307-314 (--CH = CH--, cis and trans); Almquist et al., 1980, J. Med. Chem. 23:1392-1398 (--CO $CH_2$ --); Jennings-White et al., Tetrahedron. Lett. 23:2533 (--CO $CH_2$ --); European Patent Application EP 45665 (1982) CA:97: 39405 (--CH(OH) $CH_2$ --); Holladay et al., 1983, Tetrahedron Lett. 24:4401-4404 ($--C(OH)CH_2--$); and Hruby, 1982, Life Sci. 31:189-199 ($--CH_2$ --S--).

**Target Molecules**

Peptides of the invention can bind any biomolecule, tissue or protein of interest. Such biomolecules or proteins of interest may be involved in lipid transport or cellular uptake e.g. apolipoprotein (a, AI, AII, AIV, B, CI, CII, CIII or E), low density lipoprotein receptor (LDL-R), cholesterol ester transfer protein, hepatic TG lipase, lipoprotein lipase, high density lipoprotein receptor p110, LDL receptor like protein, ARP1, LDL-R protein kinase, apolipoprotein E receptor or oncostatin M. The biomolecule may be involved in the uptake of modified lipoproteins e.g. LDL-R, scavenger receptor, advanced glycosylated end-product receptor or macrophage FC receptor. The biomolecule may be involved in lipid metabolism e.g. AMP-activated protein kinase, AMP-activated protein kinase kinase, acetyl CoA cholesterol ester transferase, lecithin-cholesterol ester transferase, cholesterol 7α-hydroxylase, hormone sensitive-lipase/cholesterol ester hydroxylase or HMG CoA reductase. The biomolecule may be involved in lipid oxidation e.g. 15-lipoxygenase, IL-4, IL-4 receptor, superoxide dismutase or 12 lipoxygenase. The biomolecule may be involved in smooth muscle cell growth such as platelet derived growth factor (PDGF-A), PDGF-B, PDGF-α receptor, PDGF-β receptor, heparin-binding EGF-like growth factor, basic fibroblast growth factor (bFGF), aFGF, FGF receptor, IL-1, IL-1 receptor p80, IL-1 receptor protein kinase, interferon gamma, TGF-β1, TGF-β2, TGF-β3, TGF receptor, tumor necrosis factor-.alpha. (TNF-α), TNF-α receptor, α-thrombin, α-thrombin receptor, 9-hydroxyoctadeca-10,12-dienoic acid (9-HODE) receptor, insulin-like growth factor, platelet factor-4, TGF-.alpha., thromboxane $A_{.2}$ receptor, 12-hydroxy-5,8,10,14-eicosatetraenoic acid (12-HETE) receptor, 13-hydoxyoctadeca-9,11-dienoic acid (13-HODE) receptor, IL6, IL-6 receptor or EGF receptor. The biomolecule may be an endothelial cell growth factor or receptor (EGF) such as vascular EGF, VEGF receptor, bFGF, aFGF, FGF receptor or platelet-derived endothelial cell growth factor. The biomolecule may be associated with macrophage growth and chemotaxis e.g. CSF-1, CSF-1 receptor, monocyte chemoattractant protein-1 (MCP-1) or MCP-1 receptor. The biomolecule associated with atherosclerosis may be associated with endothelial cell adhesion such as VCAM-1, VLA-4 $\alpha_4$ subunit, VLA-4 $\beta_1$ subunit, ELAM-1, ICAM-1, LFA-1 $\alpha_L$ subunit, LFA-1 $\beta_2$ subunit, GMP-140 (PADGEM), neuropeptide Y, VLA-4 $\alpha_1$ subunit, vitronectin receptor or 13-hydoxyoctadeca-9,11-dienoic acid (13-HODE) receptor. The biomolecule associated with a peptide of the invention may also be phosphoenolpyruvate carboxykinase (PEPCK).

In one embodiment the biomolecule is an 82 kilodalton protein that binds to the CAPGPSKSC (SEQ ID NO:4) peptide. This 82 kilodalton protein is referred to herein as the P82 protein. It may be a membrane protein. In another the biomolecule is an 120 kilodalton protein that binds to the CAPGPSKSC (SEQ ID NO:4) peptide. This 120 kilodalton protein is referred to herein as the P120 protein.

Peptides of the invention can bind to any target. Peptides conjugated to reporter molecules can be used *in vivo* to locate, image or otherwise identify where such targets are present in mammals and to ascertain what biological processes, physiological structures, intracellular structures, diseases, and the like with which the target is associated. Peptides conjugated to therapeutic agents can be used *in vivo* to treat any site where such targets are located within a mammal.

**Therapeutic Methods**

The peptides of the invention can be used in any therapeutic procedure available to one of skill in the art to treat any disease or physiological problem with which a target molecule bound by of a peptide of the invention is

associated. An association with a disease or physiological problem means that the target molecule is localized at the site of the disease or physiological problem. The target molecule may be directly or indirectly involved in the disease or the physiological problem. For example, the target molecule can have an activity or structure that contributes to the disease or the physiological problem. However, the target molecule may also be merely localized to the region of a disease or physiological problem and may not be a direct or indirect cause of the disease or physiological problem. Thus, the target molecule may have no obvious involvement in the pathogenesis of the disease or the physiological problem, but rather have the potential to modify cell function and thereby minimize the pathogenetic process.

Any disease or physiological problem associated with a target molecule or cell bound by of a peptide of the invention can be treated with the present peptides through conjugation of the peptides to one or more therapeutic agents. For example, peptides conjugated to therapeutic agents can be used to treat stroke, atherosclerosis, peripheral arterial diseases, peripheral limb disease, acute coronary syndromes including unstable angina, thrombosis and myocardial infarction, plaque rupture, both primary and secondary (in-stent) restenosis in coronary or peripheral arteries, vein graft stenosis, transplantation-induced sclerosis, reperfusion injuries, ischemic vascular diseases, myocardial ischemia, intermittent claudication and diabetic complications (including ischemic heart disease, peripheral artery disease, congestive heart failure, retinopathy, neuropathy and nephropathy), or thrombosis. This invention also can be used to treat bypass grafts or access port stenosis (e.g. dialysis access ports) in any vascular location. The methods and peptides of the invention can be used to stabilize atherosclerotic plaques at risk of rupture or other clinical events.

To treat these diseases and physiological conditions, peptides are conjugated to therapeutic agents using procedures provided herein or any other method known to one of skill in the art. Such peptide conjugates are then administered to a mammal in a therapeutically effective amount. Peptide conjugates can be administered for a time sufficient to beneficially treat the disease or physiological condition.

For example, the invention makes available a method of treating atherosclerosis in a mammal that includes administering a therapeutically effective amount of a peptide conjugated to a therapeutic agent, wherein the peptide can bind to an atherosclerotic lesion in the mammal and the therapeutic agent can beneficially treat atherosclerosis. Such therapeutic agents include, for example, thrombolytic agents such as streptokinase, tissue plasminogen activator, plasmin and urokinase, anti-thrombotic agents such as tissue factor protease inhibitors (TFPI), nematode-extracted anticoagulant proteins (NAPs) and the like, metalloproteinase inhibitors, anti-inflammatory agents or liposomes that contain thrombolytic agents such as streptokinase, tissue plasminogen activator, plasmin and urokinase, anti-thrombotic agents such as tissue factor protease inhibitors (TFPI), nematode-extracted anticoagulant proteins (NAPs) and the like, metalloproteinase inhibitors, or anti-inflammatory agents.

In another embodiment, the invention makes available a method of preventing heart attack in a mammal that includes administering a therapeutically effective amount of a peptide conjugated to a therapeutic agent, wherein the peptide can bind to an atherosclerotic lesion in the mammal and the therapeutic agent can help prevent heart attack. Such therapeutic agents include, for example, thrombolytic agents such as streptokinase, tissue plasminogen activator, plasmin and urokinase, anti-thrombotic agents such as tissue factor protease inhibitors (TFPI), nematode-extracted anticoagulant proteins (NAPs) and the like, metalloproteinase inhibitors, anti-inflammatory agents or liposomes that contain thrombolytic agents such as streptokinase, tissue plasminogen activator, plasmin and urokinase, anti-thrombotic agents such as tissue factor protease inhibitors (TFPI), nematode-extracted anticoagulant proteins (NAPs) and the like, metalloproteinase inhibitors, or anti-inflammatory agents.

The peptides and peptide conjugates of the invention can be formulated as pharmaceutical compositions and administered to a mammalian host, such as a human patient in a variety of dosage forms adapted to the chosen route of administration, i.e., orally or parenterally, by intravenous, intramuscular, topical or subcutaneous routes.

Thus, the peptides and peptide conjugates may be systemically administered, for example, intravenously or intraperitoneally by infusion or injection. Solutions of the peptide or peptide conjugate can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

The pharmaceutical dosage forms suitable for injection or infusion can include sterile aqueous solutions or dispersions or sterile powders comprising the active ingredient(s) that are adapted for the extemporaneous preparation of sterile injectable or infusible solutions or dispersions, optionally encapsulated in liposomes. In all cases, the ultimate dosage form must be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about

by the use in the compositions of agents delaying absorption, for example, aluminum monostearate and gelatin.

Sterile injectable solutions are prepared by incorporating the peptide or peptide conjugate in the required amount in the appropriate solvent with various of the other ingredients enumerated above, as required, followed by filter sterilization. In the case of sterile powders for the preparation of sterile injectable solutions, the preferred methods for preparation of such powders are vacuum drying and freeze drying techniques, which yield a powder of the active ingredient plus any additional desired ingredient present in the previously sterile-filtered solutions.

In some instances, the peptides and peptide conjugates can also be administered orally, in combination with a pharmaceutically acceptable vehicle such as an inert diluent or an assimilable edible carrier. The peptides may be enclosed in hard or soft shell gelatin capsules, may be compressed into tablets, or may be incorporated directly with the food of the patient's diet. For oral therapeutic administration, the peptide or peptide conjugate may be combined with one or more excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% to about 90% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices.

Useful solid carriers include finely divided solids such as talc, clay, microcrystalline cellulose, silica, alumina and the like. Useful liquid carriers include water, alcohols or glycols or water-alcohol/glycol blends, in which the present compounds can be dissolved or dispersed at effective levels, optionally with the aid of non-toxic surfactants. Adjuvants such as fragrances and additional antimicrobial agents can be added to optimize the properties for a given use.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Useful dosages of the peptides and peptide conjugates can be determined by correlating their *in vitro* activity, and *in vivo* activity in animal models described herein.

The therapeutically effective amount of peptide or peptide conjugate necessarily varies with the subject and the disease or physiological problem to be treated. For example, a therapeutic amount between 30 to 112,000 μg per kg of body weight can be effective for intravenous administration. As one skilled in the art would recognize, the amount can be varied depending on the method of administration. The amount of the peptide or peptide conjugate, required for use in treatment will vary not with the route of administration, but also the nature of the condition being treated and the age and condition of the patient and will be ultimately at the discretion of the attendant physician or clinician.

The compound can conveniently be administered in unit dosage form; for example, containing 1 to 1000 mg, conveniently 10 to 750 mg, most conveniently, 20 to 500 mg of peptide or peptide conjugate per unit dosage form.

Ideally, the active ingredient should be administered to achieve peak plasma concentrations of the active compound of from about 0.1 to about 75 μM, preferably, about 1 to 50 μM, most preferably, about 2 to about 30 μM. This may be achieved, for example, by the intravenous injection of a 0.05 to 5% solution of the active ingredient, optionally in saline, or orally administered as a bolus containing about 1-100 mg of the active ingredient. Desirable blood levels may be maintained by continuous infusion to provide about 0.01-5.0 mg/kg/hr or by intermittent infusions containing about 0.4-15 mg/kg of the active ingredient(s).

The desired dose may conveniently be presented in a single dose, as divided doses, or as a continuous infusion. The desired dose can also be administered at appropriate intervals, for example, as two, three, four or more sub-doses per day.

## Methods for Isolating Peptides

The invention further contemplates *in vivo* methods of identifying and/or isolating peptides that can bind to a biomolecule of interest. Such methods involve contacting the biomolecule *in vivo* with a phage display library and isolating

phage that selectively adhere to the biomolecule. See Pasqualini et al., 380 Nature 364-66 (1996).

The methods are performed *in vivo* by placing the phage display library within a suitable animal in a manner that permits the phage to come into contact with the biomolecule. For example, the phage can be injected into the tissues of an animal within the general region of the biomolecule. Preferably, the phage display library is placed or injected into a cavity or vessel of the animal. Such cavities or vessels preferably are filled or partially filled with fluids that permit diffusion and/or circulation of the phage. For example, phage libraries can be injected or placed within the vascular system, digestive system, lymph system, spinal fluid and the like.

In one embodiment, the invention makes available a method of identifying a peptide capable of binding to mammalian vascular tissues that includes circulating a phage display library through the vascular tissues of a mammal. Phage that selectively adhere to the vascular tissues of the mammal can be isolated and the peptides displayed on the phage can be identified. Such methods are useful when identifying peptides that are capable of binding to the vascular tissues of the mammal, or to a biomolecule present in discrete portions of to the vascular tissues of the mammal.

In many instances, the tissues or biomolecules can more advantageously be contacted and saturated with wild type phage identical to those used for the phage display library, but without peptide inserts. Such exposure blocks or saturates the tissue or biomolecules with wild type phage so that, when the tissue or biomolecules are contacted with phage displaying peptides, any phage bound will likely bind through the displayed peptide. Therefore, exposure to phage without peptide inserts can, for example, saturate the reticuloendothelial system of the animal, and diminish non-specific phage binding.

Prior to injection or exposure to the phage display library, the tissues or biomolecules of the animal are exposed to a sufficient number of wild type phage for a sufficient time to permit binding of wild type phage to all sites that have a propensity to bind such wild type phage.

A sufficient number of wild type phage to saturate all sites in the tissue or biomolecules of the animal that have a propensity to bind such wild type phage can be determined by one of skill in the art. Such a number of wild type phage depends on the size of the animal, the time for exposure to the phage and the complexity of biomolecules within the animal. For example, when mice are used as the experimental animal, about $10^3$ pfu to about $10^{13}$ pfu of wild type helper phage can be injected into the mice. Alternatively, about $10^6$ pfu to about $10^{12}$ pfu, about $10^9$ pfu to about $10^{12}$ pfu, or about $10^{11}$ pfu to about $10^{12}$ pfu of wild type helper phage are injected into the mice.

A sufficient time to permit binding of wild type phage to all sites that have a propensity to bind such wild type phage and then binding of phage display libraries can also be determined by one of skill in the art. Such a time depends on the size of the animal and the complexity of biomolecules within the animal. For example, when mice are used as the experimental animal, about ten to about sixty minutes can be used. Preferably, the time to permit binding of wild type phage to all sites that have a propensity to bind such wild type phage, is about fifteen to about forty five minutes. In one embodiment $10^{12}$ pfu of wild type helper phage were injected into the atherosclerotic mice and were allowed to circulate for about thirty minutes. Such exposure was sufficient to diminish non-specific phage binding and saturate the reticuloendothelial system of the mice. Injection of wild type phage can be performed just prior to injection of the phage display library.

After injection or exposure to wild type phage, the tissues or biomolecules of the mammal are exposed to a sufficient number of the library phage or peptides for a sufficient time to permit binding of displayed peptides to biomolecules. A sufficient number of library phage to permit binding to biomolecules depends on the size of the animal and the complexity of phage in the library within the animal. In general, a selected library of phage will have less complexity than a library of all possible peptide sequences. Lower numbers of phage from such a selected library can be used. For example, when mice are used as the experimental animal, about $10^3$ pfu to about $10^{13}$ pfu of library phage can be injected into the mice. Preferably, about $10^{10}$ pfu to about $10^{12}$ pfu of unselected library phage are injected into the mice initially. After selection of phage bound to the tissue or biomolecule of interest, about $10^3$ pfu to about $10^7$ pfu from a preselected library can be injected for a subsequent round of selection.

A sufficient time to permit binding of library phage to biomolecules can be determined by one of skill in the art. Such a time depends on the size of the animal and the complexity of library. For example, when mice are used as the experimental animal, about 10 to about 120 minutes can be used. Preferably, the time to permit binding of library phage to biomolecules is about fifteen to about sixty minutes.

The methods of the invention for identifying peptides can include several rounds of selection for adherence to the biomolecule, lesion or tissue of interest. For example, after isolating phage that selectively adhere to the biomolecule, lesion or tissue of interest, those phage can be replicated and again contacted with the biomolecule or tissue of interest. Preferably, the phage are minimally amplified to minimize the possibility of genetic selection during amplification that may lead to loss or mutation of the peptide. These methods provide a population of phage that selectively adhere the biomolecule or tissue of interest. After contacting this population with the biomolecule or tissue *in vivo*, one or more second selected phage are isolated. When one of skill in the art has performed sufficient rounds of selection, the peptide displayed on the selected phage can be identified and further characterized or tested to evaluate the peptide for its capability for binding to the biomolecule or tissues of interest.

In some instances, the methods of the invention are used to identify peptides that bind to a particular biomolecule. In other instances, the methods of the invention are used to identify peptides that bind to a particular tissue or a local lesion or site of pathology, but the biomolecule to which the peptides bind is not known. The biomolecule to which the peptide binds can be identified by methods readily available to one of skill in the art. For example, a biomolecule within a tissue bound by a peptide can be identified by first homogenizing the tissue to create a mixture of biomolecules. Such biomolecules can be separated and then contacted by a selected peptide. The peptides will then bind to the biomolecule to which is adhered when the peptide bound to the tissue and the biomolecule can be identified and charecterized.

Hence, in one embodiment, the invention makes available a method of identifying a protein or other biomolecule bound by a peptide that is capable of binding to the vascular tissues of a mammal. This method involves separating a mixture of proteins prepared from the vascular tissues of a mammal and contacting the separated mixture of proteins with a peptide that is capable of binding to the vascular tissues of the mammal. The protein that binds to peptide is then identified, and can be characterized.

The invention has particular utility for identifying and isolating peptides that bind to atherosclerotic lesions and biomolecules that are uniquely expressed on the surface of these lesions. Such methods involve circulating a phage display library through the vascular tissues of a mammal and isolating a phage that selectively adheres to atherosclerotic lesions in the mammal. The peptide displayed on the phage is then identified. As described above, further rounds of selection for adhesion to atherosclerotic lesions can be performed to insure that the peptide is capable of specifically binding to only atherosclerotic lesions or to particular types of atherosclerotic lesions in the mammal.

The protein or biomolecule within an atherosclerotic lesion that is bound by the peptide can also be identified. For example, a mixture of proteins or biomolecules prepared from atherosclerotic lesions of a mammal can be separated. The separated mixture of proteins or biomolecules can then be contacted with a peptide that is capable of binding to the atherosclerotic lesions in the mammal. The protein or biomolecule that binds the peptide is identified.

A peptide within a phage that selectively binds to a tissue or biomolecule of interest can be identified and/or isolated by dissecting out the tissue of interest or the tissue containing the bound phage. Such dissected tissue can be washed to remove phage that are non-specifically bound. To isolate phage displaying a selectively bound peptide, the phage that remain associated with the tissue of interest are eluted. Such bound phage can be amplified and titered. After the desired rounds of selections are performed, the phage can be plated out and individual clones can be selected, recloned and the peptide inserts sequenced.

The potential significance of an isolated peptide, the ability of the phage displaying that peptide to localize *in vivo* can be assessed by injection of the cloned phage, sometimes mixed with wild type, into an appropriate animal model, such as mice, rats and later, rodents bearing human skin grafts with human vasculature. About 10 to about 60 min after injection, the animals were euthanized, the vasculature perfused, and the target and control tissues recovered for analysis by: (1) recovery of phage and determining the ratio of specific phage to wild type control phage by blue/ white colony counts; and (2) confocal microscopy of cryostat sections of frozen tissue blocks to determine that the specific phage associates with endothelium and to determine the specific pattern of localization in the vasculature (e.g. capillaries vs. venules vs arterioles).

In one series of experiments, one hundred thirty individual phage clones were isolated and sequenced using these procedures. Two clones occurred twice and two additional clones occurred three times. Ninety-three independent sequence "sets" have been defined. The total number of recovered phage clones from three cycles of *in vivo* selection is about several hundred, indicating that a relatively large number of initial candidates can readily be obtained with these methods.

Peptide sequences isolated by the present methods can be scrutinized for homologies to known peptide and protein sequences. Database searches for common or similar protein domains are within the ken of one of skill in the art.

## Animals

Any animal containing a tissue of interest or a tissue containing a biomolecule of interest can be used for the *in vivo* selection procedures of the invention. Such animals include mice, rats, rabbits, cats, dogs, goats, horses, pigeons and the like. In some instances, animals of a particular genotype and/or animals that exhibit a particular phenotype are used. One of skill in the art can readily identify the appropriate animal for use in the present methods. For example, for identification of peptides that bind to atherosclerotic lesions, hypercholesterolemic animals that are accepted models for atherosclerosis can be used. Such animals include: Apo E mice, LDL-R deficient mice, combined Apo E/LDL-R deficient mice, Watanabe rabbits, and pigeons.

## Phage Display Libraries

While the invention contemplates identification of peptides capable of binding to biomolecules by any mechanism, one method involves the use of phage display libraries. Any available phage display library can be used.

For example, filamentous bacteriophages can be used to express a library of peptides on the surface of the bacteriophage. Filamentous bacteriophages are a group of related viruses that infect bacteria. They are termed filamentous because they are long and thin particles comprised of an elongated capsule that envelopes the deoxyribonucleic acid (DNA) that forms the bacteriophage genome. The F pili filamentous bacteriophage (Ff phage) infect only gram-negative bacteria by specifically adsorbing to the tip of F pili, and include fd, fl and M13.

The mature capsule of Ff phage is comprised of a coat of five phage-encoded gene products: cpVIII, the major coat protein product of gene VIII that forms the bulk of the capsule; and four minor coat proteins, cpIII and cpIV at one end of the capsule and cpVII and cpIX at the other end of the capsule. The length of the capsule is formed by 2500 to 3000 copies of cpVIII in an ordered helix array that forms the characteristic filament structure. About five copies each of the minor coat proteins are present at the ends of the capsule. The gene III-encoded protein (cpIII) is typically present in 4 to 6 copies at one end of the capsule and serves as the receptor for binding of the phage to its bacterial host in the initial phase of infection. For detailed reviews of Ff phage structure, see Rasched et al., Microbiol. Rev., 50:401-427 (1986); and Model et al., in "The Bacteriophages, Volume 2", R. Calendar, Ed., Plenum Press, pp. 375-456 (1988). One of skill in the art can fuse a combinatorial peptide library to any of these proteins to create a phage display library.

However, one of skill may prefer to choose to fuse peptide libraries with the cpVIII or cpIII proteins. No phage particles are assembled within a host cell; rather, they are assembled during extrusion of the viral genome through the host cell's membrane. Prior to extrusion, the major coat protein cpVIII and the minor coat protein cpIII are synthesized and transported to the host cell's membrane. Both cpVIII and cpIII are anchored in the host cell membrane prior to their incorporation into the mature particle. In addition, the viral genome is produced and coated with cpV protein. During the extrusion process, cpV-coated genomic DNA is stripped of the cpV coat and simultaneously re-coated with the mature coat proteins.

Both cpIII and cpVIII proteins include two domains that provide signals for assembly of the mature phage particle. The first domain is a secretion signal that directs the newly synthesized protein to the host cell membrane. The secretion signal is located at the amino terminus of the polypeptide and targets the polypeptide at least to the cell membrane. The second domain is a membrane anchor domain that provides signals for association with the host cell membrane and for association with the phage particle during assembly. This second signal for both cpVIII and cpIII comprises at least a hydrophobic region for spanning the membrane.

The cpVIII has been extensively studied as a model membrane protein because it can integrate into lipid bilayers such as the cell membrane in an asymmetric orientation with the acidic amino terminus toward the outside and the basic carboxyl terminus toward the inside of the membrane. The mature protein is about 50 amino acid residues in length of which 11 residues provide the carboxyl terminus, 19 residues provide the hydrophobic transmembrane region, and the remaining residues comprise the amino terminus.

The sequence of cpIII indicates that the C-terminal 23 amino acid residue stretch of hydrophobic amino acids normally responsible for a membrane anchor function can be altered in a variety of ways and still retain the capacity to associate with membranes. Ff phage-based expression vectors are available in which the entire cpIII amino acid residue sequence is modified by insertion of short polypeptide "epitopes" [Parmely et al., Gene, 73:305-318 (1988); and Cwirla et al., Proc. Natl. Acad. Sci. USA, 87:6378-6382 (1991)] or an amino acid residue sequence defining a single chain antibody domain. McCafferty et al., Science, 348:552-554 (1990). These hybrid proteins were synthesized and assembled onto phage particles in amounts of about 5 copies per particle, a density at which normal cpIII is usually found. One of skill in the art can use these teachings to incorporate combinatorial peptide-encoding polynucleotide libraries into the normal cpIII or cpVIII genes. Further teachings in this regard are found in U.S. Patent 6,235,469.

A gene repertoire useful for the practice of the invention is a collection of different peptide-encoding genes, which can be isolated from natural sources or can be generated artificially. A gene repertoire useful in practicing the present invention contains at least $10^3$, preferably at least $10^4$, more preferably at least $10^5$, and most preferably at least $10^7$ different genes. Methods for evaluating the diversity of a repertoire of genes are well known to one skilled in the art.

For example, the present invention contemplates testing a library of peptides from known adhesive proteins. Many such proteins involved in cell attachment are members of a large family of related proteins termed integrins. Integrins are heterodimers comprised of a beta and an alpha subunit. Members of the integrin family include the cell surface glycoproteins platelet receptor GpIIb-IIIa, vitronectin receptor (VnR), fibronectin receptor (FnR) and the leukocyte adhesion receptors LFA-1, Mac-1, Mo-1 and 60.3. Rouslahti et al., Science, 238:491-497 (1987). Nucleic acid and protein sequence data demonstrate that regions of conserved sequences exit in the members of these families, particularly between the beta chain of GpIIb-IIIa, VnR and FnR, and between the alpha subunit of VnR, Mac-1, LFA-1, FnR and GpIIb-IIIa. Suzuki et al., Proc. Natl. Acad. Sci. USA, 83:8614-8618, 1986; Ginsberg et al., J. Biol. Chem., 262: 5437-5440, 1987. Any of these proteins or peptides derived therefrom can be used to make peptide display libraries.

A library of DNA molecules can be produced where each DNA molecule comprises a cistron for expressing a fusion polypeptide on the surface of a filamentous phage particle. This can be done, for example, by (a) forming a ligation admixture by combining in a ligation buffer (i) a repertoire of peptide encoding genes and (ii) a plurality of DNA expression vectors in linear form adapted to form a fusion polypeptide, and (b) subjecting the admixture to ligation conditions for a

time period sufficient for the repertoire of genes to become operatively linked (ligated) to the plurality of vectors to form the library.

At this point, the repertoire of peptide encoding genes is in the form of double-stranded (ds) DNA and each member of the repertoire has cohesive termini adapted for directional ligation. In addition, the plurality of DNA expression vectors are each linear DNA molecules having upstream and downstream cohesive termini that are (a) adapted for directionally receiving the polypeptide genes in a common reading frame, and (b) operatively linked to respective upstream and downstream translatable DNA sequences. The upstream translatable DNA sequence encodes a secretion signal, for example, a pelB secretion signal, and the downstream translatable DNA sequence encodes a filamentous phage coat protein membrane anchor for a peptide. The translatable DNA sequences are also operatively linked to respective upstream and downstream DNA expression control sequences as defined for a DNA expression vector.

The library so produced can be utilized for expression and screening of the fusion polypeptides encoded by the resulting library of cistrons represented in the library by the screening methods described herein.

### Nucleic Acids

The present invention provides purified and isolated nucleic acids encoding the peptides of the invention. Nucleic acids of the invention (both sense and anti-sense strands thereof) include genomic DNAs, cDNAs, and RNAs, as well as completely or partially synthetic nucleic acids. Nucleic acids of the invention include any DNA encoding peptides having even-numbered SEQ ID NO, as well as nucleic acids that hybridize to those DNAs under standard stringent hybridization conditions. DNAs encoding the peptides of the invention also include the odd-numbered SEQ ID NOs described herein as well as DNAs that, due to the degeneracy of the code, also encode the peptides of the invention. The odd-numbered SEQ ID NOs correspond to the phage display library DNAs that encode the even-numbered peptides isolated by the present methods.

Exemplary stringent hybridization conditions are as follows: hybridization at 65° C in 50% formamide, 5 × SSC, 20 mM sodium phosphate, pH 6.8 and washing in 0.2 × SSC at 65 °C. More preferred stringent hybridization conditions include hybridization in 6 × SSC and at 55°C. It is understood by those of skill in the art that variation in these conditions occurs based on the length and GC nucleotide content of the sequences to be hybridized. Formulas standard in the art are appropriate for determining exact hybridization conditions. *See* Sambrook et al., §§ 9.47-9.51 in Molecular Cloning: A Laboratory Manual, Cold Spring Harbor Laboratory Press, Cold Spring Harbor, N.Y. (1989); Sambrook et al., Molecular Cloning: A Laboratory Manual, Cold Spring Harbor Laboratory Press, Cold Spring Harbor, N.Y. (2001). Preferred nucleic acids of the invention include DNAs encoding peptides having SEQ ID NO:2, SEQ ID NO:4, SEQ ID NO:6, SEQ ID NO: 8, SEQ ID NO:10, SEQ ID NO:12, SEQ ID NO:336, SEQ ID NO:344 and/or SEQ ID NO:464.

The following examples further illustrate the invention and are not intended to limit it in any way.

### EXAMPLE 1: Screening Peptides for Binding to Atherosclerotic Lesions

The surface characteristics of endothelial cells from atherosclerotic lesions of atherosclerotic ApoE knockout mice were investigated by *in vivo* selection of phage displaying seven amino acid peptides flanked by cysteine residues. Apo-E knockout and LDL receptor knockout mice on atherogenic diets develop atherosclerotic lesions within 10-12 weeks at the aortic arch and the branch point of renal arteries. These animals are accepted animal models of human atherosclerotic lesions that have similar developmental and biochemical pathways.

### Material and Methods

### Phage Display Libraries

Gene III-fused libraries used included New England lab PHD™, 7-mer linear, 12-mer linear, and 7-mer cyclized libraries. The PHD™ phage peptide library was from New England Biolabs (Beverly, MA). Gene VIII fused libraries used were designed and constructed as 4-mer, 5-mer and 6-mer cyclized libraries. The inserted combinatorial elements were linear peptides of 4-7 amino acids in length, constrained at each end by cysteine residues.

### Animals

The parental stock of C57BL/6J ApoE gene inactivated (knockout) mice was obtained from Jackson Laboratories. These and wild type C57BL/6J mice were bred in the Rodent Breeding Facility of The Scripps Research Institute and fed *ad lib* standard chow diet (No. 5015, Harlan Tekland, Madison, WI). To produce the atherosclerotic ApoE model lesions, the mice were fed atherogenic diet No. TD 88051 containing 15.8% (wt/wt) fat, 1.25% (wt/wt) cholesterol, and 0.5% (wt/wt) sodium cholate. All studies were reviewed and approved by the Institutional Animal Care and Use Committee

and conducted in the Institutional facilities accredited by AAALAC, with an assurance from the Public Health Service, registered with the U.S. Department of Agriculture and in compliance with regulations.

**In Vivo Panning with Phage Display Libraries**

*In vivo* biopanning was performed in atherosclerotic ApoE knockout mice, after 12 weeks on the high fat diet. All animals had grossly visible atherosclerotic lesions of the aortic arch and the branch point of renal arteries within 10-12 weeks after being fed the high fat diet. Thirty minutes prior injection of the phage library, $10^{12}$ pfu of irradiated helper phage were infused via the tail vein of the mice and allowed to circulate for 30 minutes to block non-specific phage binding and to saturate the mouse reticuloendothelial system. Then, $10^{11}$ pfu of viable peptide library phage were injected and allowed to circulate for 30 min. The mouse was perfused via the heart at arterial pressure with warm physiological saline (Phosphate Buffered Saline (PBS), pH 7.4) for about 5 min. The aorta was removed, washed, and opened to expose lesions at the arch and the branch point of renal arteries. These were dissected free and bound phage were eluted with elution buffer (0.1 M glycine, pH 2.2) and neutralized with 0.1 volume ofTris buffer (2 M tns-HCl, pH 8). The eluted phage were amplified by infection of E. *coli* in medium, then plated and titered. Basic phage protocols used are from "Phage Display of Peptides and Proteins, A Laboratory Manual" (Brian K. Kay, Jill Winter, and John McCafferty, Academic Press). Up to $10^4$ of library phage were recovered from the first round of selection. Three additional rounds of selection were performed. Phage recovered from the fourth round were plated and individual clones were selected for sequence analysis.

**Analysis of peptide sequences.**

Peptide sequences were analyzed with ClustalW (12) software from the European Molecular Biology Laboratory-to identify amino acid motifs that are shared among multiple peptides. The peptide sequences were also searched by online databases using the BLAST program accessible at NCBI (website at ncbi.nim.nih.gov/BLAST). The value of a "hit" sequence was assessed by the following ordered set of criteria: (1) a sequence exhibits significant similarity to extracellular protein domains, including a variety of proteins with the same type of domain; (2) the sequence exhibits similarity with a known protein candidate for binding to cells; (3) scoring is increased if multiple phage hit sequences exhibit similarity to the same or closely adjacent linear sequence of a given protein; (4) a phage sequence shows similarity, overlap, or a common smaller motif with other "Mt" phage inserts. Independent of the exact sequence of the insert, or its similarity to known sequences, an insert that was recovered repeatedly from progressive *in vivo* selection experiments, was considered of interest and meritorious of further analysis.

The distribution of the account X of success in n independent trials with probability p of success on each trial is a binomial distribution B (*n*, *p*), and was approximated with equation:

$$P(X=k) = \{ n!/k!(n-k)! \} p^k (1-p)^{n-k}$$

Here X is the number of times a given peptide motif occurred in n independent sequencing trials, and *p* is the probability of this peptide occurring in each trail.

**Peptides**

To assess weather a peptide recovered by the selection procedures provided above is truly specific for atherosclerotic lesions, selected peptides were synthesized with a triple-Gly spacer terminating in a biotin reporter molecule. Thus, peptides ACAPGPSKSCGGSYK-Biotin (SEQ ID NO:468), ACNHRYMQMCGGSYK-Bioitin (SEQ ID NO:470), ACNQRHQMSCGGSYK-Biotin (SEQ ID NO:47 2) and ACVNRSDGMCGGSYK-Biotin (SEQ ID NO:474) were synthesized by the Scripps Peptide Core facility using Fmoc chemistry. A control peptide with a scrambled sequence but the same amino acid composition was also synthesized. An N-terminal alanine (underlined) that is encoded by the phage and present on the phage protein was added to the N-terminus of the peptides. An extension of GGSYK was added to the C-terminus of the peptides (underlined). The tyrosine permitted iodination and the lysine was added for biotinylation. Biotin was attached through the side chain amino group of the C-terminal while the peptides were attached to the beads. The peptides were deblocked, cleaved, and HPLC purified. The purified peptides were characterized by mass spectrometry. The molecular weight of the peptides exactly matched the predicted mass. Control and experimental peptides were tested to insure that they could bind streptavidin.

Immunohistochemistry with confocal microscopy was used to observe whether the experimental and/or control peptides localize selectively to the endothelium. Streptavidin alone was used as a further control to assess background

staining.

### Intermolecular Disulfide Bond Formation in Cys-Containing Peptides

Analysis of the role of intact disulfide bond constraints was assessed by reduction and re-oxidization of the peptides. Two ml PBS was used to dissolve every five mg peptide, and 2-mercaptoethanol was added to a final concentration of 0.05 M. The reduction reaction was carried out for four hours at room temperature. The reaction mixture was then lyophilized to remove the 2-mercaptoethanol. The reduced peptide preparations were analyzed and divided into two aliquots. One aliquot was stored as the reduced, lyophilized peptide preparation. The second aliquot was air oxidized in 0.1 mM in folding buffer (2 M Guanidinium HCl, 0.2 M Tris-HCl, pH 8.5) while being stirred vigorously with air overnight. The progress of intermolecular disulfide formation was monitored by the Ellman's reaction. The re-oxidized and folded peptides and the control reduced peptides were dialyzed against phosphate buffered saline. For *in vivo* experiments the peptides were dialyzed against sterile clinical physiologic saline and sterile filtered.

Molecular modeling of the peptide with sequence CAPGPSKSC (SEQ ID NO:4) suggested that it forms a very rigid ring structure. The refolding experiment also indicated that, under oxidizing condition, the peptide quickly circularizes, and the monomeric cysteine loop structure is the favored conformation for this peptide. Most peptides do not have such a defined and restricted conformation in solution.

### Immunohistochemical analysis.

Immunohistochemical analysis for binding was performed on frozen 5 micron sections of ApoE mouse atherosclerotic aortic valve and aorta on poly-L-lysine coated slides. Biotinylated peptide binding to aortic lesions was detected by strepavidin conjugated peroxidase and was developed with DAB substrate (Vector, Burlingame, CA). For endothelial identification, biotinylated rat CD-31 specific mouse monoclonal antibody was used and was detected with Texas red conjugated strepavidin. Phage staining was performed with rabbit anti-phage antibody followed by a fluorescein conjugated goat anti-rabbit antibody. The sections were analyzed by laser scanning confocal microscopy.

### Phage Localization in Atherosclerotic Lesions

To observe phage or peptide association with atherosclerotic lesions, either phage carrying a homing peptide sequence or a circular synthetic peptide were injected into the mice via the tail vein. After 30 min circulation, the mice were perfused with sterile PBS (pH 7.4) through a cannula to the left ventricle, and the perfusate was drained out through an incision made in the inferior vena cava. Fixation was *in situ* by perfusion with 4% paraformaldehyde. The aortas were dissected under a microscope, and the associated fat tissue cleaned while attached. The aorta was then longitudinally opened from aortic valve to iliac bifurcation. The detached aorta was pinned flat.

Biotinylated anti-phage antibody (Sigma) diluted 1:500 was layered over the vascular tissue, followed by 1: 500 dilution of avidin-peroxidase (Vector, Burlingame, CA), to detect phage that were associated with the atherosclerotic lesion in the aorta. The aorta samples were then washed three times in PBS and developed using DAB substrate as suggested by the manufacturer.

The atherosclerotic regions of human vessels were similarly dissected and pinned on to the dissecting pan. These tissues were then incubated with refolded biotinylated peptides (10.0 μg/ml) overnight at 4˚C. The samples were washed three times. An avidin-peroxidase conjugate was used as a second tier probe, and each specimen was visualized with DAB substrate.

### Target Protein Identification

Human umbilical cord venous endothelial cells (HUVEC) and EGM™ culture medium were from Clonetics, Walkersville, MD. Cells were cultivated in EGM with 5% fetal bovine serum (FBS) in flasks coated with 0.2% gelatin. The bEND.3 cells (ATCC) were cultured in Dulbecco's minimum essential medium (DMEM) (Invitrogen, Carlsbad, CA) supplemented with 10% FBS and 2 mM L-glutamine. The HT1080 cells were from ATCC and cultured in DMEM supplemented with 10% FBS, 2 mM L- glutamine, 1.5 g/L sodium bicarbonate, and 0.1 mM NEAA (Non-essential amino acids, Invitrogen, Carlsbad, CA). The ECV304 were from ATCC and cultured in DMEM, 10% FBS.

The membrane fractions of endothelial cell lines were prepared using Triton 114 extraction. Endothelial cell pellets of approximately $5 \times 10^6$ cells were lysed with 1 ml 1% triton X-114 in 0.1 M Tris, 10 mM EDTA, 2000 U/ml Aprotinin, 100 μM PMSF by incubating with repeated mixing on ice for 15 min. The debris was removed by centrifugation at 16,000 g at 4˚C. The resultant supernatant was then incubated for 5 min at 37˚C to permit phase separation and then centrifuged at 16,000 g for 2 min at room temperature. The lower membrane phase was recovered and re-extracted with 1% Triton X 114 solution as described above. Extracted membrane fractions were precipitated with cold acetone

(-20 °C) and centrifuged for 10 minutes at 16,000 g. The membrane protein pellet was air dried and re-suspended in SDS sample buffer. The membrane protein preparation was separated by either polyacrylamide gel electrophoresis under non-denaturing conditions or SDS polyacrylamide gel electrophoresis using a gradient Tris-glycine gel (8-16%). Separated proteins were transferred from the gel onto a nitrocellulose membrane and non-specific interactions were then blocked with non-fat milk. Biotinylated peptides identified by the *in vivo* panning procedures were used as probes. Nitrocellulose membranes were incubated with 10 μg/ml biotinylated peptide probes overnight at room temperature with gentle agitation. Nitrocellulose membranes were then washed three times with PBS, incubated with streptavidin-peroxidase for 15-30 min and developed with DAB substrate.

**Peptide binding assay**

Analysis of synthetic peptide binding to cells was performed in 96 well plates. Cells ($10^5$) were plated into each well, incubated overnight then briefly fixed with cold ethanol for 10 seconds. Serial concentrations of peptide in 100 μl PBS were added and incubated at room temperature with gentle shaking for four hours. The wells were washed three times with PBS and reaction developed using strepavidin-peroxidase substrate (Vector, Burlingame, CA). The reactions were quantified at 405 nm in a plate reader (Molecular Devices, Sunnyvale, CA). In the inhibition assay, TIMP-2 protein was included with the peptide in the initial incubation.

**Results**

**Atherosclerotic Lesions in Apo E Knockout Mice have Intact Endothelial Surfaces**

Immunohistochemical staining with antibody against an endothelial marker CD 31 was used to determine whether the endothelial lining is intact over the atherosclerotic lesions of Apo E knockout mice. The presence of endothelium was detected in about 50 % of the early atherosclerotic lesions sample stained. However as the lesion progressed, gaps in endothelial coverage were sometimes observed. In humans, these gaps in endothelial coverage are believed to represent potential or actual thrombogenic sites that encourage formation of platelet mural thrombi. However, in atherosclerotic Apo E knockout mice, lesions of this severity do not occur and no such thrombogenic activity has been reported, indicating that the lesions observed in ApoE mice correspond to early human lesions.

**Peptides Isolated by In Vivo Panning of Phage Displayed Peptide Libraries**

After four rounds of panning, approximately 150 individual phage isolates were separately selected, cloned and sequenced. Of these 150 isolates, fifty had no peptide insert. Four phage isolates were repeatedly isolated. These data suggest that multiple target binding sites exist on the surface of the endothelium and that several rounds of selection can be used for enrichment of phage displaying peptides that truly bind to atherosclerotic lesions. Use of gene III-linked libraries limited the number of rounds of panning, because this phage vector did not tolerate cysteine-containing peptides well and insert-containing phage were rapidly out-grown by contaminating wild type phage.

The following peptides were isolated as being displayed on phage that repeatedly bound to atherosclerotic lesions:

CAPGPSKSC (SEQ ID NO:4)
CQEPTRLKC (SEQ ID NO:8)
CKEPTRAHC (SEQ ID NO:12).

Four repeated phage clones were isolated having CAPGPSKSC (SEQ ID NO:4) by preferential binding to atherosclerotic lesions *in vivo.* A purified isolate of phage displaying the CAPGPSKSC (SEQ ID NO:4) sequence were amplified, then injected into atherogenic Apo E knockout mice. After circulation for 30 min, the mice were perfused with 4% paraformaldehyde, and aortic tissue was removed and stained with anti-phage antibody. Phage displaying the CAPGPSKSC (SEQ ID NO:4) sequence showed preferential binding to atherosclerotic lesions compared to wild type phage. No binding is detected in similarly-treated aortas from normal Balb/C mice and young Apo E knockout mice on a normal diet. These data indicate that phage displaying the CAPGPSKSC (SEQ ID NO:4) peptide are associated with an endothelial surface molecule that is expressed as atherosclerosis develops. A low degree of homology to some regions of laminin and laminin-like molecules was detected when protein databases were searched for homology to CAPGPSKSC (SEQ ID NO:4).

Two other peptides were separately isolated that had distinct but related sequences CQEPTRLKC (SEQ ID NO:8) and CKEPTRAHC (SEQ ID NO:12). These two peptides were homologous in an internal sequence of four amino acids EPTR (SEQ ID NO:450). Recovery of different phage that bear overlapping or homologous peptidyl sequences

is statistically unlikely because of the diversity of the library and the complexity of target molecules for binding. Homologous clones were rarely observed. Such observations indicate that homologous clones may bind to the same or similar targets that are consistently associated with atherosclerotic lesions.

The following nucleic acids and peptides were identified using the methods described above.

**Table 4: Peptides and Nucleic Acids Isolated**

| Name | Sequence | SEQIDNO: |
|---|---|---|
| | GCGCCGGGTCCGTCTAAGAGT<br>CGCGGCCCAGGCAGATTCTCA | 1 |
| | APGPSKS | 2 |
| | TGTGCGCCGGGTCCGTCTAAGAGTTGC<br>ACACGCGGCCCAGGCAGATTCTCAACG | 3 |
| | CAPGPSKSC | 4 |
| | CAGGAGCCGACGCGGCTGAAG<br>GTCCTCGGCTGCGCCGACTTC | 5 |
| | QEPTRLK | 6 |
| | TGTCAGGAGCCGACGCGGCTGAAGTGC<br>ACAGTCCTCGGCTGCGCCGACTTCACG | 7 |
| | CQEPTRLKC | 8 |
| | AAGGAGCCTACGCGGGCGCAT<br>TTCCTCGGATGCGCCCGCGTA | 9 |
| | KEPTRAH | 10 |
| | TGTAAGGAGCCTACGCGGGCGCATTGC<br>ACATTCCTCGGATGCGCCCGCGTAACG | 11 |
| | CKEPTRAHC | 12 |
| Eo2-1 | TTGGCGATGCTTATGGATACG<br>AACCGCTACGAATACCTATGC | 13 |
| Eo2-1 | LAMLMDT | 14 |
| Eo2-1 | TGTTTGGCGATGCTTATGGATACGTGC<br>ACAAACCGCTACGAATACCTATGCACG | 15 |
| Eo2-1 | CLAMLMDTC | 16 |
| Eo2-2 | AATAAGCATACTAGGCCGCTT<br>TTATTCGTATGATCCGGCGAA | 17 |
| Eo2-2 | NKHTRPL | 18 |
| Eo2-2 | TGTAATAAGCATACTAGGCCGCTTTGC<br>ACATTATTCGTATGATCCGGCGAAACG | 19 |
| Eo2-2 | CNKHTRPLC | 20 |
| Eo2-3 | GTGCATAAGCTGCCTGAGTCT<br>TTCGACGGACTCAGAACGCCA | 21 |
| Eo2-3 | VHKLPES | 22 |
| Eo2-3 | TGTGTGCATAAGCTGCCTGAGTCTTGC<br>ACATTCGACGGACTCAGAACGCCAACG | 23 |

(continued)

| Name | Sequence | SEQIDNO: |
|---|---|---|
| Eo2-3 | CVHKLPESC | 24 |
| Eo2-4 | CCGACTCAGGCTTCTCTTCAT<br>GGCTGAGTCCGAAGAGAAGTA | 25 |
| Eo2-4 | PTQASLH | 26 |
| Eo2-4 | TGTCCGACTCAGGCTTCTCTTCATTGC<br>ACAGGCTGAGTCCGAAGAGAAGTAACG | 27 |
| Eo2-4 | CPTQASLHC | 28 |
| Eo2-5 | GATACTGCGCCTCCGTCGTCG<br>CTATGACGCGGAGGCAGCAGC | 29 |
| Eo2-5 | DTAPPSS | 30 |
| Eo2-5 | TGTGATACTGCGCCTCCGTCGTCGTGC<br>ACACTATGACGCGGAGGCAGCAGCACG | 31 |
| Eo2-5 | CDTAPPSSC | 32 |
| Eo2-6 | GGGGTGCAGACTCTGCTTGCT<br>CCCCACGTCTGAGACGAACGA | 33 |
| Eo2-6 | GVQTLLA | 34 |
| Eo2-6 | TGTGGGGTGCAGACTCTGCTTGCTTGC<br>ACACCCCACGTCTGAGACGAACGAACG | 35 |
| Eo2-6 | CGVQTLLAC | 36 |
| Eo2-7 | GATCCTGTGACGAAGCATACT<br>CTAGGACACTGCTTCGTATGA | 37 |
| Eo2-7 | DPVTKHT | 38 |
| Eo2-7 | TGTGATCCTGTGACGAAGCATACTTGC<br>ACACTAGGACACTGCTTCGTATGAACG | 39 |
| Eo2-7 | CDPVTKHTC | 40 |
| Eo2-8 | GATCAGAGTACGATTCGGGCG<br>CTAGTCTCATGCTAAGCCCGC | 41 |
| Eo2-8 | DQSTIRA | 42 |
| Eo2-8 | TGTGATCAGAGTACGATTCGGGCGTGC<br>ACACTAGTCTCATGCTAAGCCCGCACG | 43 |
| Eo2-8 | CDQSTIRAC | 44 |
| Eo2-9 | CGGGCTGCTACTCCTTCGATT<br>GCCCGACGATGAGGAAGCTAA | 45 |
| Eo2-9 | RAATPSI | 46 |
| Eo2-9 | TGTCGGGCTGCTACTCCTTCGATTTGC<br>ACAGCCCGACGATGAGGAAGCTAAACG | 47 |
| Eo2-9 | CRAATPSIC | 48 |

(continued)

| Name | Sequence | SEQIDNO: |
|---|---|---|
| Eo2-10 | AAGACGTCGCATGCGCAGGAG<br>TTCTGCAGCGTACGCGTCCTC | 49 |
| Eo2-10 | KTSHAQE | 50 |
| Eo2-10 | TGTAAGACGTCGCATGCGCAGGAGTGC<br>ACATTCTGCAGCGTACGCGTCCTCACG | 51 |
| Eo2-10 | CKTSHAQEC | 52 |
| Eo3-3 | AAGCATCCTGTTGGNCGGGTG<br>TTCGTAGGACAACCNGCCCAC | 53 |
| Eo3-3 | KHPVGRV | 54 |
| Eo3-3 | TGTAAGCATCCTGTTGGNCGGGTGTGC<br>ACATTCGTAGGACAACCNGCCCACACG | 55 |
| Eo3-3 | CKHPVGRVC | 56 |
| Eo3-5 | ACGGATACTAAGAATTCGCAG<br>TGCCTATGATTCTTAAGCGTC | 57 |
| Eo3-5 | TDTKNSQ | 58 |
| Eo3-5 | TGTACGGATACTAAGAATTCGCAGTGC<br>ACATGCCTATGATTCTTAAGCGTCACG | 59 |
| Eo3-5 | CTDTKNSQC | 60 |
| Eo3-11 | CAGCCGCCGATGGGGCGGTAT<br>GTCGGCGGCTACCCCGCCATA | 61 |
| Eo3-11 | QPPMGRY | 62 |
| Eo3-11 | TGTCAGCCGCCGATGGGGCGGTATTGC<br>ACAGTCGGCGGCTACCCCGCCATAACG | 63 |
| Eo3-11 | CQPPMGRYC | 64 |
| Eo3-13 | AATGAGAGGCTGAATAAGGAT | 65 |
| | TTACTCTCCGACTTATTCCTA | |
| Eo3-13 | NERLNKD | 66 |
| Eo3-13 | TGTAATGAGAGGCTGAATAAGGATTGC<br>ACATTACTCTCCGACTTATTCCTAACG | 67 |
| Eo3-13 | CNERLNKDC | 68 |
| Eo3-15 | CCGCCGTCGAATAAGCAGATG<br>GGCGGCAGCTTATTCGTCTAC | 69 |
| Eo3-15 | PPSNKQM | 70 |
| Eo3-15 | TGTCCGCCGTCGAATAAGCAGATGTGC<br>ACAGGCGGCAGCTTATTCGTCTACACG | 71 |
| Eo3-15 | CPPSNKQMC | 72 |
| Eo3-27 | GATTCTTCGTCGCCTGCTCGG<br>CTAAGAAGCAGCGGACGAGCC | 73 |

(continued)

| Name | Sequence | SEQIDNO: |
|---|---|---|
| Eo3-27 | DSSSPAR | 74 |
| Eo3-27 | TGTGATTCTTCGTCGCCTGCTCGGTGC ACACTAAGAAGCAGCGGACGAGCCACG | 75 |
| Eo3-27 | CDSSSPARC | 76 |
| Eo3-28 | ACGCAGTCTGATAATAGGCGT TGCGTCAGACTATTATCCGCA | 77 |
| Eo3-28 | TQSDNRR | 78 |
| Eo3-28 | TGTACGCAGTCTGATAATAGGCGTTGC ACATGCGTCAGACTATTATCCGCAACG | 79 |
| Eo3-28 | CTQSDNRRC | 80 |
| Eco3-29 | AAGGGTCTGCCGGCTAAGACT TTCCCAGACGGCCGATTCTGA | 81 |
| Eco3-29 | KGLPAKT | 82 |
| Eco3-29 | TGTAAGGGTCTGCCGGCTAAGACTTGC ACATTCCCAGACGGCCGATTCTGAACG | 83 |
| Eco3-29 | CKGLPAKTC | 84 |
| Eo3-31 | TTGCAGCCGCATCTGAGTCTT AACGTCGGCGTAGACTCAGAA | 85 |
| Eo3-31 | LQPHLSL | 86 |
| Eo3-31 | TGTTTGCAGCCGCATCTGAGTCTTTGC ACAAACGTCGGCGTAGACTCAGAAACG | 87 |
| Eo3-31 | CLQPHLSLC | 88 |
| Eo3-33 | GCGGTTCCGTAGAATCGTTCT CGCCAAGGCATCTTAGCAAGA | 89 |
| Eo3-33 | AVPQNRS | 90 |
| Eo3-33 | TGTGCGGTTCCGTAGAATCGTTCTTGC ACACGCCAAGGCATCTTAGCAAGAACG | 91 |
| Eo3-33 | CAVPQNRSC | 92 |
| Eo3-34 | ATGAATCAGACTCCTGATTTG TACTTAGTCTGAGGACTAAAC | 93 |
| Eo3-34 | MNQTPDL | 94 |
| Eo3-34 | TGTATGAATCAGACTCCTGATTTGTGC ACATACTTAGTCTGAGGACTAAACACG | 95 |
| Eo3-34 | CMNQTPDLC | 96 |
| Eo3-36 | TTTCAGATGCAGCCTACTCTT AAAGTCTACGTCGGATGAGAA | 97 |
| Eo3-36 | FQMQPTL | 98 |
| Eo3-36 | TGTTTTCAGATGCAGCCTACTCTTTGC ACAAAAGTCTACGTCGGATGAGAAACG | 99 |

(continued)

| Name | Sequence | SEQIDNO: |
|---|---|---|
| Eo3-36 | CFQMQPTLC | 100 |
| Eo3-37 | AGTGGGGCTTCTAATAAGACG TCACCCCGAAGATTATTCTGC | 101 |
| Eo3-37 | SGASNKT | 102 |
| Eo3-37 | TGTAGTGGGGCTTCTAATAAGACGTGC ACATCACCCCGAAGATTATTCTGCACG | 103 |
| Eo3-37 | CSGASNKTC | 104 |
| Eo3-38 | ACTAAGATGCGGTTGGAGCAG TGATTCTACGCCAACCTCGTC | 105 |
| Eo3-38 | TKMRLEQ | 106 |
| Eo3-38 | TGTACTAAGATGCGGTTGGAGCAGTGC ACATGATTCTACGCCAACCTCGTCACG | 107 |
| Eo3-38 | CTKMRLEQC | 108 |
| Eo3-41 | ACGTCTCCTATTTATCCGGGT TGCAGAGGATAAATAGGCCCA | 109 |
| Eo3-41 | TSPIYPG | 110 |
| Eo3-41 | TGTACGTCTCCTATTTATCCGGGTTGC ACATGCAGAGGATAAATAGGCCCAACG | 111 |
| Eo3-41 | CTSPIYPGC | 112 |
| Eo3-43 | AAGACTCCGTCGCAGAGTCAG TTCTGAGGCAGCGTCTCAGTC | 113 |
| Eo3-43 | KTPSQSQ | 114 |
| Eo3-43 | TGTAAGACTCCGTCGCAGAGTCAGTGC ACATTCTGAGGCAGCGTCTCAGTCACG | 115 |
| Eo3-43 | CKTPSQSQC | 116 |
| Eo3-46 | CTTCAGGCTTTTAAGGCGACT GAAGTCCGAAAATTCCGCTGA | 117 |
| Eo3-46 | LQAFKAT | 118 |
| Eo3-46 | TGTCTTCAGGCTTTTAAGGCGACTTGC ACAGAAGTCCGAAAATTCCGCTGAACG | 119 |
| Eo3-46 | CLQAFKATC | 120 |
| Eo3-47 | TCTACTACTGAGCTTAATAAG AGATGATGACTCGAATTATTC | 121 |
| Eo3-47 | STTELNK | 122 |
| Eo3-47 | TGTTCTACTACTGAGCTTAATAAGTGC ACAAGATGATGACTCGAATTATTCACG | 123 |
| Eo3-47 | CSTTELNKC | 124 |

(continued)

| Name | Sequence | SEQIDNO: |
|---|---|---|
| Eo3-48 | AGGACTCATAGTTCTCCGACT<br>TCCTGAGTATCAAGAGGCTGA | 125 |
| Eo3-48 | RTHSSPT | 126 |
| Eo3-48 | TGTAGGACTCATAGTTCTCCGACTTGC<br>ACATCCTGAGTATCAAGAGGCTGAACG | 127 |
| Eo3-48 | CRTHSSPTC | 128 |
| Eo3-50 | AATGAGAATTTTAAGGGGCTG<br>TTACTCTTAAAATTCCCCGAC | 129 |
| Eo3-50 | NENFKGL | 130 |
| Eo3-50 | TGTAATGAGAATTTTAAGGGGCTGTGC<br>ACATTACTCTTAAAATTCCCCGACACG | 131 |
| Eo3-50 | CNENFKGLC | 132 |
| Eo3-51 | AGTAAGACTAATCATGCTTCT<br>TCATTCTGATTAGTACGAAGA | 133 |
| Eo3-51 | SKTNHAS | 134 |
| Eo3-51 | TGTAGTAAGACTAATCATGCTTCTTGC<br>ACATCATTCTGATTAGTACGAAGAACG | 135 |
| Eo3-51 | CSKTNHASC | 136 |
| Eo3-52 | ACGCCGTATCCTTCTAATTCG<br>TGCGGCATAGGAAGATTAAGC | 137 |
| Eo3-52 | TPYPSNS | 138 |
| Eo3-52 | TGTACGCCGTATCCTTCTAATTCGTGC<br>ACATGCGGCATAGGAAGATTAAGCACG | 139 |
| Eo3-52 | CTPYPSNSC | 140 |
| Eo3-53 | ACGCTGTCTGCTGCTCCGCAT<br>TGCGACAGACGACGAGGCGTA | 141 |
| Eo3-53 | TLSAAPH | 142 |
| Eo3-53 | TGTACGCTGTCTGCTGCTCCGCATTGC<br>ACATGCGACAGACGACGAGGCGTAACG | 143 |
| Eo3-53 | CTLSAAPHC | 144 |
| Eo3-54 | CTTAATAATTCTCAGGCTCAT<br>GAATTATTAAGAGTCCGAGTA | 145 |
| Eo3-54 | LNNSQAH | 146 |
| Eo3-54 | TGTCTTAATAATTCTCAGGCTCATTGC<br>ACAGAATTATTAAGAGTCCGAGTAACG | 147 |
| Eo3-54 | CLNNSQAHC | 148 |
| Eo3-56 | ATTGAGCATTCGGCGCAGCAG<br>TAACTCGTAAGCCGCGTCGTC | 149 |

(continued)

| Name | Sequence | SEQIDNO: |
|---|---|---|
| Eo3-56 | IEHSAQQ | 150 |
| Eo3-56 | TGTATTGAGCATTCGGCGCAGCAGTGC<br>ACATAACTCGTAAGCCGCGTCGTCACG | 151 |
| Eo3-56 | CIEHSAQQC | 152 |
| Eo3-57 | TCGGCTGCGGGGCATCATACT<br>AGCCGACGCCCCGTAGTATGA | 153 |
| Eo3-57 | SAAGHHT | 154 |
| Eo3-57 | TGTTCGGCTGCGGGGCATCATACTTGC<br>ACAAGCCGACGCCCCGTAGTATGAACG | 155 |
| Eo3-57 | CSAAGHHTC | 156 |
| Eo3-58 | CATAATCAGAAGTTGAATCGT<br>GTCTTCAACTTAGCAACGCCA | 157 |
| Eo3-58 | HNQKLNR | 158 |
| Eo3-58 | TGTCATAATCAGAAGTTGAATCGTTGC<br>ACAGTCTTCAACTTAGCAACGCCAACG | 159 |
| Eo3-58 | CHNQKLNRC | 160 |
| Eo3-59 | AAGTCGACTTCTCATAGTATG<br>TTCAGCTGAAGAGTATCATAC | 161 |
| Eo3-59 | KSTSHSM | 162 |
| Eo3-59 | TGTAAGTCGACTTCTCATAGTATGTGC | 163 |
| | ACATTCAGCTGAAGAGTATCATACACG | |
| Eo3-59 | CKSTSHSMC | 164 |
| Eo3-60 | CCTAATAATAAGTCGGCTTCG<br>GGATTATTATTCAGCCGAAGC | 165 |
| Eo3-60 | PNNKSAS | 166 |
| Eo3-60 | TGTCCTAATAATAAGTCGGCTTCGTGC<br>ACAGGATTATTATTCAGCCGAAGCACG | 167 |
| Eo3-60 | CPNNKSASC | 168 |
| Eo3-62 | GAGGATCCTACGTTGAAGGTG<br>CTCCTAGGATGCAACTTCCAC | 169 |
| Eo3-62 | EDPTLKV | 170 |
| Eo3-62 | TGTGAGGATCCTACGTTGAAGGTGTGC<br>ACACTCCTAGGATGCAACTTCCACACG | 171 |
| Eo3-62 | CEDPTLKVC | 172 |
| Eo3-63 | ATGTCTGCGATGTCTCGTCAG<br>TACAGACGCTACAGAGCAGTC | 173 |
| Eo3-63 | MSAMSRQ | 174 |

(continued)

| Name | Sequence | SEQIDNO: |
|---|---|---|
| Eo3-63 | TGTATGTCTGCGATGTCTCGTCAGTGC ACATACAGACGCTACAGAGCAGTCACG | 175 |
| Ego3-63 | CMSAMSRQC | 176 |
| Eo3-64 | CCTGGGAAGATTTCGCGTAGT GGACCCTTCTAAAGCGCATCA | 177 |
| Eo3-64 | PGKISRS | 178 |
| Eo3-64 | TGTCCTGGGAAGATTTCGCGTAGTTGC ACAGGACCCTTCTAAAGCGCATCAACG | 179 |
| Eo3-64 | CPGKISRSC | 180 |
| Eo3-65 | CTGAAGCTGGGGTCGAAGCAG GACTTCGACCCCAGCTTCGTC | 181 |
| Eo3-65 | LKLGSKQ | 182 |
| Eo3-65 | TGTCTGAAGCTGGGGTCGAAGCAGTGC ACAGACTTCGACCCCAGCTTCGTCACG | 183 |
| Eo3-65 | CLKLGSKQC | 184 |
| Eo3-66 | AAGACTAGTCCGGAGTCTACT TTCTGATCAGGCCTCAGATGA | 185 |
| Eo3-66 | KTSPEST | 186 |
| Eo3-66 | TGTAAGACTAGTCCGGAGTCTACTTGC ACATTCTGATCAGGCCTCAGATGAACG | 187 |
| Eo3-66 | CKTSPESTC | 188 |
| Eo3-67 | ACGCTTTTTCCGGGGAATTCT TGCGAAAAAGGCCCCTTAAGA | 189 |
| Eo3-67 | TLFPGNS | 190 |
| Eo3-67 | TGTACGCTTTTTCCGGGGAATTCTTGC ACATGCGAAAAAGGCCCCTTAAGAACG | 191 |
| Eo3-67 | CTLFPGNSC | 192 |
| Eo3-68 | TTGCCGTCGTCTACGAGGCTG AACGGCAGCAGATGCTCCGAC | 193 |
| Eo3-68 | LPSSTRL | 194 |
| Eo3-68 | TGTTTGCCGTCGTCTACGAGGCTGTGC ACAAACGGCAGCAGATGCTCCGACACG | 195 |
| Eo3-68 | CLPSSTRLC | 196 |
| Eo3-69 | TCGAGTCAGAGGACTCCTCCG AGCTCAGTCTCCTGAGGAGGC | 197 |
| Eo3-69 | SSQRTPP | 198 |
| Eo3-69 | TGTTCGAGTCAGAGGACTCCTCCGTGC ACAAGCTCAGTCTCCTGAGGAGGCACG | 199 |

(continued)

| Name | Sequence | SEQIDNO: |
|---|---|---|
| Eo3-69 | CSSQRTPPC | 200 |
| Eo3-70 | CTGCCTACGATGACGCCGACG<br>GACGGATGCTACTGCGGCTGC | 201 |
| Eo3-70 | LPTMTPT | 202 |
| Eo3-70 | TGTCTGCCTACGATGACGCCGACGTGC<br>ACAGACGGATGCTACTGCGGCTGCACG | 203 |
| Eo3-70 | CLPTMTPTC | 204 |
| Eo3-71 | CTTATGACGCCGTCGAAGAGG<br>GAATACTGCGGCAGCTTCTCC | 205 |
| Eo3-71 | LMTPSKR | 206 |
| Eo3-71 | TGTCTTATGACGCCGTCGAAGAGGTGC<br>ACAGAATACTGCGGCAGCTTCTCCACG | 207 |
| Eo3-71 | CLMTPSKRC | 208 |
| Eo3-72 | GAGCATTTTTTTAGTCGGTCT<br>CTCGTAAAAAAATCAGCCAGA | 209 |
| Eo3-72 | EHFFSRS | 210 |
| Eo3-72 | TGTGAGCATTTTTTTAGTCGGTCTTGC<br>ACACTCGTAAAAAAATCAGCCAGAACG | 211 |
| Eo3-72 | CEHFFSRSC | 212 |
| Eo3-73 | ACGAATCAGTTTTTGCAGCAG<br>TGCTTAGTCAAAAACGTCGTC | 213 |
| Eo3-73 | TNQFLQQ | 214 |
| Eo3-73 | TGTACGAATCAGTTTTTGCAGCAGTGC<br>ACATGCTTAGTCAAAAACGTCGTCACG | 215 |
| Eo3-73 | CTNQFLQQC | 216 |
| Eo3-75 | CCTGCGAATAAGTCTTCGTTT<br>GGACGCTTATTCAGAAGCAAA | 217 |
| Eo3-75 | PANKSSF | 218 |
| Eo3-75 | TGTCCTGCGAATAAGTCTTCGTTTTGC<br>ACAGGACGCTTATTCAGAAGCAAAACG | 219 |
| Eo3-75 | CPANKSSFC | 220 |
| Eo3-77 | AGTACGACGCAGTCTAGTTGG<br>TCATGCTGCGTCAGATCAACC | 221 |
| Eo3-77 | STTQSSW | 222 |
| Eo3-77 | TGTAGTACGACGCAGTCTAGTTGGTGC<br>ACATCATGCTGCGTCAGATCAACCACG | 223 |
| Eo3-77 | CSTTQSSWC | 224 |

(continued)

| Name | Sequence | SEQIDNO: |
|---|---|---|
| Eo3-78 | GTGACTCCGGATCGGCTGACG CACTGAGGCCTAGCCGACTGC | 225 |
| Eo3-78 | VTPDRLT | 226 |
| Eo3-78 | TGTTTGACTCCGGATCGGCTGACGTGC ACACACTGAGGCCTAGCCGACTGCACG | 227 |
| Eo3-78 | CVTPDRLTC | 228 |
| Eo3-80 | ACGTGGCAGACTTAGAGGTCG TGCACCGTCTGAATCTCCAGC | 229 |
| Eo3-80 | TWQTQRS | 230 |
| Eo3-80 | TGTACGTGGCAGACTTAGAGGTCGTGC ACATGCACCGTCTGAATCTCCAGCACG | 231 |
| Eo3-80 | CTWQTQRSC | 232 |
| Eo3-81 | CCGCATCCTGGGACTCGTCAT GGCGTAGGACCCTGAGCAGTA | 233 |
| Eo3-81 | PHPGTRH | 234 |
| Eo3-81 | TGTCCGCATCCTGGGACTCGTCATTGC ACAGGCGTAGGACCCTGAGCAGTAACG | 235 |
| Eo3-81 | CPHPGTRHC | 236 |
| Eo3-82 | GCGCCGAAGCCGCAGTCTCAG CGCGGCTTCGGCGTCAGAGTC | 237 |
| Eo3-82 | APKPQSQ | 238 |
| Eo3-82 | TGTGCGCCGAAGCCGCAGTCTCAGTGC ACACGCGGCTTCGGCGTCAGAGTCACG | 239 |
| Eo3-82 | CAPKPQSQC | 240 |
| Eo3-84 | AGTCAGGCGTAGATTCCTGCG TCAGTCCGCATCTAAGGACGC | 241 |
| Eo3-84 | SQAQIPA | 242 |
| Eo3-84 | TGTAGTCAGGCGTAGATTCCTGCGTGC ACATCAGTCCGCATCTAAGGACGCACG | 243 |
| Eo3-84 | CSQAQIPAC | 244 |
| Eo3-85 | CCGCAGAATAAGGGGAAGGCT GGCGTCTTATTCCCCTTCCGA | 245 |
| Eo3-85 | PQNKGKA | 246 |
| Eo3-85 | TGTCCGCAGAATAAGGGGAAGGCTTGC ACAGGCGTCTTATTCCCCTTCCGAACG | 247 |
| Eo3-85 | CPQNKGKAC | 248 |
| Eo3-86 | CATACTGCGCATCCGCGGTCT GTATGACGCGTAGGCGCCAGA | 249 |
| Eo3-86 | HTAHPRS | 250 |

(continued)

| Name | Sequence | SEQIDNO: |
|---|---|---|
| Eo3-86 | TGTCATACTGCGCATCCGCGGTCTTGC<br>ACAGTATGACGCGTAGGCGCCAGAACG | 251 |
| Eo3-86 | CHTAHPRSC | 252 |
| Eo3-87 | AAGCAGTCTGGTCCTGTTTCT<br>TTCGTCAGACCAGGACAAAGA | 253 |
| Eo3-87 | KQSGPVS | 254 |
| Eo3-87 | TGTAAGCAGTCTGGTCCTGTTTCTTGC<br>ACATTCGTCAGACCAGGACAAAGAACG | 255 |
| Eo3-87 | CKQSGPVSC | 256 |
| Eo3-88 | AGTCAGTATCCGTCGCGTTCT<br>TCAGTCATAGGCAGCGCAAGA | 257 |
| Eo3-88 | SQYPSRS | 258 |
| Eo3-88 | TGTAGTCAGTATCCGTCGCGTTCTTGC<br>ACATCAGTCATAGGCAGCGCAAGAACG | 259 |
| Eo3-88 | CSQYPSRSC | 260 |
| Eo3-89 | TCGAGGGATGGGAAGACTACG<br>AGCTCCCTACCCTTCTGATGC | 261 |
| Eo3-89 | SRDGKTT | 262 |
| Eo3-89 | TGTTCGAGGGATGGGAAGACTACGTGC<br>ACAAGCTCCCTACCCTTCTGATGCACG | 263 |
| Eo3-89 | CSRDGKTTC | 264 |
| Eo3-90 | ACGACGCTGATGCCTAATATT<br>TGCTGCGACTACGGATTATAA | 265 |
| Eo3-90 | TTLMPNI | 266 |
| Eo3-90 | TGTACGACGCTGATGCCTAATATTTGC<br>ACATGCTGCGACTACGGATTATAAACG | 267 |
| Eo3-90 | CTTLMPNIC | 268 |
| Eo3-92 | ACGAATAAGCTTGATAATACT<br>TGCTTATTCGAACTATTATGA | 269 |
| Eo3-92 | TNKLDNT | 270 |
| Eo3-92 | TGTACGAATAAGCTTGATAATACTTGC<br>ACATGCTTATTCGAACTATTATGAACG | 271 |
| Eo3-92 | CTNKLDNTC | 272 |
| Eo3-93 | ACTAAGATGCGGTTGGAGCAG<br>TGATTCTACGCCAACCTCGTC | 273 |
| Eo3-93 | TKMRLEQ | 274 |
| Eo3-93 | TGTACTAAGATGCGGTTGGAGCAGTGC<br>ACATGATTCTACGCCAACCTCGTCACG | 275 |

(continued)

| Name | Sequence | SEQIDNO: |
|---|---|---|
| Eo3-93 | CTKMRLEQC | 276 |
| Eo3-94 | TCGCCGGATCCGGGTAGTAAG<br>AGCGGCCTAGGCCCATCATTC | 277 |
| Eo3-94 | SPDPGSK | 278 |
| Eo3-94 | TGTTCGCCGGATCCGGGTAGTAAGTGC<br>ACAAGCGGCCTAGGCCCATCATTCACG | 279 |
| Eo3-94 | CSPDPGSKC | 280 |
| Eo3-97 | GAGCATTTTTTAGTCGGTCT<br>CTCGTAAAAAATCAGCCAGA | 281 |
| Eo3-97 | EHFFSRS | 282 |
| Eo3-97 | TGTGAGCATTTTTTAGTCGGTCTTGC<br>ACACTCGTAAAAAATCAGCCAGAACG | 283 |
| Eo3-97 | CEHFFSRSC | 284 |
| Eo3-98 | GGGGCGCCGTCTGATCATGTG<br>CCCCGCGGCAGACTAGTACAC | 285 |
| Eo3-98 | GAPSDHV | 286 |
| Eo3-98 | TGTGGGGCGCCGTCTGATCATGTGTGC<br>ACACCCCGCGGCAGACTAGTACACACG | 287 |
| Eo3-98 | CGAPSDHVC | 288 |
| Eo3-99 | CCGCATCCTGGGACTCGTCAT<br>GGCGTAGGACCCTGAGCAGTA | 289 |
| Eo3-99 | PHPGTRH | 290 |
| Eo3-99 | TGTCCGCATCCTGGGACTCGTCATTGC | 291 |
| | ACAGGCGTAGGACCCTGAGCAGTAACG | |
| Eo3-99 | CPHPGTRHC | 292 |
| Eo3-100 | ATTAAGCAGTCGCTGTCTCGT<br>TAATTCGTCAGCGACAGAGCA | 293 |
| Eo3-100 | IKQSLSR | 294 |
| Eo3-100 | TGTATTAAGCAGTCGCTGTCTCGTTGC<br>ACATAATTCGTCAGCGACAGAGCAACG | 295 |
| Eo3-100 | CIKQSLSRC | 296 |
| Eo3-101 | ACGACGCATAATGCGAAGTGG<br>TGCTGCGTATTACGCTTCACC | 297 |
| Eo3-101 | TTHNAKW | 298 |
| Eo3-101 | TGTACGACGCATAATGCGAAGTGGTGC<br>ACATGCTGCGTATTACGCTTCACCACG | 299 |
| Eo3-101 | CTTHNAKWC | 300 |

(continued)

| Name | Sequence | SEQIDNO: |
|---|---|---|
| Eo3-102 | CTGACTACGAAGCCTAGGATG<br>GACTGATGCTTCGGATCCTAC | 301 |
| Eo3-102 | LTTKPRM | 302 |
| Eo3-102 | TGTCTGACTACGAAGCCTAGGATGTGC<br>ACAGACTGATGCTTCGGATCCTACACG | 303 |
| Eo3-102 | CLTTKPRMC | 304 |
| Eo3-103 | AAGCTGAAGTCGGGGTCGCTG<br>TTCGACTTCAGCCCCAGCGAC | 305 |
| Eo3-103 | KLKSGSL | 306 |
| Eo3-103 | TGTAAGCTGAAGTCGGGGTCGCTGTGC<br>ACATTCGACTTCAGCCCCAGCGACACG | 307 |
| Eo3-103 | CKLKSGSLC | 308 |
| Eo3-105 | CTTCCGTCGAAGGTGTCTCGG<br>GAAGGCAGCTTCCACAGAGCC | 309 |
| Eo3-105 | LPSKVSR | 310 |
| Eo3-105 | TGTCTTCCGTCGAAGGTGTCTCGGTGC<br>ACAGAAGGCAGCTTCCACAGAGCCACG | 311 |
| Eo3-105 | CLPSKVSRC | 312 |
| Eo3-106 | GCGCCGGGTCCGTCTAAGAGT<br>CGCGGCCCAGGCAGATTCTCA | 313 |
| Eo3-106 | APGPSKS | 314 |
| Eo3-106 | TGTGCGCCGGGTCCGTCTAAGAGTTGC<br>ACACGCGGCCCAGGCAGATTCTCAACG | 315 |
| Eo3-106 | CAPGPSKSC | 316 |
| Eo3-107 | TCTCCGCTTAAGTCTCTTTCG<br>AGAGGCGAATTCAGAGAAAGC | 317 |
| Eo3-107 | SPLKSLS | 318 |
| Eo3-107 | TGTTCTCCGCTTAAGTCTCTTTCGTGC<br>ACAAGAGGCGAATTCAGAGAAAGCACG | 319 |
| Eo3-107 | CSPLKSLSC | 320 |
| Eo3-108 | GCGCCGGGTCCGTCTAAGAGT<br>CGCGGCCCAGGCAGATTCTCA | 321 |
| Eo3-108 | APGPSKS | 322 |
| Eo3-108 | TGTGCGCCGGGTCCGTCTAAGAGTTGC<br>ACACGCGGCCCAGGCAGATTCTCAACG | 323 |
| Eo3-108 | CAPGPSKSC | 324 |
| Eo3-109 | CCGTCGGGTCTTACTAAGCAG<br>GGCAGCCCAGAATGATTCGTC | 325 |

(continued)

| Name | Sequence | SEQIDNO: |
|---|---|---|
| Eo3-109 | PSGLTKQ | 326 |
| Eo3-109 | TGTCCGTCGGGTCTTACTAAGCAGTGC ACAGGCAGCCCAGAATGATTCGTCACG | 327 |
| Eo3-109 | CPSGLTKQC | 328 |
| Eo3-111 | AAGTCGAATATGCCTCTGAGT TTCAGCTTATACGGAGACTCA | 329 |
| Eo3-111 | KSNMPLT | 330 |
| Eo3-111 | TGTAAGTCGAATATGCCTCTGAGTTGC ACATTCAGCTTATACGGAGACTCAACG | 331 |
| Eo3-111 | CKSNMPLTC | 332 |
| Eo3-112 | AATCAGCGGCATTAGATGTCT TTAGTCGCCGTAATCTACAGA | 333 |
| Eo3-112 | NQRHQMS | 334 |
| Eo3-112 | TGTAATCAGCGGCATTAGATGTCTTGC ACATTAGTCGCCGTAATCTACAGAACG | 335 |
| Eo3-112 | CNQRHQMSC | 336 |
| Eo3-113 | CAGCGGGCGGATCAGAAGCAG GTCGCCCGCCTAGTCTTCGTC | 337 |
| Eo3-113 | QRADQKQ | 338 |
| Eo3-113 | TGTCAGCGGGCGGATCAGAAGCAGTGC ACAGTCGCCCGCCTAGTCTTCGTCACG | 339 |
| Eo3-113 | CQRADQKQC | 340 |
| Eo3-114 | AATCATCGGTATATGCAGATG TTAGTAGCCATATACGTCTAC | 341 |
| Eo3-114 | NHRYMQM | 342 |
| Eo3-114 | TGTAATCATCGGTATATGCAGATGTGC ACATTAGTAGCCATATACGTCTACACG | 343 |
| Eo3-114 | CNHRYMQMC | 344 |
| Eo3-115 | ATTACTCCTATGTCTCGTACT TAATGAGGATACAGAGCATGA | 345 |
| Eo3-115 | ITPMSRT | 346 |
| Eo3-115 | TGTATTACTCCTATGTCTCGTACTTGC ACATAATGAGGATACAGAGCATGAACG | 347 |
| Eo3-115 | CITPMSRTC | 348 |
| Eo3-116 | AGTCCTACGATTGGGCAGAAG TCAGGATGCTAACCCGTCTTC | 349 |
| Eo3-116 | SPTIGQK | 350 |

(continued)

| Name | Sequence | SEQIDNO: |
|---|---|---|
| Eo3-116 | TGTAGTCCTACGATTGGGCAGAAGTGC TAATCAGGATGCTAACCCGTCTTCACG | 351 |
| Eo3-116 | CSPTIGQKC | 352 |
| Eo3-117 | TCTAATTATTCGCTGGGTATG AGATTAATAAGCGACCCATAC | 353 |
| Eo3-117 | SNYSLGM | 354 |
| Eo3-117 | TGTTCTAATTATTCGCTGGGTATGTGC ACAAGATTAATAAGCGACCCATACACG | 355 |
| Eo3-117 | CSNYSLGMC | 356 |
| Eo3-118 | ACGAATACGGGGCATAGGCAT TGCTTATGCCCCGTATCCGTA | 357 |
| Eo3-118 | TNTGHRH | 358 |
| Eo3-118 | TGTACGAATACGGGGCATAGGCATTGC ACATGCTTATGCCCCGTATCCGTAACG | 359 |
| Eo3-118 | CTNTGHRHC | 360 |
| Eo3-119 | ACTATGCGTACTAATTCTAGT TGATACGCATGATTAAGATCA | 361 |
| Eo3-119 | TMRTNSS | 362 |
| Eo3-119 | TGTACTATGCGTACTAATTCTAGTTGC ACATGATACGCATGATTAAGATCAACG | 363 |
| Eo3-119 | CTMRTNSSC | 364 |
| Eo3-120 | ACGGCGCCGTTGGAGCGGAGG TGCCGCGGCAACCTCGCCTCC | 365 |
| Eo3-120 | TAPLERR | 366 |
| Eo3-120 | TGTACGGCGCCGTTGGAGCGGAGGTGC ACATGCCGCGGCAACCTCGCCTCCACG | 367 |
| Eo3-120 | CTAPLERRC | 368 |
| Eo3-122 | CTGCTTGGGGAGCCTCGGACT GACGAACCCCTCGGAGCCTGA | 369 |
| Eo3-122 | LLGEPRT | 370 |
| Eo3-122 | TGTCTGCTTGGGGAGCCTCGGACTTGC ACAGACGAACCCCTCGGAGCCTGAACG | 371 |
| Eo3-122 | CLLGEPRTC | 372 |
| Eo3-123 | TCTCGTGCTAGTACTAATGAT AGAGCACGATCATGATTACTA | 373 |
| Eo3-123 | SRASTND | 374 |
| Eo3-123 | TGTTCTCGTGCTAGTACTAATGATTGC ACAAGAGCACGATCATGATTACTAACG | 375 |
| Eo3-123 | CSRASTNDC | 376 |

(continued)

| Name | Sequence | SEQIDNO: |
|---|---|---|
| Eo3-124 | AATAAGTCGAATAAGGAGTTT<br>TTATTCAGCTTATTCCTCAAA | 377 |
| Eo3-124 | NKSNKEF | 378 |
| Eo3-124 | TGTAATAAGTCGAATAAGGAGTTTTGC<br>ACATTATTCAGCTTATTCCTCAAAACG | 379 |
| Eo3-124 | CNKSNKEFC | 380 |
| Eo3-125 | CATGCGCGGGTGCCGCTGGTT<br>GTACGCGCCCACGGCGACCAA | 381 |
| Eo3-125 | HARVPLV | 382 |
| Eo3-125 | TGTCATGCGCGGGTGCCGCTGGTTTGC<br>ACAGTACGCGCCCACGGCGACCAAACG | 383 |
| Eo3-125 | CHARVPLVC | 384 |
| Eo3-126 | CTTAATAATTCTCAGGCTCAT<br>GAATTATTAAGAGTCCGAGTA | 385 |
| Eo3-126 | LNNSQAH | 386 |
| Eo3-126 | TGTCTTAATAATTCTCAGGCTCATTGC<br>ACAGAATTATTAAGAGTCCGAGTAACG | 387 |
| Eo3-126 | CLNNSQAHC | 388 |
| Eo3-127 | AATCCGTCGCGTTCTACGTCG | 389 |
| | TTAGGCAGCGCAAGATGCAGC | |
| Eo3-127 | NPSRSTS | 390 |
| Eo3-127 | TGTAATCCGTCGCGTTCTACGTCGTGC<br>ACATTAGGCAGCGCAAGATGCAGCACG | 391 |
| Eo3-127 | CNPSRSTSC | 392 |
| Eo3-128 | ACGCCGACTTAGAAGAGTTTG<br>TGCGGCTGAATCTTCTCAAAC | 393 |
| Eo3-128 | TPTQKSL | 394 |
| Eo3-128 | TGTACGCCGACTTAGAAGAGTTTGTGC<br>ACATGCGGCTGAATCTTCTCAAACACG | 395 |
| Eo3-128 | CTPTQKSLC | 396 |
| Eo3-129 | TCGCAGCGGCCTGTTCAGATG<br>AGCGTCGCCGGACAAGTCTAC | 397 |
| Eo3-129 | SQRPVQM | 398 |
| Eo3-129 | TGTTCGCAGCGGCCTGTTCAGATGTGC<br>ACAAGCGTCGCCGGACAAGTCTACACG | 399 |
| Eo3-129 | CSQRPVQMC | 400 |
| Eo3-130 | GCGCCGGGTCCGTCTAAGAGT<br>CGCGGCCCAGGCAGATTCTCA | 401 |
| Eo3-130 | APGPSKS | 402 |

(continued)

| Name | Sequence | SEQIDNO: |
|---|---|---|
| Eo3-130 | TGTGCGCCGGGTCCGTCTAAGAGTTGC ACACGCGGCCCAGGCAGATTCTCAACG | 403 |
| Eo3-130 | CAPGPSKSC | 404 |
| Eo3-132 | AAGGGGTCGTCTATTCTTAAT TTCCCCAGCAGATAAGAATTA | 405 |
| Eo3-132 | KGSSILN | 406 |
| Eo3-132 | TGTAAGGGGTCGTCTATTCTTAATTGC ACATTCCCCAGCAGATAAGAATTAACG | 407 |
| Eo3-132 | CKGSSILNC | 408 |
| Eo3-133 | GTTAATCGGAGTGATGGGATG CAATTAGCCTCACTACCCTAC | 409 |
| Eo3-133 | VNRSDGM | 410 |
| Eo3-133 | TGTGTTAATCGGAGTGATGGGATGTGC ACACAATTAGCCTCACTACCCTACACG | 411 |
| Eo3-133 | CVNRSDGMC | 412 |
| Eo3-134 | CCGCATCCTGGGACTCGTCAT GGCGTAGGACCCTGAGCAGTA | 413 |
| Eo3-134 | PHPGTRH | 414 |
| Eo3-134 | TGTCCGCATCCTGGGACTCGTCATTGC ACAGGCGTAGGACCCTGAGCAGTAACG | 415 |
| Eo3-134 | CPHPGTRHC | 416 |
| Eo3-135 | ATGAATCAGCGGGTTCAGAAT TACTTAGTCGCCCAAGTCTTA | 417 |
| Eo3-135 | MNQRVQN | 418 |
| Eo3-135 | TGTATGAATCAGCGGGTTCAGAATTGC ACATACTTAGTCGCCCAAGTCTTAACG | 419 |
| Eo3-135 | CMNQRVQNC | 420 |
| Eo3-137 | AATCAGTGGAAGTCGGTGTCT TTAGTCACCTTCAGCCACAGA | 421 |
| Eo3-137 | NQWKSVS | 422 |
| Eo3-137 | TGTAATCAGTGGAAGTCGGTGTCTTGC ACATTAGTCACCTTCAGCCACAGAACG | 423 |
| Eo3-137 | CNQWKSVSC | 424 |
| Eo3-138 | CAGACGCATGCTCGGCATGTT GTCTGCGTACGAGCCGTACAA | 425 |
| Eo3-138 | QTHARHV | 426 |
| Eo3-138 | TGTCAGACGCATGCTCGGCATGTTTGC ACAGTCTGCGTACGAGCCGTACAAACG | 427 |

(continued)

| Name | Sequence | SEQIDNO: |
|---|---|---|
| Eo3-138 | CQTHARHVC | 428 |
| Eo3-139 | TTTCAGAATCGTCAGCCGATG<br>AAAGTCTTAGCAGTCGGCTAC | 429 |
| Eo3-139 | FQNRQPM | 430 |
| Eo3-139 | TGTTTTCAGAATCGTCAGCCGATGTGC<br>ACAAAAGTCTTAGCAGTCGGCTACACG | 431 |
| Eo3-139 | CFQNRQPMC | 432 |
| Eo3-140 | AGGGCTCTGGATACGGCGAAT<br>TCCCGAGACCTATGCCGCTTA | 433 |
| Eo3-140 | RALDTAN | 434 |
| Eo3-140 | TGTAGGGCTCTGGATACGGCGAATTGC<br>ACATCCCGAGACCTATGCCGCTTAACG | 435 |
| Eo3-140 | CRALDTANC | 436 |
| Eo3-141 | CAGGAGCCGACGCGGCTGAAG<br>GTCCTCGGCTGCGCCGACTTC | 437 |
| Eo3-141 | QEPTRLK | 438 |
| Eo3-141 | TGTCAGGAGCCGACGCGGCTGAAGTGC<br>ACAGTCCTCGGCTGCGCCGACTTCACG | 439 |
| Eo3-141 | CQEPTRLKC | 440 |
| Eo3-142 | AAGGAGCCTACGAAGGCGCAT<br>TTCCTCGGATGCTTCCGCGTA | 441 |
| Eo3-142 | KEPTKAH | 442 |
| Eo3-142 | TGTAAGGAGCCTACGAAGGCGCATTGC<br>ACATTCCTCGGATGCTTCCGCGTAACG | 443 |
| Eo3-142 | CKEPTKAHC | 444 |
| Eo3-143 | AATGGTAAGGCTAATTGGAAG<br>TTACCATTCCGATTAACCTTC | 445 |
| Eo3-143 | NGKANWK | 446 |
| Eo3-143 | TGTAATGGTAAGGCTAATTGGAAGTGC<br>ACATTACCATTCCGATTAACCTTCACG | 447 |
| Eo3-143 | CNGKANWKC | 448 |

The sequences were analyzed with ClustalW software to identify amino acid motifs that are shared by different peptides. The NCBI/NIH/NLM database was searched for each amino acid peptide sequence. The peptidyl sequence data set was also prioritized according to similarity, that is: a. exact, b. similar, or c. homologous to known proteins. The peptides with greater than 50% homology were aligned and the shared motifs were searched in the online database using BLAST.

Some sequences bore significant homology to the sequences of known proteins. For example, SEQ ID NO: 334 (Eo3-112), SEQ ID NO:336 (Eo3-112), SEQ ID NO:342 (Eo3-114), SEQ ID NO:344 (Eo3-114), SEQ ID NO:410 (Eo3-133) and SEQ ID NO:412 (Eo3-133) were similar to Tissue Inhibitor of Metalloproteinase 2 (TIMP-2). In particular, SEQ ID NO:412 with sequence CVNRSDGMC is homologous to CIKRSDGSC (SEQ ID NO:452) of TIMP-2. Similarly, SEQ ID NO:334 and 336 with sequence NQRHQMSC is homologous to TIMP-2 at positions 145 -152 (NQRYQMGC,

SEQ ID NO:454). Also, SEQ ID NO:342 and 344 with sequence NHRYMQM is homologous to chicken TIMP-2 at positions 145 -152 (NHRY---QMGC, SEQ ID NO:456).

Some proteins were homologous to peptide CAPGPSKSC (SEQ ID NO:4), including subtilisin inhibitor at positions 66-77 (CAPGPS, SEQ ID NO:458), chymase at positions 46-51 (GPSKSC, SEQ ID NO:460) and CDA05 (Genbank Accession No. AAK14929.1) at positions 221- 226 (APGPSK, SEQ ID NO:462).

The considerable number of peptidyl sequences found to associate with plaque surfaces suggests expression of multiple membrane targets on these endothelial cells, indicative of a positional gene expression or molecular processing different from endothelium elsewhere in the vascular tree.

### In Vivo Imaging Using Phage Displaying Peptides

Mice were injected with about $10^{11}$ phage bearing CAPGDSKSD (SEQ ID NO:4) or with control phage without this peptide sequence. After about 30 min, the aortic tissue was perfusion-fixed with paraformaldehyde and removed. Aortic tissue was then stained with streptavidin hydroxylase conjugate.

Figure 1 provides images of aortic specimens from mice injected with such phage. Binding of phage to aorta was visualized with biotinylated anti-phage antibody and avidin-linked enzyme activation of DAB. Phage bearing the CAPGDSKSD (SEQ ID ÑO:4) peptide showed preferential binding to atherosclerotic lesions (Figure 1D). Figure 1A shows a young non-atherosclerotic Apo E knockout mouse injected with CAPGPSKSC (SEQ ID NO:4) phage. In the aorta of these mice, atherosclerotic lesions have not yet developed, and no phage binding to the aorta is detectable. Figure 1B shows a normal aorta from a Balb/C mouse injected with CAPGPSKSC (SEQ ID NO:4) phage. No association of phage with the aortic surface is observed, suggesting that the molecule that binds CAPGPSKSC (SEQ ID NO:4) is not present in detectable quantity on the surface of normal aorta endothelium. Figure 1C shows an aorta from an Apo B knockout mouse fed a high fat diet that was injected with $10^{11}$ control phage. Atherosclerotic lesions are clearly visible, however no detectable association of control phage with the lesions is observed. Figure 1D shows an aorta of an atherosclerotic Apo E knockout mouse fed a high fat diet and injected with $10^{11}$ CAPGPSKSC (SEQ ID NO:4) phage. Phage binding to the lesions is readily visualized.

Immunostaining showed that the phage expressing CAPGPSKSC (SEQ D NO:4) preferentially bound to atherosclerotic lesions in the Apo B knockout mouse aorta. Binding of the CAPGPSKSC (SEQ ID NO:4) expressing phage was more intense at the edge of the lesions.

The intensity of the phage binding at the edge of lesions indicates that the target for this peptide must be present on the surface of these potentially activated endothelial cells. Endothelial cells grow as strict monolayers. When wounding occurs that disrupts the endothelial monolayer at the center of atherosclerotic lesions, cells at the edge of the lesion will replicate and migrate into the lesion to reestablish the integrity of the endothelial lining. In aortic arch lesions, binding of the peptide was consistently absent in the center of lesions that coincide with endothelial quiescence, possibly denudation and/or wounding.

These data suggest the CAPGPSKSC (SEQ ID NQ:4) expressing phage is recognizing a target molecule on the endothelial cell surface, rather than binding to the sub-endothelial matrix proteins, and/or that the target molecule may be associated with activated endothelial cells.

### Peptide CAPGPSKSC (SEQ ID NO:4) Binds *In Vivo* to Atherosclerotic Lesions

To verify that the peptidyl sequence CAPGPSKSC (SEQ ID NO:4) that is expressed on phage protein III was responsible for the selective localization to atherosclerotic lesions, a biotinylated peptide containing the core sequence CAPGPSKSC (SEQ ID NO:4) was synthesized, folded, disulfide bonds constituted by oxidization, and compositional validity established by mass spectrometry. The peptide was injected into atherosclerotic ApoE knockout mice via tail vein, and the tissues were fixed by *in vivo* perfusion, and aortas were freed by dissection. The binding of biotinylated peptide to the aorta was visualized with strepavidin conjugated peroxidase and DAB substrate. The peptide binding replicated the CAPGPSKSC (SEQ ID NO:4) phage binding to the lesions (Figure 2A), supporting that the association of CAPGPSKSC (SEQ ID NO:4) phage with atherosclerotic lesions was mediated by the fused peptidyl sequence.

The association of this peptide to the atherosclerotic lesions was also analyzed on tissue sections. The experiment was replicated in atherosclerotic ApoE mice by infusing CAPGPSKSC (SEQ ID NO:4) peptide and sections of the aorta were prepared, detected with strepavidin-conjugated peroxidase and developed with DAB substrate. Peptide binding to the surface of atherosclerotic lesions (Figure 2B) was quite evident. In contrast, no substantial signal was evident elsewhere. Further, there was a differential pattern observed in which the peptide appeared to bind more to the endothelium at the periphery of the lesions and less to the central aspect of the lesions. This suggests that the endothelium at the periphery express the target at greater density. These areas are likely involved in lesion formation and differ to some degree in their phenotype compared to that of the overlying endothelium.

### Peptide CAPGPSKSC (SEQ ID NO:4) Binds to Human Atherosclerotic Lesions

The biotinylated CAPGPSKSC (SEQ ID NO:4) peptide was used to probe the human vascular samples with atherosclerotic lesions. Figure 3B shows an atherosclerotic lesion in a human arterial specimen after application of biotinylated CAPGPSKSC (SEQ ID NO:4) peptide directly to the luminal surface. The biotinylated CAPGPSKSC (SEQ ID NO:4) peptide exhibited no observable binding to non-atherosclerotic arterial samples or veins. Instead, the peptide preferentially bound to the atherosclerotic plaque (compare Figures 3A and 3B) indicating the molecular target of this peptide exists in human lesions. In contrast, a peptide control with the same amino acid composition as CAPGPSKSC (SEQ ID NO:4) but with a scrambled sequence exhibited no observable binding to atherosclerotic arterial samples.

### An 82 Kd Protein Is The Target of the CAPGPSKSC (SEQ ID NO:4) Peptide

Peptide CAPGPSKSC (SEQ ID NO:4) bound two protein bands on a blot of a protein lysate of the mouse endothelial cells (bEND.3). See Figure 4. The sizes of the two proteins that bound the CAPGPSKSC (SEQ ID NO:4) peptide were 82 kilodaltons (P82) and 120 kilodaltons (P120). The sharpness of the detected bands suggests that these target proteins are not glycoproteins. The P82 protein was also detected in membrane fractions partially purified by Triton X 114 extraction. These data suggest that the P82 protein is a membrane protein. The sizes of these proteins indicates that neither one is vascular cell adhesion molecule-1 (VCAM-1), which is a member of the immunoglobulin (Ig) supergene family expressed on activated, but not resting, endothelium.

Figure 4 is a photograph of a Western blot identifying the target bi omolecule of the CAPGPSKSC (SEQ ID NO:4) peptide in mouse endothelial cells (Bend-3). The total protein lysate (lane 1) and membrane preparations were separated on a SDS polyacrylamide gel and then transferred to a nitrocellulose membrane. The membrane was probed with biotinylated peptide CAPGPSKSC (SEQ ID NO:4). Two sharp bands were observed on a Western blot of a whole cell lysate of mouse endothelial cell line (Bend-3), using the biotinylated CAPGPSKSC (SEQ ID NO:4) peptide to detect target proteins. The sizes of the proteins bound by CAPGPSKSC (SEQ ID NO:4) peptide were about 82 kilodaltons (P82) and about 120 kilodaltons (P120). The sharpness of the detected bands suggests that these target proteins are not glycoproteins. The P82 protein was also detected, and apparently enriched, in membrane fractions partially purified by Triton X 114 extraction. These data suggest that the P82 protein is a membrane protein.

### References

1. Libby, P. 2000. Changing concepts of atherogenesis. J. Intern. Med. 247:349-358
2. Faggiotto, A., and Ross, R.. 1984. Studies of hypercholesterolemia in the nonhuman primate. II. Fatty streak conversion to fibrous plaque. Arteriosclerosis 4:341-356.
3. Rosenfeld, M.E., Tsukada, T., Gown, A.M., and Ross, R. 1987. Fatty streak initiation in Watanabe Heritable Hyperlipemic and comparably hypercholesterolemic fat-fed rabbits. Arteriosclerosis 7:9-23.
4. Ross, R. 1993. The pathogenesis of atherosclerosis: a perspective for the 1990s. Nature. 362:801-809.
5. Ross, R. 1993. Atherosclerosis: current understanding of mechanisms and future strategies in therapy. Transplant. Proc. 25:2041-2043.
6. Fuster, V. 1994. Lewis A. Conner Memorial Lecture. Mechanisms leading to myocardial infarction: insights from studies of vascular biology [published erratum appears in Circulation 1995 Jan 1;91(1):256]. Circulation. 90: 2126-2146.
7. Dong, Z.M., Chapman, S.M., Brown, A.A., Frenette, P.S., Hynes, R.O., and Wagner, D.D. 1998. The combined role ofP- and E-selectins in atherosclerosis. J. Clin. Invest. 102:145-152.
8. Cybulsky, M.I., and Gimbrone, M.A., Jr. 1991. Endothelial expression of a mononuclear leukocyte adhesion molecule during atherogenesis. Science. 251:788-791.
9. Boisvert, W.A., Black, A.S., and Curtiss, L.K. 1999. ApoA1 reduces free cholesterol accumulation in atherosclerotic lesions of ApoE-deficient mice transplanted with ApoE-expressing macrophages. Arterioscler. Thromb. Vasc. Biol. 19:525-530.
10. Curtiss, L.K., and Boisvert, W.A. 2000. Apolipoprotein E and atherosclerosis. Curr. Opin. Lipidol. 11:243-251.
11. Pasqualini, R., and Ruoslahti, E. 1996. Organ targeting in vivo using phage display peptide libraries. Nature 380:364-366.
12. Vachon, V., Pouliot. J.F., Laprade, R., and Beliveau, R. 1991. Fractionation of renal brush border membrane proteins with Triton X-114 phase partitioning. Biochem. Cell Biol. 69:206-211.
13. Fernandez-Catalan, C., Bode. W., Huber, R., Turk, D., Calvete, J.J., Lichte, A., Tschesche, H., and Maskos, K. 1998. Crystal structure of the complex formed by the membrane type 1-matrix metalloproteinase with the tissue

inhibitor ofmetalloproteinases-2, the soluble progelatinase A receptor. Embo J. 17:5238-5248.
14. Chesler, L., Golde, D.W., Bersch, N., and Johnson, M.D. 1995. Metalloproteinase inhibition and erythroid potentiation are independent activities of tissue inhibitor of metalloproteinases-1. Blood 86:4506-4515.
15. Corcoran, M.L., and Stetler-Stevenson, W.G. 1995. Tissue inhibitor of metalloproteinase-2 stimulates fibroblast proliferation via a cAMP-dependent mechanism. J. Biol. Chem. 270:13453-13459.
16. Kolonin, M., Pasqualini, R., and Arap, W. 2001. Molecular addresses in blood vessels as targets for therapy. Curr. Opin. Chem. Biol. 5:308-313.
17. Tressler, R.J., Belloni, P.N., and Nicolson, G.L. 1989. Correlation of inhibition of adhesion of large cell lymphoma and hepatic sinusoidal endothelial cells by RGD-containing peptide polymers with metastatic potential: role of integrin-dependent and -independent adhesion mechanisms. Cancer Commun. 1:55-63.
18. van der Wal, A.C., Becker, A.E., van der Loos, C.M., and Das, P.K. 1994. Site of intimal rupture or erosion of thrombosed coronary atherosclerotic plaques is characterized by an inflammatory process irrespective of the dominant plaque morphology. Circulation 89:36-44.
19. Lendon, C.L., Davies, M.J., Born, G.V., and Richardson, P.D. 1991. Atherosclerotic plaque caps are locally weakened when macrophages density is increased. Atherosclerosis 87:87-90.
20. Krieger, M., Acton, S., Ashkenas, J., Pearson, A., Penman, M., and Resnick, D. 1993. Molecular flypaper, host defense, and atherosclerosis. Structure, binding properties, and functions of macrophage scavenger receptors. J. Biol. Chem. 268:4569-4572.
21. Endemann, G., Stanton, L.W., Madden, K.S., Bryant, C.M., White, R.T., and Protter, A.A. 1993. CD36 is a receptor for oxidized low density lipoprotein. J. Biol. Chem. 268:11811-11816.
22. Ottnad, E., Parthasarathy, S., Sambrano, G.R., Ramprasad, M.P., Quehenberger, O., Kondratenko, N., Green, S., and Steinberg, D. 1995. A macrophage receptor for oxidized low density lipoprotein distinct from the receptor for acetyl low density lipoprotein: partial purification and role in recognition of oxidatively damaged cells. Proc. Natl. Acad. Sci. USA. 92:1391-1395.
23. Ramprasad, M.P., Fischer, W., Witztum, J.L., Sambrano, G.R., Quehenberger, O., and Steinberg, D. 1995. The 94- to 97-KDa mouse macrophage membrane protein that recognizes oxidized low density lipoprotein and phosphatidylserine-rich liposomes is identical to macrosialin, the mouse homologue of human CD68. Proc. Natl. Acad. Sci. USA. 92:9580-9584.
24. Acton, S., Rigotti, A., Landschulz, K.T., Xu, S., Hobbs, H.H., and Krieger, M. 1996. Identification of scavenger receptor SR-BI as a high density lipoprotein receptor. Science 271:518-520.
25. Gu, X., Trigatti, B., Xu, S., Acton, S., Babitt, J., and Krieger, M.. 1998. The efficient cellular uptake of high density lipoprotein lipids via scavenger receptor class B type I requires not only receptor-mediated surface binding but also receptor-specific lipid transfer mediated by its extracellular domain. J. Biol. Chem. 273:26338-26348.
26. Watson, A.D., Leitinger, N., Navab; M., Faull, K.F., Horkko, S., Witztum, J.L., Palinski, W., Schwenke, D., Salomon, R.G., Sha, W., Subbanagounder, G., Fogelman; A.M., and Berliner, J.A. 1997. Structural identification by mass spectrometry of oxidized phospholipids in minimally oxidized low density lipoprotein that induce monocyte/endothelial interactions and evidence for their presence in vivo. J. Biol. Chem. 272:13597-13607.
27. Witztum, J.L., and Berliner, J.A. 1998. Oxidized phospholipids and isoprostanes in atherosclerosis. Curr. Opin. Lipidol. 9:441-448.
28. Berliner, J., Leitinger, N., Watson, A., Huber, J., Fogelman, A., and Navab, M. 1997. Oxidized lipids in atherogenesis: formation, destruction and action. Thromb. Haemost. 78:195-199.
29. Gimbrone, M.A., Jr., Topper, J.N., Nagel, T., Anderson, K.R., and Garcia-Cardena, G. 2000. Endothelial dysfunction, hemodynamic forces, and atherogenesis. Ann. N Y Acad. Sci. 902:230-239; discussion 239-240.
30. Gimbrone, M.A., Jr., Nagel, T., and Topper, J.N. 1997. Biomechanical activation: an emerging paradigm in endothelial adhesion biology. J. Clin. Invest. 99:1809-1813.
31. Carlos, T.M., and Harlan, J.M. 1994. Leukocyte-endothelial adhesion molecules. Blood 84:2068-2101.
32. Frenette, P.S., and Wagner, D.D. 1996. Adhesion molecules-Part 1. N. Engl. J. Med. 334:1526-1529.
33. Frenette, P.S., and Wagner, D.D. 1996. Adhesion molecules--Part II: Blood vessels and blood cells. N. Engl. J. Med. 335:43-45.

## Claims

1. An isolated peptide comprising SEQ ID NO:4, which peptide is capable of binding to an atherosclerotic lesion in a mammal.

2. The isolated peptide according to claim 1 wherein the peptide is conjugated to a therapeutic agent or a reporter molecule.

3. Use of a peptide conjugated to a therapeutic agent according to claim 2 for the preparation of a medicament for the treatment of atherosclerosis in a mammal.

4. The isolated peptide according to claim 2 wherein the therapeutic agent is:

   a) an agent that can modulate lipid levels in mammalian serum selected from the group consisting of an HMG-CoA reductase inhibitor, a thyromimetic, a fibrate, and an agonist of peroxisome proliferator-activated receptors (PPAR);
   b) an agent that can modulate an oxidative process in a mammal;
   c) an agent that can modulate insulin resistance or glucose metabolism in a mammal selected from the group consisting of PPAR-alpha, PPAR-gamma, PPAR-delta, a modifier of DPP-IV, and a modifier of a glucocorticoid receptor;
   d) an agent that can modulate expression of an endothelial cell receptor, an endothelial cell adhesion molecule, an endothelial cell integrin, a smooth muscle cell receptor, a smooth muscle cell adhesion molecule or a smooth muscle cell integrin;
   e) an agent that can modulate the proliferation of an endothelial cell or a smooth muscle cell in a mammalian blood vessel;
   f) an agent that can modulate an inflammation associated receptor selected from the group consisting of a chemokine receptor, a RAGE receptor, a toll-like receptor, an angiotensin receptor, a TGF receptor, an interleukin receptor, a TNF receptor, a C-reactive protein receptor, and a receptor that can activate NF-κb;
   g) an agent that can modulate proliferation, apoptosis or necrosis of endothelial cells, vascular smooth muscle cells, lymphocytes, monocytes, or neutrophils;
   h) an agent that can modulate production, degradation, or cross-linking of an extracellular matrix protein selected from the group consisting of collagen, an elastin, and a proteoglycan;
   i) an agent that can modulate activation, secretion or lipid loading of a cell within a mammalian blood vessel;
   j) an agent that can modulate activation or proliferation of a dendritic cell within a mammalian blood vessel;
   k) an agent that can modulate activation or adhesion of a platelet at a mammalian blood vessel wall;
   l) an agent consisting of a nucleic acid that encodes a protein therapeutic agent, wherein the protein therapeutic agent has an *in vivo* activity that is beneficial to a mammal suffering from an atherosclerotic lesion.

5. A pharmaceutical composition comprising the peptide according to claims 1,2 or 4 and a pharmaceutically acceptable carrier.

6. An isolated nucleic acid encoding a peptide comprising SEQ ID NO:4, wherein the encoded peptide is capable of binding to an atherosclerotic lesion in a mammal.

7. An isolated nucleic acid capable of hybridizing under stringent conditions to the isolated nucleic acid of claim 6,

wherein the stringent hybridization conditions comprise hybridization in 6 x SSC and at 55˚C.

## Patentansprüche

1. Isoliertes Peptid, das ein SEQ ID NR:4 umfasst, wobei dieses Peptid zur Bindung an eine atherosklerotische Läsion bei einem Säuger befähigt ist.

2. Isoliertes Peptid nach Anspruch 1, worin das Peptid an ein therapeutisches Mittel oder ein Reportermolekül konjugiert ist.

3. Verwendung eines Peptids, das an ein therapeutisches Mittel konjugiert ist, nach Anspruch 2 zur Herstellung eines Arzneimittels für die Behandlung von Atherosklerose bei einem Säuger.

4. Isoliertes Peptid nach Anspruch 2, worin das therapeutische Mittel folgendes ist:

   a) ein Mittel, das Lipidspiegel in einem Säugerserum modulieren kann und das ausgewählt ist aus der Gruppe, die besteht aus einem HMG-CoA Reduktaseinhibitor, einem Thyromimetikum, einem Fibrat und einem Agonisten von Peroxisom-Proliferator-aktivierten Rezeptoren (PPAR),
   b) ein Mittel, das einen oxidativen Prozess in einem Säuger modulieren kann,
   c) ein Mittel, das eine Insulinresistenz oder einen Glucosemetabolismus in einem Säuger modulieren kann, wobei dieses Mittel ausgewählt ist aus der Gruppe, die besteht aus PPAR-alpha, PPAR-gamma, PPAR-delta, einem Modifikator von DPP-IV und einem Modifikator eines Glucocorticoidrezeptors,
   d) ein Mittel, das eine Expression eines Endothelzellrezeptors, eines Endolthelzelladhäsionsmoleküls, eines Endothelzellintegrins, eines Zellrezeptors einer glatten Muskulatur, eines Zelladhäsionsmoleküls einer glatten Muskulatur oder eines Zellintegrins einer glatten Muskulatur modulieren kann,
   e) ein Mittel, das die Proliferation einer Endothelzelle oder einer Zelle einer glatten Muskulatur in einem Blutgefäß eines Säugers modulieren kann,
   f) ein Mittel, das einen mit einer Inflammation assoziierten Rezeptor modulieren kann, der ausgewählt ist aus der Gruppe, die besteht aus einem Chemokinrezeptor, einem RAGE Rezeptor, einem Toll-like Rezeptor, einem Angiotensinrezeptor, einem TGF Rezeptor, einem Interleukinrezeptor, einem TNF Rezeptor, einem C-reaktiven Proteinrezeptor und einem Rezeptor, der NF-κb aktivieren kann,
   g) ein Mittel, das eine Proliferation, eine Apoptose oder Nekrose von Endothelzellen, vaskularen glatten Muskelzellen, Lymphozyten, Monozyten oder Neutrophilen modulieren kann,
   h) ein Mittel, das eine Produktion, einen Abbau oder eine Vernetzung eines extrazellularen Matrixproteins modulieren kann, das aus der Gruppe ausgewählt ist, die besteht aus Kollagen, einem Elastin und einem Proteoglycan,
   i) ein Mittel, das eine Aktivierung, Sekretion oder Lipidbeladung einer Zelle innerhalb eines Blutgefäßes eines Säugers modulieren kann,
   j) ein Mittel, das eine Aktivierung oder Proliferation einer dendritischen Zelle innerhalb eines Blutgefäßes eines Säugers modulieren kann,
   k) ein Mittel, das eine Aktivierung oder Adhäsion eines Plättchens an einer Wand eines Blutgefäßes bei einem Säuger modulieren kann, und
   l) ein Mittel, das aus einer Nukleinsäure besteht, die für ein therapeutisch wirksames Protein kodiert, worin das therapeutisch wirksame Protein eine in vivo Aktivität aufweist, die für einen Säuger vorteilhaft ist, der an einer atherosklerotischen Läsion leidet.

5. Pharmazeutische Zusammensetzung, umfassend das Peptid nach einem der Ansprüche 1, 2 oder 4, und einen pharmazeutisch akzeptablen Träger.

6. Isolierte Nukleinsäure, die für ein Peptid kodiert, das SEQ ID NR:4 umfasst, worin das kodierte Peptid zu einer Bindung an eine atherosklerotische Läsion bei einem Säuger befähigt ist.

7. Isolierte Nukleinsäure, die unter stringenten Bedingungen zu einer Hybridisierung an die isolierte Nukleinsäure nach Anspruch 6 fähig ist, worin die stringenten Bedingungen einer solchen Hybridisierung eine Hybridisierung in 6 x SSC und bei 55 ˚C umfassen.

**Revendications**

1. Peptide isolé comprenant la SEQ ID N° 4, ledit peptide étant capable de se lier à une lésion athéroscléreuse chez un mammifère.

2. Peptide isolé selon la revendication 1, dans lequel le peptide est conjugué à un agent thérapeutique ou à une molécule rapportrice.

3. Utilisation d'un peptide conjugué à un agent thérapeutique selon la revendication 2 pour la préparation d'un médicament pour le traitement de l'athérosclérose chez un mammifère.

4. Peptide isolé selon la revendication 2, dans lequel l'agent thérapeutique est :

   a) un agent qui peut moduler les niveaux de lipides dans le sérum d'un mammifère, choisi dans le groupe constitué par un inhibiteur de HMG-CoA-réductase, un thyromimétique, un fibrate, et un agoniste des récepteurs aux facteurs activés de prolifération du peroxysome (PPAR) ;
   b) un agent qui peut moduler un processus oxydatif chez un mammifère ;
   c) un agent qui peut moduler la résistance à l'insuline ou le métabolisme du glucose chez un mammifère, choisi dans le groupe constitué par PPAR-alpha, PPAR-gamma, PPAR-delta, un modificateur de DPP-IV, et un modificateur d'un récepteur de glucocorticoïde ;
   d) un agent qui peut moduler l'expression d'un récepteur de cellules endothéliales, d'une molécule d'adhérence de cellules endothéliales, d'une intégrine de cellules endothéliales, d'un récepteur cellulaire du muscle lisse, d'une molécule d'adhérence cellulaire du muscle lisse ou d'une intégrine cellulaire du muscle lisse ;
   e) un agent qui peut moduler la prolifération d'une cellule endothéliale ou d'une cellule du muscle lisse dans un vaisseau sanguin d'un mammifère ;
   f) un agent qui peut moduler un récepteur associé à une inflammation, choisi dans le groupe constitué par un récepteur de chimiokine, un récepteur de RAGE, un récepteur TLR (Toll-like receptor), un récepteur d'angiotensine, un récepteur de TGF, un récepteur d'interleukine, un récepteur de TNF, un récepteur de la protéine C réactive (CRP), et un récepteur qui peut activer NF-κb ;
   g) un agent qui peut moduler la prolifération, l'apoptose ou la nécrose de cellules endothéliales, de cellules vasculaires du muscle lisse, de lymphocytes, de monocytes, ou de neutrophiles ;
   h) un agent qui peut moduler la production, la dégradation ou la réticulation d'une protéine de matrice extracellulaire choisie dans le groupe constitué par le collagène, l'élastine, et un protéoglycane ;
   i) un agent qui peut moduler l'activation, la sécrétion ou la charge lipidique d'une cellule à l'intérieur d'un vaisseau sanguin de mammifère ;
   j) un agent qui peut moduler l'activation ou la prolifération d'une cellule dendritique à l'intérieur d'un vaisseau sanguin de mammifère ;
   k) un agent qui peut moduler l'activation ou l'adhérence d'une plaquette à la paroi d'un vaisseau sanguin de mammifère ;
   l) un agent constitué d'un acide nucléique qui code pour un agent thérapeutique protéique, l'agent thérapeutique protéique présentant une activité *in vivo* qui est bénéfique pour un mammifère souffrant d'une lésion athéroscléreuse.

5. Composition pharmaceutique comprenant le peptide selon la revendication 1, 2 ou 4, et un véhicule pharmaceutiquement acceptable.

6. Acide nucléique isolé codant pour un peptide comprenant la SEQ ID N° 4, dans lequel le peptide codé est capable de se lier à une lésion athéroscléreuse chez un mammifère.

7. Acide nucléique isolé capable de s'hybrider dans des conditions stringentes à l'acide nucléique isolé selon la revendication 6, les conditions d'hybridation stringentes comprenant une hybridation dans 6 x SSC et une température de 55 °C.

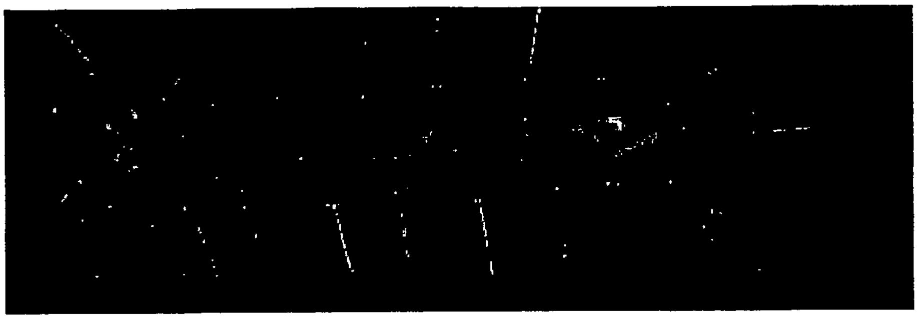

FIG. 1A

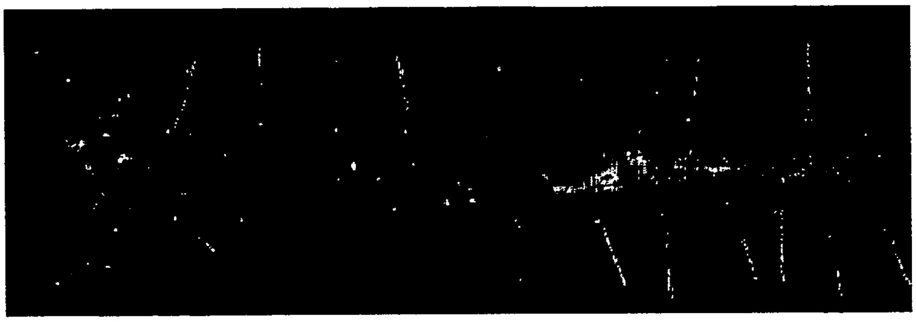

FIG. 1B

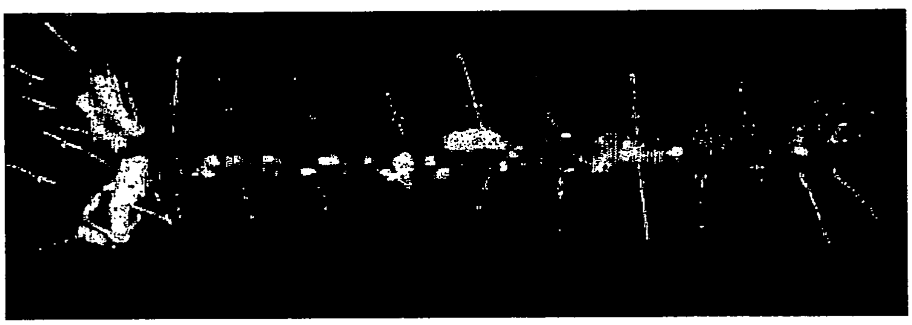

FIG. 1C

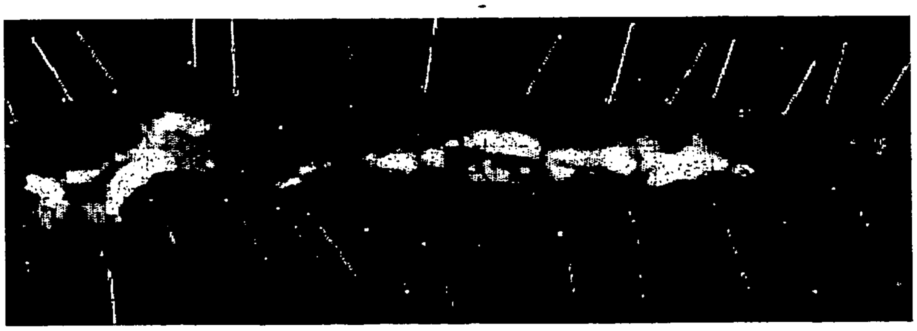

FIG. 1D

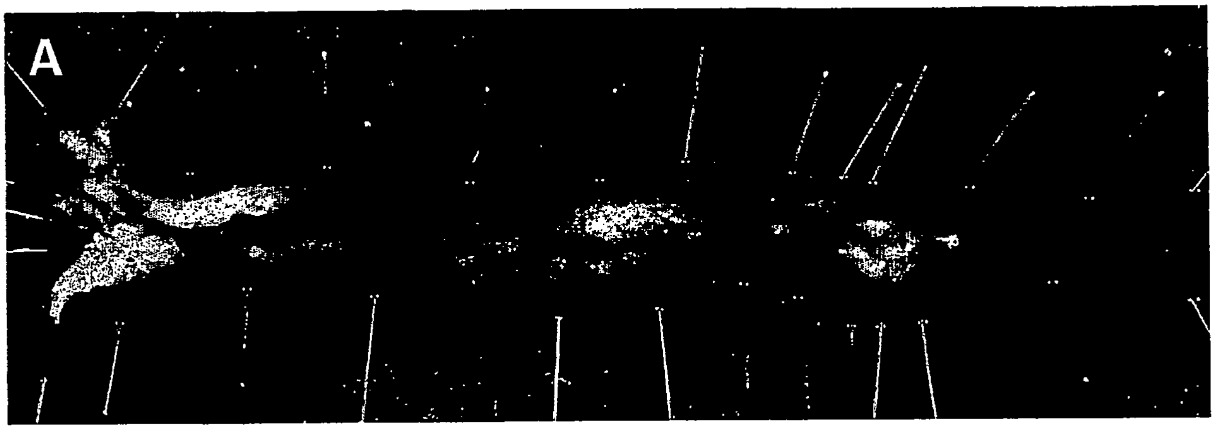


FIG. 2A

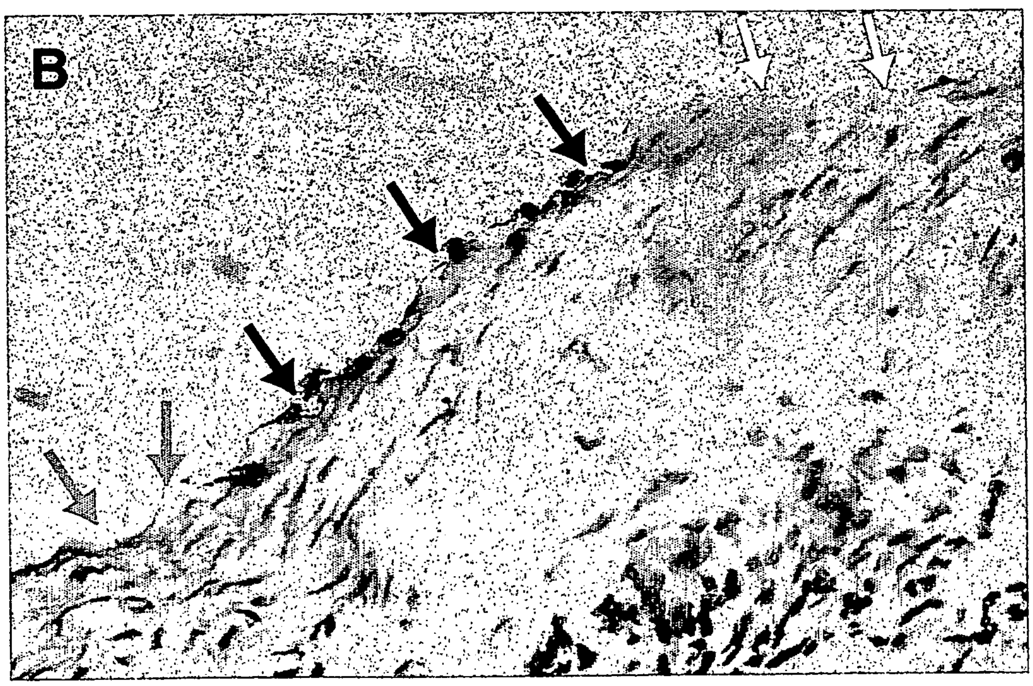


FIG. 2B

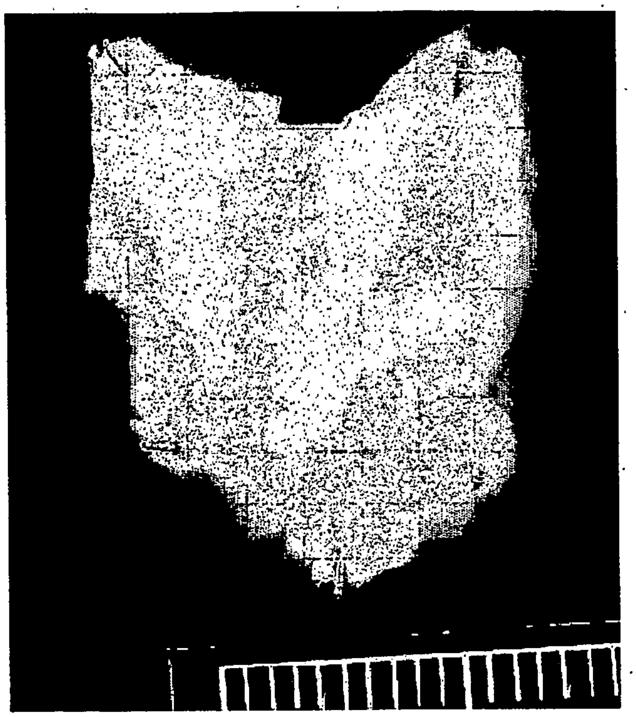

FIG. 3A

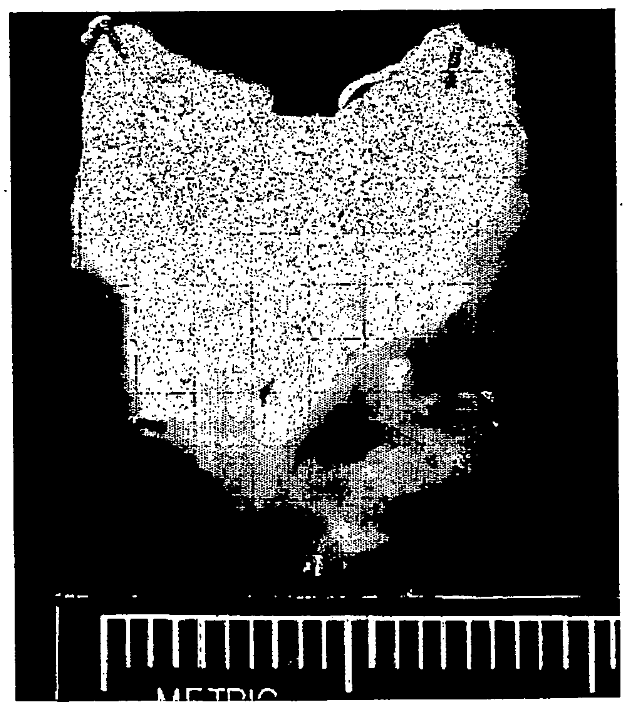

FIG. 3B

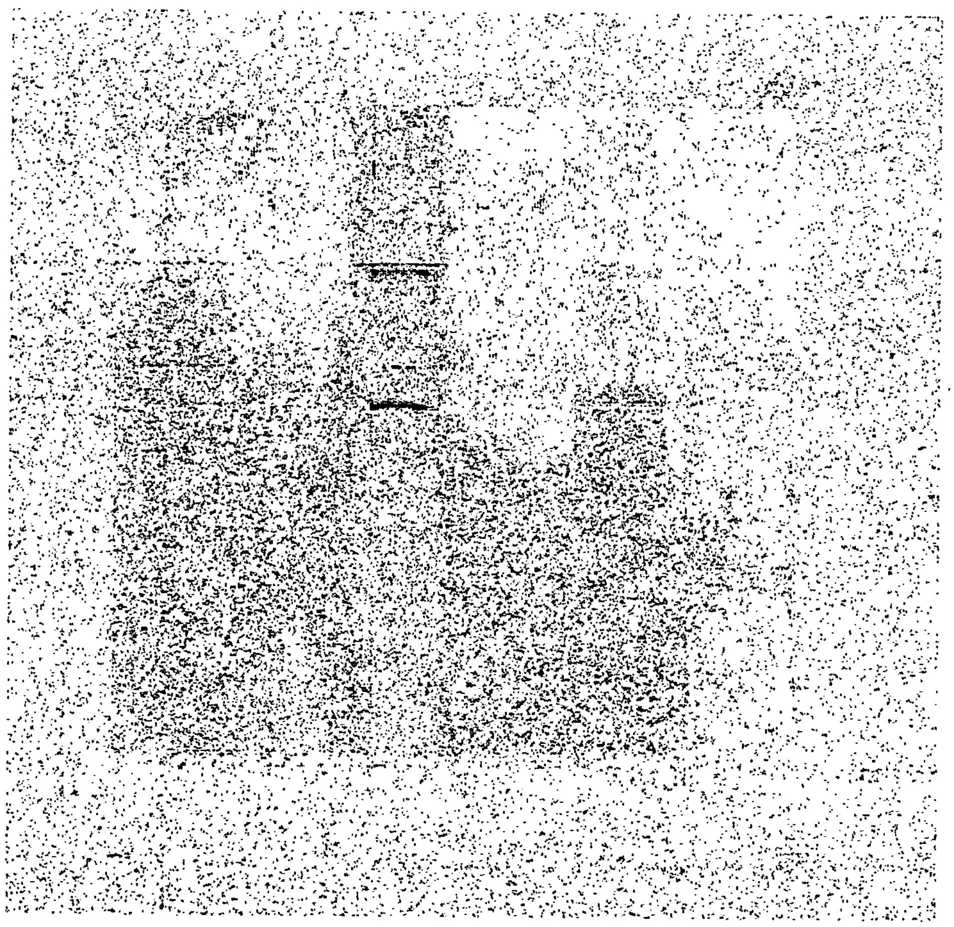

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5827516 A
- WO 9429333 A
- WO 0132853 A
- WO 9413327 A
- US 3817837 A
- US 3850752 A
- US 3939350 A
- US 3996345 A
- US 4277437 A
- US 4275149 A
- US 4366241 A
- US 6139819 A, Unger
- US 6107459 A, Dean
- US 5501979 A
- WO 0144458 A
- US 09734836 B
- US 6277633 B
- US 5380830 B
- EP 680320 A
- EP 491226 A
- US 5354772 A
- EP 304063 A
- EP 22478 A
- GB 2077264 A
- EP 33538 A
- US 6008239 A
- WO 9727847 A
- WO 9727857 A
- WO 9728115 A
- WO 9728137 A
- WO 9728149 A
- WO 9908501 A
- WO 97128137 A
- WO 9819998 A
- DE 19616486 A1
- WO 0034241 A
- WO 9515309 A
- WO 0172290 A
- WO 0152825 A
- WO 9310127 A
- WO 9925719 A
- WO 9938501 A
- WO 9946272 A
- WO 9967278 A
- WO 9967279 A
- US 6110949 A
- WO 9529691 A
- EP 580550 A
- US 5654468 A
- US 5569674 A
- US 5401772 A
- US 4069343 A
- US 4554290 A
- US 4766121 A
- US 4826876 A
- US 4910305 A
- US 5061798 A
- US 5232947 A
- US 5284971 A
- WO 0058279 A
- WO 0051971 A
- EP 45665 A
- CA 9739405
- US 6235469 B
- US 60311507 B

### Non-patent literature cited in the description


- **LIBBY, P.** Changing concepts of atherogenesis. *J. Internal Medicine,* 2000, vol. 247, 349-58
- **GEYSEN et al.** *Proc. Natl. Acad. Sci. USA,* 1984, vol. 81, 3998-4002
- **MAEJI et al.** *J. Immunol. Methods,* 1992, vol. 146, 83-90
- **FODOR et al.** *Science,* 1991, vol. 251, 767-775
- **LAM et al.** *Nature,* 1991, vol. 354, 82-84
- **HOUGHTEN et al.** *Nature,* 1991, vol. 354, 84-86
- **CWIRLA et al.** *Proc. Natl. Acad. Sci. USA,* 1990, vol. 87, 6378-6382
- **SCOTT et al.** *Science,* 1990, vol. 249, 386-390
- **DEVLIN et al.** *Science,* 1990, vol. 249, 404-406
- **FELICI et al.** *Gene,* 1993, vol. 128, 21-27
- **O'NEIL et al.** *Proteins: Structure, Function and Genetics,* 1992, vol. 14, 509-515
- **FRANK. R.** *Tetrahedron,* 1992, vol. 48 (42), 9217-9232

- **FAVRE M. et al.** *J. Am. Chem. Soc.,* vol. 121, 2679-2685
- **BENGTSSON M et al.** *Glycoconjugate Journal,* 1998, vol. 15, 223-231
- **FASMAN.** CRC Practical Handbook of Biochemistry and Molecular Biology. CRC Press, Inc, 1989
- **BACIC, G. ; M. R. NIESMAN et al.** NMR and ESR study of liposome delivery of Mn2+ to murine liver. *Magn Reson Med,* 1990, vol. 13 (1), 44-61
- **BARTOLOZZI, C. ; F. DONATI et al.** MnDPDP-enhanced MRI vs dual-phase spiral CT in the detection of hepatocellular carcinoma in cirrhosis. *Eur Radiol,* 2000, vol. 10 (11), 1697-702
- **BOCKHORST, K. ; M. HOEHN-BERLAGE et al.** NMR-contrast enhancement of experimental brain tumors with MnTPPS: qualitative evaluation by in vivo relaxometry. *Magn Reson Imaging,* 1993, vol. 11 (5), 655-63
- **COLET, J. M. ; L. VANDER ELST et al.** Dynamic evaluation of the hepatic uptake and clearance of manganese-based MRI contrast agents: a 31P NMR study on the isolated and perfused rat liver. *J Magn Reson Imaging,* 1998, vol. 8 (3), 663-9
- **DIEHL, S. J. ; K. J. LEHMANN et al.** MR imaging of pancreatic lesions. Comparison of manganese-DPDP and gadolinium chelate. *Invest Radiol,* 1999, vol. 34 (9), 589-95
- **FIEL, R. ; E. MARK et al.** Tumor-selective contrast enhancing agent, Mn(III)meso- [tri(4-sulfonatophenyl)phenyl]porphine (MnTPPS3. *Magn Reson Imaging,* 1993, vol. 11 (7), 1079-81
- **KIM, S. W. ; T. KOZUKA.** Mn-TPPS4; a potential MRI contrast agent for localizing the normal aortic wall in rabbits. *Nippon Igaku Hoshasen Gakkai Zasshi,* 1990, vol. 50 (2), 192-94
- **LANIADO, M. ; A. F. KOPP.** Current status of the clinical development of MR contrast media. *Rofo Fortschr Geb Rontgenstr Neuen Bildgeb Verfahr,* 1997, vol. 167 (6), 541-50
- **MARCHAL, G. ; X. ZHANG et al.** Comparison between Gd-DTPA, Gd-EOB-DTPA, and Mn-DPDP in induced HCC in rats: a correlation study of MR imaging, microangiography, and histology. *Magn Reson Imagine,* 1993, vol. 11 (5), 665-74
- **MAURER, J. ; A. STRAUSS et al.** Contrast-enhanced high resolution magnetic resonance imaging of pigmented malignant melanoma using Mn-TPPS4 and Gd-DTPA: experimental results. *Melanoma Res,* 2000, vol. 10 (1), 40-6
- **MAURER, J. ; A. STRAUSS et al.** Mn-TPPS4 in the diagnosis of malignant skin tumors. In vivo studies with high resolution magnetic resonance tomography in melanotic melanoma. *Radiologe,* 1999, vol. 39 (5), 422-7
- **NAVON, G. ; R. PANIGEL et al.** Liposomes containing paramagnetic macromolecules as MRI contrast agents. *Magn Reson Med,* 1986, vol. 3 (6), 876-80
- **NI, Y. ; G. MARCHAL et al.** Prolonged positive contrast enhancement with Gd-EOB-DTPA in experimental liver tumors: potential value in tissue characterization. *J Magn Reson Imaging,* 1994, vol. 4 (3), 355-63
- **PLOWCHALK, D. R. ; J. P. JORDAN et al.** Effects of manganese (Mn++) and iron (Fe+++) on magnetic resonance imaging (MRI) characteristics of human placenta and amniotic fluid. *Physiol Chem Phys Med NMR,* 1987, vol. 19 (1), 35-41
- **ROFSKY, N. M. ; J. C. WEINREB.** Manganese (II) N,N'-dipyridoxylethylene-diamine-N,N'-diacetate 5,5'-bis(phosphate): clinical experience with a new contrast agent. *Magn Reson Q,* 1992, vol. 8 (3), 156-68
- **RUNGE, V. M.** Safety of approved MR contrast media for intravenous injection. *J Magn Reson Imaging,* 2000, vol. 12 (2), 205-13
- **SAEED, M. ; S. WAGNER et al.** Occlusive and reperfused myocardial infarcts: differentiation with Mn-DPDP--enhanced MR imaging. *Radiology,* 1989, vol. 172 (1), 59-64
- **SCHMIEDL, U. P. ; J. A. NELSON et al.** Hepatic contrast-enhancing properties of manganese-mesoporphyrin and manganese-TPPS4. A comparative magnetic resonance imaging study in rats. *Invest Radiol,* 1992, vol. 27 (7), 536-42
- **SCHWENDENER, R. A. ; R. WUTHRICH et al.** A pharmacokinetic and MRI study of unilamellar gadolinium-, manganese-, and iron-DTPA-stearate liposomes as organ-specific contrast agents. *Invest Radiol,* 1990, vol. 25 (8), 922-32
- **WANG, C.** Mangafodipir trisodium (MnDPDP)-enhanced magnetic resonance imaging of the liver and pancreas. *Acta Radiol,* 1998, vol. 415, 1-31
- **WILMES, L. J. ; M. HOEHN-BERLAGE et al.** In vivo relaxometry of three brain tumors in the rat: effect of Mn-TPPS, a tumor-selective contrast agent. *J Magn Reson Imaging,* 1993, vol. 3 (1), 5-12
- **WOLF, G. L. ; K. R. BURNETT et al.** Contrast agents for magnetic resonance imaging. *Magn Reson Annu,* 1985, 231-66
- **WYTTENBACH, R. ; M. SAEED et al.** Detection of acute myocardial ischemia using first-pass dynamics of MnDPDP on inversion recovery echoplanar imaging. *J Magn Reson Imaging,* 1999, vol. 9 (2), 209-14
- **YAMAMOTO, T. ; A. MATSUMURA et al.** Manganese-metalloporphyrin (ATN-10) as a tumor-localizing agent: magnetic resonance imaging and inductively coupled plasma atomic emission spectroscopy study with experimental brain tumors. *Neurosurgery,* 1998, vol. 42 (6), 1332-71337-8
- **ZENA ; MICHAEL A. WIENER.** Clinical imaging of the high-risk or vulnerable atherosclerotic plaque. *CIRCULATION RESEARCH,* 17 August 2001, vol. 89 (4), 305-16

- **LEYEN, BRAUN-DULLAEUS et al.** A pressure-mediated nonviral method for efficient arterial gene and oligonucleotide transfer. *Hum. Gene Ther.,* 1999, vol. 10, 2355-64
- **FIRE, A. ; XU, S. ; MONTGOMERY, M. K. ; KOSTAS, S. A. ; DRIVER, S. E. ; MELLO, C. C.** *Nature (London),* 1998, vol. 391, 806-811
- **BERNSTEIN, E. ; DENLI, A. M. ; HANNON, G. J.** *RNA,* 2001, vol. 7, 1509-1521
- **SHARP, P. A.** *Genes Dev.,* 2001, vol. 15, 485-490
- **DOUGLAS et al.** *Hum Gene Ther.,* 2001, vol. 12 (4), 401-413
- **MIYOSHI et al.** *Virol.,* 1999, vol. 72, 8150-8157
- **GARVEY et al.** *Virology,* 1990, vol. 175, 391-409
- **BERKOWITZ et al.** *J. Virol.,* 2001, vol. 7 (7), 3371-3382
- **J. SAMBROOK et al.** Molecular Cloning: A Laboratory Manual. Cold Spring Harbor Laboratory Press, 1989
- **LEFEBVRE et al.** *Arterioscler. Thromb. Vasc. Biol.,* 1997, vol. 17, 1756
- **STAELS ; AUWERX.** *Atherosclerosis,* 1998, vol. 137, 19
- **GUERRE-MILLO et al.** *J. Biol. Chem.,* 2000, vol. 275, 16638
- **DJOUADI et al.** *J. Clin. Invest.,* 1998, vol. 102, 1083
- **KERSTEN et al.** *Nature,* 2000, vol. 405, 421
- **ISSEMANN.** *J. Mol. Endocrinol.,* 1993, vol. 11, 37
- **MURAKAMI et al.** *Biochem. Biophy. Res. Comm.,* 1999, vol. 260, 609
- **COSTET et al.** *J. Biol. Chem.,* 1998, vol. 273, 29577
- **DEVCHAND et al.** *Nature,* 1996, vol. 384, 39
- **POYNTER ; DAYNES.** *J. Biol. Chem.,* 1998, vol. 273, 32833
- **BARGER et al.** *Trends Cardiovasc. Med.,* 2001, vol. 10, 238
- **BUCHAN et al.** *Med. Res. Rev.,* 2000, vol. 20, 350
- **GLASS.** *J. Endocrinol.,* 2001, vol. 169, 461
- **ZHOU et al.** *Proc. Natl. Acad. Sci. U.S.A.,* 1998, vol. 95, 8898
- **TONTONOZ et al.** *Cell,* 1994, vol. 79, 1147
- **LEHMANN et al.** *J. Biol. Chem.,* 1995, vol. 270, 12953
- **MACDOUGALD ; LANE.** *Annu. Rev. Biochem.,* 1995, vol. 64, 345
- **ROBINSON et al.** *Biochem. Biophys. Res. Commun.,* 1998, vol. 244, 671
- **CLARKE.** *Br. J. Nutr.,* 2000, vol. 83 (1), 59
- **ZHOU et al.** *Metabolism,* 1996, vol. 45, 981
- **SHIMABUKURO et al.** *Proc. Natl. Acad. Sci. U.S.A.,* 1998, vol. 95, 2498
- **SATOH et al.** *Biochem. Biophys. Res. Commun.,* 1999, vol. 254, 757
- **BARROSO et al.** *Nature,* 1999, vol. 402, 880
- **WILLSON et al.** *Annu. Rev. Biochem.,* 2001, vol. 70, 341
- **LEIBOWITZ et al.** *FEBS Letters,* 2000, vol. 473, 333
- **OLIVER et al.** *Proc. Nat. Acad. Sci.,* 2001, vol. 98, 5306
- **CHINETTI et al.** *Nature Medicine,* 2001, vol. 7, 53
- **HULIN et al.** *Current Pharm. Design,* 1996, vol. 2, 85-102
- **WILLSON et al.** *J. Med. Chem.,* 1996, vol. 39, 665-669
- **FUKUI.** *Diabetes,* 2000, vol. 49 (5), 759-767
- **BERGER et al.** Novel peroxisome proliferator-activated receptor (PPAR-gamma) and PPAR-delta ligands produce distinct biological effects. *J. Biol. Chem.,* 1999, vol. 274, 6718-6725
- **BERGER et al.** Novel peroxisome proliferator-activated receptor-γ (PPAR-gamma) and PPAR-δ (PPAR-delta) ligands produce distinct biological effects. *J Biol Chem,* 1999, vol. 274, 6718 6725
- **YOKOYAMA N. et al.** *Journal of Medicinal Chemistry,* 1995, vol. 38 (4), 695-707
- **STEPHAN Z. F. et al.** *Atherosclerosis,* 1996, vol. 126, 53-63
- Peptide Backbone Modifications. **SPATOLA.** Chemistry and Biochemistry of Amino Acids Peptides and Proteins. Marcel Dekker, 1983, 267
- **MORLEY.** *Trends Pharm. Sci.,* 1980, vol. 1, 463-468
- **HUDSON et al.** *Int. J. Prot. Res.,* 1979, vol. 14, 177-185
- **SPATOLA et al.** *Life Sci.,* 1986, vol. 38, 1243-1249
- **HANN.** *J. Chem. Soc. Perkin Trans. I.,* 1982, vol. 1, 307-314
- **ALMQUIST et al.** *J. Med. Chem.,* 1980, vol. 23, 1392-1398
- **JENNINGS-WHITE et al.** *Tetrahedron. Lett.,* vol. 23, 2533
- **HOLLADAY et al.** *Tetrahedron Lett.,* 1983, vol. 24, 4401-4404
- **HRUBY.** *Life Sci.,* 1982, vol. 31, 189-199
- **PASQUALINI et al.** *Nature,* 1996, vol. 380, 364-66
- **RASCHED et al.** *Microbiol. Rev.,* 1986, vol. 50, 401-427
- **MODEL et al.** The Bacteriophages. Plenum Press, 1988, vol. 2, 375-456
- **PARMELY et al.** *Gene,* 1988, vol. 73, 305-318
- **CWIRLA et al.** *Proc. Natl. Acad. Sci. USA,* 1991, vol. 87, 6378-6382

- **MCCAFFERTY et al.** *Science,* 1990, vol. 348, 552-554
- **ROUSLAHTI et al.** *Science,* 1987, vol. 238, 491-497
- **SUZUKI et al.** *Proc. Natl. Acad. Sci. USA,* 1986, vol. 83, 8614-8618
- **GINSBERG et al.** *J. Biol. Chem.,* 1987, vol. 262, 5437-5440
- **SAMBROOK et al.** Molecular Cloning: A Laboratory Manual. Cold Spring Harbor Laboratory Press, 1989, 9.47-9.51
- **SAMBROOK et al.** Molecular Cloning: A Laboratory Manual. Cold Spring Harbor Laboratory Press, 2001
- **BRIAN K. KAY ; JILL WINTER ; JOHN MCCAFFERTY.** Phage Display of Peptides and Proteins, A Laboratory Manual. Academic Press
- **LIBBY, P.** Changing concepts of atherogenesis. *J. Intern. Med.,* 2000, vol. 247, 349-358
- **FAGGIOTTO, A. ; ROSS, R.** Studies of hypercholesterolemia in the nonhuman primate. II. Fatty streak conversion to fibrous plaque. *Arteriosclerosis,* 1984, vol. 4, 341-356
- **ROSENFELD, M.E. ; TSUKADA, T. ; GOWN, A.M. ; ROSS, R.** Fatty streak initiation in Watanabe Heritable Hyperlipemic and comparably hypercholesterolemic fat-fed rabbits. *Arteriosclerosis,* 1987, vol. 7, 9-23
- **ROSS, R.** The pathogenesis of atherosclerosis: a perspective for the 1990s. *Nature,* 1993, vol. 362, 801-809
- **ROSS, R.** Atherosclerosis: current understanding of mechanisms and future strategies in therapy. *Transplant. Proc.,* 1993, vol. 25, 2041-2043
- **FUSTER, V. ; LEWIS A.** Mechanisms leading to myocardial infarction: insights from studies of vascular biology. *Conner Memorial Lecture,* 1994
- *Circulation,* 01 January 1995, vol. 91 (1), 256
- *Circulation,* vol. 90, 2126-2146
- **DONG, Z.M. ; CHAPMAN, S.M. ; BROWN, A.A. ; FRENETTE, P.S. ; HYNES, R.O. ; WAGNER, D.D.** The combined role ofP- and E-selectins in atherosclerosis. *J. Clin. Invest.,* 1998, vol. 102, 145-152
- **CYBULSKY, M.I. ; GIMBRONE, M.A., JR.** Endothelial expression of a mononuclear leukocyte adhesion molecule during atherogenesis. *Science,* 1991, vol. 251, 788-791
- **BOISVERT, W.A. ; BLACK, A.S. ; CURTISS, L.K.** ApoA1 reduces free cholesterol accumulation in atherosclerotic lesions of ApoE-deficient mice transplanted with ApoE-expressing macrophages. *Arterioscler. Thromb. Vasc. Biol.,* 1999, vol. 19, 525-530
- **CURTISS, L.K. ; BOISVERT, W.A.** Apolipoprotein E and atherosclerosis. *Curr. Opin. Lipidol.,* 2000, vol. 11, 243-251
- **PASQUALINI, R. ; RUOSLAHTI, E.** Organ targeting in vivo using phage display peptide libraries. *Nature,* 1996, vol. 380, 364-366
- **VACHON, V. ; POULIOT. J.F. ; LAPRADE, R. ; BELIVEAU, R.** Fractionation of renal brush border membrane proteins with Triton X-114 phase partitioning. *Biochem. Cell Biol.,* 1991, vol. 69, 206-211
- **FERNANDEZ-CATALAN, C. ; BODE. W. ; HUBER, R. ; TURK, D. ; CALVETE, J.J. ; LICHTE, A. ; TSCHESCHE, H. ; MASKOS, K.** Crystal structure of the complex formed by the membrane type 1-matrix metalloproteinase with the tissue inhibitor ofmetalloproteinases-2, the soluble progelatinase A receptor. *Embo J.,* 1998, vol. 17, 5238-5248
- **CHESLER, L. ; GOLDE, D.W. ; BERSCH, N. ; JOHNSON, M.D.** Metalloproteinase inhibition and erythroid potentiation are independent activities of tissue inhibitor of metalloproteinases-1. *Blood,* 1995, vol. 86, 4506-4515
- **CORCORAN, M.L. ; STETLER-STEVENSON, W.G.** Tissue inhibitor of metalloproteinase-2 stimulates fibroblast proliferation via a cAMP-dependent mechanism. *J. Biol. Chem.,* 1995, vol. 270, 13453-13459
- **KOLONIN, M. ; PASQUALINI, R. ; ARAP, W.** Molecular addresses in blood vessels as targets for therapy. *Curr. Opin. Chem. Biol.,* 2001, vol. 5, 308-313
- **TRESSLER, R.J. ; BELLONI, P.N. ; NICOLSON, G.L.** Correlation of inhibition of adhesion of large cell lymphoma and hepatic sinusoidal endothelial cells by RGD-containing peptide polymers with metastatic potential: role of integrin-dependent and -independent adhesion mechanisms. *Cancer Commun.,* 1989, vol. 1, 55-63
- **VAN DER WAL, A.C. ; BECKER, A.E. ; VAN DER LOOS, C.M. ; DAS, P.K.** Site of intimal rupture or erosion of thrombosed coronary atherosclerotic plaques is characterized by an inflammatory process irrespective of the dominant plaque morphology. *Circulation,* 1994, vol. 89, 36-44
- **LENDON, C.L. ; DAVIES, M.J. ; BORN, G.V. ; RICHARDSON, P.D.** Atherosclerotic plaque caps are locally weakened when macrophages density is increased. *Atherosclerosis,* 1991, vol. 87, 87-90
- **KRIEGER, M. ; ACTON, S. ; ASHKENAS, J. ; PEARSON, A. ; PENMAN, M. ; RESNICK, D.** Molecular flypaper, host defense, and atherosclerosis. Structure, binding properties, and functions of macrophage scavenger receptors. *J. Biol. Chem.,* 1993, vol. 268, 4569-4572
- **ENDEMANN, G. ; STANTON, L.W. ; MADDEN, K.S. ; BRYANT, C.M. ; WHITE, R.T. ; PROTTER, A.A.** CD36 is a receptor for oxidized low density lipoprotein. *J. Biol. Chem.,* 1993, vol. 268, 11811-11816

- **OTTNAD, E. ; PARTHASARATHY, S. ; SAMBRANO, G.R. ; RAMPRASAD, M.P. ; QUEHENBERGER, O. ; KONDRATENKO, N. ; GREEN, S. ; STEINBERG, D.** A macrophage receptor for oxidized low density lipoprotein distinct from the receptor for acetyl low density lipoprotein: partial purification and role in recognition of oxidatively damaged cells. *Proc. Natl. Acad. Sci. USA.,* 1995, vol. 92, 1391-1395
- **RAMPRASAD, M.P. ; FISCHER, W. ; WITZTUM, J.L. ; SAMBRANO, G.R. ; QUEHENBERGER, O. ; STEINBERG, D.** The 94- to 97-KDa mouse macrophage membrane protein that recognizes oxidized low density lipoprotein and phosphatidylserine-rich liposomes is identical to macrosialin, the mouse homologue of human CD68. *Proc. Natl. Acad. Sci. USA.,* 1995, vol. 92, 9580-9584
- **ACTON, S. ; RIGOTTI, A. ; LANDSCHULZ, K.T. ; XU, S. ; HOBBS, H.H. ; KRIEGER, M.** Identification of scavenger receptor SR-BI as a high density lipoprotein receptor. *Science,* 1996, vol. 271, 518-520
- **GU, X. ; TRIGATTI, B. ; XU, S. ; ACTON, S. ; BABITT, J. ; KRIEGER, M.** The efficient cellular uptake of high density lipoprotein lipids via scavenger receptor class B type I requires not only receptor-mediated surface binding but also receptor-specific lipid transfer mediated by its extracellular domain. *J. Biol. Chem.,* 1998, vol. 273, 26338-26348
- **WATSON, A.D. ; LEITINGER, N. ; NAVAB; M. ; FAULL, K.F. ; HORKKO, S. ; WITZTUM, J.L. ; PALINSKI, W. ; SCHWENKE, D. ; SALOMON, R.G. ; SHA, W.** Structural identification by mass spectrometry of oxidized phospholipids in minimally oxidized low density lipoprotein that induce monocyte/endothelial interactions and evidence for their presence in vivo. *J. Biol. Chem.,* 1997, vol. 272, 13597-13607
- **WITZTUM, J.L. ; BERLINER, J.A.** Oxidized phospholipids and isoprostanes in atherosclerosis. *Curr. Opin. Lipidol.,* 1998, vol. 9, 441-448
- **BERLINER, J. ; LEITINGER, N. ; WATSON, A. ; HUBER, J. ; FOGELMAN, A. ; NAVAB, M.** Oxidized lipids in atherogenesis: formation, destruction and action. *Thromb. Haemost.,* 1997, vol. 78, 195-199
- **GIMBRONE, M.A., JR. ; TOPPER, J.N. ; NAGEL, T. ; ANDERSON, K.R. ; GARCIA-CARDENA, G.** Endothelial dysfunction, hemodynamic forces, and atherogenesis. *Ann. N Y Acad. Sci.,* 2000, vol. 902, 230-239239-240
- **GIMBRONE, M.A., JR. ; NAGEL, T. ; TOPPER, J.N.** Biomechanical activation: an emerging paradigm in endothelial adhesion biology. *J. Clin. Invest.,* 1997, vol. 99, 1809-1813
- **CARLOS, T.M. ; HARLAN, J.M.** Leukocyte-endothelial adhesion molecules. *Blood,* 1994, vol. 84, 2068-2101
- **FRENETTE, P.S. ; WAGNER, D.D.** Adhesion molecules-Part 1. *N. Engl. J. Med.,* 1996, vol. 334, 1526-1529
- **FRENETTE, P.S. ; WAGNER, D.D.** Adhesion molecules--Part II: Blood vessels and blood cells. *N. Engl. J. Med.,* 1996, vol. 335, 43-45